# EUROPEAN PATENT APPLICATION

(11) **EP 3 786 290 A1**
(43) Date of publication of application: **03.03.2021**
(21) Application number: 19194807.4
(22) Date of filing: 31.08.2019
(51) Int. Cl.: C12N 9/88, C12P 7/22, C12P 7/42, C12P 11/00, C12P 13/00

(54) **PEPTIDES AND METHODS FOR THE CARBON-CARBON BOND FORMATION**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to methods for the preparation of α-hydroxyacyl compounds, and peptides for the catalyzed formation of α-hydroxyacyl compounds as well as their use in the preparation of α-hydroxyacyl compounds.

## Description

The present invention relates to methods for the preparation of α-hydroxyacyl compounds, and peptides for the catalyzed formation of α-hydroxyacyl compounds as well as their use in the preparation of α-hydroxyacyl compounds.

### Background of the invention

One of biotechnology's central goals is the synthesis of multicarbon compounds under mild and sustainable conditions from renewable resources. This requires biocatalysts and methods that enable selective C-C bond formation (carboligation) between two carbon units.

Moreover, optically active compounds are useful for optical resolving agents. For example, optically active compounds can be used for optically resolving agents of medical or agriculture supplies such as 2-amino-1-butanol which is a starting material of the antituberculous drug ethambutol, diltiazem hydrochloride which is a coronary vasodilator and tetramizol which is effective as an anthelmintic. Further, optically active compounds can also be used as starting materials or intermediates for the synthesis of optically pure therapeutic agents.

In the international patent application WO 2016/069929 A1, the formation of α-hydroxyacyl-CoA thioester using α-hydroxyacyl-CoA lyase (HACL1) is disclosed, and is thus not directed to aromatic α-hydroxyacyl-CoA or even less to aromatic α-hydroxy carboxylic acids such as mandelic acid or its derivatives. The conversion and even less the yield of the product is not taught. Furthermore, the enantioselectivity of the reaction and the stereochemical information of the stereocenter of the product are not described.

The US patent application US 2003/0119173 A1 refers to the synthesis of mandelic acid derivatives by enzymatic reduction of α-keto acids. It is not described that the reaction can be performed enantioselective.

Albeit enantioselective synthesis of *(R)*-mandelic acid derivatives is well known in the state of the art, the enzymatic enantioselective synthesis of *(S)*-mandelic acid derivatives is very limited. In the US patent application US 2010/0036150 the synthesis of *(S)*-mandelic acid derivatives are described, wherein the reaction starts from acetylated α-hydroxy carboxylic acid ester.

Therefore, there is an interest in further synthesis procedure for the preparation of aromatic α-hydroxy carboxylic acids, salts thereof or their esters in particular enantioselective and especially selective for the *(S)*-configuration.

It is the objective of the present invention to provide methods and peptides for the preparation of aromatic α-hydroxy carboxylic acids, salts thereof or their esters in particular enantioselective, and especially selective for the *(S)*-enantiomer.

The objective of the present invention is solved by the teaching of the independent claims. Further advantageous features, aspects and details of the invention are evident from the dependent claims, the description, the figures, and the examples of the present application.

### Brief description of the invention

The objective of the present invention can be solved by a method for the preparation of an α-hydroxyacyl compound of the formula (**I**') 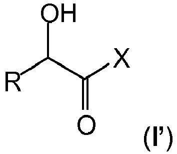 wherein **R** represents 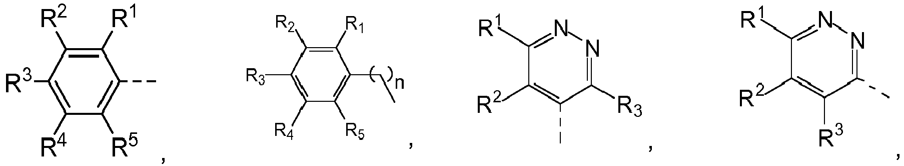 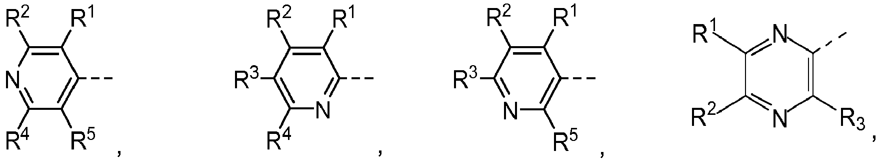 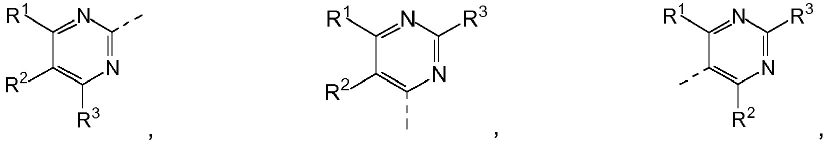 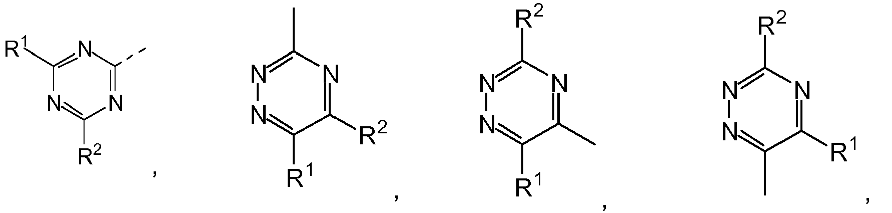 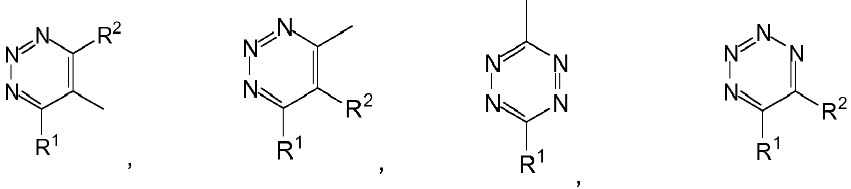 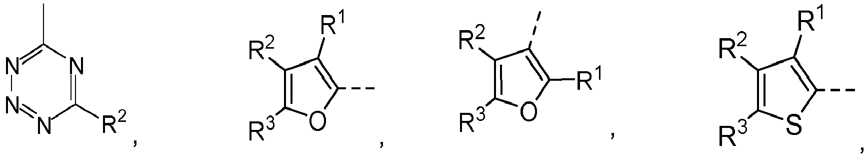 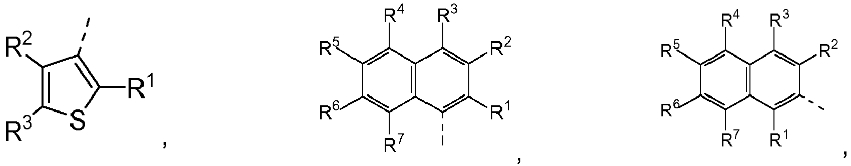 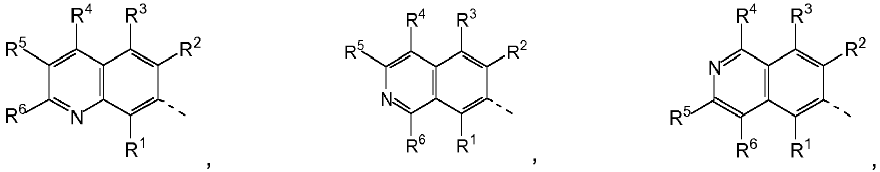 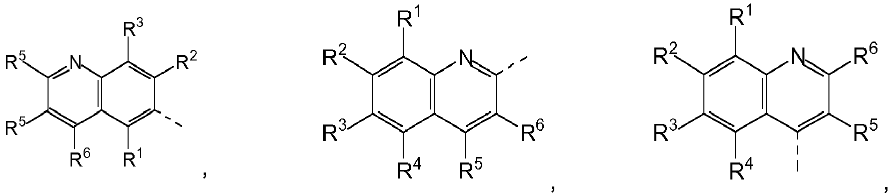 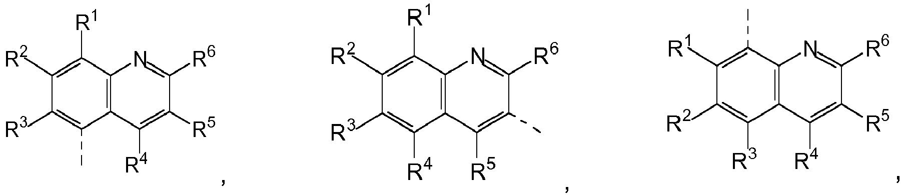 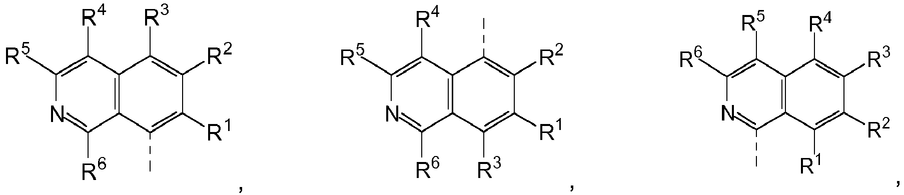 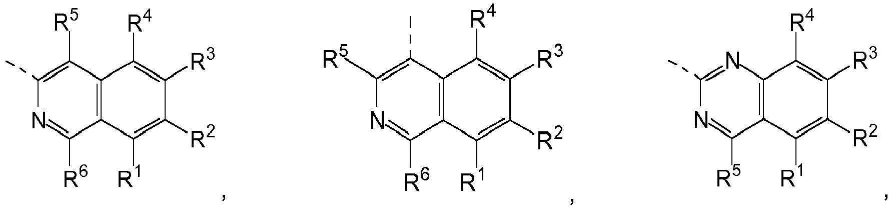 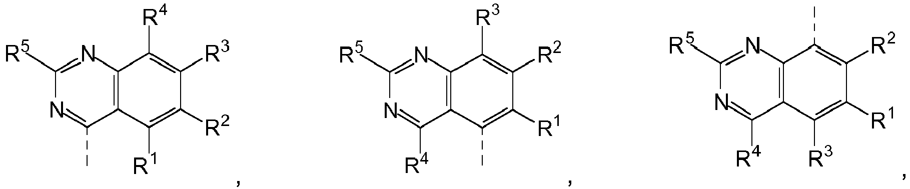 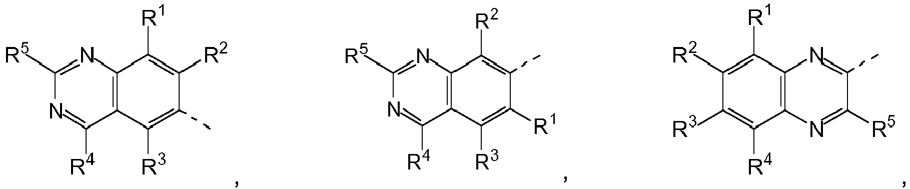 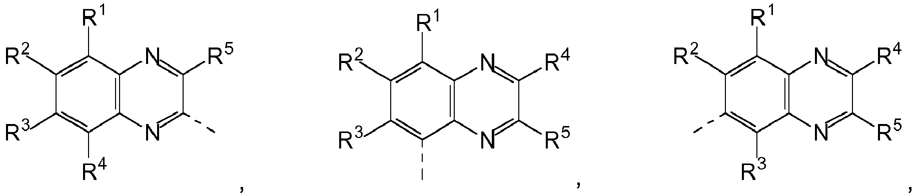 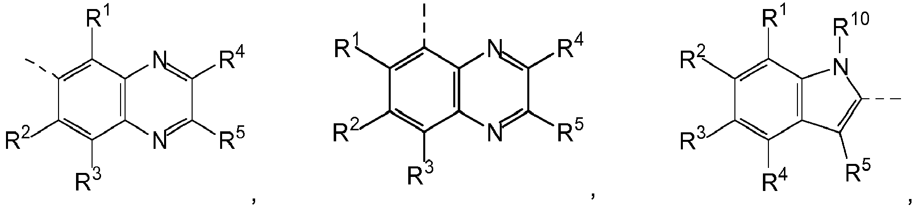 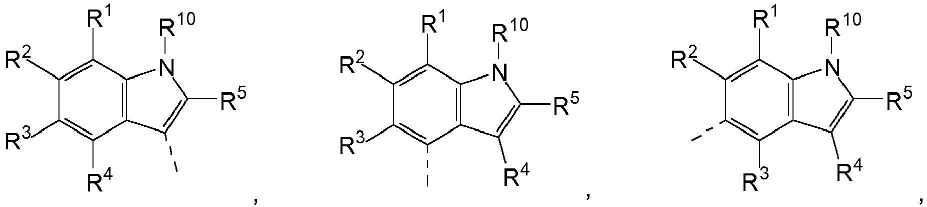 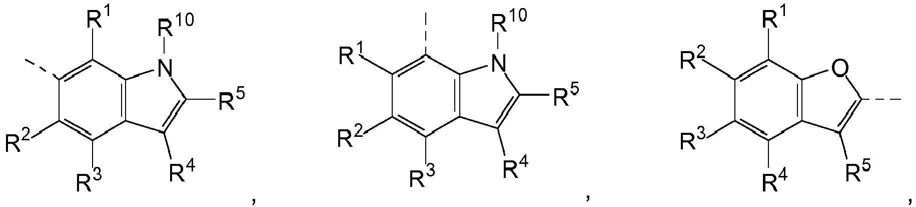 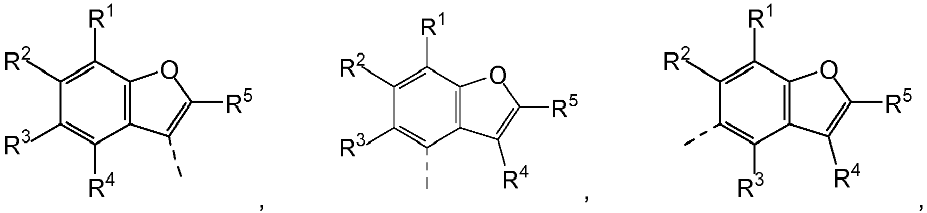 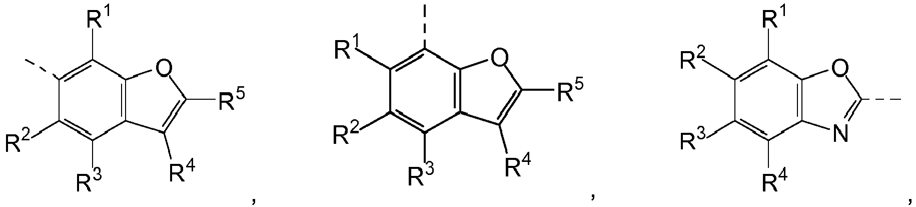 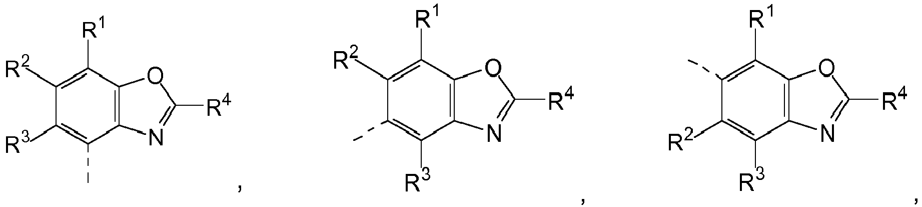 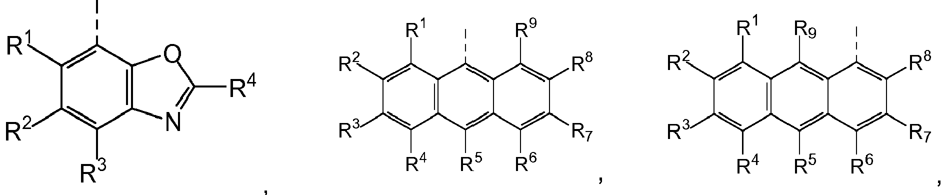 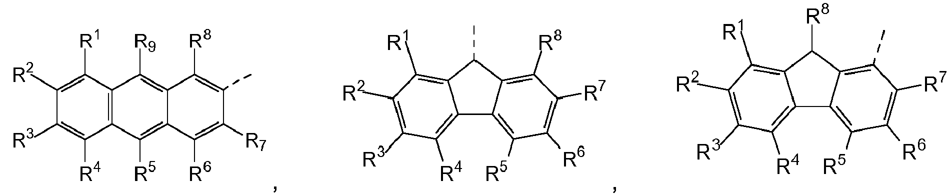 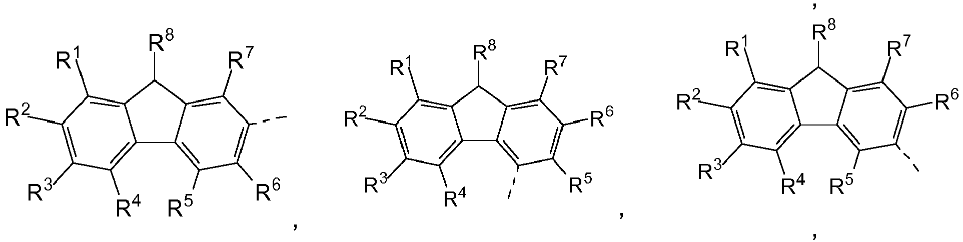 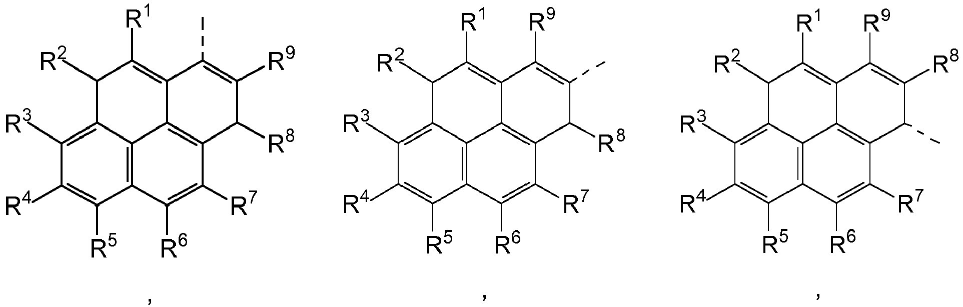 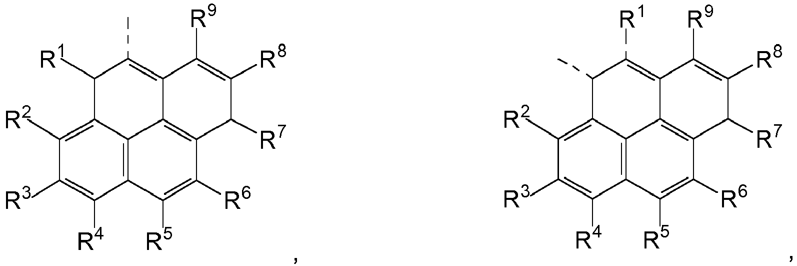 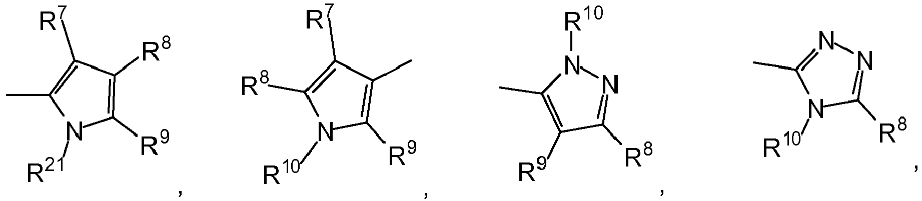 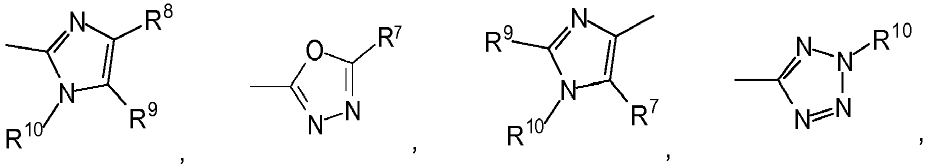 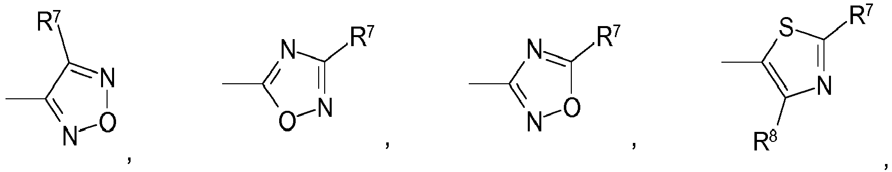 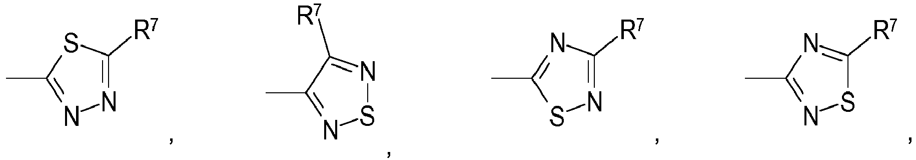 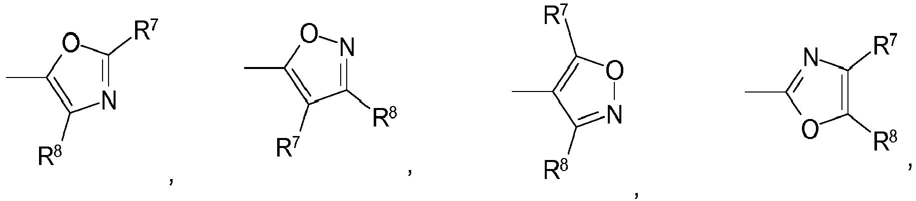 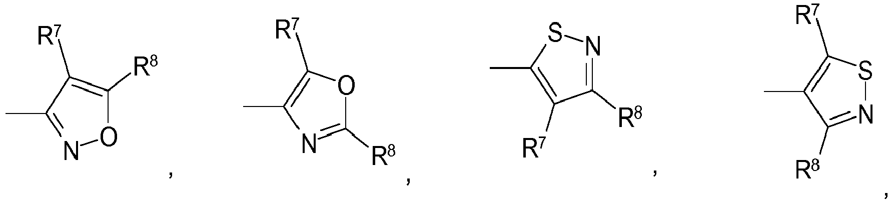 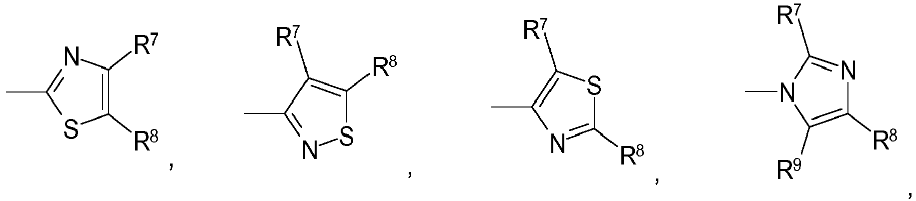 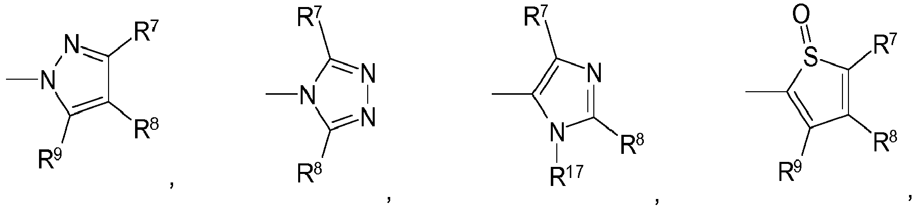 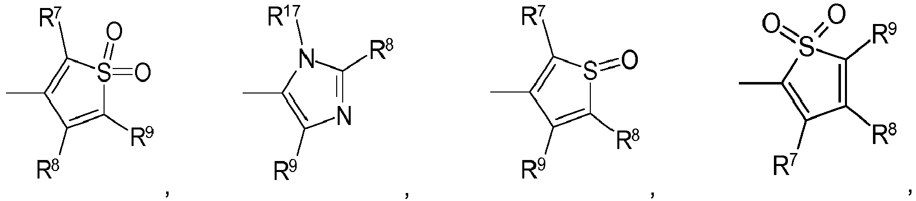 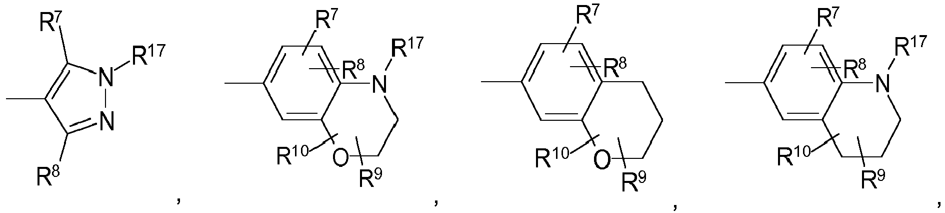 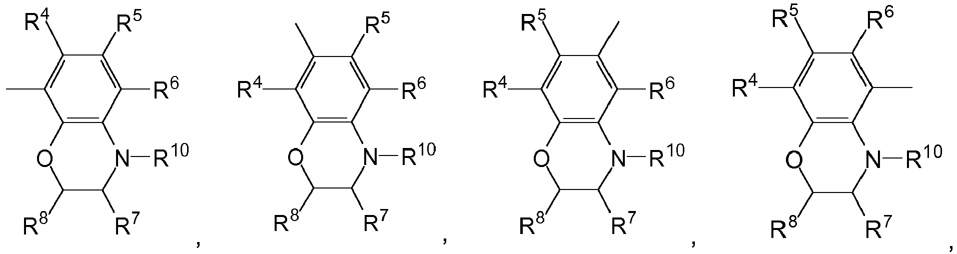 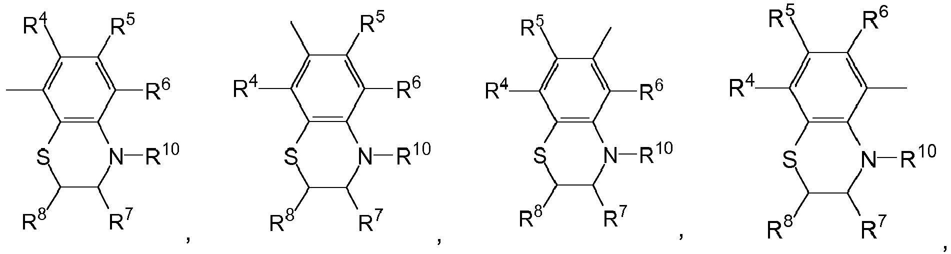 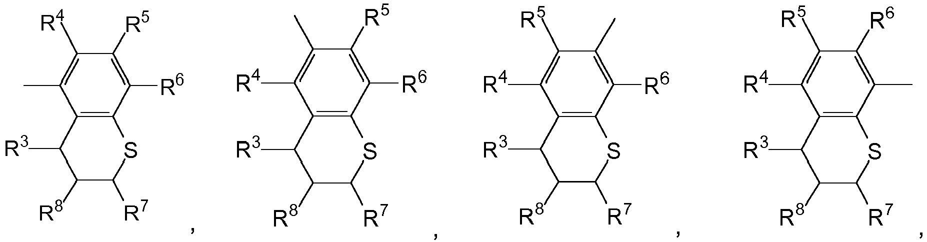 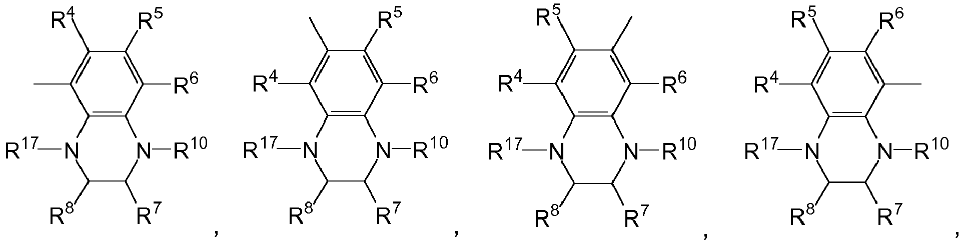 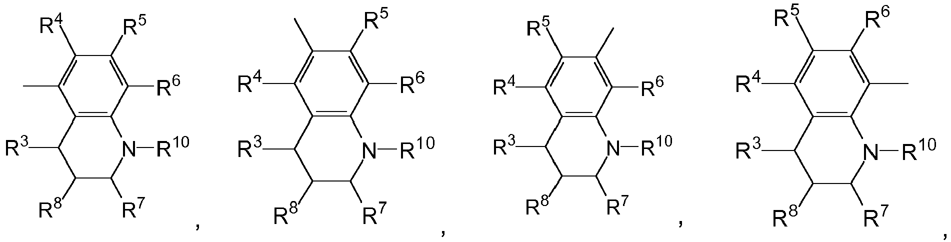 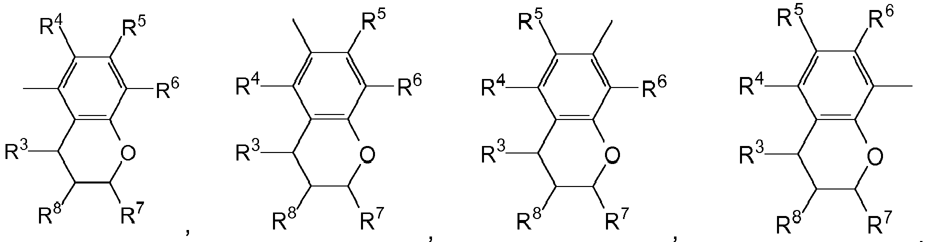 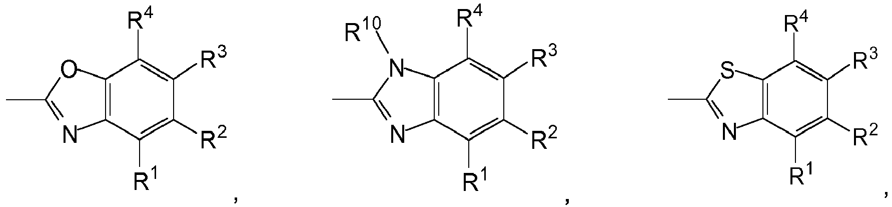 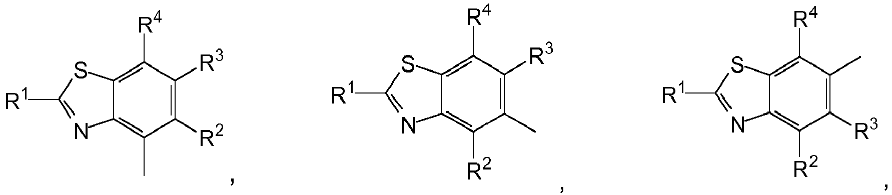 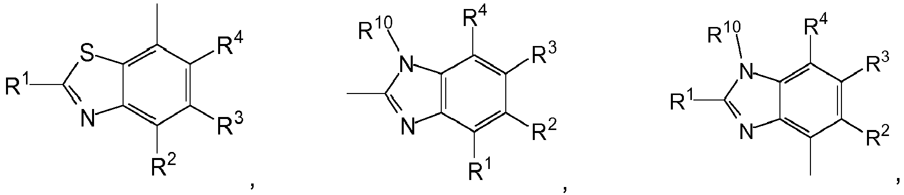 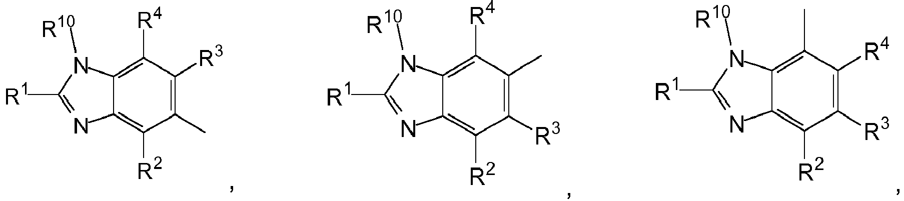 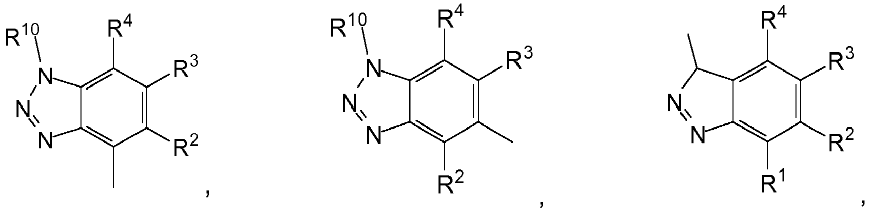 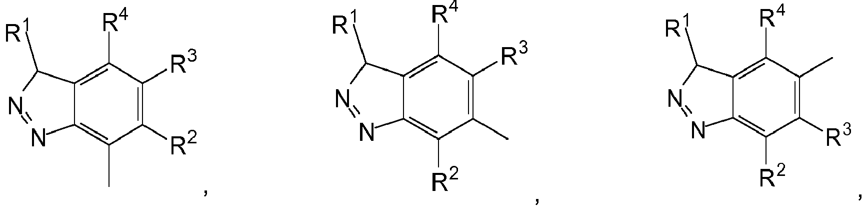 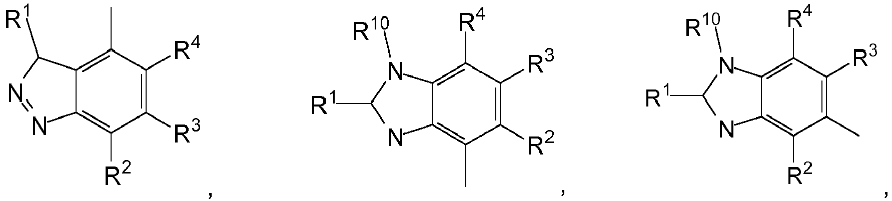 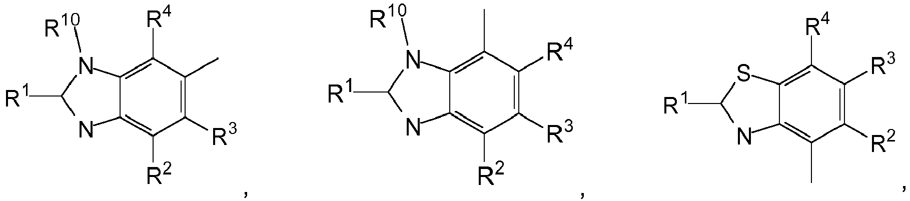 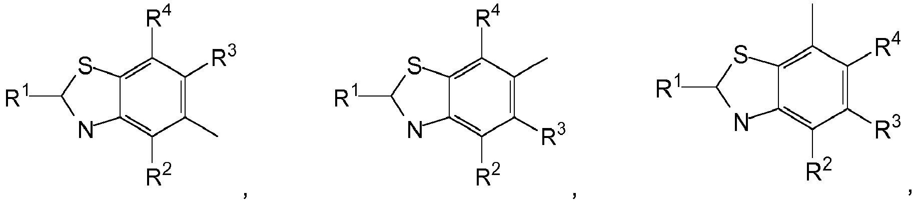 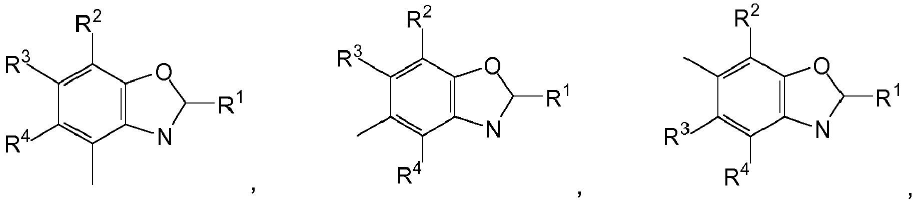 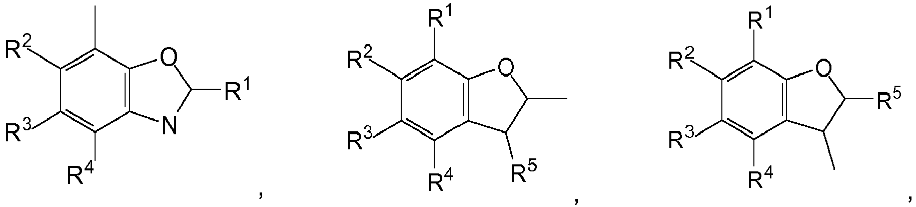 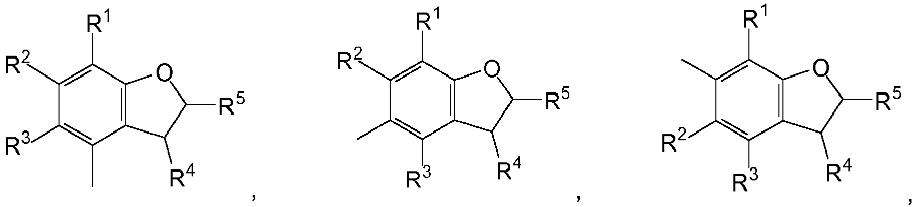 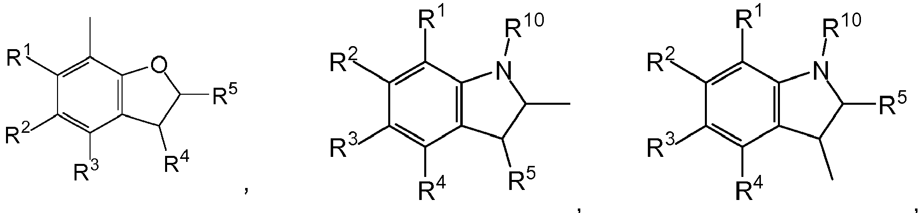 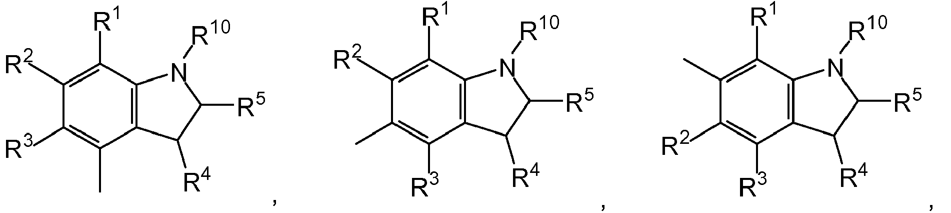 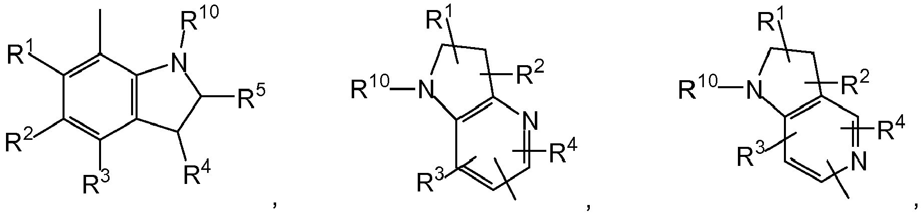 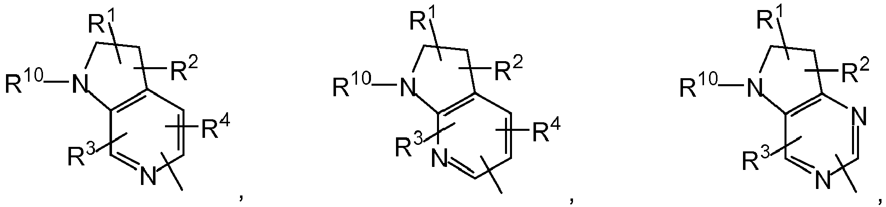 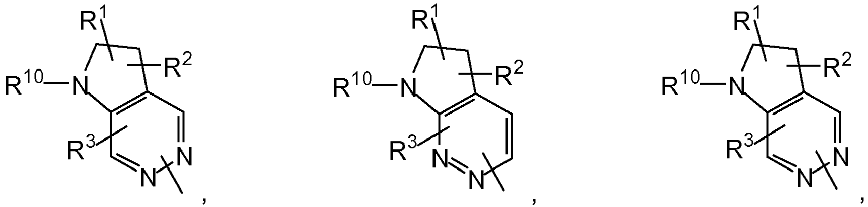 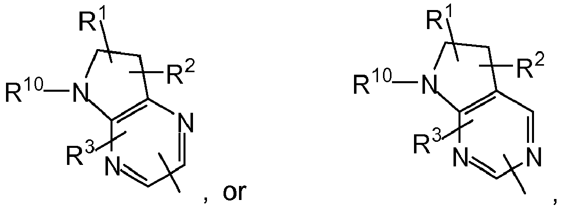 wherein **R¹, R², R³, R⁴, R⁵, R⁶, R⁷, R⁸,** and **R⁹represent** independently of each other -H, -F, -CI, -Br, -I, -Ph, -OH, -OCH₃, -OC₂H₅, -OC₃H₇, -OCH(CH₃)₂, -OC₄H₉, -OCH(CH₃)CH₂CH₃, -OCH₂CH(CH₃)₂, -OC(CH₃)₃, -O-cyclo-C₃H₅, -O-cyclo-C₄H₇, -O-cyclo-C₅H₉, -O-cyclo-C₆H₁₁, -OPh, -OCH₂-Ph, -OCH₂CH₂-Ph -OCH=CH₂, -OCH₂-CH=CH₂, -OCH₂CH₂-CH=CH₂, -OCF₃, -OC₂F₅, -SCH₃, -SC₂H₅, -SC₃H₇, -SCH(CH₃)₂, -SC₄H₉, -SCH(CH₃)CH₂CH₃, -SCH₂CH(CH₃)₂, -SC(CH₃)₃, -NO₂, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(C₄H₉)₂, -N(cyclo-C₃H₅)₂, -N[CH(CH₃)₂]₂, -N[C(CH₃)₃]₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH₂-CH₂-CH(CH₃)₂, -CH(CH₃)-CH(CH₃)-CH₃, -C(CH₃)₂-C₂H₅, -C₆H₁₃, -CH(CH₃)-C₄H₉, -CH₂-CH(CH₃)-C₃H₇, -CH₂-CH₂-CH₂-CH(CH₃)₂, -CH(CH₃)-CH(CH₃)-C₂H₅, -C(CH₃)₂-C₃H₇, -C₇H₁₅, -CH(CH₃)-C₅H₁₁, -CH₂-CH(CH₃)-C₄H₉, -CH₂-CH₂-CH₂-CH₂-CH(CH₃)₂, -C₈H₁₇, -C₉H₁₉, -C₁₀H₂₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -CH₂F, -CF₂I, -CHF₂, -CF₃, -CH₂I, -CH₂Br, -CH₂I, -CH₂-CH₂F, -CH₂-CHF₂, -CH₂-CF₃, -CH₂-CH₂I, -CH₂-CH₂Br, -CH₂-CH₂I, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CHO, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COO-cyclo-C₄H₇, -COO-cyclo-C₅H₉, -COO-cyclo-C₆H₁₁, -COOCH(CH₃)₂, -COOC(CH₃)₃, -O-COOCH₃, -O-COOC₂H₅, -O-COOC₃H₇, -O-COO-cyclo-C₃H₅, -O-COOCH(CH₃)₂, -O-COOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, 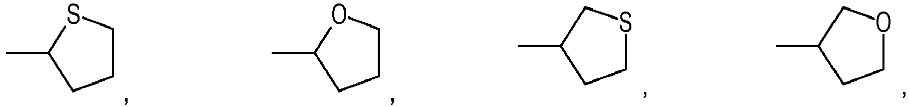 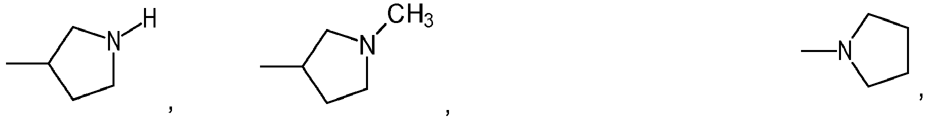 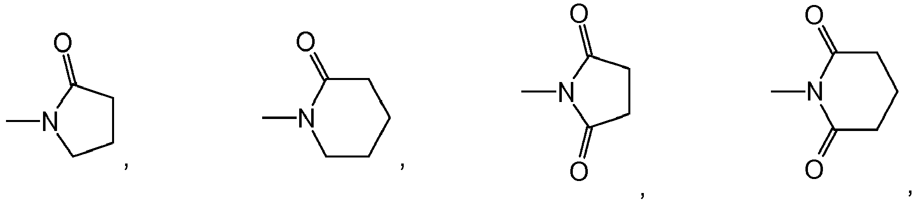 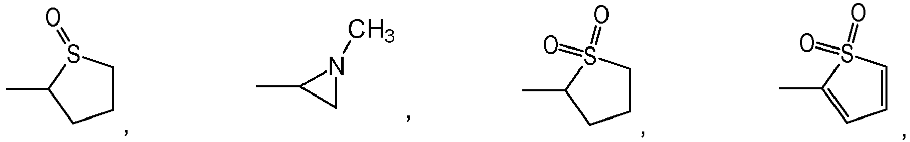 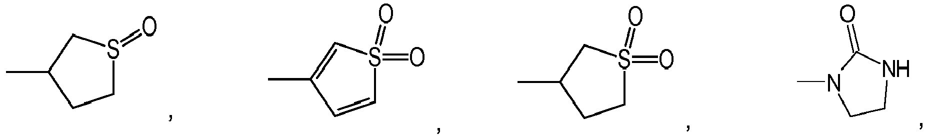 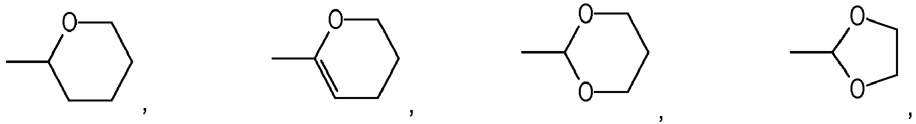 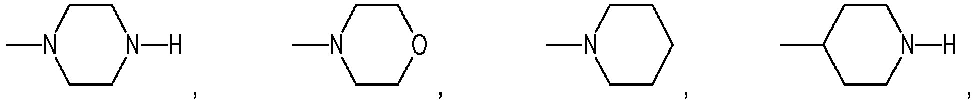 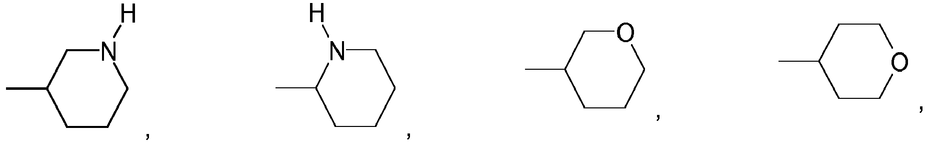 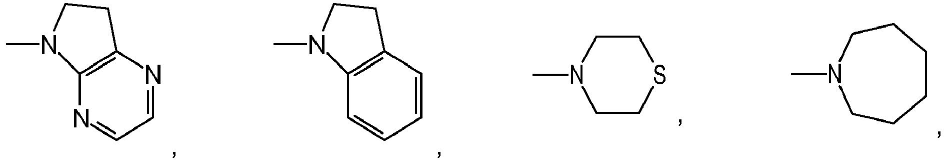 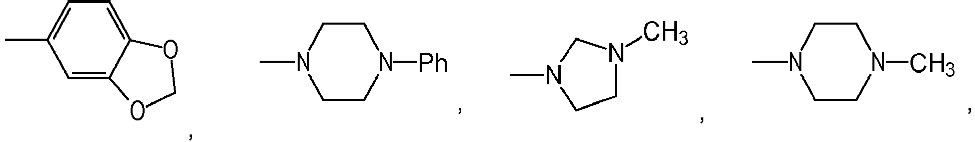 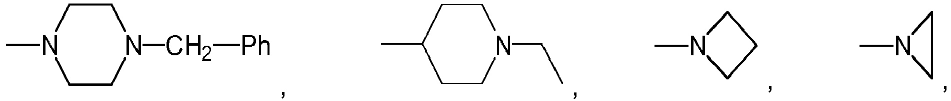 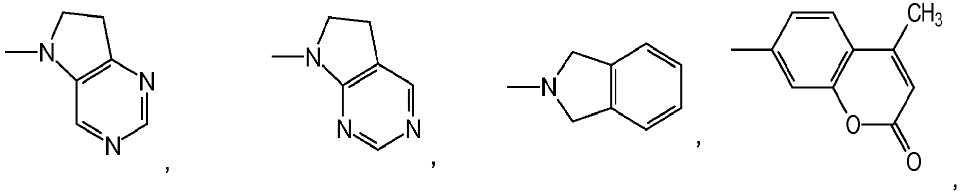 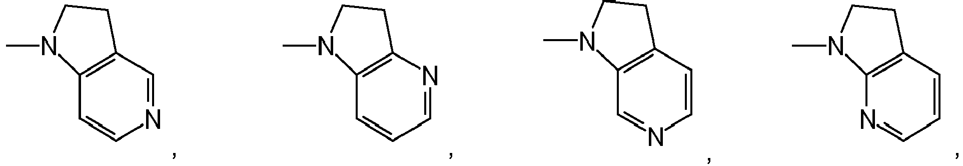 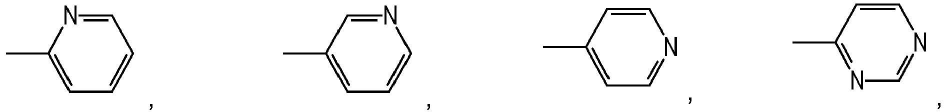 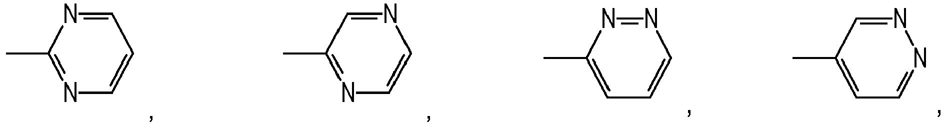 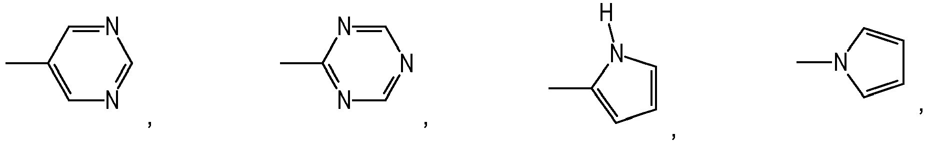 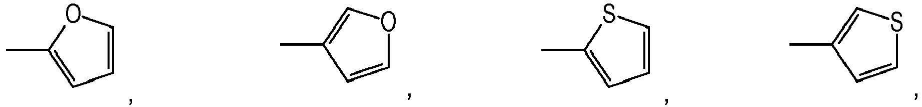 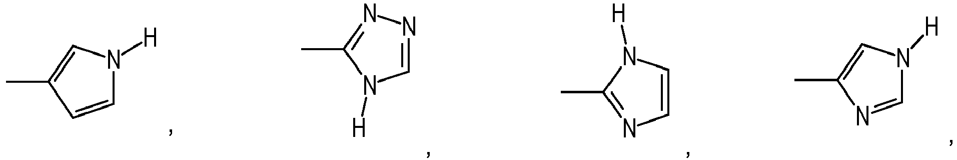 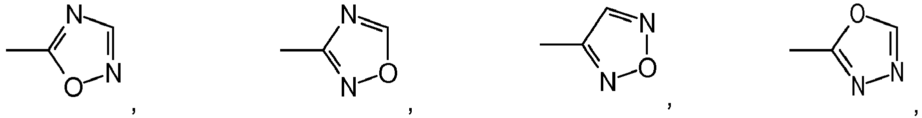 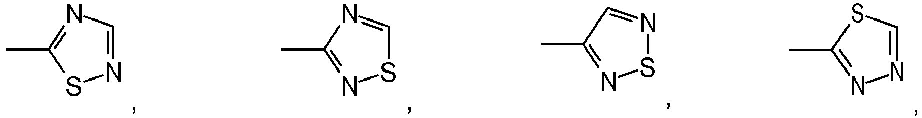 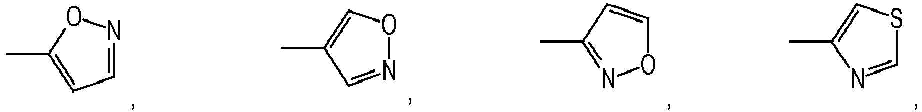 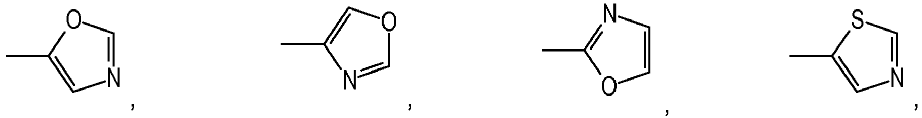 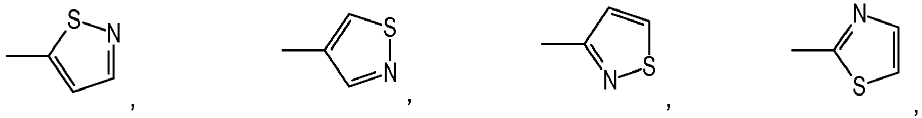 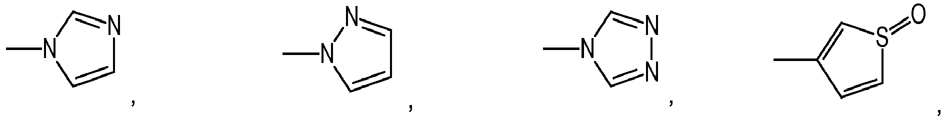 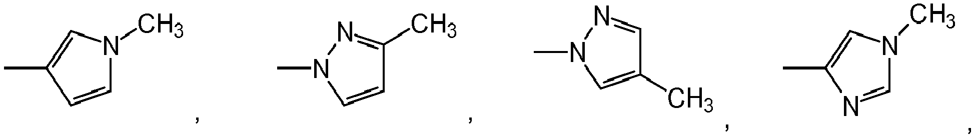 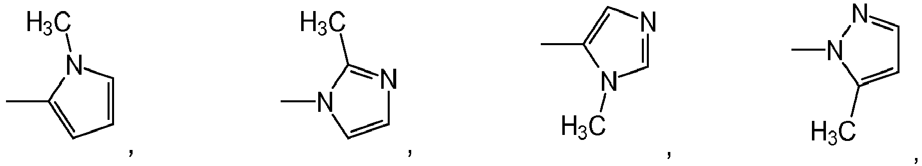 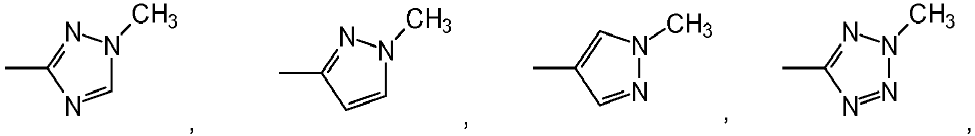 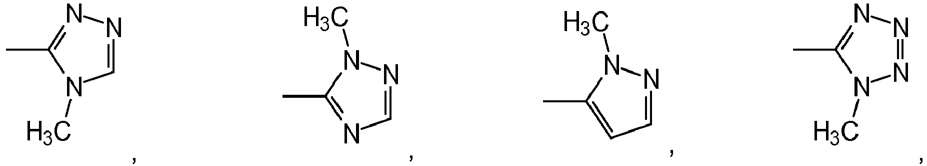 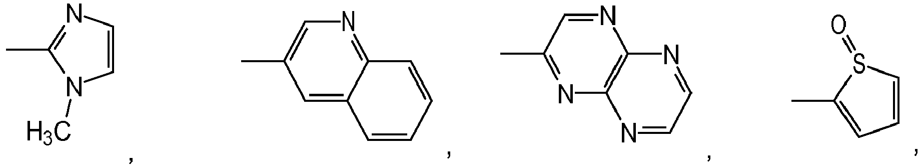 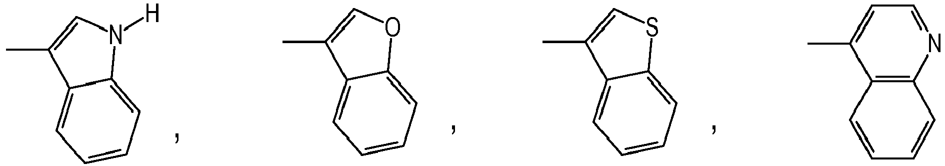 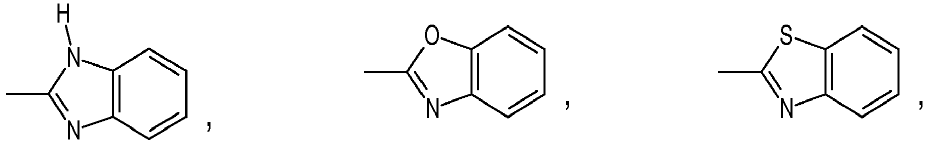 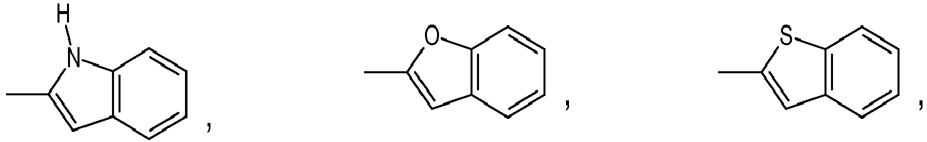 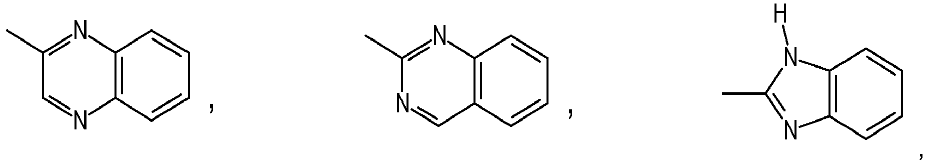 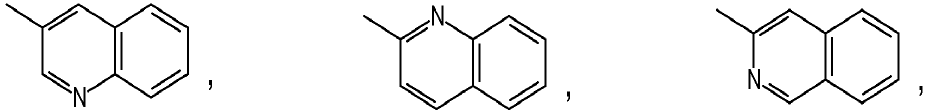 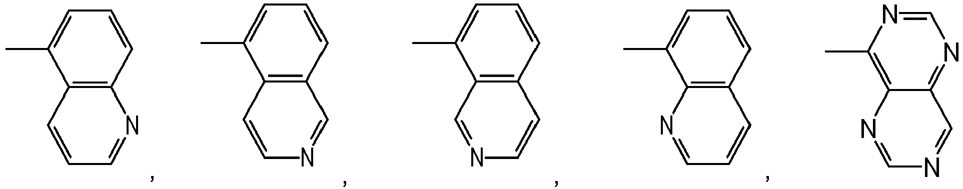 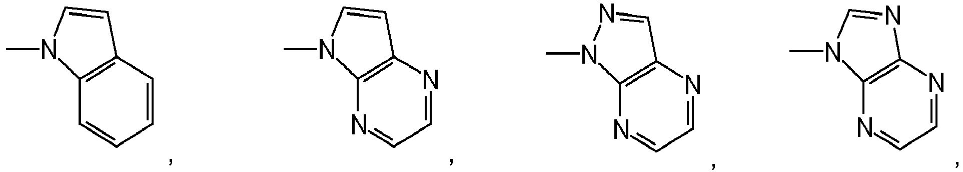 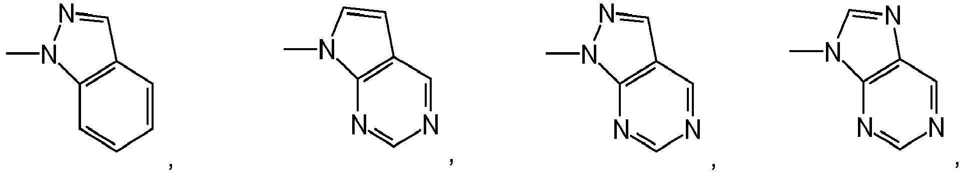 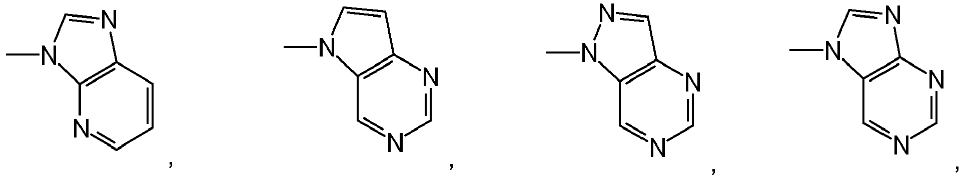 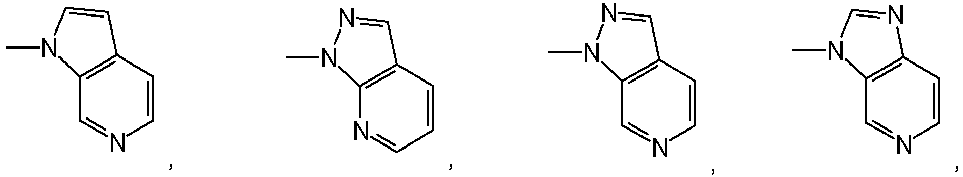 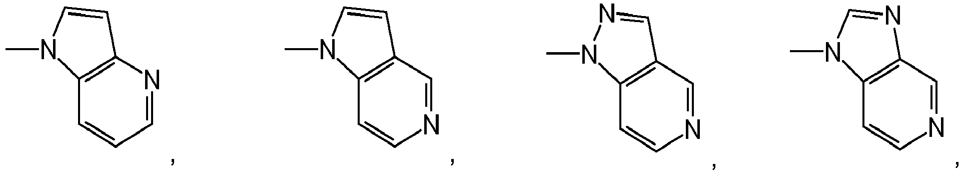 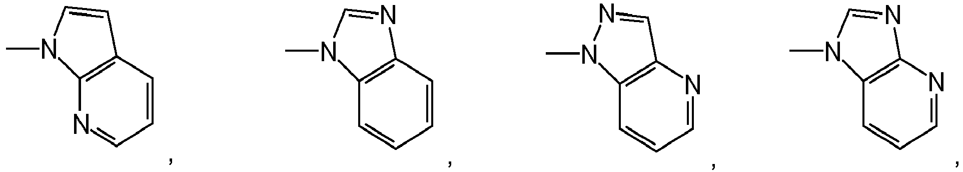 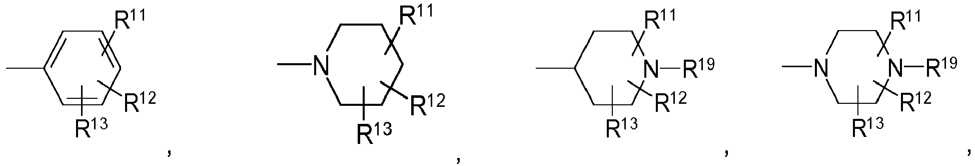 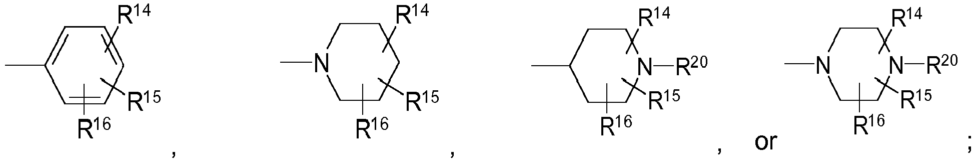
**R¹¹** to **R¹⁶** represents independently of each other:
   -H, -NH₂, -OH, -OCH₃, -OC₂H₅, -OC₃H₇, -OCF₃, -CF₃, -F, -CI, -Br, -I, -CH₃, -C₂H₅, -C₃H₇, -CH(CH₃)₂, -Ph, or -CN;
**R¹⁰, R¹⁷, R¹⁸, R¹⁹, R²⁰, and R²¹** represents independently of each other -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, CH₂-CH₂F, -CH₂-CHF₂, -CH₂-CF₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CHO, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, preferably R²¹ cannot be -H;
**X** represents -OH, -O⁻ or -SCoA,
wherein n is an integer 1, 2, or 3; or a salt thereof,
comprising or consisting of:
   **A**) providing a compound R-CHO (**1**) and an acyl-CoA (**2**) 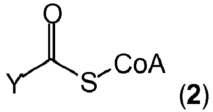
      wherein Y represents -H, or -COO⁻,
      in a buffer solution with a pH-value in the range of 5.0 to 7.5, optionally together with at least one co-solvent;
   **B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the α-hydroxyacyl-CoA thioester (**I'**) 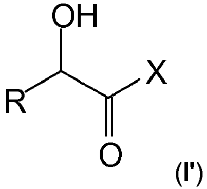
      wherein X represents -S-CoA,
      and the oxalyl-CoA decarboxylase (**3a**) is a peptide encoded by a gene selected from *Methylobacterium extorquens* or a mutant thereof; and optionally comprising the additional step **C**)
   **C**) cleaving the coenzyme A moiety of said α-hydroxyacyl-CoA thioester (**I**') by a hydrolase, α-hydroxyacyl-CoA:oxalate CoA-transferase and/or α-hydroxyacyl-CoA:formate CoA-transferase to obtain the compound of the formula (**I'**) or a salt thereof 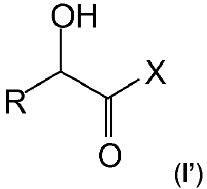
      wherein X represents -OH or O⁻,
      and the hydrolase is acyl-CoA thioester hydrolase YciA (**4**) encoded by a gene selected from *E. coli.*

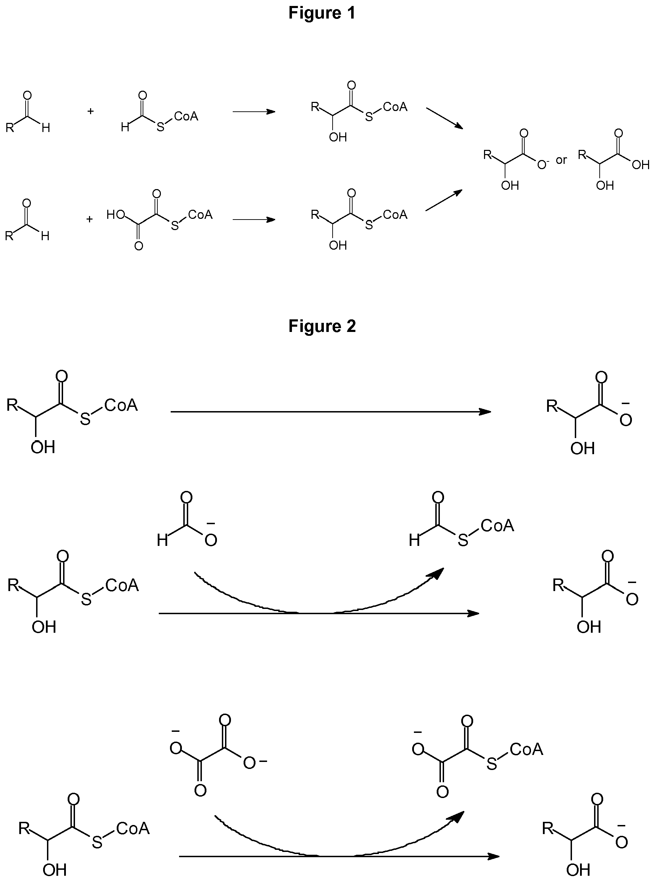

An example for the carbon-carbon-bond formation is depicted in figures 1 and 2.

The use of oxalyl-CoA decarboxylase from ***Methylobacterium extorquens*** or a mutant thereof (**3a**) were not known in the state of the art. Remarkably, the oxalyl-CoA decarboxylase is normally used for the carbon-carbon-bond cleavage. In particular, the oxalyl-CoA decarboxylase from *Methylobacterium extorquens* exhibits a high activity towards aromatic aldehydes in comparison to aliphatic aldehydes (example 7, and table 4).

*Methylobacterium extorquens* is also known as *Methylorubrum extorquens.*

A further aspect of the present invention is related to the use of peptide OXC_{Me} encoded by a gene from *Methylobacterium extorquens* or mutant thereof for the carbon-carbon-bond formation in particular for the synthesis of an α-hydroxy carboxylic acid, salts thereof or an α-hydroxyacyl-CoA thioester.

In addition, it could be shown that the α-hydroxyacyl-CoA thioester formed can be hydrolyzed by a hydrolase YciA from *E. coli* (**4**) to yield an α-hydroxy carboxylic acid, or its salts. The process can be carried out either in subsequent manner or in one pot.
Surprisingly, the hydrolase YciA from *E. coli* is selective for aromatic α-hydroxyacyl-CoA thioester, i.e. the hydrolysis of oxalyl-CoA or formyl-CoA is slower than for α-hydroxyacyl-CoA thioester.

A further aspect of the present invention is related to the use of a composition comprising or consisting of a peptide oxalyl-CoA decarboxylase OXC_{Me} encoded by a gene from *Methylobacterium extorquens* or a mutant thereof for the preparation of an α-hydroxy carboxylic acid, salt thereof or α-hydroxyacyl-CoA thioester starting from oxalyl-CoA and an aldehyde.

### Description of the invention

"Aromatic aldehydes", as used herein, refer to a compound of the formula (I), (I'), (II), (II"), (3), (3'), wherein the substituent R is an aromatic substituent including benzyl, homo-benzyl and bis-homo-benzyl substituents.

"Aliphatic aldehydes", as used herein, refer to a compound of (I), (I'), (II), (II"), (3), (3'), wherein the substituent R is an aliphatic substituent.

The substituent -SCoA as used herein refers to the coenzyme, and not to the disulfide. The sulphur is depicted in order to emphasize the position where it bonds.

The stereochemistry of formula (I), formula (II) and formula (3) as depicted in the following refers to an enantiomeric excess of the depicted enantiomer.

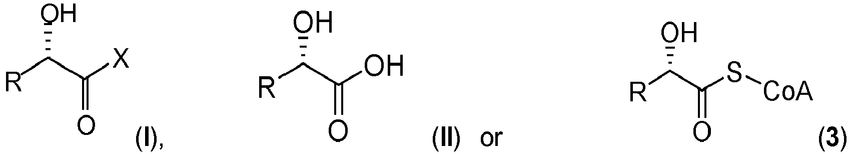

Thus, the compounds can also be a mixture of enantiomers but with an excess of the depicted enantiomer.

It could be shown that the enantiomer having the *(S)*-configuration can be prepared in an enantiomeric excess. Thus, the methods according to the invention preferably lead to α-hydroxyacylcompound of the formula (I)

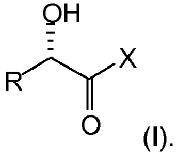

Thus, an embodiment of the invention is related to a method for preparation of the compounds according to a method described herein represented by formula (I)

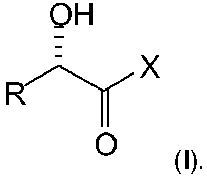

The methods according to the invention are preferably used to prepare an α-hydroxyacyl compound of the formula (I) with an enantiomeric excess of the (S)-enantiomer.

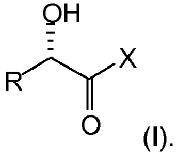

More preferably the method according to the invention is used to prepare a compound of the formula (**I**), wherein the compound can be mixture of the enantiomers, and wherein the *(S)*-enantiomer is present in an enantiomeric excess. Preferably, the enantiomeric excess (ee) is at least 5%, preferably 10%, more preferably 15%, more preferably 20%, more preferably 25%, more preferably 30%, more preferably 35%, more preferably 40%, more preferably 45%, more preferably 50%, more preferably 55%, more preferably 60%, more preferably 65%, more preferably 65%, more preferably 70%, more preferably 75%, more preferably 80%, more preferably 85%, more preferably 90%, more preferably 91%, more preferably 92%, more preferably 93%, more preferably 94%, more preferably % 95%, more preferably 96%, more preferably 97%, more preferably 98%, and most preferably 99%.

Moreover, the methods according to the invention preferably lead to α-hydroxyacyl compound of the formula (**3**)

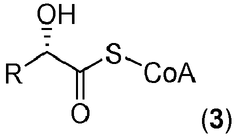

The methods according to the invention are preferably used to prepare an α-hydroxyacyl compound of the formula (I) with an enantiomeric excess of the (S)-enantiomer.

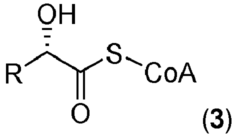

More preferably the method according to the invention is used to prepare a compound of the formula (**3**), wherein the compound can be mixture of the enantiomers, and wherein the *(S)*-enantiomer is present in an enantiomeric excess. Preferably, the enantiomeric excess (ee) is at least 5%, preferably 10%, more preferably 15%, more preferably 20%, more preferably 25%, more preferably 30%, more preferably 35%, more preferably 40%, more preferably 45%, more preferably 50%, more preferably 55%, more preferably 60%, more preferably 65%, more preferably 65%, more preferably 70%, more preferably 75%, more preferably 80%, more preferably 85%, more preferably 90%, more preferably 91%, more preferably 92%, more preferably 93%, more preferably 94%, more preferably % 95%, more preferably 96%, more preferably 97%, more preferably 98%, and most preferably 99%.

Moreover, the methods according to the invention preferably lead to α-hydroxyacyl compound of the formula (**II**)

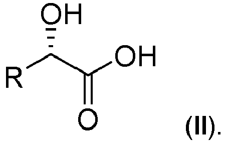

The methods according to the invention are preferably used to prepare an α-hydroxyacyl compound of the formula (II) with an enantiomeric excess of the (S)-enantiomer

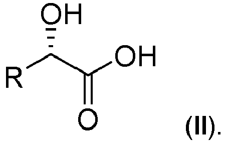

More preferably the method according to the invention is used to prepare a compound of the formula (**II**), wherein the compound can be mixture of the enantiomers, and wherein the *(S)*-enantiomer is present in an enantiomeric excess. Preferably, the enantiomeric excess (ee) is at least 5%, preferably 10%, more preferably 15%, more preferably 20%, more preferably 25%, more preferably 30%, more preferably 35%, more preferably 40%, more preferably 45%, more preferably 50%, more preferably 55%, more preferably 60%, more preferably 65%, more preferably 65%, more preferably 70%, more preferably 75%, more preferably 80%, more preferably 85%, more preferably 90%, more preferably 91%, more preferably 92%, more preferably 93%, more preferably 94%, more preferably % 95%, more preferably 96%, more preferably 97%, more preferably 98%, and most preferably 99%.

Therefore, an embodiment according to the invention is related to a method for the preparation of an α-hydroxyacyl compound of the formula (**I**) 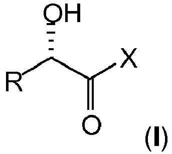 comprising:
**A**) providing a compound R-CHO (**1**) and an acyl-CoA (**2**) 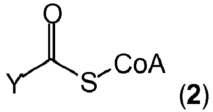
   wherein Y represents -H, or -COO⁻,
   in a buffer solution with a pH-value in the range of 5.0 to 7.5 optionally together with at least one co-solvent;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the *(S)*-α-hydroxyacyl-CoA thioester (**I**) 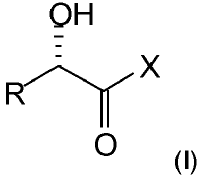
   wherein X represents -S-CoA,
   and the oxalyl-CoA decarboxylase (**3a**) is a peptide encoded by a gene selected from *Methylobacterium extorquens* or a mutant thereof; and optionally comprising the additional step **C**)
**C**) cleaving the coenzyme A moiety of said *(S)*-α-hydroxyacyl-CoA thioester (**I**) by a hydrolase, α-hydroxyacyl-CoA:oxalate CoA-transferase and/or α-hydroxyacyl-CoA:formate CoA-transferase to obtain the compound of the formula (**I**) or a salt thereof 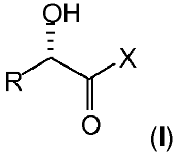
   wherein X represents -OH or O⁻,
   and the hydrolase is acyl-CoA thioester hydrolase YciA (**4**) encoded by a gene selected from *E. coli.*

The peptide in a method according to the invention is preferably used in its isolated form, i.e. the peptide is isolated from the microbe or it is not contained in a microbe.

Herein, the first time the enantioselective carbon-carbon-formation of an α-hydroxyacyl compound according to formula (**I**), formula (II) or formula (3) by means of an oxalyl-CoA decarboxylase could be shown. In particular, the conditions are suitable for the synthesis of the *(S)*-enantiomer (example 14, table 5).

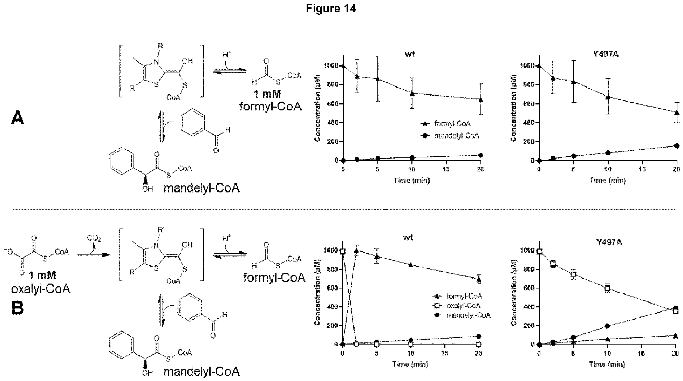

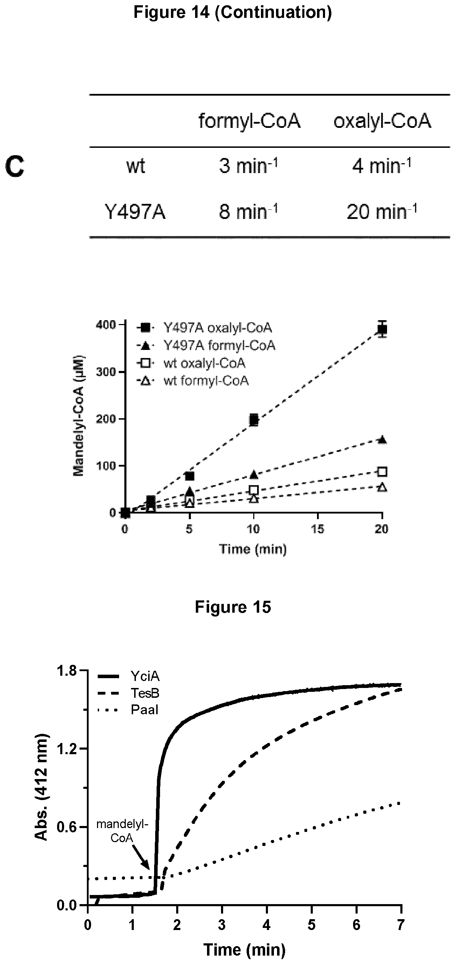

It could be shown that the synthesis of α-hydroxyacyl-CoA thioester is faster when oxalyl-CoA thioester is provided as a C₁-unit source (see figure 14). Thus, in step **A**) of a method according to the invention acyl-CoA (**2**) being provided is preferably an oxalyl-CoA (**2'**).

Thus, an embodiment of the invention is related to a method for the preparation of the compounds according to a method described herein represented by formula (**I'**) 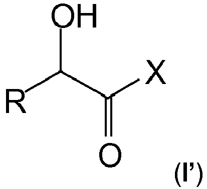 and wherein in step A) an acyl-CoA (2) is provided 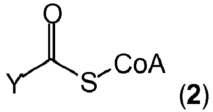 wherein Y represents -COO⁻.

A further embodiment of the invention is related to a method for the preparation of the compounds according to a method described herein represented by formula (**I**) 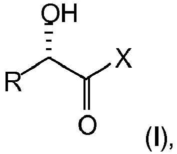 and wherein in step A) an acyl-CoA (2) is provided 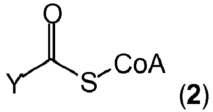 wherein Y represents -COO⁻.

The oxally-CoA according to formula (**2**) 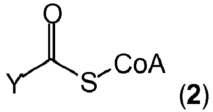 wherein Y represents -COO⁻ is also represented by formula (**2'**)

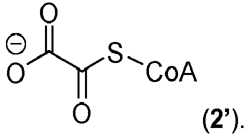

The α-hydroxyacyl-CoA thioester according to formula (**I'**) 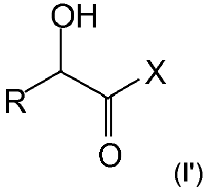 wherein X represents -S-CoA is also represented by formula (**3'**)

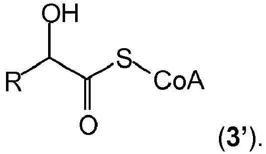

A compound according to formula (**I'**) 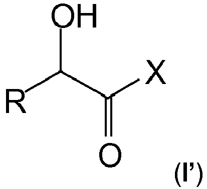 wherein X represents -OH or O⁻ is also represented by formula (**II'**)

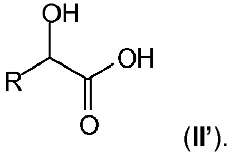

The *(S)*-α-hydroxyacyl-CoA thioester according to formula (**I**) 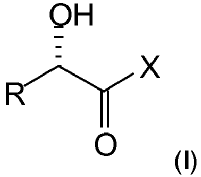 wherein X represents -S-CoA is also represented by formula (**3**)

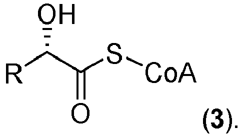

A compound according to formula (I) 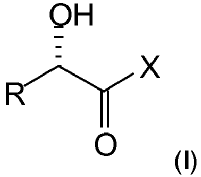 wherein X represents -OH or O⁻ is also represented by formula (**II**)

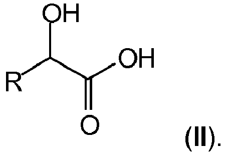

Thus, an embodiment of the invention is directed to a method for preparation of a compound of the formula (**I'**) 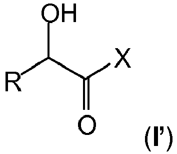 comprising:
**A**) providing a compound R-CHO **(1)** and an oxalyl-CoA (**2'**) 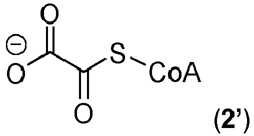 in a buffer solution with a pH-value in the range of 5.0 to 7.5, optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the α-hydroxyacyl-CoA thioester (**3'**) 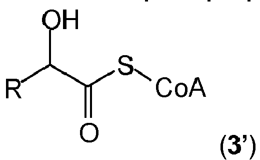 wherein the oxalyl-CoA decarboxylase (**3a**) is a peptide encoded by a gene selected from *Methylobacterium extorquens* or a mutant thereof; and optionally comprising the additional step **C**)
**C**) cleaving the coenzyme A moiety of said α-hydroxyacyl-CoA thioester (**3'**) by a hydrolase , α-hydroxyacyl-CoA:oxalate CoA-transferase, and/or α-hydroxyacyl-CoA:formate CoA-transferase to obtain the compound of the formula (II') or a salt thereof 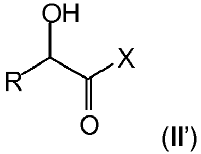 wherein the hydrolase is acyl-CoA thioester hydrolase YciA (**4**) encoded by a gene selected from *E. coli.*

Further, an embodiment of the invention is directed to a method for preparation of a compound of the formula (**I**) 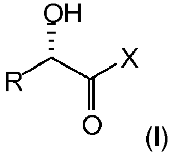 comprising:
**A**) providing a compound R-CHO **(1)** and an oxalyl-CoA (**2**) 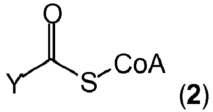
   wherein Y represents -COO⁻;
   in a buffer solution with a pH-value in the range of 5.0 to 7.5, and optionally together with at least one co-solvent;
**B**) performing a coupling reaction of R-CHO (**1**) and oxalyl-CoA (**2**) by an oxalyl-CoA decarboxylase (**3a**) in the presence of thiamine diphosphate (**3b**) and a magnesium (II) cation to obtain the *(S)*-α-hydroxyacyl-CoA thioester (**I**) 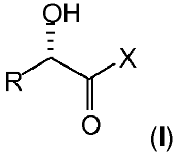
   wherein X represents -S-CoA,
   and the oxalyl-CoA decarboxylase (**3a**) is a peptide encoded by a gene selected from *Methylobacterium extorquens* or a mutant thereof; and optionally comprising the additional step **C**)
**C**) cleaving the coenzyme A moiety of said *(S)*-α-hydroxyacyl-CoA thioester (**3**) by a hydrolase , α-hydroxyacyl-CoA:oxalate CoA-transferase and/or α-hydroxyacyl-CoA:formate CoA-transferase to obtain the compound of the formula (I) or a salt thereof 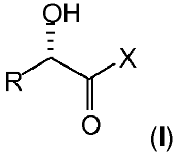
   wherein X represents -OH or O⁻,
   and the hydrolase is acyl-CoA thioester hydrolase YciA (**4**) encoded by a gene selected from *E. coli.*

The method according to the invention is preferably used to synthesis a compound of the formula (**II'**) 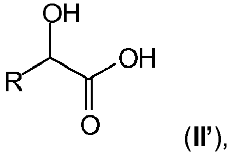 and preferably a compound according to formula (**II**)

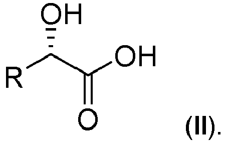

Thus, a preferred embodiment of the present invention is related to a method for preparation of a compound of the formula (II') 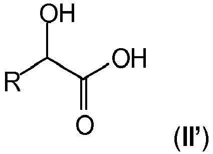 comprising:
**A**) providing a compound R-CHO **(1)** and an oxalyl-CoA (2') 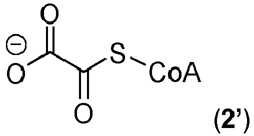 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the α-hydroxyacyl-CoA thioester (**3'**) 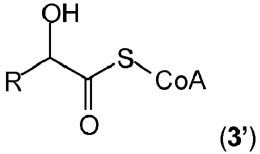 wherein the oxalyl-CoA decarboxylase (**3a**) is a peptide encoded by a gene selected from *Methylobacterium extorquens* or a mutant thereof; and
**C**) cleaving the coenzyme A moiety of said α-hydroxyacyl-CoA thioester (**3'**) by a hydrolase, α-hydroxyacyl-CoA:oxalate CoA-transferase and/or α-hydroxyacyl-CoA:formate CoA-transferase to obtain the compound of the formula (**II'**) or a salt thereof 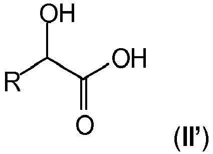 wherein the hydrolase is acyl-CoA thioester hydrolase YciA (**4**) encoded by a gene selected from *E. coli.*

A more preferred embodiment of the present invention is related to a method for preparation of a compound of the formula (II) 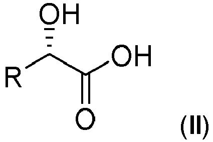 wherein **R** represents 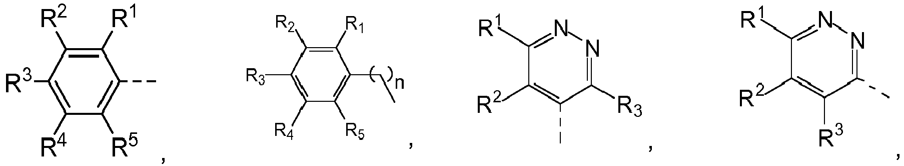 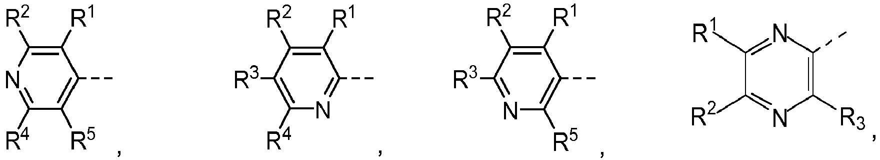 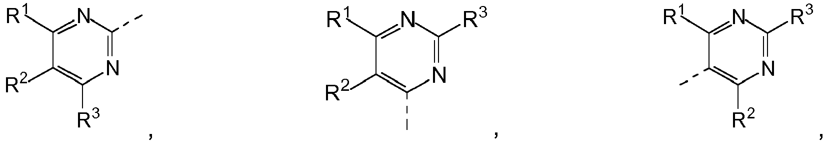 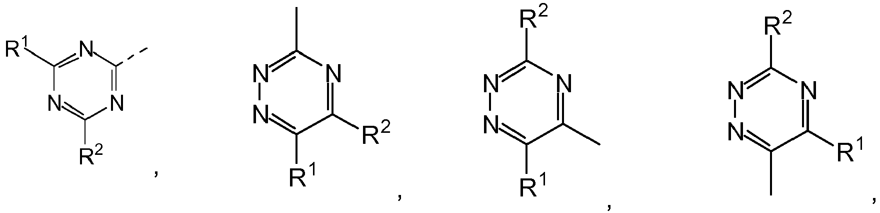 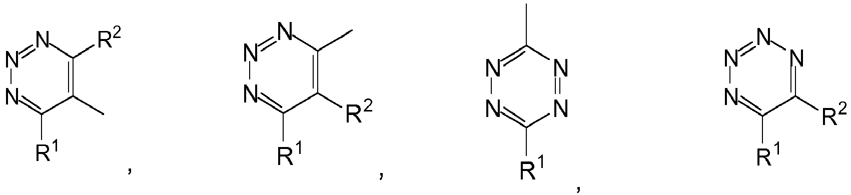 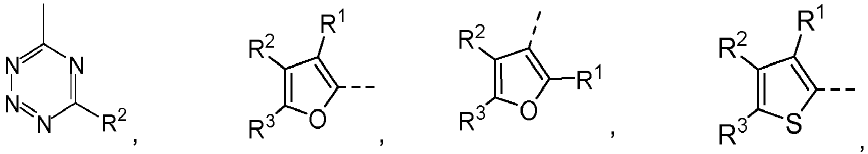 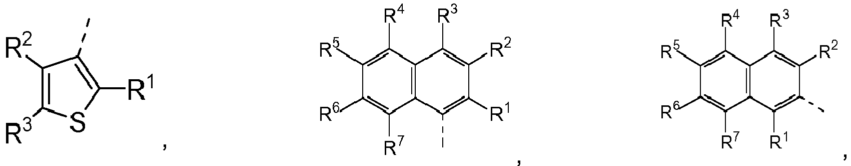 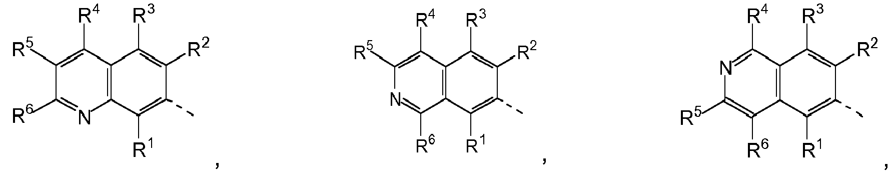 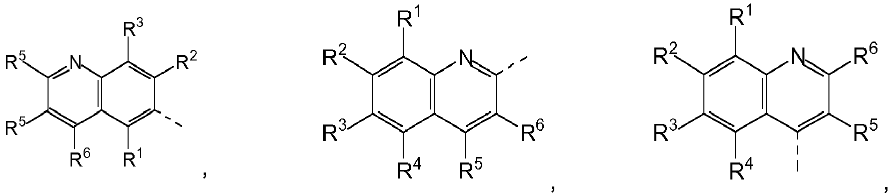 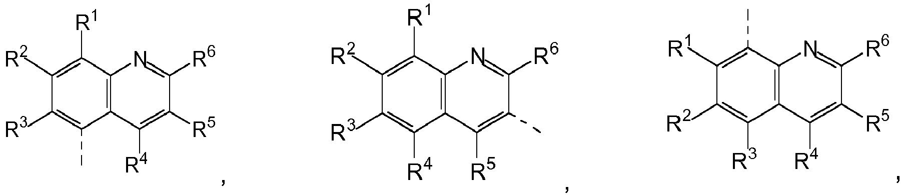 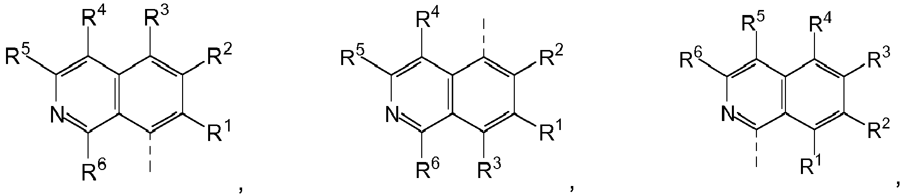 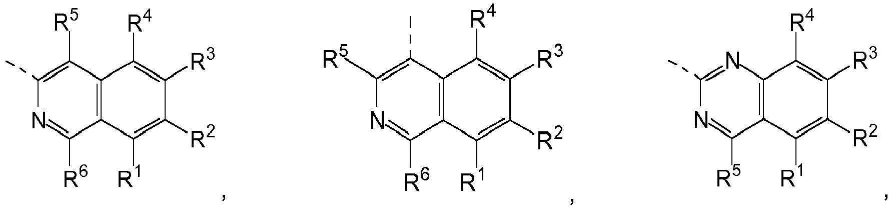 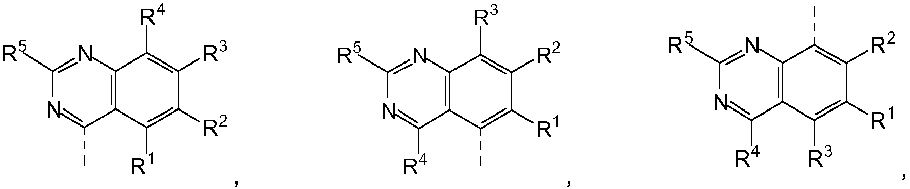 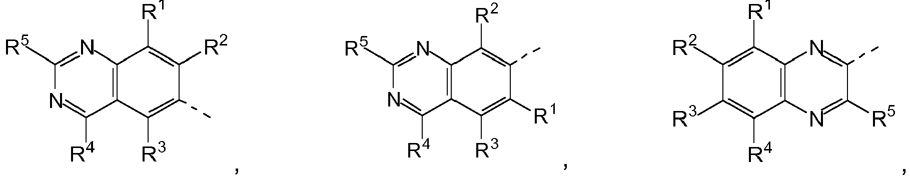 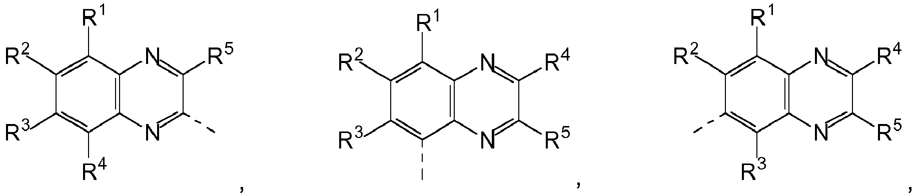 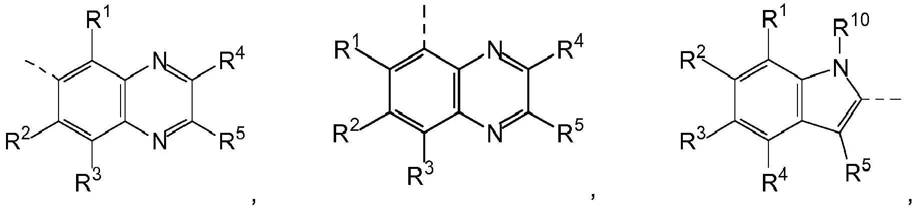 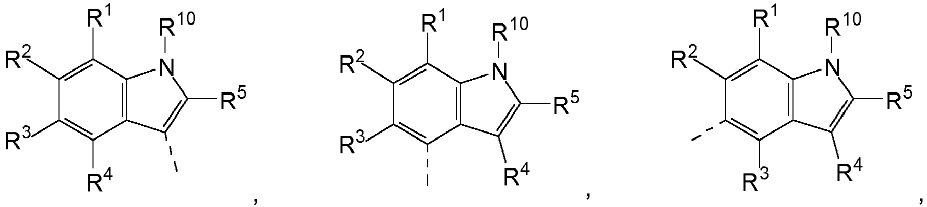 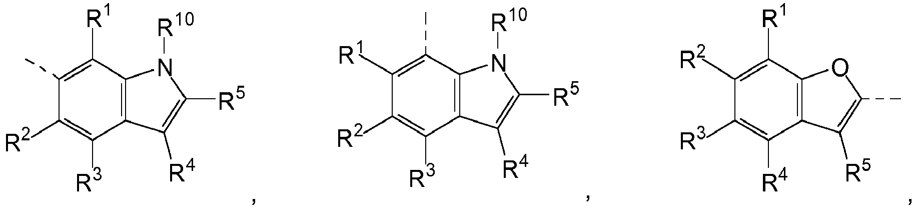 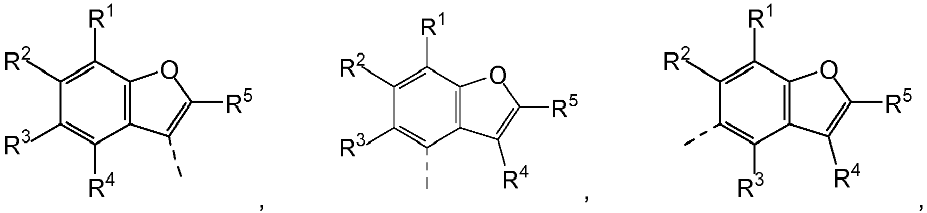 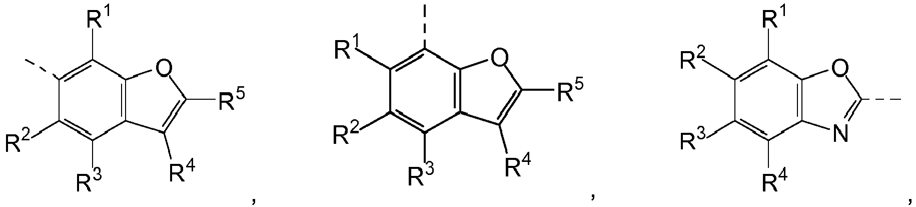 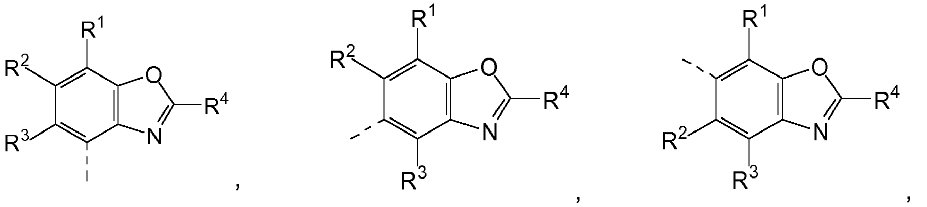 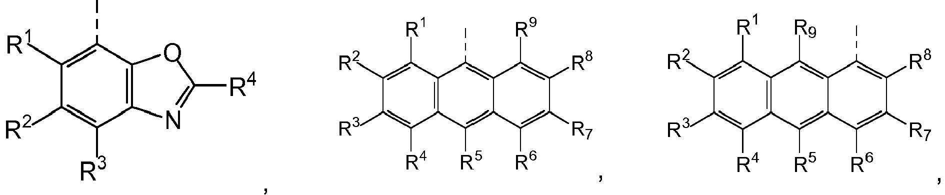 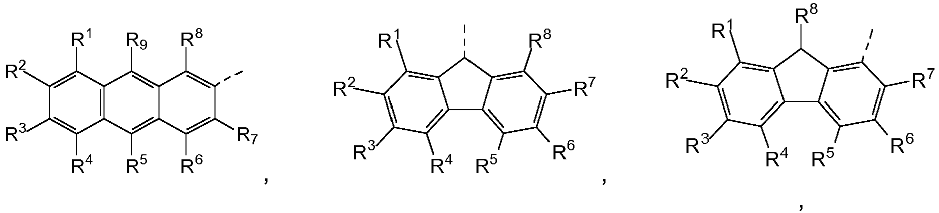 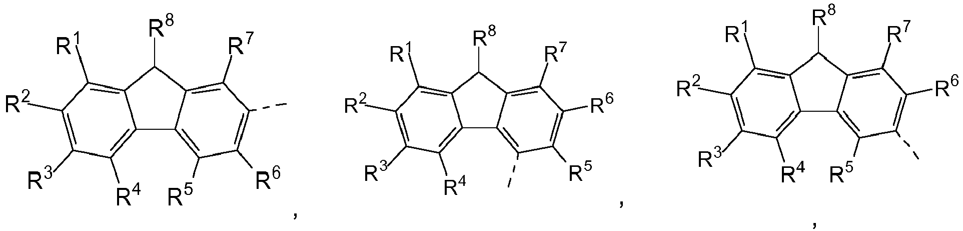 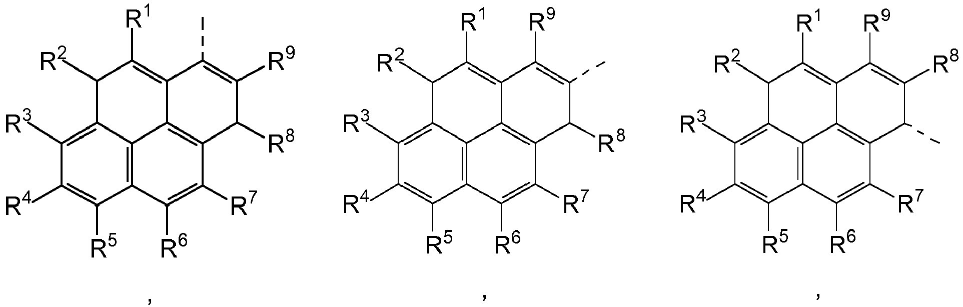 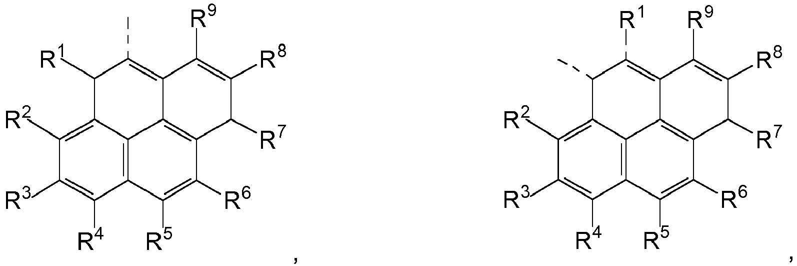 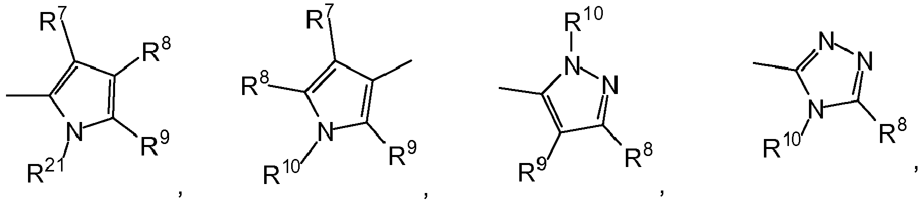 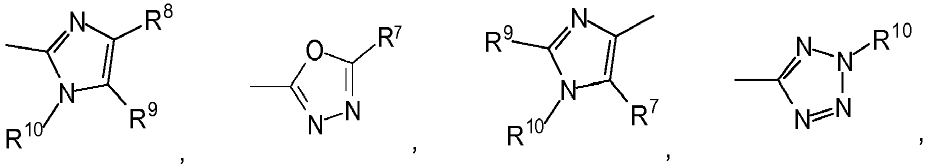 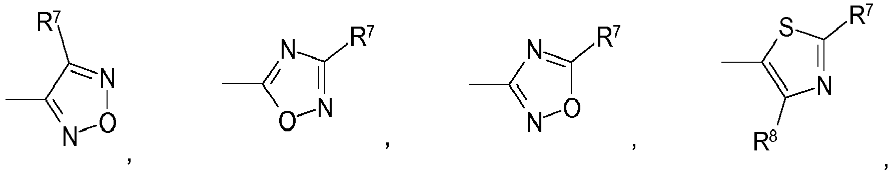 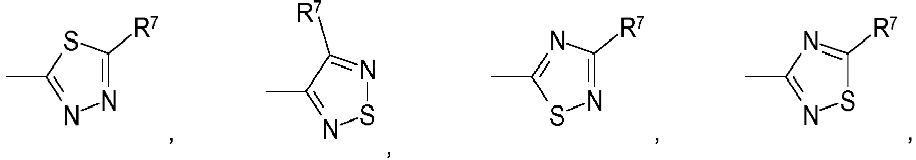 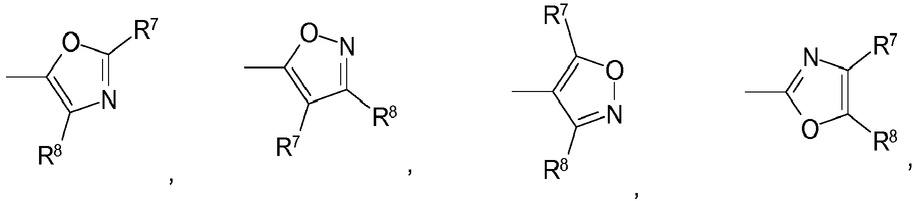 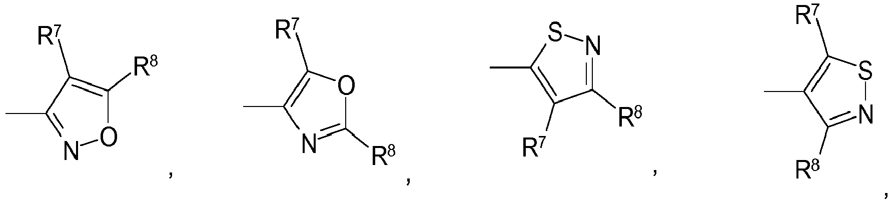 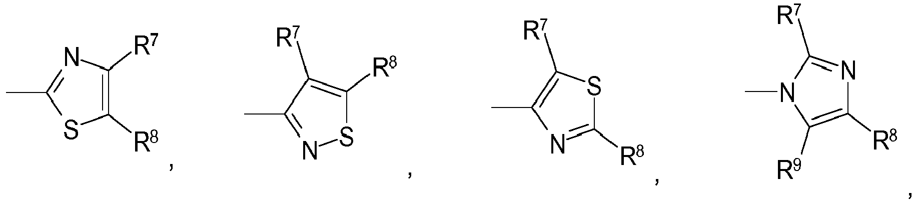 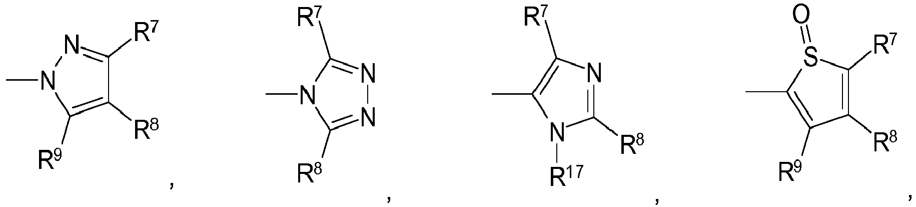 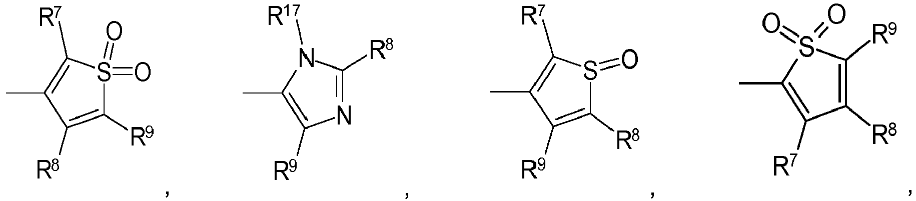 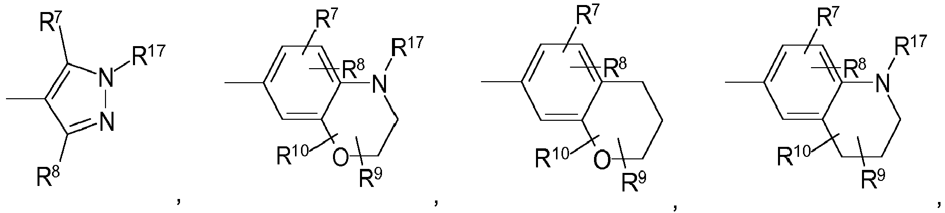 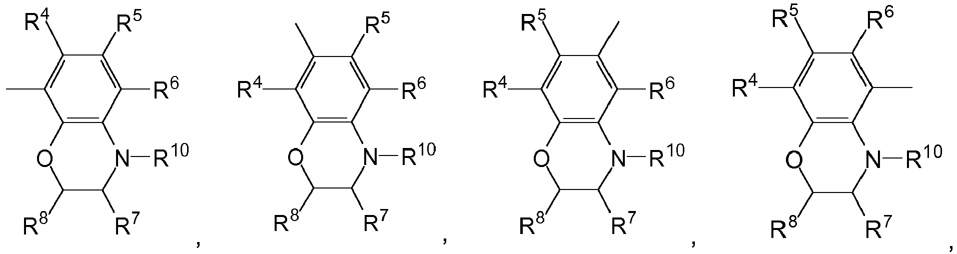 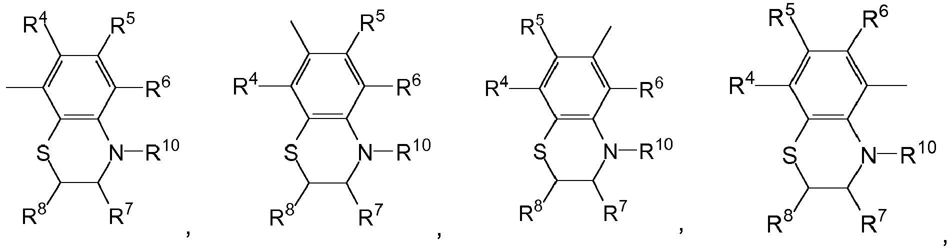 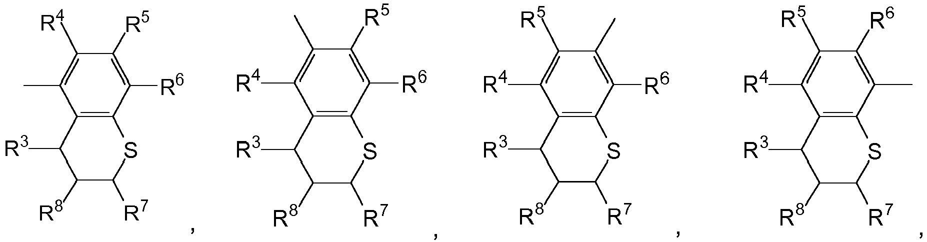 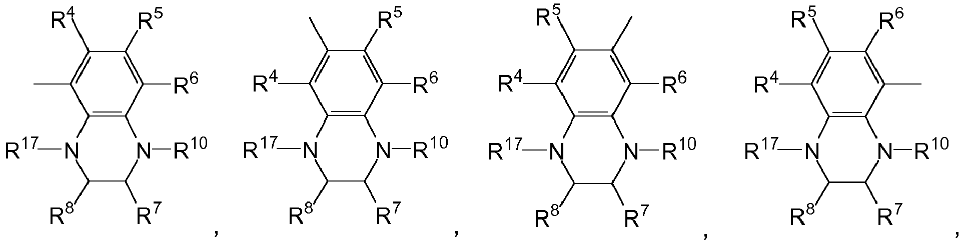 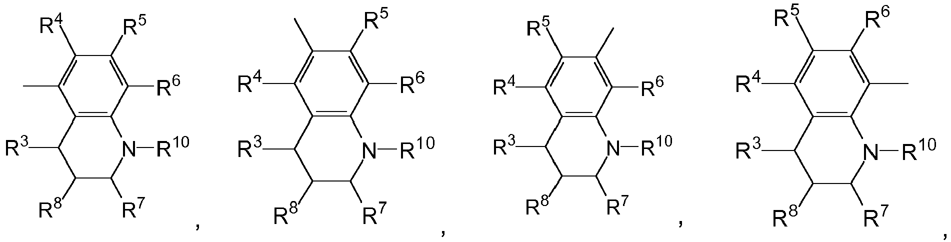 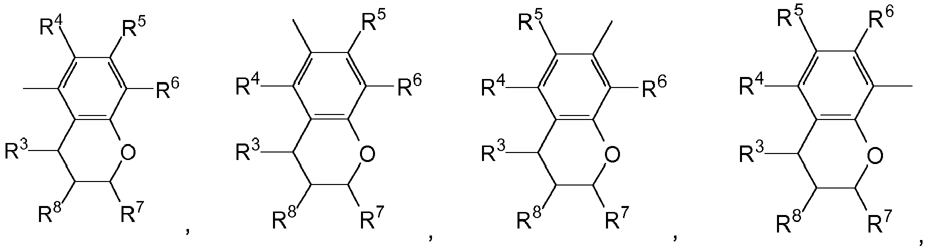 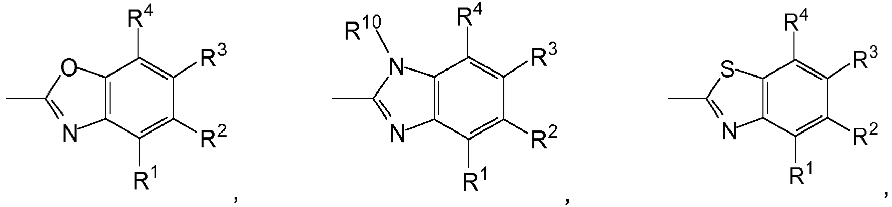 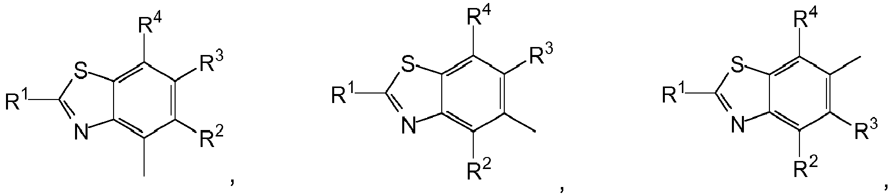 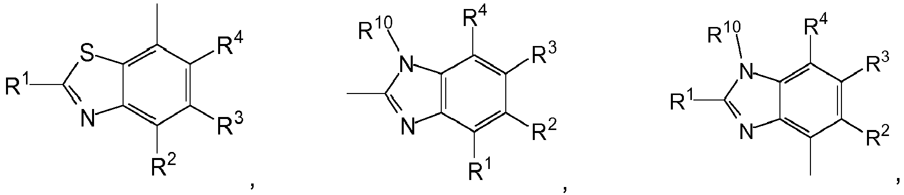 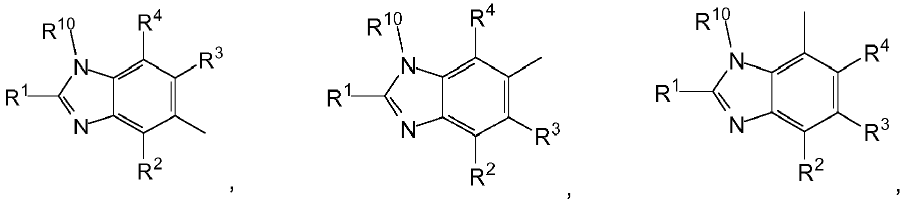 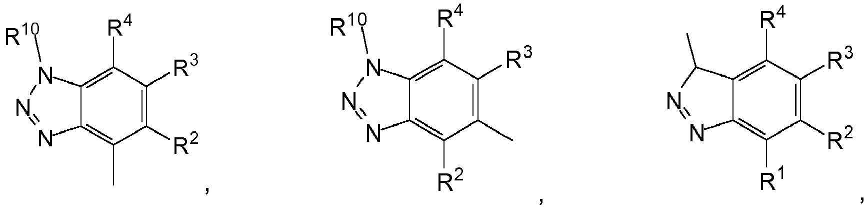 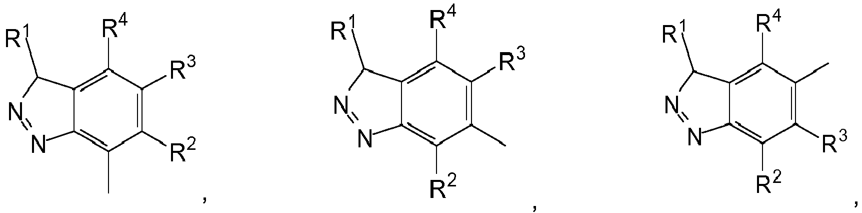 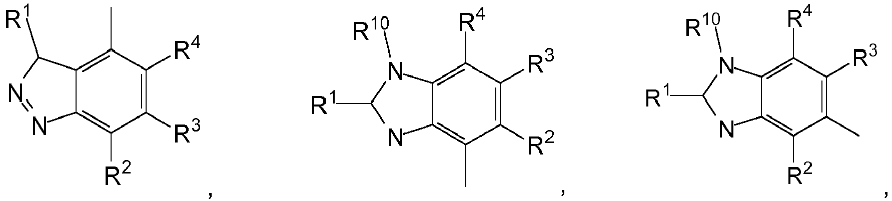 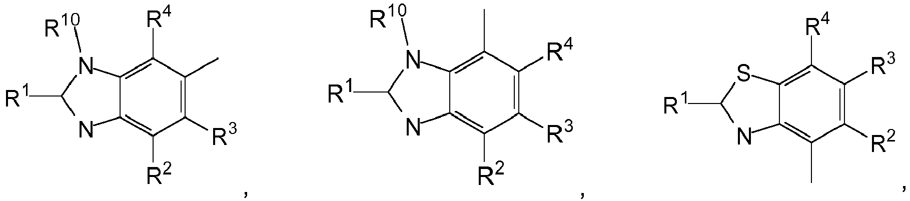 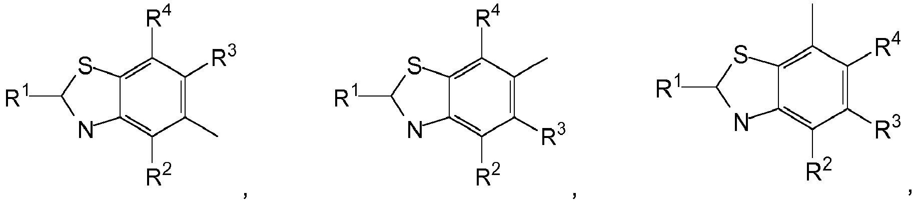 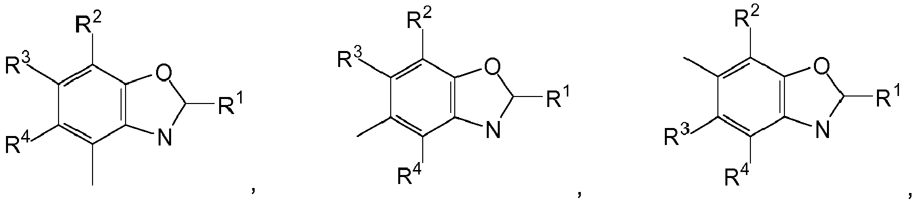 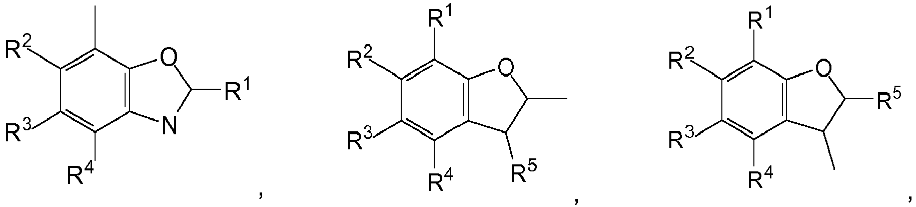 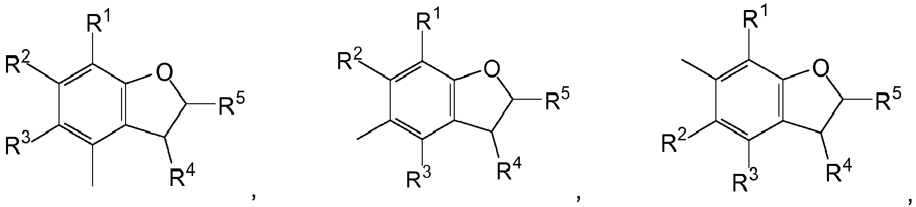 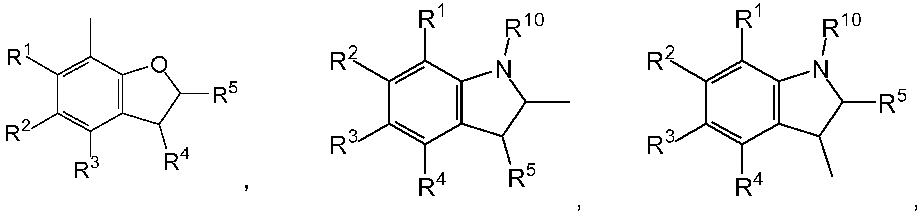 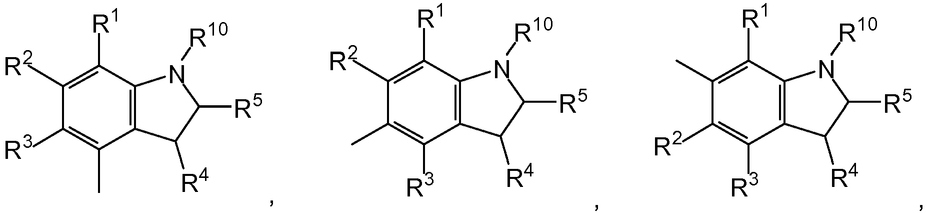 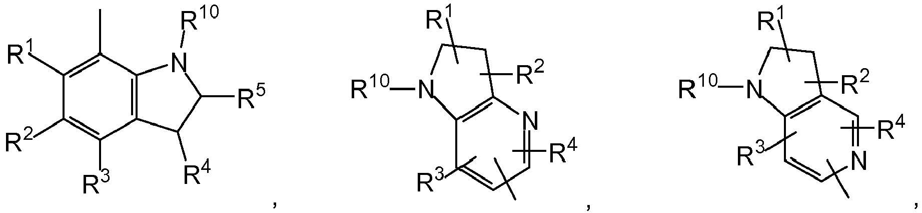 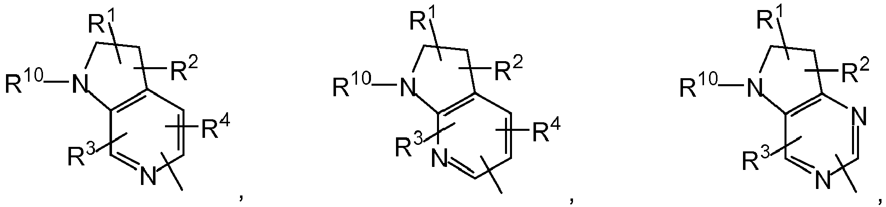 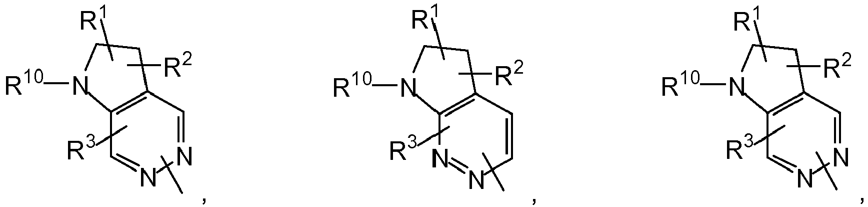 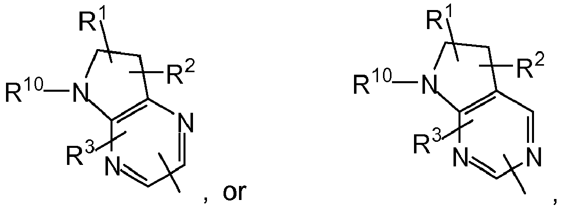 wherein **R¹, R², R³, R⁴, R⁵, R⁶, R⁷, R⁸, and R⁹** represent independently of each other -H, -F, -CI, -Br, -I, -Ph, -OH, -OCH₃, -OC₂H₅, -OC₃H₇, -OCH(CH₃)₂, -OC₄H₉, -OCH(CH₃)CH₂CH₃, -OCH₂CH(CH₃)₂, -OC(CH₃)₃, -O-cyclo-C₃H₅, -O-cyclo-C₄H₇, -O-cyclo-C₅H₉, -O-cyclo-C₆H₁₁, -OPh, -OCH₂-Ph, -OCH₂CH₂-Ph -OCH=CH₂, -OCH₂-CH=CH₂, -OCH₂CH₂-CH=CH₂, -OCF₃, -OC₂F₅, -SCH₃, -SC₂H₅, -SC₃H₇, -SCH(CH₃)₂, -SC₄H₉, -SCH(CH₃)CH₂CH₃, -SCH₂CH(CH₃)₂, -SC(CH₃)₃, -NO₂, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(C₄H₉)₂, -N(cyclo-C₃H₅)₂, -N[CH(CH₃)₂]₂, -N[C(CH₃)₃]₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH₂-CH₂-CH(CH₃)₂, -CH(CH₃)-CH(CH₃)-CH₃, -C(CH₃)₂-C₂H₅, -C₆H₁₃, -CH(CH₃)-C₄H₉, -CH₂-CH(CH₃)-C₃H₇, -CH₂-CH₂-CH₂-CH(CH₃)₂, -CH(CH₃)-CH(CH₃)-C₂H₅, -C(CH₃)₂-C₃H₇, -C₇H₁₅, -CH(CH₃)-C₅H₁₁, -CH₂CH(CH₃)-C₄H₉, -CH₂-CH₂-CH₂-CH₂CH(CH₃)₂, -C₈H₁₇, -C₉H₁₉, -C₁₀H₂₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -CH₂F, -CF₂I, -CHF₂, -CF₃, -CH₂I, -CH₂Br, -CH₂I, -CH₂-CH₂F, -CH₂-CHF₂, -CH₂-CF₃, -CH₂-CH₂I, -CH₂-CH₂Br, -CH₂-CH₂I, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CHO, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COO-cyclo-C₄H₇, -COO-cyclo-C₅H₉, -COO-cyclo-C₆H₁₁, -COOCH(CH₃)₂, -COOC(CH₃)₃, -O-COOCH₃, -O-COOC₂H₅, -O-COOC₃H₇, -O-COO-cyclo-C₃H₅, -O-COOCH(CH₃)₂, -O-COOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, 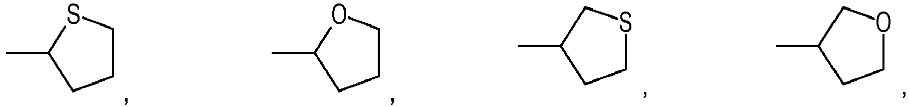 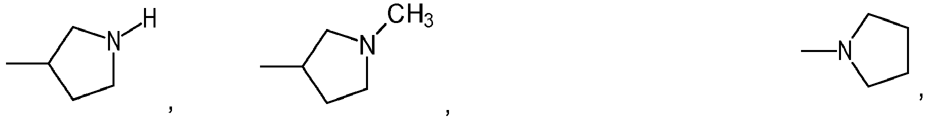 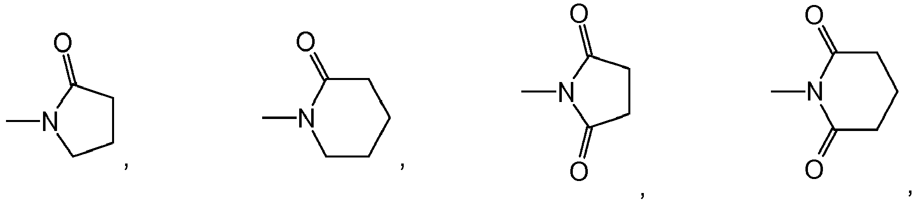 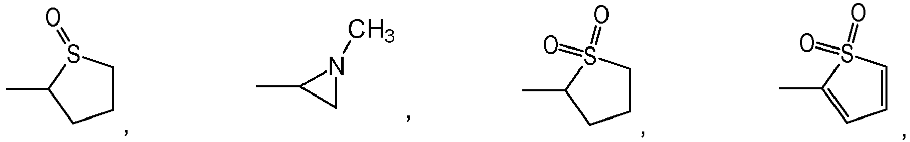 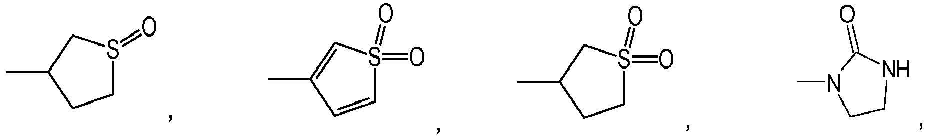 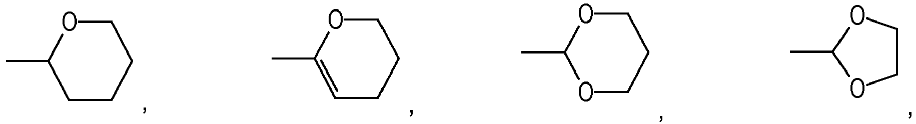 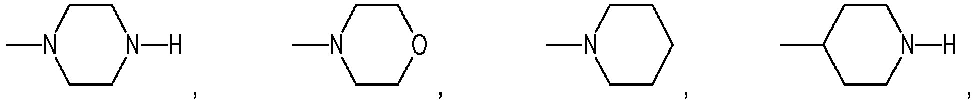 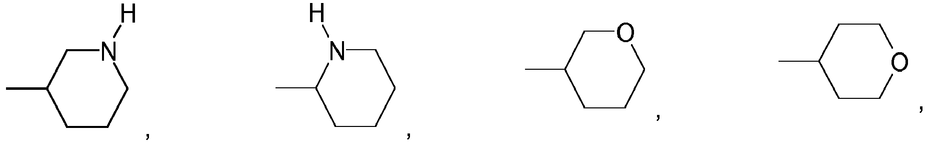 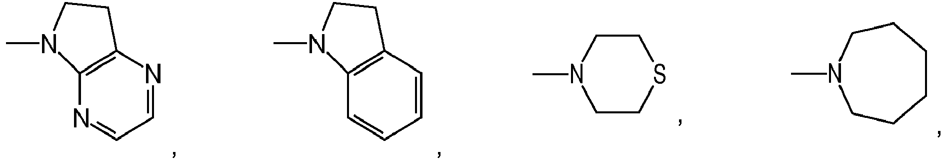 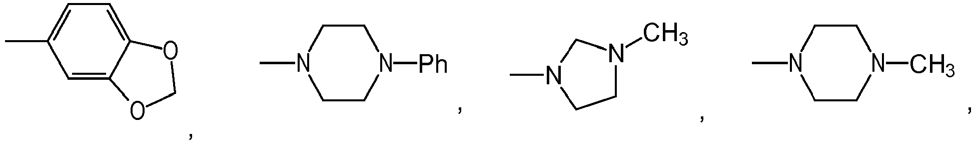 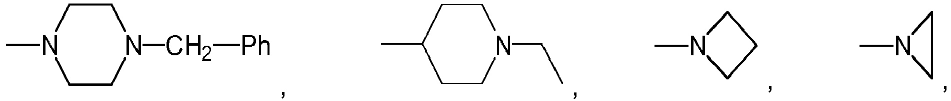 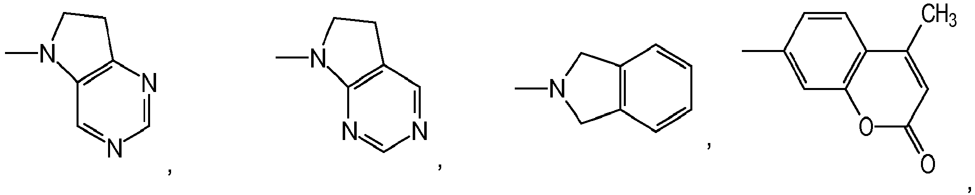 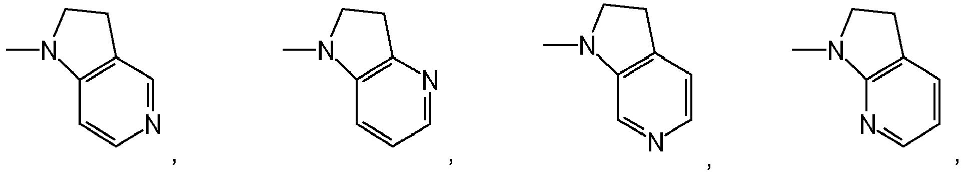 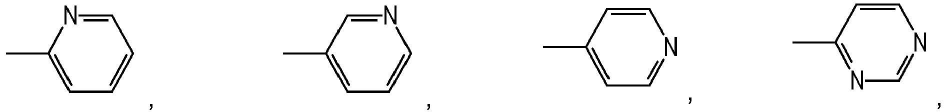 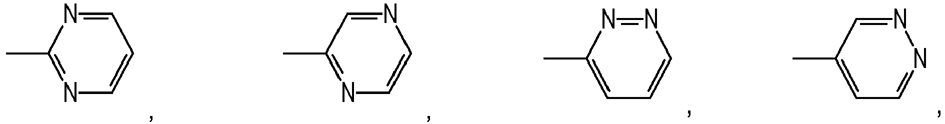 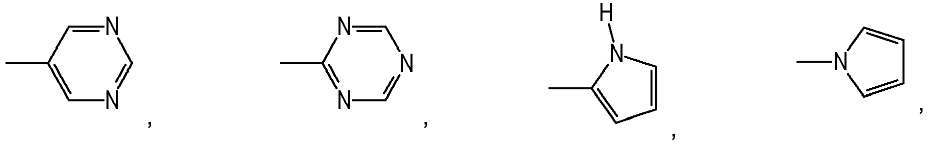 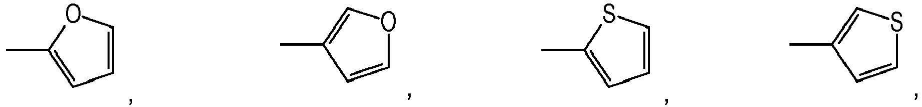 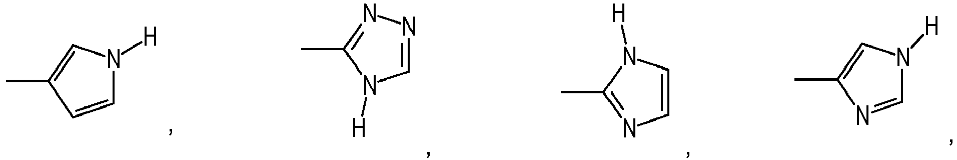 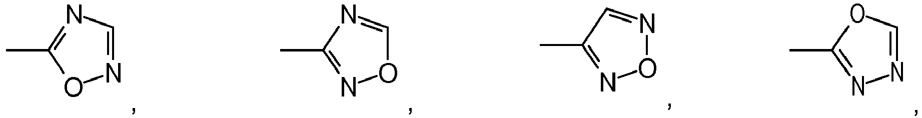 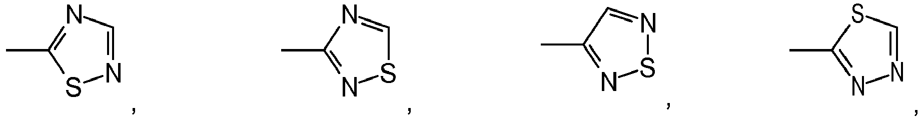 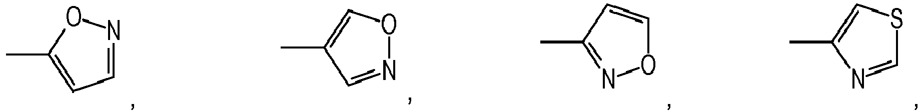 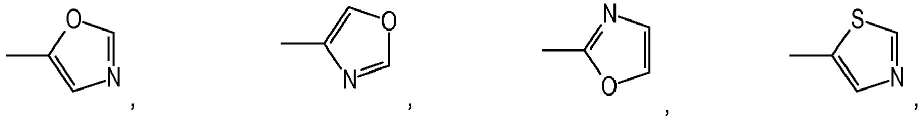 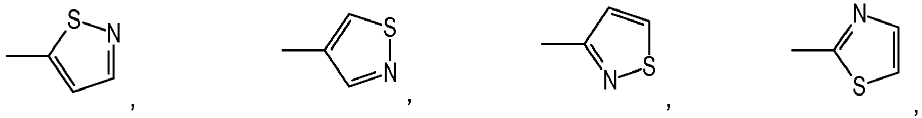 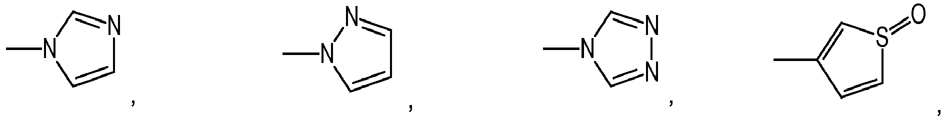 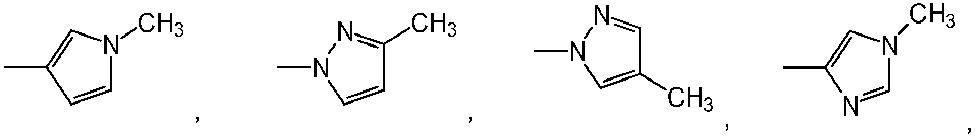 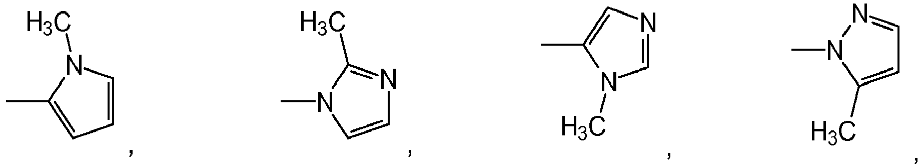 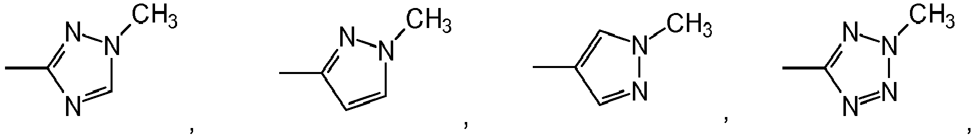 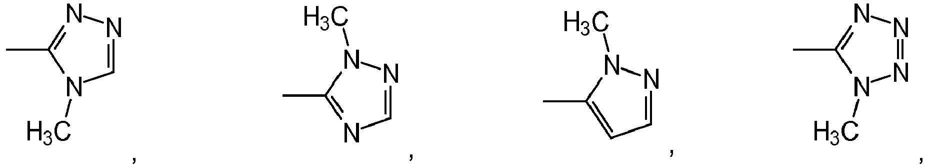 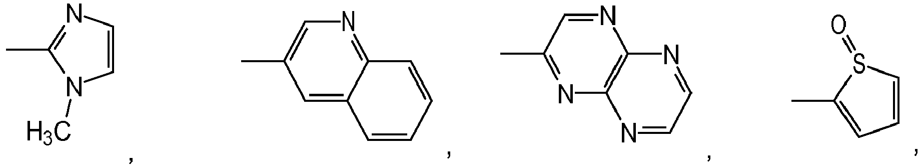 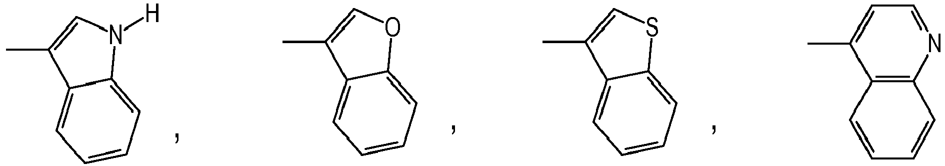 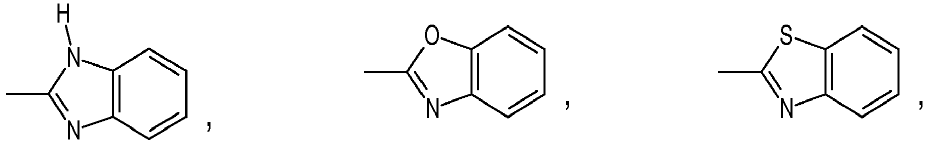 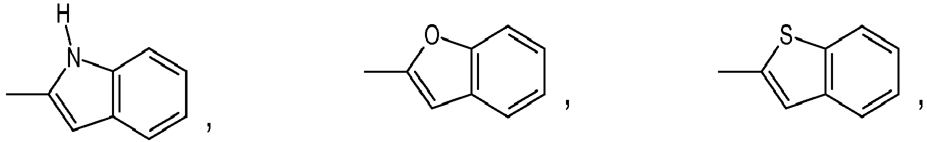 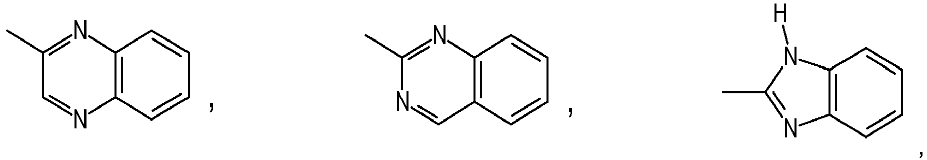 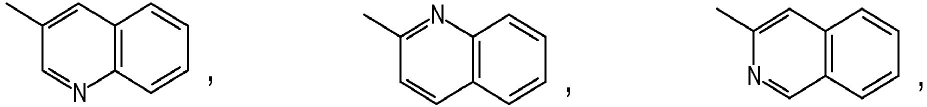 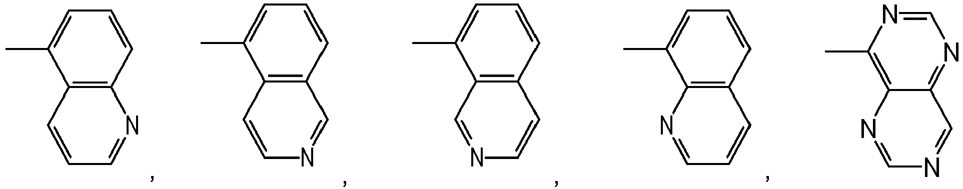 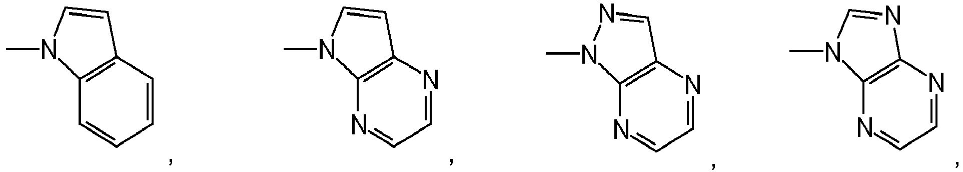 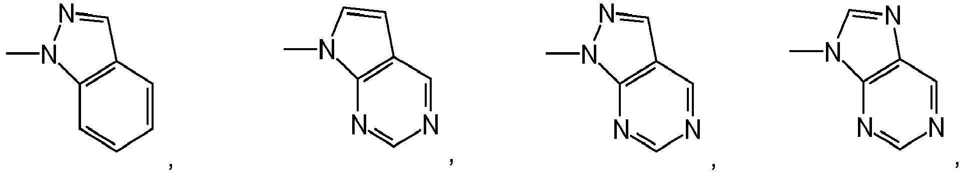 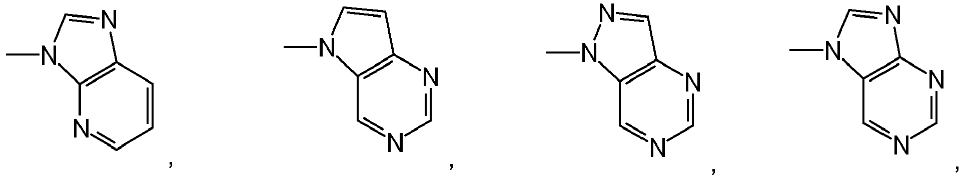 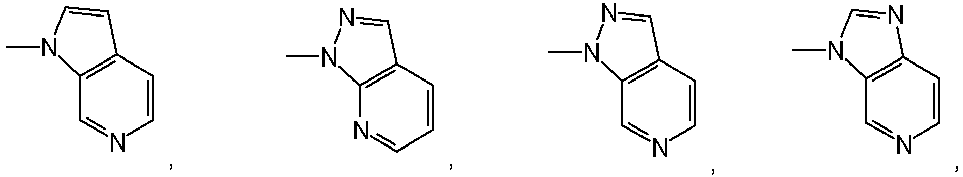 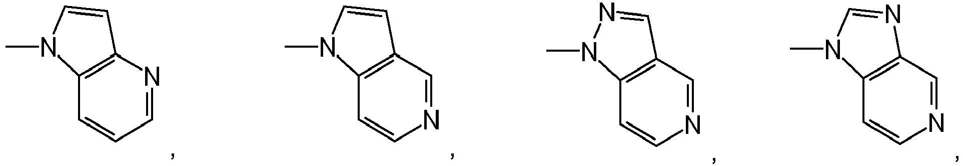 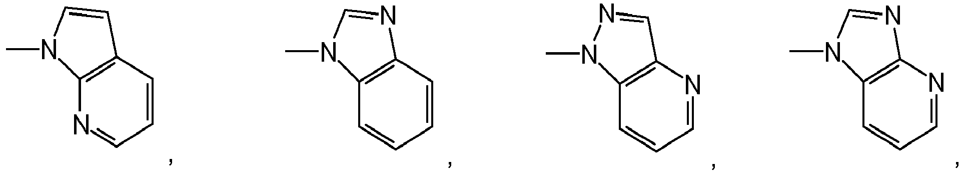 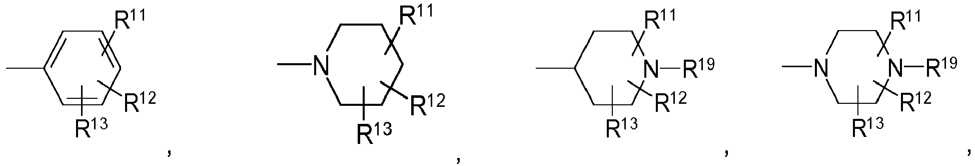 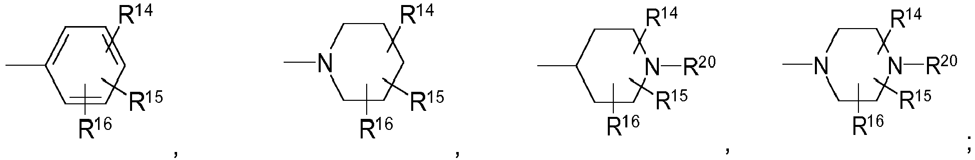
**R¹¹** to **R¹⁶** represents independently of each other:
   -H, -NH₂, -OH, -OCH₃, -OC₂H₅, -OC₃H₇, -OCF₃, -CF₃, -F, -CI, -Br, -I, -CH₃, -C₂H₅, -C₃H₇, -CH(CH₃)₂, -Ph, or -CN;
**R¹⁰, R¹⁷, R¹⁸, R¹⁹, R²⁰, and R²¹** represents independently of each other -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, CH₂-CH₂F, -CH₂-CHF₂, -CH₂-CF₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -C≡CH, -C≡C-CH₃, -CH₂-C≡CH, -C₂H₄-C≡CH, -CH₂C≡C-CH₃, -C≡C-C₂H₅, -C₃H₆-C≡CH, -C₂H₄-C≡C-CH₃, -CH₂-C≡C-C₂H₅, -C≡C-C₃H₇, -CHO, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, preferably R²¹ cannot be -H;
wherein n is an integer 1, 2, or 3; or a salt thereof,
comprising:
   **A**) providing a compound R-CHO (**1**) and an oxalyl-CoA (**2**) 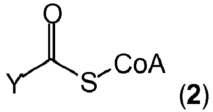
      wherein Y represents -COO⁻;
      in a buffer solution with a pH-value in the range of 5.0 to 7.5 optionally together with at least one co-solvent;
   **B**) performing a coupling reaction of R-CHO (**1**) and oxalyl-CoA (**2**) by an oxalyl-CoA decarboxylase (**3a**) in the presence of thiamine diphosphate (**3b**) and a magnesium (II) cation to obtain the *(S)*-α-hydroxyacyl-CoA thioester (**I**) 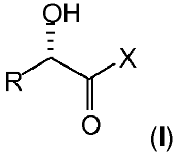
      wherein X represents -S-CoA,
      and the oxalyl-CoA decarboxylase (**3a**) is a peptide encoded by a gene selected from *Methylobacterium extorquens* or a mutant thereof; and
   **C**) cleaving the coenzyme A moiety of said *(S)*-α-hydroxyacyl-CoA thioester (**I**) by a hydrolase, α-hydroxyacyl-CoA:oxalate CoA-transferase and/or α-hydroxyacyl-CoA:formate CoA-transferase to obtain the compound of the formula (I) or a salt thereof 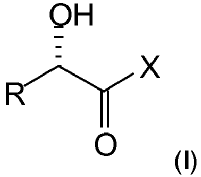
      wherein X represents -OH or O⁻,
      and the hydrolase is acyl-CoA thioester hydrolase YciA (**4**) encoded by a gene selected from *E. coli.*

Step B) can be performed for at least 0.5 hour, preferably 1 hour, more preferably 2 hours, more preferably 3 hours, more preferably 4 hours, more preferably 5 hours, more preferably 6 hours, more preferably 7 hours, more preferably 8 hours, more preferably 9 hours, more preferably 10 hours, more preferably 11 hours, more preferably 12 hours, more preferably 13 hours, more preferably 14 hours, more preferably 15 hours, more preferably 16 hours, more preferably 17 hours, more preferably 18 hours, more preferably 19 hours, more preferably 20 hours, more preferably 21 hours, more preferably 22 hours, more preferably 23 hours, more preferably 24 hours, more preferably 25 hours, more preferably 26 hours, more preferably 27 hours, and more preferably 28 hours.

Step B) can be performed from 0.5 hour to 48 hours, preferably from 1 hour to 40 hours, more preferably from 2 hours to 35 hours, more preferably from 3 hours to 30 hours, more preferably from 4 hours to 28 hours, more preferably form 5 hours to 26 hours, more preferably from 6 hours to 24 hours, more preferably form 8 hours to 24 hours, more preferably from 10 hours to 24 hours, more preferably form 12 hours to 24 hours, more preferably from 14 hours to 24 hours, more preferably from 16 hours to 24 hours, and most preferably form 18 hours to 24 hours.

In general, after performing step B), i.e. the carbon-carbon-bond forming step, the formed α-hydroxyacyl-CoA thioester can be isolated, and hydrolyzed in a subsequent step C) to obtain the α-hydroxy carboxylic acid, or salts thereof.

In order to increase the efficiency of the method, the steps in particular the steps A), B) and C) of a method according to invention described herein can be carried out as cascade reaction in one-pot system. If in a method according to the invention the steps A), B) and C) is performed as cascade reaction in one-pot system preferably ATP, coenzyme A and/or carboxylate such as oxalate and formate is added last. More preferably oxalate is added last.

For this, an enzyme such as thioesterase capable of hydrolyzing the non-natural metabolite aromatic α-hydroxyacyl-CoA thioester in particular mandelic acid and its derivatives, and it particular capable of selectively hydrolyzing said compound would be necessary since the acyl-CoA (**2**) as well as the α-hydroxyacyl-CoA thioester encompass a thioester moiety.

Surprisingly, it could be found that YciA from *E. coli* has shown a relatively high α-hydroxyacyl-CoA in particular mandelyl-CoA thioesterase activity, low activity with formyl-CoA and no activity with oxalyl-CoA (example 11, figure 15).

A further aspect of the invention is directed to a composition comprising or consisting of a peptide oxalyl-CoA decarboxylase (OXC_{Me}) encoded by a gene from *Methylobacterium extorquens* or a mutant thereof, wherein in the mutant at least one tyrosine or serine is replaced independently of each other by alanine, phenylalanine, and/or glycine, and a thioesterase YciA encoded by a gene selected from *E. coli.* Preferably, at least one tyrosine or serine is replaced by alanine. It could be shown that the formation of mandelyl-CoA is higher using said mutant than the wild type OXC_{Me} (example 9, and figure 14A and 14B).

Further, an aspect of the present invention is directed to use of YciA for the hydrolysis of non-natural α-hydroxyacyl-CoA thioester in particular mandelyl-CoA or for the synthesis non-natural α-hydroxy carboxylic acids, or salts thereof in particular mandelic acid and its derivatives.

Another aspect of the present invention is related to use of a composition comprising or consisting of a peptide oxalyl-CoA decarboxylase (OXC_{Me}) encoded by a gene from *Methylobacterium extorquens* or a mutant thereof, wherein in the mutant at least one tyrosine or serine is replaced by an amino acid selected independently of each other from the group comprising or consisting of alanine, phenylalanine and glycine and a thioesterase YciA encoded by a gene selected from *E*. *coli* for the preparation of an α-hydroxy carboxylic acid, or its salt preferably starting from oxalyl-CoA and an aldehyde. Preferably, at least one tyrosine or serine is replaced by alanine.

A preferred embodiment of the invention is directed to a method for preparation of a compound of the formula (**II**) 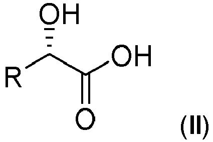 comprising:
**A**) providing a compound R-CHO **(1)** and an oxalyl-CoA (**2'**) 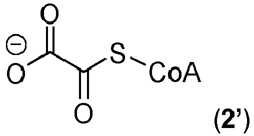 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (**II**) cation to obtain the *(S)*-α-hydroxyacyl-CoA thioester (**3**) 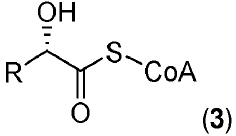 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 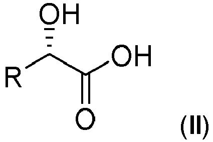 wherein the hydrolase is acyl-CoA thioester hydrolase YciA (**4**) encoded by a gene selected from *E. coli;* and
   wherein steps A), B) and C) are carried out as cascade reaction in one-pot system.

In the methods according to invention as described herein, the acyl-CoA (**2**) can be prepared by the following step A'):
**A')** reacting a carboxylate (**2a**) with a free coenzyme A (**2b**) by acyl-CoA synthetase (**2c**) in the presence of ATP and a magnesium (II) cation (**2e**) to produce an acyl-CoA (**2**).

The carboxylate (**2a**) can be an oxalate or a formate. As a source for the carboxylate the protonated form of the carboxylate can be used if the carboxylate is generated in solution. In case of the oxalate it is possible to use an oxalic acid (H₂C₂O₄) or a hydrogen oxalate (HC₂O₄⁻) as a source for the oxalate. For the formate, formic acid could be used as a source for the formate.

Thus, in the methods according to invention as described herein, the acyl-CoA (**2**) can be prepared by the following step A'):
**A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**) or reacting a formate (HCOO⁻) (**2a**") with a free coenzyme A (**2b**) by formyl-CoA synthetase (**2c"**) optional in the presence of ATP and magnesium(II) cation to produce formyl-CoA (**2"**).

In the methods according to invention as described herein, the oxalyl-CoA (**2'**) or the formyl-CoA (**2"**) can be prepared by the following step A'):
**A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**) or reacting a formate (HCOO⁻) (**2a"**) with a free coenzyme A (**2b**) by formyl-CoA synthetase (**2c"**) to produce formyl-CoA (**2"**).

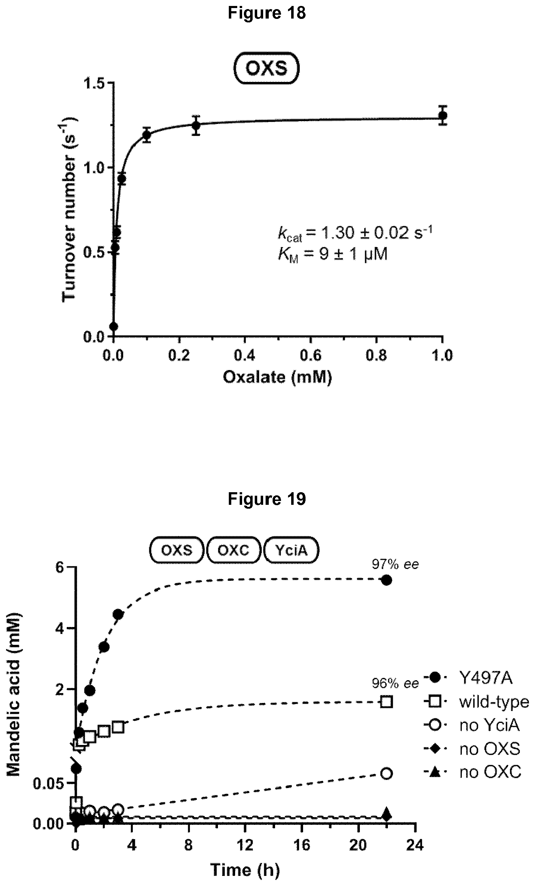

The steady-state kinetic parameters are shown in figure 18.

Thus, the step A') is preferably included in the method according to the invention as described herein.

The term "acyl-CoA synthetase" refers to an enzyme capable of catalyzing the formation of acyl-CoA (**2**) starting from a carboxylate (**2a**). This includes oxalyl-CoA synthetase, acetyl-CoA synthetase and formyl-CoA synthetase.

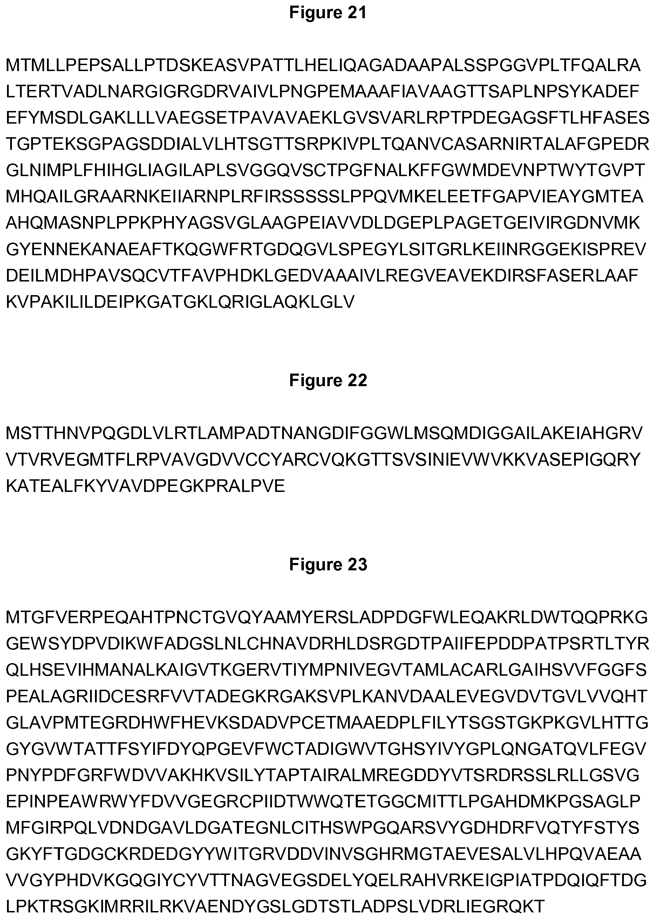

The oxalyl-CoA synthetase is preferably encoded by a gene selected from *Methylobacterium extorquens* preferably having the sequence SEQ ID NO. 5 (figure 21).

Therefore, another aspect of the invention is related to the use of an oxalyl-CoA synthetase encoded by a gene selected from *Methylobacterium extorquens* preferably having the sequence SEQ ID NO. 5 (figure 21)) for the synthesis of an α-hydroxycarboxylic acid, salts thereof or an α-hydroxyacyl-CoA thioester.

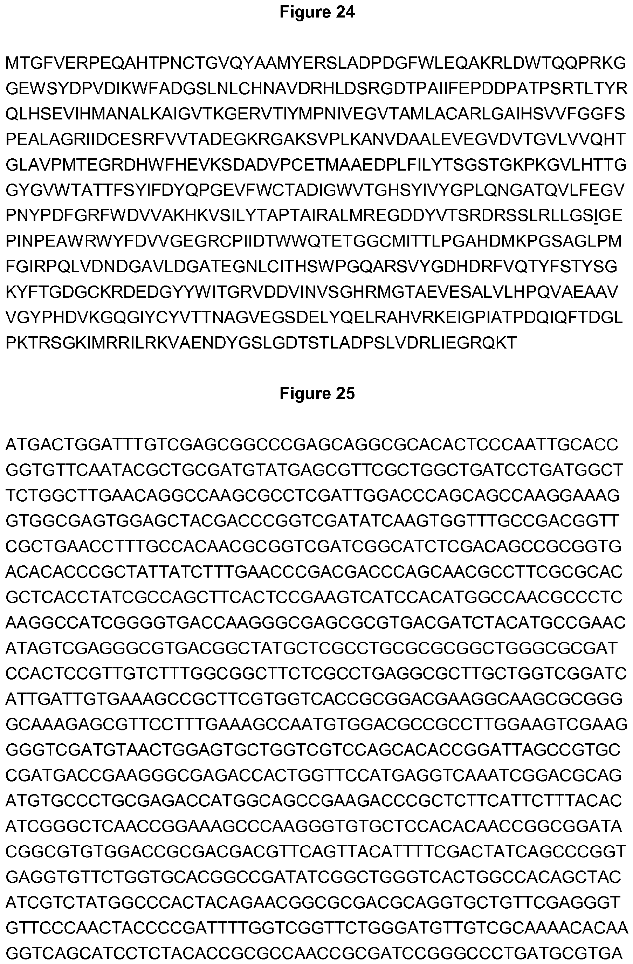

As a formyl-CoA synthetase, an acyl-CoA-synthetase can be used preferably encoded by a gene selected from *Erythrobacter* sp. having the sequence SEQ ID NO. 18 (figure 23) or mutants thereof can be used. More preferably, the formyl-CoA synthetase is an acyl-CoA synthetase is a mutant of the formyl-CoA synthetase encoded by a gene selected from *Erythrobacter* sp. Still more preferably, the formyl-CoA synthetase is an acyl-CoA synthetase is a mutant of the formyl-CoA synthetase encoded by a gene selected from *Erythrobacter* sp., wherein at least one amino acid is replaced by another proteinogenic amino acid. Still more preferably, the formyl-CoA synthetase is an acyl-CoA synthetase is a mutant of the formyl-CoA synthetase encoded by a gene selected from *Erythrobacter* sp., wherein at position 379 valine is replaced by a proteinogenic amino acid. Most preferably, the formyl-CoA synthetase is an acyl-CoA synthetase is a mutant of the formyl-CoA synthetase encoded by a gene selected from *Erythrobacter* sp. having the sequence SEQ ID NO. 19 (figure 24), i.e. most preferably, the formyl-CoA synthetase is an acyl-CoA synthetase is a mutant of the formyl-CoA synthetase encoded by a gene selected from *Erythrobacter* sp., wherein at position 379 valine is replaced by isoleucine.

Thus, another aspect of the invention is directed to a mutant of the acyl-CoA synthetase encoded by a gene selected from *Erythrobacter* sp. having the sequence SEQ ID NO. 19. A further aspect is related to the use of a mutant of the acyl-CoA synthetase encoded by a gene selected from *Erythrobacter* sp. having the sequence SEQ ID NO. 19 as an oxalyl-CoA synthetase.

An embodiment according to the invention is related to a method for the preparation of α-hydroxyacyl compound of the formula (I) 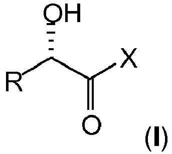 comprising:
**A**) providing a compound R-CHO (**1**) and an acyl-CoA (**2**) 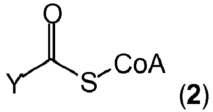
   wherein Y represents -H, or -COO⁻,
   in a buffer solution with a pH-value in the range of 5.0 to 7.5 and optionally together with at least one co-solvent, and wherein acyl-CoA (**2**) can be prepared by the following step A'):
      **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**) or reacting a formate (HCOO⁻) (**2a"**) with a free coenzyme A (2b) by formyl-CoA synthetase to produce formyl-CoA (**2"**);
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 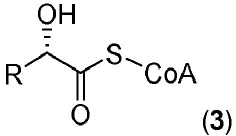 wherein the oxalyl-CoA decarboxylase (**3a**) is a peptide encoded by a gene selected from *Methylobacterium extorquens* or a mutant thereof; and optionally comprising the additional step **C**)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 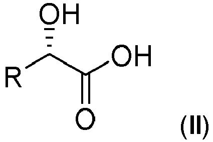 wherein the hydrolase is acyl-CoA thioester hydrolase YciA (**4**) encoded by a gene selected from *E. coli.*

An embodiment of the invention is directed to a method for preparation of a compound of the formula (**I**) 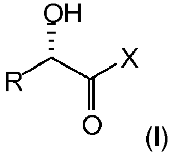 comprising:
**A**) providing a compound R-CHO (**1**) and an oxalyl-CoA (**2'**) 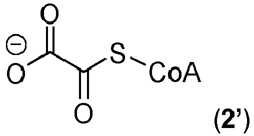 in a buffer solution with a pH-value in the range of 5.0 to 7.5, optionally together with at least one co-solvent, wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 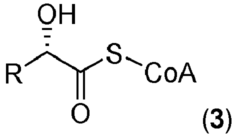 wherein the oxalyl-CoA decarboxylase (**3a**) is a peptide encoded by a gene selected from *Methylobacterium extorquens* or a mutant thereof; and optionally comprising the additional step **C**)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 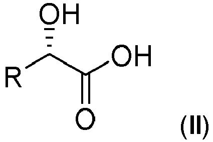 wherein the hydrolase is acyl-CoA thioester hydrolase YciA (**4**) encoded by a gene selected from *E. coli.*

A preferred embodiment of the invention is related to a method for preparation of a compound of the formula (**II**) 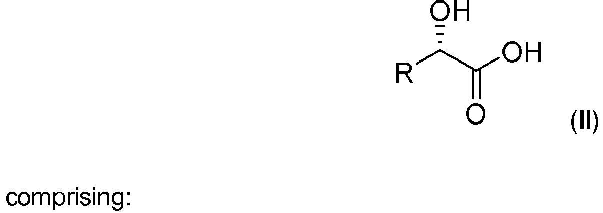
**A**) providing a compound R-CHO **(1)** and an oxalyl-CoA (**2'**) 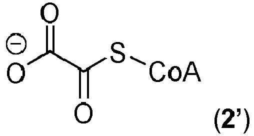 in a buffer solution with a pH-value in the range of 5.0 to 7.5 optionally together with at least one co-solvent, wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 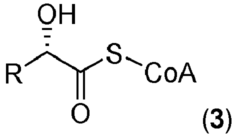 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoAoxalate CoA-transferase and/or α-hydroxyacyl-CoA:formate CoA-transferase to obtain the compound of the formula (**II**) or a salt thereof 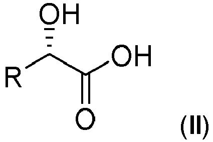 wherein the hydrolase is acyl-CoA thioester hydrolase YciA (**4**) encoded by a gene selected from *E. coli.*

A more preferred embodiment of the invention is directed to a method for preparation of a compound of the formula (**II**) 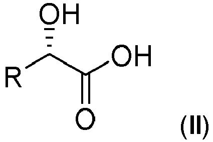 comprising:
**A**) providing a compound R-CHO (**1**) and an oxalyl-CoA (**2'**) 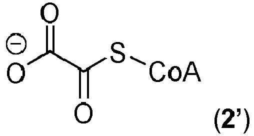 in a buffer solution with a pH-value in the range of 5.0 to 7.5 optionally at least one co-solvent, wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the *(S)*-α-hydroxyacyl-CoA thioesteer (**3**) 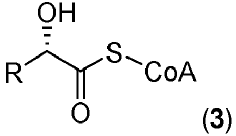 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 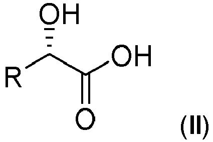 wherein the hydrolase is acyl-CoA thioester hydrolase YciA (**4**) encoded by a gene selected from *E. coli;* and
   wherein steps A), B) and C) are carried out as cascade reaction in one-pot system.

In general, the compounds generated in step A') or B) can be isolated, and used in step C). In order to increase the efficiency of the method, the steps **A'**), **A**), and **B**) or **A'**), **A**), **B**), and **C**) of a method according to the invention can be carried out as cascade reaction in one-pot system. Preferably, **A'**), **A**), **B**), and **C**) of a method according to the invention can be carried out as cascade reaction in one-pot system.

An embodiment of the present invention is directed to a method for preparation of a compound of the formula (I) 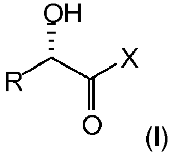 comprising:
**A**) providing a compound R-CHO (**1**) and an acyl-CoA (**2**) 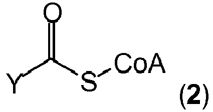
   wherein Y represents -H, or -COO⁻,
   in a buffer solution with a pH-value in the range of 5.0 to 7.5, optionally together with least one co-solvent, and wherein acyl-CoA (**2**) can be prepared by the following step A'):
      **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**) or reacting a formate (HCOO⁻) (**2a"**) with a free coenzyme A (2b) by formyl-CoA synthetase to produce formyl-CoA (**2"**);
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 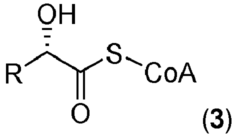 wherein the oxalyl-CoA decarboxylase (**3a**) is a peptide encoded by a gene selected from *Methylobacterium extorquens* or a mutant thereof; and optionally comprising the additional step **C**)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 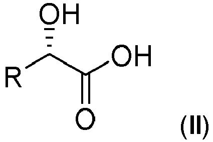 wherein the hydrolase is acyl-CoA thioester hydrolase YciA (**4**) encoded by a gene selected from *E. coli;* and
   wherein steps **A'**), **A**), and **B**), or steps **A'**), **A**), and **B**), and **C**) are carried out as cascade reaction in one-pot system.

An embodiment of the invention is directed to a method for preparation of a compound of the formula (**I**) 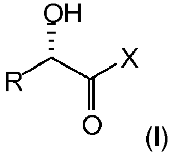 comprising:
**A**) providing a compound R-CHO (**1**) and an oxalyl-CoA (2') 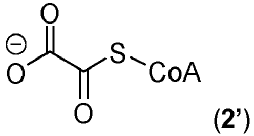 in a buffer solution with a pH-value in the range of 5.0 to 7.5, optionally together with at least one co-solvent, wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (1) and oxalyl-CoA (**2'**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 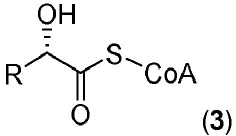 wherein the oxalyl-CoA decarboxylase (**3a**) is a peptide encoded by a gene selected from *Methylobacterium extorquens* or a mutant thereof; and optionally comprising the additional step C)
C) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 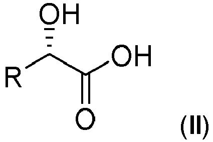 wherein the hydrolase is acyl-CoA thioester hydrolase YciA (**4**) encoded by a gene selected from *E. coli*; and
   wherein steps **A'**), **A**), and **B**), or steps **A'**), **A**), and **B**), and **C**) are carried out as cascade reaction in one-pot system.

A preferred embodiment of the invention is related to a method for preparation of a compound of the formula (**II**) 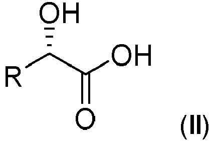 comprising:
**A**) providing a compound R-CHO (**1**) and an oxalyl-CoA (**2'**) 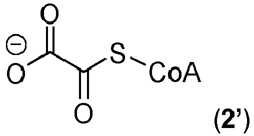 in a buffer solution with a pH-value in the range of 5.0 to 7.5 optionally together with at least one co-solvent, wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 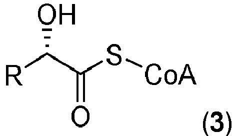 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoAoxalate CoA-transferase and/or α-hydroxyacyl-CoA:formate CoA-transferase to obtain the compound of the formula (**II**) or a salt thereof 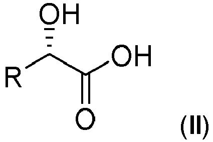 wherein the hydrolase is acyl-CoA thioester hydrolase YciA (**4**) encoded by a gene selected from *E. coli;* and
   wherein steps **A'**), **A**), **B**), and **C**) are carried out as cascade reaction in one-pot system.

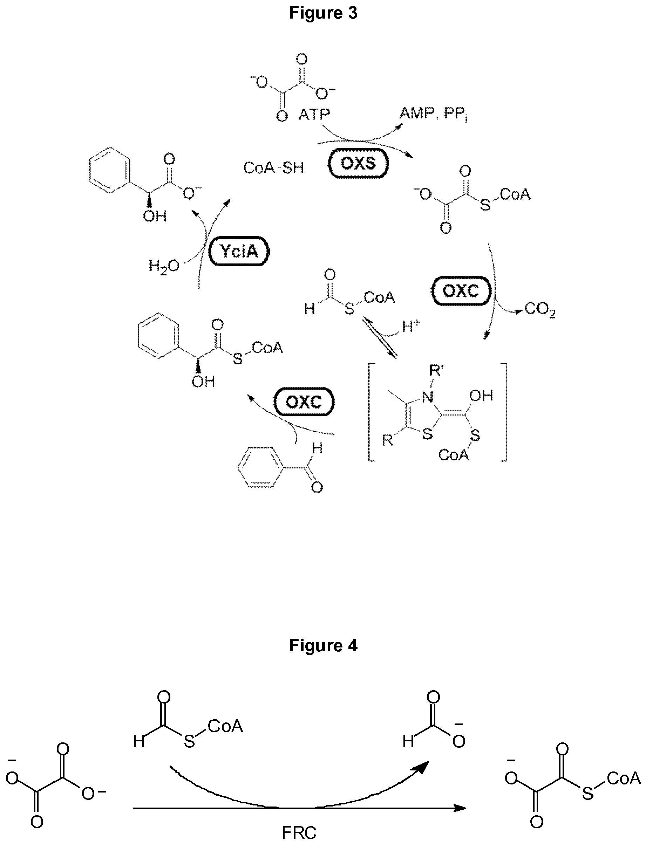

Since the coenzyme A is cleaved during the hydrolysis of the α-hydroxyacyl-CoA thioester, the free coenzyme A can be used in small amounts (catalytic amounts). Thus, the tree enzymes form a catalytic cycle (figure 3, 4 and 5). Therefore, in a method according to the invention, wherein the steps **A'**), **A**), **B**), and **C**) are carried out as cascade reaction in one-pot system, a ratio of R-CHO (1) and the free coenzyme A (**2b**) is 10 to 1000, preferably 20 to 500, and more preferably 40 to 250.

In order to receive a method in which the coenzyme A can be used in small amounts (catalytic amounts) or in other words a catalytic cycle, an enzyme composition has to be provided which is adapted to the various reaction demands such as selectivity, activity or stability of the enzyme, the starting material or the product.

Another aspect of the invention is thus directed to a composition comprising or consisting of a peptide OXC_{Me} encoded by a gene from *Methylobacterium extorquens* or a mutant thereof, wherein in the mutant at least one tyrosine or serine is replaced by alanine, phenylalanine, and/or glycine, and a thioesterase YciA encoded by a gene selected from *E. coli*, and an acyl-CoA synthetase in particular oxalyl-CoA synthetase. Preferably, tyrosine or serine is replaced by alanine, and more preferably, the at least one tyrosine or serine is replaced by alanine.

A further aspect of the present invention is related to use of a composition comprising or consisting of a peptide OXC_{Me} encoded by a gene from *Methylobacterium extorquens* or its mutant, wherein in the mutant at least one tyrosine or serine is replaced by alanine, phenylalanine, and/or glycine, and a thioesterase YciA encoded by a gene selected from *E*. *coli*, and an acyl-CoA synthetase in particular oxalyl-CoA synthetase for the preparation of an α-hydroxy carboxylic acid or salts thereof in particular starting from an oxalate, hydrogen oxalate, or oxalic acid and an aldehyde. Preferably, at least one tyrosine or serine is replaced by alanine.

An embodiment according to the invention is related to a method for the preparation of α-hydroxyacylcompound of the formula (**I**) 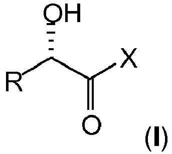 comprising:
**A**) providing a compound R-CHO (**1**) and an acyl-CoA (**2**) 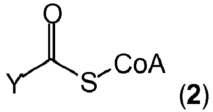
   wherein Y represents -H, or -COO⁻,
   in a buffer solution with a pH-value in the range of 5.0 to 7.5 optionally together with at least one co-solvent, and wherein acyl-CoA (**2**) can be prepared by the following step A'):
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**) or reacting a formate (HCOO⁻) (**2a"**) with a free coenzyme A (**2b**) by formyl-CoA synthetase to produce formyl-CoA (**2"**);
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 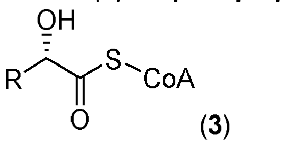 wherein the oxalyl-CoA decarboxylase (**3a**) is a peptide encoded by a gene selected from *Methylobacterium extorquens* or a mutant thereof; or additionally comprising the step **C**)
**C**) cleaving the coenzyme A moiety of said *(S)*-α-hydroxyacyl-CoA thioester (3) by a hydrolase, α-hydroxyacyl-CoA:oxalate CoA-transferase and/or α-hydroxyacyl-CoA:formate CoA-transferase to obtain the compound of the formula (**II**) or a salt thereof 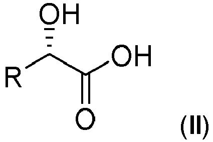 wherein the hydrolase is acyl-CoA thioester hydrolase YciA (**4**) encoded by a gene selected from *E. coli*; ; and
   wherein the steps **A'**), **A**), and **B**), and **C**) are carried out as cascade reaction in one-pot system, and wherein a ratio of R-CHO (**1**) and the free coenzyme A (**2b**) is 10 to 1000.

An embodiment of the invention is directed to a method for preparation of a compound of the formula (I) 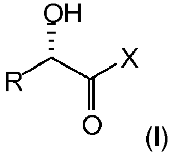 comprising:
**A**) providing a compound R-CHO (**1**) and an oxalyl-CoA (**2'**) 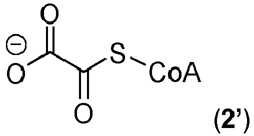 in a buffer solution with a pH-value in the rangein the range of 5.0 to 7.5 optionally together with at least one co-solvent, wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain *(S)*-α-hydroxyacyl-CoA thioester (**3**) 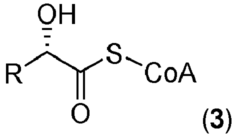 wherein the oxalyl-CoA decarboxylase (**3a**) is a peptide encoded by a gene selected from *Methylobacterium extorquens* or a mutant thereof; or additionally comprising the step **C**)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (I**I)** or a salt thereof 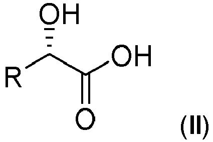 wherein the hydrolase is acyl-CoA thioester hydrolase YciA (**4**) encoded by a gene selected from *E. coli*; and
   wherein steps **A'**), **A**), **B**), and **C**) are carried out as cascade reaction in one-pot system, and wherein a ratio of R-CHO (1) and the free coenzyme A (**2b**) is 10 to 1000.

A preferred embodiment of the invention is related to a method for preparation of a compound of the formula (**II**) 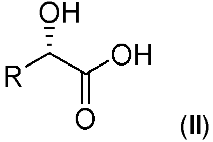 comprising:
**A**) providing a compound R-CHO (**1**) and an oxalyl-CoA (**2'**) 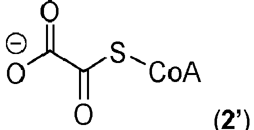 in a buffer solution with a pH-value in the range of 5.0 to 7.5 optionally together with at least one co-solvent, wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 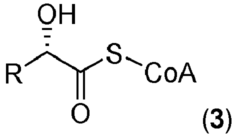 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 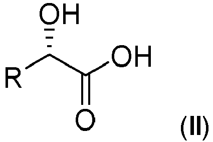 wherein the hydrolase is acyl-CoA thioester hydrolase YciA (**4**) encoded by a gene selected from *E. coli;* and
   wherein steps **A'**), **A**), **B**), and **C**) are carried out as cascade reaction in one-pot system, and wherein a ratio of R-CHO (**1**) and the free coenzyme A (**2b**) is 10 to 1000.

In a method according to the invention wherein the steps **A'**), **A**), **B**), and **C**) are carried out as cascade reaction in one-pot system a ratio of the carboxylate (**2a**) and the free coenzyme A (**2b**) is preferably 10 to 10,000.

An embodiment of the present invention is directed to a method for preparation of a compound of the formula (**I**) 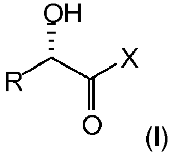 comprising:
**A**) providing a compound R-CHO (**1**) and an acyl-CoA (**2**) 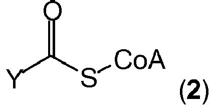
   wherein Y represents -H, or -COO⁻,
   in a buffer solution with a pH-value in the range of 5.0 to 7.5, optionally together with at least one co-solvent, and wherein acyl-CoA (**2**) can be prepared by the following step A'):
      **A'**) reacting an carboxylate (**2a**) with a free coenzyme A (**2b**) by acyl-CoA synthetase (**2c**) in the presence of ATP (**2e**) to produce an acyl-CoA (**2**);
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 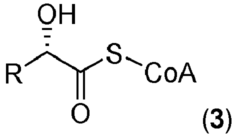 wherein the oxalyl-CoA decarboxylase (**3a**) is a peptide encoded by a gene selected from *Methylobacterium extorquens* or a mutant thereof; or additionally comprising the step **C**)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 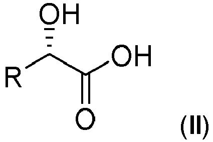 wherein the hydrolase is acyl-CoA thioester hydrolase YciA (**4**) encoded by a gene selected from *E. coli*; and
   wherein the steps **A'**), **A**), and **B**), and **C**) are carried out as cascade reaction in one-pot system, and wherein a ratio of the carboxylate (**2a**) and the free coenzyme A (**2b**) is preferably 10 to 10,000.

In a method according to the invention, wherein the steps **A'**), **A**), **B**), and **C**) are carried out as cascade reaction in one-pot system a ratio of the oxalate (**2a'**) and the free coenzyme A (**2b**) is preferably 10 to 10,000.

The reduction of the used amount of the free coenzyme A leads to a substrate economic procedure. Moreover, due to the reduction of the amount of starting material solvent can be reduced, and in total a higher amount of the starting material aldehyde can be transformed in an apparatus of the same size.

An embodiment of the invention is directed to a method for preparation of a compound of the formula (**I**) 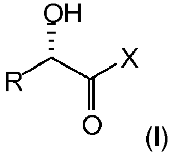 comprising:
**A**) providing a compound R-CHO (**1**) and an oxalyl-CoA (**2'**) 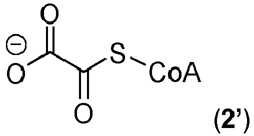 in a buffer solution with a pH-value in the range of 5.0 to 7.5 optionally together with at least one co-solvent wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 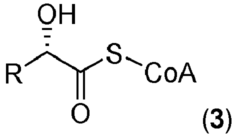 wherein the oxalyl-CoA decarboxylase (**3a**) is a peptide encoded by a gene selected from *Methylobacterium extorquens* or a mutant thereof; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 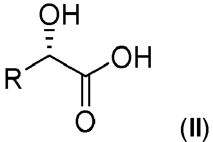 wherein the hydrolase is acyl-CoA thioester hydrolase YciA (**4**) encoded by a gene selected from *E. coli*; and
   wherein steps **A'**), **A**), **B**), and **C**) are carried out as cascade reaction in one-pot system, and wherein a ratio of the oxalate (**2a'**) and the free coenzyme A (**2b**) is 10 to 10,000.

A preferred embodiment of the invention is related to a method for preparation of a compound of the formula (**II**) 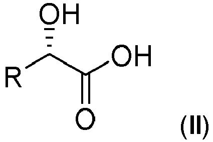 comprising:
**A**) providing a compound R-CHO (**1**) and an oxalyl-CoA (**2'**) 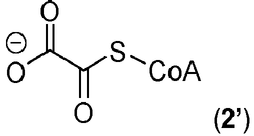 in a buffer solution with a pH-value in the range of 5.0 to 7.5 optionally together with at least one co-solvent, wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 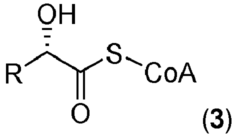 wherein the oxalyl-CoA decarboxylase (**3a**) is a peptide encoded by a gene selected from *Methylobacterium extorquens* or a mutant thereof; and
**C**) cleaving the coenzyme moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 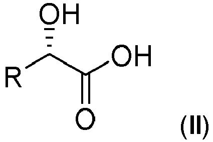 wherein the hydrolase is acyl-CoA thioester hydrolase YciA (**4**) encoded by a gene selected from *E. coli*; and
   wherein steps **A'**), **A**), **B**), and **C**) are carried out as cascade reaction in one-pot system, and wherein a ratio of the oxalate (**2a'**) and the free coenzyme A (**2b**) is 10 to 10,000.

In step A) of a method according to the invention, a concentration of R-CHO (**1**) can be in the range of 5 mM to 50 mM. Preferably the concentration is in the range of 10 mM to 45 mM, more preferably 15 mM to 40 mM, more preferably 20 mM to 35 mM, more preferably 20 mM to 30 mM, more preferably 22.5 mM to 30 mM, and most preferably 25 mM to 30 mM.

An embodiment of the present invention is directed to a method for preparation of a compound of the formula (I) 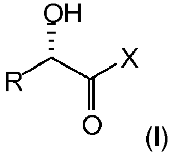 comprising:
**A**) providing a compound R-CHO (**1**) and an acyl-CoA (**2**) 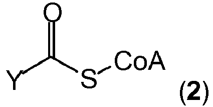
   wherein Y represents -H, or -COO⁻,
   in a buffer solution with a pH-value in the range of 5.0 to 7.5 optionally together with at least one co-solvent, wherein a concentration of R-CHO (1) is in the range of 5 mM to 50 mM (25 mM);
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 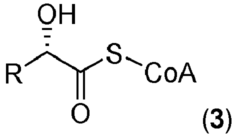 wherein the oxalyl-CoA decarboxylase (**3a**) is a peptide encoded by a gene selected from *Methylobacterium extorquens* or a mutant thereof; and optionally comprising the additional step c)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 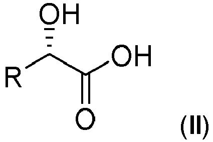 wherein the hydrolase is acyl-CoA thioester hydrolase YciA (**4**) encoded by a gene selected from *E. coli.*

An embodiment of the invention is directed to a method for preparation of a compound of the formula (**I**) 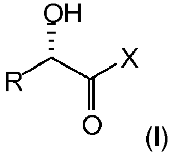 comprising:
**A**) providing a compound R-CHO **(1)** and an oxalyl-CoA (**2'**) 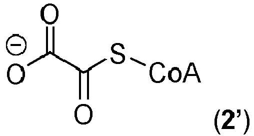 in a buffer solution with a pH-value in the range of 5.0 to 7.5 optionally together with at least one co-solvent, wherein a concentration of R-CHO (**1**) is in the range of 5 mM to 50 mM (25 mM);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 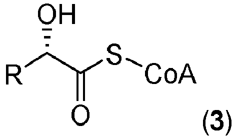 wherein the oxalyl-CoA decarboxylase (**3a**) is a peptide encoded by a gene selected from *Methylobacterium extorquens* or a mutant thereof, ; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 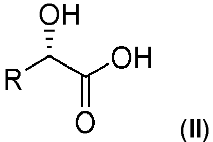 wherein the hydrolase is acyl-CoA thioester hydrolase YciA (**4**) encoded by a gene selected from *E. coli.*

Another embodiment of the present invention is related to a method for preparation of a compound of the formula (II) 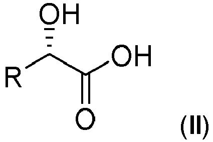 comprising:
**A**) providing a compound R-CHO **(1)** and an oxalyl-CoA (**2'**) 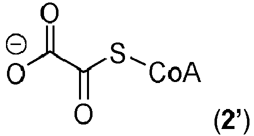 in a buffer solution with a pH-value in the range of 5.0 to 7.5 optionally together with at least one co-solvent, wherein a concentration of R-CHO (1) is in the range of 5 mM to 50 mM (25 mM);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 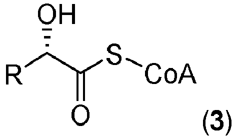 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 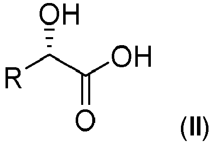 wherein the hydrolase is acyl-CoA thioester hydrolase YciA (**4**) encoded by a gene selected from *E. coli.*

A preferred embodiment of the invention is related to a method for preparation of a compound of the formula (**II**) 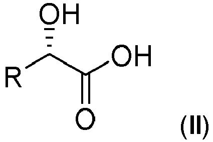 comprising:
**A**) providing a compound R-CHO (**1**) and an oxalyl-CoA (**2'**) 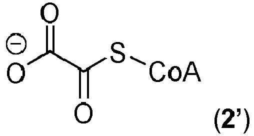 in a buffer solution with a pH-value in the range of 5.0 to 7.5 optionally together with at least one co-solvent, wherein a concentration of R-CHO (**1**) is in the range of 5 mM to 50 mM (25 mM), wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 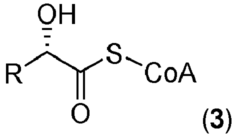 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 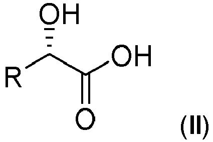 wherein the hydrolase is acyl-CoA thioester hydrolase YciA (**4**) encoded by a gene selected from *E. coli;* and
   wherein steps **A'**), **A**), **B**), and **C**) are carried out as cascade reaction in one-pot system, and wherein a ratio of R-CHO (**1**) and the free coenzyme A (**2b**) is 10 to 10,000.

In step **B**) of a method according to invention as described herein, a molar ratio of the thiamine diphosphate (**3b**) compared to R-CHO (**1**) can be from 0.001 to 0.01, preferably from 0.002 to 0.008, more preferably from 0.003 to 0.006 with a concentration in the range of 0.05 mM to 0.5 mM, and most preferably 0.1 mM to 0.25 mM.

An embodiment of the present invention is directed to a method for preparation of a compound of the formula (**I**) 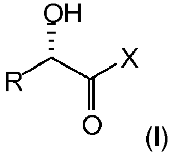 comprising:
**A**) providing a compound R-CHO (**1**) and an acyl-CoA (**2**) 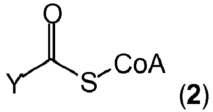
   wherein Y represents -H, or -COO⁻,
   in a buffer solution with a pH-value in the range of 5.0 to 7.5 optionally together with at least one co-solvent;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 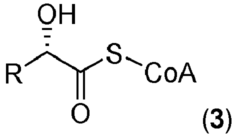 wherein the oxalyl-CoA decarboxylase (**3a**) is a peptide encoded by a gene selected from *Methylobacterium extorquens* or a mutant thereof, and wherein a molar ratio of the thiamine diphosphate (**3b**) compared to R-CHO (1) is 0.001 to 0.01 with a concentration in the range of 0.05 mM to 0.5 mM; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 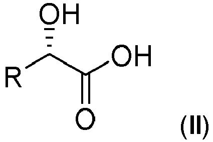 wherein the hydrolase is acyl-CoA thioester hydrolase YciA (**4**) encoded by a gene selected from *E. coli.*

An embodiment of the invention is directed to a method for preparation of a compound of the formula (**I**) 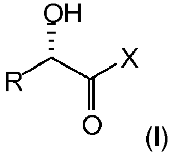 comprising:
**A**) providing a compound R-CHO (**1**) and an oxalyl-CoA (**2'**) 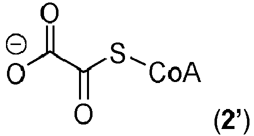 in a buffer solution with a pH-value in the range of 5.0 to 7.5, optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the *(S)*-α-hydroxyacyl-CoA thioester (**3**) 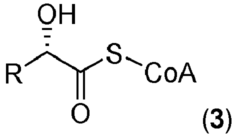 wherein the oxalyl-CoA decarboxylase (**3a**) is a peptide encoded by a gene selected from *Methylobacterium extorquens* or a mutant thereof, and wherein a molar ratio of the thiamine diphosphate (**3b**) compared to R-CHO (**1**) is 0.001 to 0.01 with a concentration in the range of 0.05 mM to 0.5 mM; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 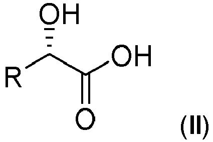 wherein the hydrolase is acyl-CoA thioester hydrolase YciA (**4**) encoded by a gene selected from *E. coli.*

Another embodiment of the present invention is related to a method for preparation of a compound of the formula (**II**) 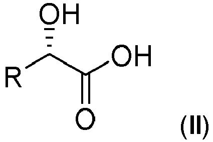 comprising:
**A**) providing a compound R-CHO (**1**) and an oxalyl-CoA (**2'**) 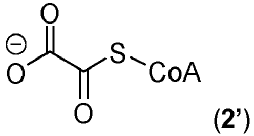 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain *(S)*-2-hydroxyacyl-CoA thioester (**3**) 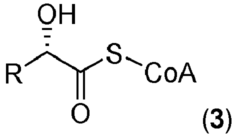 wherein the oxalyl-CoA decarboxylase (**3a**) is a peptide encoded by a gene selected from *Methylobacterium extorquens* or a mutant thereof, and wherein a molar ratio of the thiamine diphosphate (**3b**) compared to R-CHO (**1**) is 0.001 to 0.01 with a concentration in the range of 0.05 mM to 0.5 mM; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 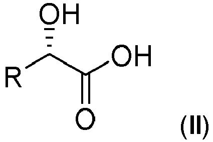 wherein the hydrolase is acyl-CoA thioester hydrolase YciA (**4**) encoded by a gene selected from *E. coli.*

A preferred embodiment of the invention is related to a method for preparation of a compound of the formula (**II**) 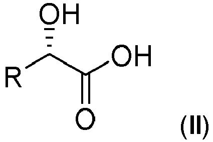 comprising:
**A**) providing a compound R-CHO (**1**) and an oxalyl-CoA (**2'**) 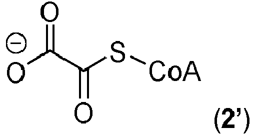 in a buffer solution with a pH-value in the range of 5.0 to 7.5 optionally together with at least one co-solvent, wherein a concentration of R-CHO (**1**) is in the range of 5 mM to 50 mM , wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 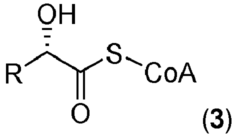 wherein the oxalyl-CoA decarboxylase (**3a**) is a peptide encoded by a gene selected from *Methylobacterium extorquens* or a mutant thereof, and wherein a molar ratio of the thiamine diphosphate (**3b**) compared to R-CHO (**1**) is 0.001 to 0.01 with a concentration in the range of 0.05 mM to 0.5 mM; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 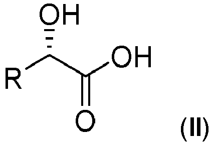 wherein the hydrolase is acyl-CoA thioester hydrolase YciA (**4**) encoded by a gene selected from *E. coli;* and
   wherein steps **A'**), **A**), **B**), and **C**) are carried out as cascade reaction in one-pot system, and wherein a ratio of R-CHO (**1**) and the free coenzyme A (**2b**) is 10 to 1000.

In step **A**) of a method according to the invention, the pH-value of the buffer solution is preferably in the range of 6.5 to 7.5, and more preferably in the range of 6.7 to 7.0.

In general, in step A) any buffer can be used to adjust the pH value, notably sodium or potassium phosphate buffer. The buffer solution can contain at least one compound selected from the group comprising or consisting of 2-[[1,3-dihydroxy-2-(hydroxymethyl)propan-2-yl]amino]ethanesulfonic acid (TES), 2-(N-morpholino)ethanesulfonic acid (MES), 2-[Bis(2-hydroxyethyl)amino]-2-(hydroxymethyl)propane-1,3-diol (Bis-Tris), 2-[(2-amino-2-oxoethyl)-(carboxymethyl)amino]acetic acid (ADA), 2-(carbamoylmethylamino)ethanesulfonic acid (ACES), 1,4-Piperazinediethanesulfonic acid (PIPES), 3-Morpholino-2-hydroxypropanesulfonic acid (MOPSO), 2,2'-(Propane-1,3-diyldiimino)bis[2-(hydroxymethyl)propane-1,3-diol] (BTP), N,N-Bis(2-hydroxyethyl)-2-aminoethanesulfonic acid (BES), 3-Morpholinopropane-1-sulfonic acid (MOPS), 2-[4-(2-hydroxyethyl)piperazin-1-yl]ethanesulfonic acid (HEPES), potassium phosphate, sodium phosphate, and imidazole, preferably 2-[[1,3-dihydroxy-2-(hydroxymethyl)propan-2-yl]amino]ethanesulfonic acid (TES).

The buffer can be prepared by use of a base such as lithium hydroxide, sodium hydroxide, potassium hydroxide, rubidium hydroxide, and/or caesium hydroxide. Preferably, potassium hydroxide is used.

In step **A**) of a method according to the invention, the buffer solution can comprise at least one compound selected from the group comprising or consisting of 2-[[1,3-dihydroxy-2-(hydroxymethyl)propan-2-yl]amino]ethanesulfonic acid, 2-(*N-*morpholino)ethanesulfonic acid (MES), 2-[Bis(2-hydroxyethyl)amino]-2-(hydroxymethyl)propane-1,3-diol (Bis-Tris), 2-[(2-amino-2-oxoethyl)-(carboxymethyl)amino]acetic acid (ADA), 2-(carbamoylmethylamino)ethanesulfonic acid (ACES), 1,4-Piperazinediethanesulfonic acid (PIPES), 3-Morpholino-2-hydroxypropanesulfonic acid (MOPSO), 2,2'-(Propane-1,3-diyldiimino)bis[2-(hydroxymethyl)propane-1,3-diol] (BTP), N,N-Bis(2-hydroxyethyl)-2-aminoethanesulfonic acid (BES), 3-Morpholinopropane-1-sulfonic acid (MOPS), 2-[4-(2-hydroxyethyl)piperazin-1-yl]ethanesulfonic acid (HEPES), potassium phosphate, sodium phosphate, and imidazole in the range of 0.25 wt% to 2.5 wt%, preferably 1 wt% to 2 wt%

Preferably, in step **A**) of a method according to the invention, the buffer solution can comprise at least one compound selected from the group comprising or consisting 2-[[1,3-dihydroxy-2-(hydroxymethyl)propan-2-yl]amino]ethanesulfonic acid (TES), 2-(*N-*morpholino)ethanesulfonic acid (MES), 2-[Bis(2-hydroxyethyl)amino]-2-(hydroxymethyl)propane-1,3-diol (Bis-Tris), 2-[(2-amino-2-oxoethyl)-(carboxymethyl)amino]acetic acid (ADA), 2-(carbamoylmethylamino)ethanesulfonic acid (ACES), 1,4-Piperazinediethanesulfonic acid (PIPES), 3-Morpholino-2-hydroxypropanesulfonic acid (MOPSO), 2,2'-(Propane-1,3-diyldiimino)bis[2-(hydroxymethyl)propane-1,3-diol] (BTP), N,N-Bis(2-hydroxyethyl)-2-aminoethanesulfonic acid (BES), 3-Morpholinopropane-1-sulfonic acid (MOPS), 2-[4-(2-hydroxyethyl)piperazin-1-yl]ethanesulfonic acid (HEPES), potassium phosphate, sodium phosphate, and imidazole in the range of 0.25 wt% to 2.5 wt%, preferably 1 wt% to 2 wt%

In step **A**) of a method according to the invention, the buffer solution can comprise 2-[[1,3-dihydroxy-2-(hydroxymethyl)propan-2-yl]amino]ethanesulfonic acid (TES) in the range of 0.25 wt% to 2.5 wt%, preferably 1 wt% to 2 wt% and the pH-value is in the range of 6.5 to 7.5, and preferably 6.7 to 7.0.

In step **A**) of a method according to the invention, the buffer solution can comprise 2-[[1,3-dihydroxy-2-(hydroxymethyl)propan-2-yl]amino]ethanesulfonic acid (TES) in the range of 0.25 wt% to 2.5 wt%, preferably 1 wt% to 2 wt% and the pH-value is in the range of 5.0 to 7.5, preferably in the range of 6.5 to 7.5, and more preferably in range of 6.7 to 7.0.

An embodiment of the present invention is directed to a method for preparation of a compound of the formula (**I**) 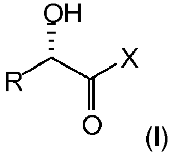 comprising:
**A**) providing a compound R-CHO (**1**) and an acyl-CoA (**2**) 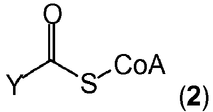 wherein Y represents -H, or -COO⁻,
   in a buffer solution with a pH-value in the range of 5.0 to 7.5 optionally together with at least one co-solvent, and wherein the buffer solution comprises at least one compound selected from the group comprising or consisting of 2-[[1,3-dihydroxy-2-(hydroxymethyl)propan-2-yl]amino]ethanesulfonic acid (TES), 2-(*N*-morpholino)ethanesulfonic acid (MES), 2-[Bis(2-hydroxyethyl)amino]-2-(hydroxymethyl)propane-1,3-diol (Bis-Tris), 2-[(2-amino-2-oxoethyl)-(carboxymethyl)amino]acetic acid (ADA), 2-(carbamoylmethylamino)ethanesulfonic acid (ACES), 1,4-Piperazinediethanesulfonic acid (PIPES), 3-Morpholino-2-hydroxypropanesulfonic acid (MOPSO), 2,2'-(Propane-1,3-diyldiimino)bis[2-(hydroxymethyl)propane-1,3-diol] (BTP), N,N-Bis(2-hydroxyethyl)-2-aminoethanesulfonic acid (BES), 3-Morpholinopropane-1-sulfonic acid (MOPS), 2-[4-(2-hydroxyethyl)piperazin-1-yl]ethanesulfonic acid (HEPES), potassium phosphate, sodium phosphate, and imidazole;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the (S)-α-hydroxyacyl-CoA thioester (**3**) 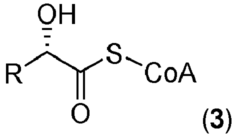 wherein the oxalyl-CoA decarboxylase (**3a**) is a peptide encoded by a gene selected from *Methylobacterium extorquens* or a mutant thereof; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 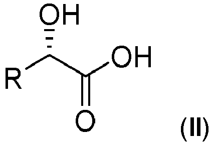 wherein the hydrolase is acyl-CoA thioester hydrolase YciA (**4**) encoded by a gene selected from *E. coli.*

A further embodiment of the present invention is directed to a method for preparation of a compound of the formula (**I**) 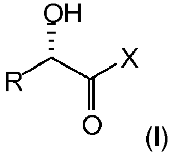 comprising:
**A**) providing a compound R-CHO (**1**) and an acyl-CoA (**2**) 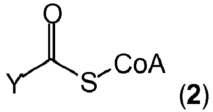
   wherein Y represents -H, or -COO⁻,
   in a buffer solution with a pH-value in the range of 6.5 to 7.5 optionally together with at least one co-solvent, and wherein the buffer solution comprises at least one compound selected from the group comprising or consisting of 2-[[1,3-dihydroxy-2-(hydroxymethyl)propan-2-yl]amino]ethanesulfonic acid (TES), 2-(*N*-morpholino)ethanesulfonic acid (MES), 2-[Bis(2-hydroxyethyl)amino]-2-(hydroxymethyl)propane-1,3-diol (Bis-Tris), 2-[(2-amino-2-oxoethyl)-(carboxymethyl)amino]acetic acid (ADA), 2-(carbamoylmethylamino)ethanesulfonic acid (ACES), 1,4-Piperazinediethanesulfonic acid (PIPES), 3-Morpholino-2-hydroxypropanesulfonic acid (MOPSO), 2,2'-(Propane-1,3-diyldiimino)bis[2-(hydroxymethyl)propane-1,3-diol] (BTP), N,N-Bis(2-hydroxyethyl)-2-aminoethanesulfonic acid (BES), 3-Morpholinopropane-1-sulfonic acid (MOPS), 2-[4-(2-hydroxyethyl)piperazin-1-yl]ethanesulfonic acid (HEPES), potassium phosphate, sodium phosphate, and imidazole in the range of 0.25 wt% to 2.5 wt%;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the (S)-α-hydroxyacyl-CoA thioester (**3**) 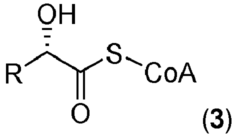 wherein the oxalyl-CoA decarboxylase (**3a**) is a peptide encoded by a gene selected from *Methylobacterium extorquens* or a mutant thereof; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 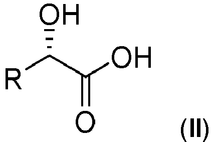 wherein the hydrolase is acyl-CoA thioester hydrolase YciA (**4**) encoded by a gene selected from *E. coli.*

An embodiment of the invention is directed to a method for preparation of a compound of the formula (**I**) 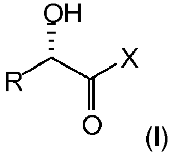 comprising:
**A**) providing a compound R-CHO (**1**) and an oxalyl-CoA (**2'**) 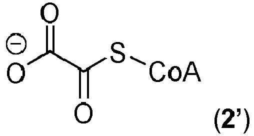 in a buffer solution with a pH-value in the range of 6.5 to 7.5 optionally together with at least one co-solvent, and wherein the buffer solution comprises at least one compound selected from the group comprising or consisting of 2-[[1,3-dihydroxy-2-(hydroxymethyl)propan-2-yl]amino]ethanesulfonic acid (TES), 2-(*N*-morpholino)ethanesulfonic acid (MES), 2-[Bis(2-hydroxyethyl)amino]-2-(hydroxymethyl)propane-1,3-diol (Bis-Tris), 2-[(2-amino-2-oxoethyl)-(carboxymethyl)amino]acetic acid (ADA), 2-(carbamoylmethylamino)ethanesulfonic acid (ACES), 1,4-Piperazinediethanesulfonic acid (PIPES), 3-Morpholino-2-hydroxypropanesulfonic acid (MOPSO), 2,2'-(Propane-1,3-diyldiimino)bis[2-(hydroxymethyl)propane-1,3-diol] (BTP), N,N-Bis(2-hydroxyethyl)-2-aminoethanesulfonic acid (BES), 3-Morpholinopropane-1-sulfonic acid (MOPS), 2-[4-(2-hydroxyethyl)piperazin-1-yl]ethanesulfonic acid (HEPES), potassium phosphate, sodium phosphate, and imidazole in the range of 0.25 wt% to 2.5 wt%;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 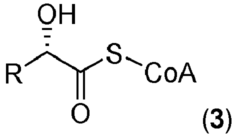 wherein the oxalyl-CoA decarboxylase (**3a**) is a peptide encoded by a gene selected from *Methylobacterium extorquens* or a mutant thereof and optionally comprising the additional step C)
**C**) cleaving coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 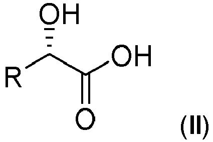 wherein the hydrolase is acyl-CoA thioester hydrolase YciA (**4**) encoded by a gene selected from *E. coli.*

Another embodiment of the present invention is related to a method for preparation of a compound of the formula (**II**) 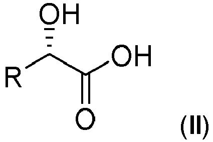 comprising:
**A**) providing a compound R-CHO (**1**) and an oxalyl-CoA (**2'**) 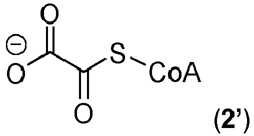 in a buffer solution with a pH-value in the range of 6.5 to 7.5 optionally together with at least one co-solvent, and wherein the buffer solution comprises at least one compound selected from the group comprising or consisting of 2-[[1,3-dihydroxy-2-(hydroxymethyl)propan-2-yl]amino]ethanesulfonic acid (TES), 2-(*N*-morpholino)ethanesulfonic acid (MES), 2-[Bis(2-hydroxyethyl)amino]-2-(hydroxymethyl)propane-1,3-diol (Bis-Tris), 2-[(2-amino-2-oxoethyl)-(carboxymethyl)amino]acetic acid (ADA), 2-(carbamoylmethylamino)ethanesulfonic acid (ACES), 1,4-Piperazinediethanesulfonic acid (PIPES), 3-Morpholino-2-hydroxypropanesulfonic acid (MOPSO), 2,2'-(Propane-1,3-diyldiimino)bis[2-(hydroxymethyl)propane-1,3-diol] (BTP), N,N-Bis(2-hydroxyethyl)-2-aminoethanesulfonic acid (BES), 3-Morpholinopropane-1-sulfonic acid (MOPS), 2-[4-(2-hydroxyethyl)piperazin-1-yl]ethanesulfonic acid (HEPES), potassium phosphate, sodium phosphate, and imidazole in the range of 0.25 wt% to 2.5 wt%;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 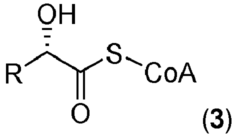 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 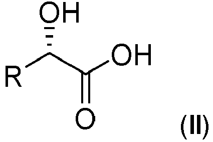 wherein the hydrolase is acyl-CoA thioester hydrolase YciA (**4**) encoded by a gene selected from *E. coli.*

A more preferred embodiment of the invention is related to a method for preparation of a compound of the formula (**II**) 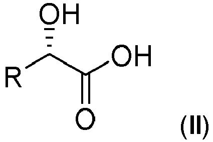 comprising:
**A**) providing a compound R-CHO (**1**) and an oxalyl-CoA (**2'**) 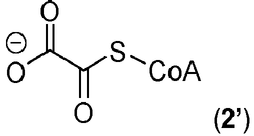 in a buffer solution with a pH-value in the range of 6.5 to 7.5 optionally together with at least one co-solvent, wherein a concentration of R-CHO (**1**) is in the range of 5 mM to 50 mM, wherein the buffer solution comprises at least one compound selected from the group comprising or consisting of 2-[[1,3-dihydroxy-2-(hydroxymethyl)propan-2-yl]amino]ethanesulfonic acid (TES), 2-(*N*-morpholino)ethanesulfonic acid (MES), 2-[Bis(2-hydroxyethyl)amino]-2-(hydroxymethyl)propane-1,3-diol (Bis-Tris), 2-[(2-amino-2-oxoethyl)-(carboxymethyl)amino]acetic acid (ADA), 2-(carbamoylmethylamino)ethanesulfonic acid (ACES), 1,4-Piperazinediethanesulfonic acid (PIPES), 3-Morpholino-2-hydroxypropanesulfonic acid (MOPSO), 2,2'-(Propane-1,3-diyldiimino)bis[2-(hydroxymethyl)propane-1,3-diol] (BTP), N,N-Bis(2-hydroxyethyl)-2-aminoethanesulfonic acid (BES), 3-Morpholinopropane-1-sulfonic acid (MOPS), 2-[4-(2-hydroxyethyl)piperazin-1-yl]ethanesulfonic acid (HEPES), potassium phosphate, sodium phosphate, and imidazole in the range of 0.25 wt% to 2.5 wt%, and wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 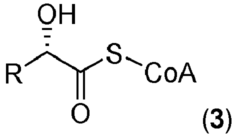 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 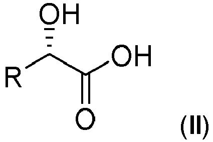 wherein the hydrolase is acyl-CoA thioester hydrolase YciA (**4**) encoded by a gene selected from *E. coli;* and
   wherein steps **A'**), **A**), **B**), and **C**) are carried out as cascade reaction in one-pot system, and wherein a ratio of R-CHO (**1**) and the free coenzyme A (**2b**) is 10 to 1000.

In step **B**) of a method according to the invention, a concentration of the oxalyl-CoA decarboxylase can be in the range of 1 to 25 µM (5 µM), preferably 5 to 20 and/or a concentration of the magnesium (II) cation is in the range of 2.5 mM to 20 mM, preferably 5 mM to 10 mM.

An embodiment of the present invention is directed to a method for preparation of a compound of the formula (**I**) 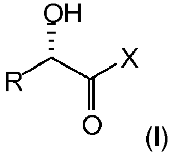 comprising:
**A**) providing a compound R-CHO (**1**) and an acyl-CoA (**2**) 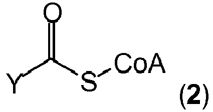
   wherein Y represents -H, or -COO⁻,
   in a buffer solution with a pH-value in the range of 5.0 to 7.5 optionally together with at least one co-solvent;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the (S)-α-hydroxyacyl-CoA thioester (**3**) 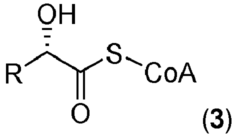 wherein the oxalyl-CoA decarboxylase (**3a**) is a peptide encoded by a gene selected from *Methylobacterium extorquens* or a mutant thereof, and wherein a concentration of the oxalyl-CoA decarboxylase is in the range of 1 to 25 µM and/or a concentration of the magnesium (II) cation is in the range of 2.5 mM to 20 mM; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 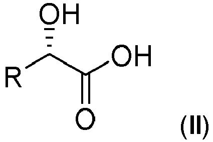 wherein the hydrolase is acyl-CoA thioester hydrolase YciA (**4**) encoded by a gene selected from *E. coli.*

An embodiment of the invention is directed to a method for preparation of a compound of the formula (**I**) 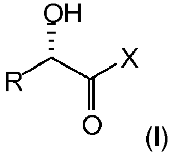 comprising:
**A)** providing a compound R-CHO (**1**) and an oxalyl-CoA (**2'**) 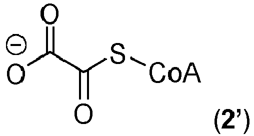 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 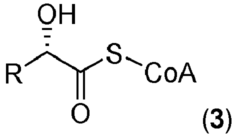 wherein the oxalyl-CoA decarboxylase (**3a**) is a peptide encoded by a gene selected from *Methylobacterium extorquens* or a mutant thereof, and wherein a concentration of the oxalyl-CoA decarboxylase is in the range of 1 to 25 µM and/or a concentration of the magnesium (II) cation is in the range of 2.5 mM to 20 mM and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 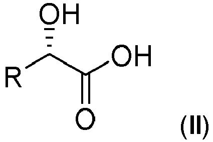 wherein the hydrolase is acyl-CoA thioester hydrolase YciA (**4**) encoded by a gene selected from *E. coli.*

Another embodiment of the present invention is related to a method for preparation of a compound of the formula (II) 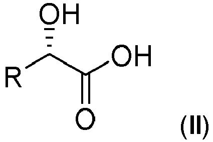 comprising:
**A**) providing a compound R-CHO (**1**) and an oxalyl-CoA (**2'**) 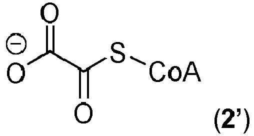 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 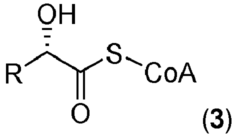 wherein the oxalyl-CoA decarboxylase (**3a**) is a peptide encoded by a gene selected from *Methylobacterium extorquens* or a mutant thereof, and wherein a concentration of the oxalyl-CoA decarboxylase is in the range of 1 to 25 µM and/or a concentration of the magnesium (II) cation is in the range of 2.5 mM to 20 mM; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 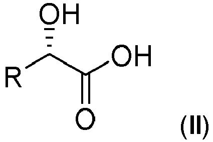 wherein the hydrolase is acyl-CoA thioester hydrolase YciA (**4**) encoded by a gene selected from *E. coli.*

A preferred embodiment of the invention is related to a method for preparation of a compound of the formula (**II**) 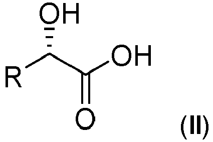 comprising:
**A)** providing a compound R-CHO (**1**) and an oxalyl-CoA (**2'**) 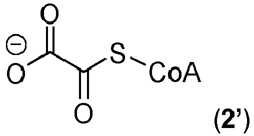 in a buffer solution with a pH-value in the range of 6.5 to 7.5 optionally together with at least one co-solvent, wherein a concentration of R-CHO (1) is in the range of 5 mM to 50 mM , wherein the buffer solution comprises 2-[[1,3-dihydroxy-2-(hydroxymethyl)propan-2-yl]amino]ethanesulfonic acid (TES) in the range of 0.25 wt% to 2.5 wt%, and wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 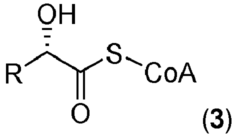 wherein the oxalyl-CoA decarboxylase (**3a**) is a peptide encoded by a gene selected from *Methylobacterium extorquens* or a mutant thereof, wherein a molar ratio of the thiamine diphosphate (**3b**) compared to R-CHO (**1**) is 0.001 to 0.01 with a concentration in the range of 0.05 mM to 0.5 mM, and wherein a concentration of the oxalyl-CoA decarboxylase is in the range of 1 to 25 µM and/or a concentration of the magnesium (II) cation is in the range of 2.5 mM to 20 mM; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 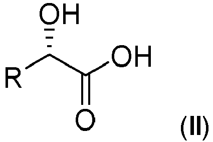 wherein the hydrolase is acyl-CoA thioester hydrolase YciA (**4**) encoded by a gene selected from *E. coli;* and
   wherein steps **A'**), **A**), **B**), and **C**) are carried out as cascade reaction in one-pot system, and wherein a ratio of R-CHO (**1**) and the free coenzyme A (**2b**) is 10 to 1000.

In step **B**), the mutant can be selected from a group comprising or consisting of OXC_{Me-Y497A} having the sequence SEQ ID NO. 2, OXC_{Me-S568A} having the sequence SEQ ID NO. 3 or OXC_{Me-Y497A-S568A} having the sequence SEQ ID NO. 4. Preferably, the used peptide is OXC_{Me-Y497A} having the sequence SEQ ID NO. 2 and/or OXC_{Me-S568A}, and most preferably OXC_{Me-Y497A} having the sequence SEQ ID NO. 2.

An embodiment of the invention is related to a method for the preparation of a-hydroxyacyl compound of the formula (**I**) 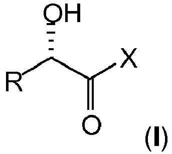 comprising:
**A**) providing a compound R-CHO (**1**) and an acyl-CoA (**2**) 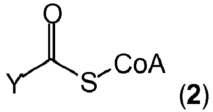
   wherein Y represents -H, or -COO⁻,
   in a buffer solution with a pH-value in the range of 5.0 to 7.5 optionally together with at least one co-solvent;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the (S)-α-hydroxyacyl-CoA thioester (**3**) 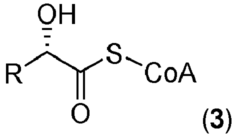 wherein the oxalyl-CoA decarboxylase (**3a**) is a peptide encoded by a gene selected from *Methylobacterium extorquens* or a mutant thereof and wherein the mutant preferably is selected from the group comprising or consisting of OXC_{Me-Y497A} having the sequence SEQ ID NO. 2, OXC_{Me-S568A} having the sequence SEQ ID NO. 3 or OXC_{Me-Y497A-S568A} having the sequence SEQ ID NO. 4; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 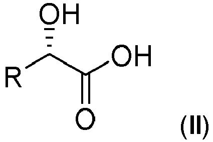 wherein the hydrolase is acyl-CoA thioester hydrolase YciA (**4**) encoded by a gene selected from *E. coli.*

An embodiment of the invention is directed to a method for preparation of a compound of the formula (**I**) 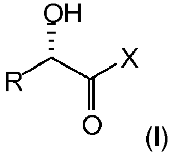 comprising:
**A)** providing a compound R-CHO (**1**) and an oxalyl-CoA (**2'**) 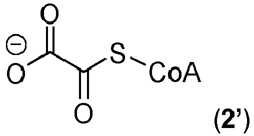 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 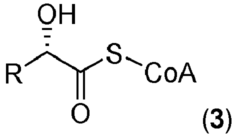 wherein the oxalyl-CoA decarboxylase (**3a**) is a peptide encoded by a gene selected from *Methylobacterium extorquens* or a mutant thereof and wherein the mutant preferably is selected from the group comprising or consisting of OXC_{Me-Y497A} having the sequence SEQ ID NO. 2, OXC_{Me-S568A} having the sequence SEQ ID NO. 3 or OXC_{Me-Y497A-S568A} having the sequence SEQ ID NO. 4; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 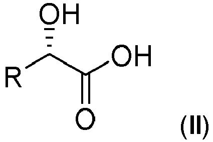 wherein the hydrolase is acyl-CoA thioester hydrolase YciA (**4**) encoded by a gene selected from *E. coli.*

Another embodiment of the present invention is related to a method for preparation of a compound of the formula (**II**) 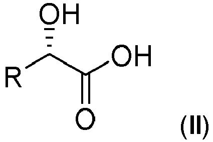 comprising:
**A**) providing a compound R-CHO (**1**) and an oxalyl-CoA (**2'**) 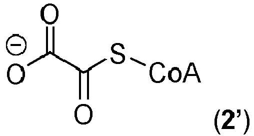 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 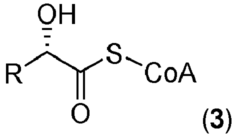 wherein the oxalyl-CoA decarboxylase (**3a**) is a peptide encoded by a gene selected from *Methylobacterium extorquens* or a mutant thereof, and wherein the mutant preferably is selected from the group comprising or consisting of OXC_{Me-Y497A} having the sequence SEQ ID NO. 2, OXC_{Me-S568A} having the sequence SEQ ID NO. 3 or OXC_{Me-Y497A-S568A} having the sequence SEQ ID NO. 4; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 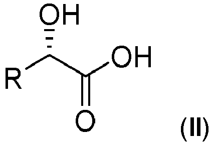 wherein the hydrolase is acyl-CoA thioester hydrolase YciA (**4**) encoded by a gene selected from *E. coli.*

A preferred embodiment of the invention is related to a method for preparation of a compound of the formula (II) 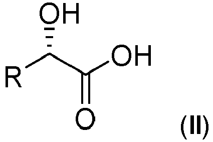 comprising:
**A**) providing a compound R-CHO (**1**) and an oxalyl-CoA (**2'**) 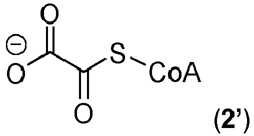 in a buffer solution with a pH-value in the range of 6.5 to 7.5 optionally together with at least one co-solvent, wherein a concentration of R-CHO (**1**) is in the range of 5 mM to 50 mM , wherein the buffer solution comprises 2-[[1,3-dihydroxy-2-(hydroxymethyl)propan-2-yl]amino]ethanesulfonic acid (TES) in the range of 0.25 wt% to 2.5 wt%, and wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 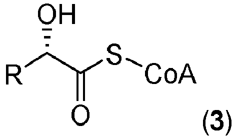 wherein the oxalyl-CoA decarboxylase (**3a**) is a peptide encoded by a gene selected from *Methylobacterium extorquens* or a mutant thereof, wherein a molar ratio of the thiamine diphosphate (**3b**) compared to R-CHO (**1**) is 0.001 to 0.01 with a concentration in the range of 0.05 mM to 0.5 mM, wherein a concentration of the oxalyl-CoA decarboxylase is in the range of 1 to 25 µM and/or a concentration of the magnesium (II) cation is in the range of 2.5 mM to 20 mM, and wherein the mutant preferably is selected from the group comprising or consisting of OXC_{Me-Y497A} having the sequence SEQ ID NO. 2, OXC_{Me-S568A} having the sequence SEQ ID NO. 3 or OXC_{Me-Y497A-S568A} having the sequence SEQ ID NO. 4; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 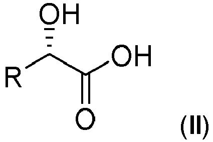 wherein the hydrolase is acyl-CoA thioester hydrolase YciA (**4**) encoded by a gene selected from *E. coli;* and
   wherein steps **A'**), **A**), **B**), and **C**) are carried out as cascade reaction in one-pot system, and wherein a ratio of R-CHO (**1**) and the free coenzyme A (**2b**) is 10 to 1000.

It is especially preferred, when the oxalyl-CoA decarboxylase (**3a**) is selected from a group comprising or consisting of OXC_{Me}, OXC_{Me-Y497A}, OXC_{Me-S568A} or OXC_{Me-Y497A-S568A}.

Decarboxylation of oxalyl-CoA proceeds via formation of the thiamine-α-hydroxyl-CoA adduct (see figure 3), which can be either protonated and released as formyl-CoA or undergo a nucleophilic attack on benzaldehyde to form α-hydroxyacyl-CoA in particular mandelyl-CoA. Surprisingly, it could be found that mandelyl-CoA formation can be increased by suppressing the unwanted protonation reaction (and subsequent release of formyl-CoA) that competes with carboligation.

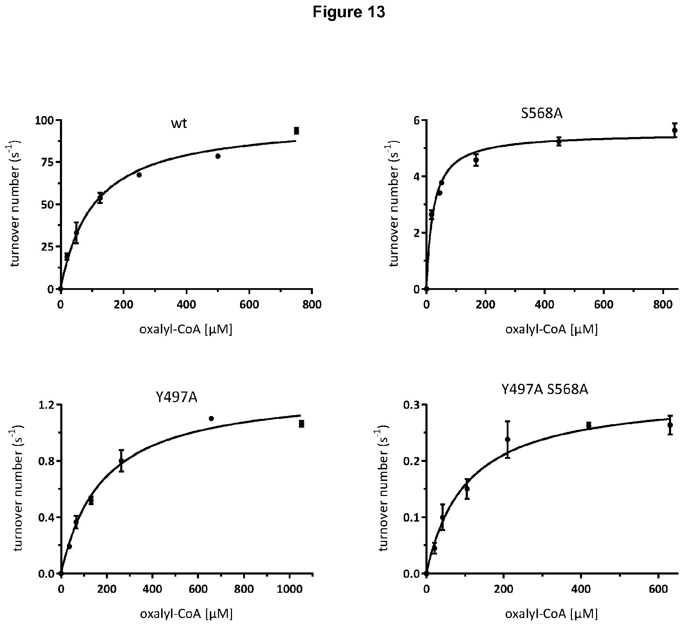

In OXC_{Me} variants OXC_{Me-Y497A} and OXC_{Me-S568A}, formyl-CoA formation was decreased 20 to 50-fold (figure 14B), while the *K*_{M} of both variants was largely unaffected (example 6, table 3, and figure 13). When starting with oxalyl-CoA and benzaldehyde as an example OXC_{Me-Y497A} showed 5-fold increased mandelyl-CoA production rate (20 min⁻¹; figure 14C), suggesting that activity of the enzyme was successfully redirected towards carboligation by suppressing protonation of thiamine-α-hydroxyl-CoA-adduct (see figure 3). Moreover, the reaction time starting from formyl-CoA could be reduced remarkably by means of OXC_{Me-Y497A} in comparison to OXC_{Me} (example 9, figure 14).
When the steps A'), A), B) and C) are carried out as cascade reaction in one-pot system, the reaction time could also be reduced significantly along with a higher enantiomeric excess by means of OXC_{Me-Y497A} in comparison to OXC_{Me.}
The variant OXC_{Me-Y497A-S568A} is also suitable for the carbon-carbon-bond formation. The kinetic data are shown example 6, table 3, and figure 13.

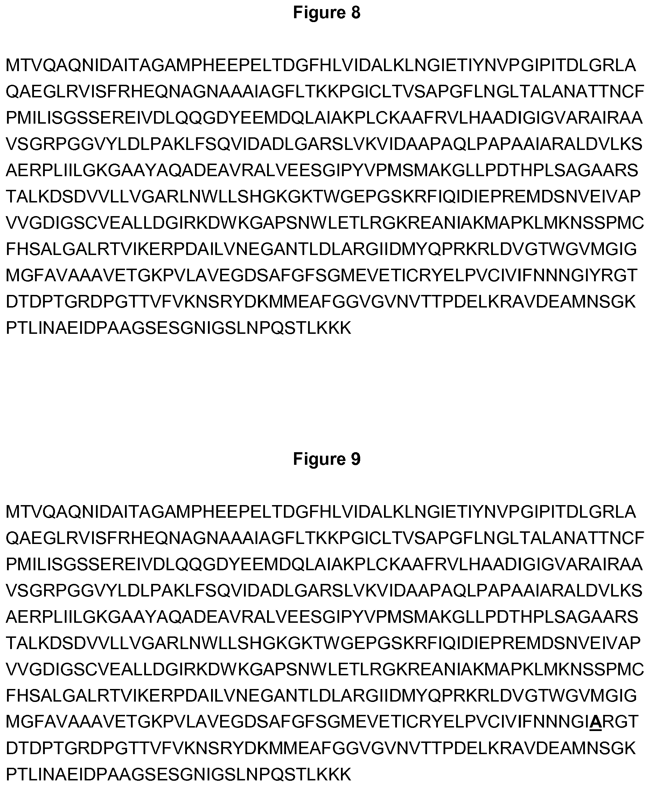

Thus, another aspect of the present invention is directed to a mutant of a peptide OXC_{Me} encoded by a gene from *Methylobacterium extorquens* having a sequence according to SEQ ID No. 1 (figure 8), wherein at least one tyrosine or serine is replaced by an amino acid selected independently of each other from the group comprising or consisting of alanine, phenylalanine, and glycine, preferably tyrosine is replaced by phenylalanine, alanine, and/or glycine, and serine is replaced by alanine and/or glycine, most preferably the tyrosine of SEQ ID NO. 1 at position 497 is replaced by alanine, phenylalanine or glycine, and/or serine of SEQ ID NO. 1 at position 568 is replaced by alanine or glycine.

The present invention is thus also directed to a peptide having a sequence SEQ ID NO. 1 (figure 8), wherein at least one tyrosine or serine is replaced independently of each other by an amino acid selected from the group comprising or consisting of phenylalanine, alanine and glycine, and preferably alanine.

A preferred embodiment according to the invention is related to a peptide OXC_{Me-Y497A} having a sequence SEQ ID NO.2, OXC_{Me-S568A} having the sequence SEQ ID NO. 3 or OXC_{Me-Y497A-S568A} having the sequence SEQ ID NO. 4.

Thus, a preferred embodiment according to the invention is directed to a peptide having a sequence SEQ ID NO. 2 (figure 9), SEQ ID NO. 3 (figure 10) and/or SEQ ID NO. 4 (figure 11).

An embodiment of the present invention is related to a mutant of the peptide oxalyl-CoA decarboxylase OXC_{Me} encoded by a gene from *Methylobacterium extorquens,* wherein preferably the position 497 and/or 568 of OXC_{Me} having the sequence SEQ ID NO. 1 is replaced independently of each other by amino acid selected from the group comprising or consisting of alanine, phenylalanine, and glycine, preferably alanine.

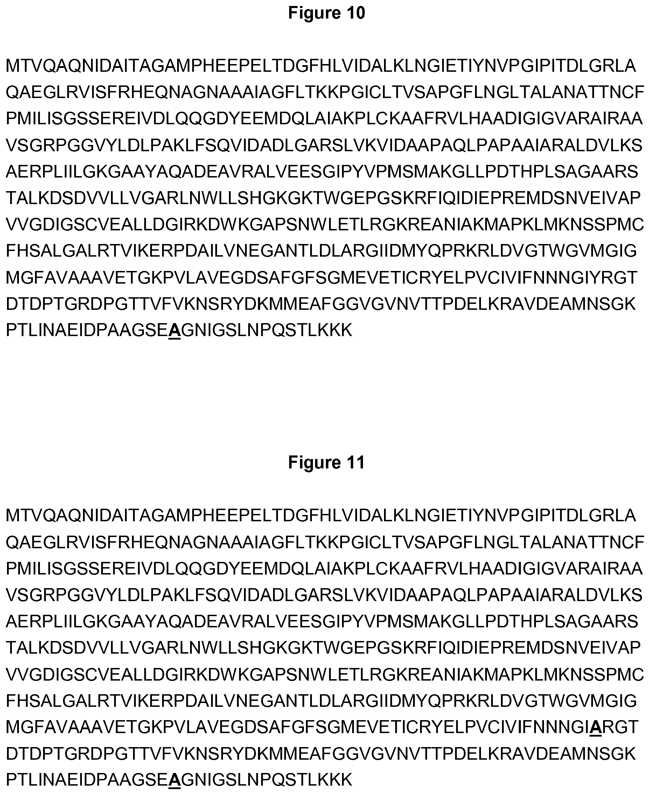

A preferred embodiment according to the present invention is related to the use of peptide having a sequence SEQ ID NO. 2 (figure 9), SEQ ID NO. 3 (figure 10) and/or SEQ ID NO. 4 (figure 11) for the synthesis of an α-hydroxycarboxylic acids, salts thereof, or α-hydroxyacyl-CoA thioester.

A preferred embodiment according to the invention is directed to a composition comprising or consisting of a peptide having a sequence SEQ ID NO. 2 (figure 9), SEQ ID NO. 3 (figure 10) and/or SEQ ID NO. 4 (figure 11), and a thioesterase YciA encoded by a gene selected from *E. coli.*

A preferred embodiment according to the present invention is related to the use of a composition comprising or consisting of a peptide having a sequence SEQ ID NO. 2 (figure 9), SEQ ID NO. 3 (figure 10) and/or SEQ ID NO. 4 (figure 11), and a thioesterase YciA encoded by a gene selected from *E. coli* for the preparation of an α-hydroxycarboxylic acid, salts thereof, or α-hydroxyacyl-CoA thioester.

Another preferred embodiment according to the invention is thus directed to a composition comprising or consisting of a peptide having a sequence SEQ ID NO. 2 (figure 9), SEQ ID NO. 3 (figure 10) and/or SEQ ID NO. 4 (figure 11), a thioesterase YciA encoded by a gene selected from *E. coli,* and an oxalyl-CoA synthetase OXS encoded by a gene selected from *Methylobacterium extorquens.*

Another preferred embodiment according to the invention is related to the use of a composition comprising or consisting of a peptide having a sequence SEQ ID NO. 2 (figure 9), SEQ ID NO. 3 (figure 10) and/or SEQ ID NO. 4 (figure 11), a thioesterase YciA encoded by a gene selected from *E. coli,* and an oxalyl-CoA synthetase (OXS) encoded by a gene selected from *Methylobacterium extorquens* for the synthesis of an α-hydroxycarboxylic acid, or salts thereof.

A further aspect of the present invention is related to a method for preparation of a mutant of oxalyl-CoA decarboxylase from *Methylobacterium extorquens* (OXC_{Me}), wherein OXC_{Me} have the sequence SEQ ID NO. 1, comprising the following steps:
I) Providing a chromosomal DNA from *Methylobacterium extorquens*;
II) Providing at least one primer;
III) Polymer chain reaction to obtain a DNA of oxalyl-CoA decarboxylase (OXC_{Me});
IV) Ligation of the DNA of oxalyl-CoA decarboxylase (OXC_{Me}) into a vector to obtain a vector ligated with the DNA of oxalyl-CoA decarboxylase;
V) mutation of ligated vector obtained in step IV) by polymer chain reaction using at least one primer and the ligated vector obtained in step IV);
VI) providing a cell culture containing a host cell;
VII) introducing the ligated vector according to step V) into the host cell and expressing a mutant of oxalyl-CoA decarboxylase from *Methylobacterium extorquens*; and
VIII) Isolating of the mutant of oxalyl-CoA decarboxylase from *Methylobacterium extorquens*.

"Vector", as used herein, refers to a natural or synthetic single or double stranded plasmid or viral nucleic acid molecule, or any other nucleic acid molecule that can be transfected or transformed into cells and replicate independently of, or within, the host cell genome. A nucleic acid can be inserted into a vector by cutting the vector with restriction enzymes and ligating the pieces together. The nucleic acid molecule can be RNA or DNA. Preferably the nucleic acid molecule is DNA. Preferably, the vector is a plasmid. Preferably, the plasmid is pET-16b.

The host cell can be any cell being suitable for the expression of a mutated oxalyl-CoA decarboxylase from *Methylobacterium extorquens.* Preferably, the host cell is *E. coli.*

In step VI) or in a step VI"), further ingredients can be added to the cell culture including induction agent for the protein expression and selection agent.
As an induction agent for protein expression L-(+)-Arabinose or Isopropyl β-D-1-thiogalactopyranoside (IPTG) can be used. Preferably, the induction agent for protein expression is IPTG.
As a selection agent an antibiotic can be used. Preferably, the antibiotic is ampicillin or chloramphenicol.
If a selection agent is used, the method contains a step VII") after step VII) cultivation of the host cells containing the ligated vector.

"Primer", as used herein, has a nucleotide sequence complementary to a portion of a targeted nucleic acid molecule, i.e., a priming sequence. The term "complementary" used herein refers to "substantially complementary", which indicates that a primer is sufficiently complementary to a template or targeted nucleic acid sequence to be selectively hybridized with the template or targeted nucleic acid sequence. Thus, the primer may have at least one mismatch with the template as long as it functions as a primer.

Preferably, the at least one primer is selected from the group comprising or consisting of the following sequences:

| |
|---|
| CAACAACGGCATCGCTCGCGGCACCGAC (SEQ ID NO. 13) |
| GTCGGTGCCGCGAGCGATGCCGTTGTTG (SEQ ID NO. 14) |
| CCGGCAGCGAGGCCGGCAATATCGG (SEQ ID NO. 15) |
| CCGATATTGCCGGCCTCGCTGCCGG (SEQ ID NO. 16) |

"DNA" refers to deoxyribonucleic acid.

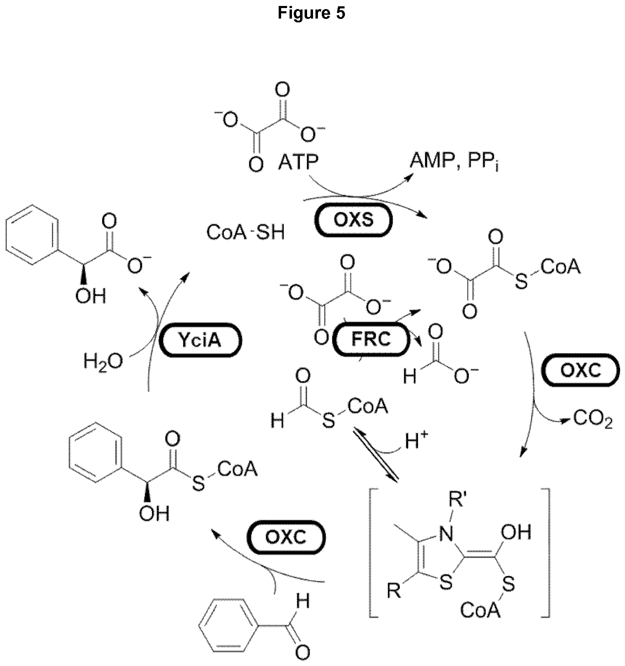

Further, in order to accelerate the carbon-carbon formation in step B) or within the cascade reaction in a one-pot system according to a method of invention since the oxalyl-CoA which is partially transformed to formyl-CoA via the protonation of the thiamine adduct during the reaction can be regenerated by means of formyl-CoA:oxalate transferase (figure 4).
Moreover, in case of a cascade reaction in a one pot system when using oxalyl-CoA the hydrolysis of the formed formyl-CoA by the thioesterase YciA can be prevented, and thus the consumption of ATP being needed for the hydrolysis by the thioesterase YciA (Figure 5).
In case of a cascade reaction in a one pot system when using oxalyl-CoA it is further preferred if the acyl-CoA thioester hydrolase YciA (4) encoded by a gene selected from *E. coli* and/or α-hydroxyacyl-CoA:oxalate CoA-transferase is used.

A "formyl-CoA:oxalate transferase" ("FRC") transfers the coenzyme A moiety from the formyl-CoA to the oxalate, and thus forms oxalyl-CoA.

Thus, preferably in step B) according to a method of the present invention a formyl-CoA:oxalate transferase (FRC) is used in addition to the oxalyl-CoA decarboxylase. More preferably, the formyl-CoA:oxalate transferase which can be used in step B) according to a method of the present invention is a formyl-CoA:oxalate transferase encoded by a gene selected from *Oxalobacter formigenes* or a mutant thereof.

In step C) of a method according to the invention, the cleavage of the coenzyme A moiety can be performed by a hydrolase such as the acyl-CoA thioester hydrolase (thioesterase) YciA (**4**).

Alternative, in step C) of a method according to the invention the cleavage can be performed by a α-hydroxyacyl-CoA:oxalate CoA-transferase or α-hydroxyacyl-CoA:formate CoA-transferase. In this case, a carboxylic acid or a salt thereof is generated by the transfer of the coenzyme A moiety to an oxalate or formate.

Thus, in step C) the coenzyme A moiety can be cleaved from the α-hydroxyacyl-CoA thioester in particular *(S)*-α-hydroxyacyl-CoA thioester by transferring of said moiety to an oxalate or a formate through α-hydroxyacyl-CoA:oxalate CoA-transferase and/or α-hydroxyacyl-CoA:formate CoA-transferase, or by hydrolysing the α-hydroxyacyl-CoA thioester by a hydrolase, wherein the hydrolase is acyl-CoA thioester hydrolase YciA (**4**) encoded by a gene selected from *E. coli.*

Hydrolysing said α-hydroxyacyl-CoA thioester (**3'**) by a hydrolase to obtain the compound of the formula (**II'**) or a salt thereof 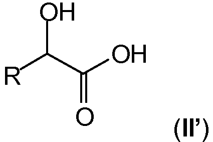 wherein the hydrolase is acyl-CoA thioester hydrolase YciA (**4**) encoded by a gene selected from *E. coli* in step C) is preferred.

Hydrolysing said *(S)*-α-hydroxyacyl-CoA thioester (**3**) by a hydrolase to obtain the compound of the formula (**II**) or a salt thereof 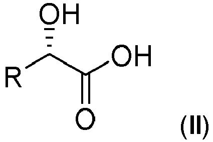 wherein the hydrolase is acyl-CoA thioester hydrolase YciA (**4**) encoded by a gene selected from *E. coli* in step C) is more preferred.

"a-hydroxyacyl-CoA:oxalate CoA-transferase" refers a peptide catalyzing the transfer of the coenzyme A of the mandelyl-CoA thioester or a derivative thereof to the oxalate, and thus forms an oxalyl-CoA thioester and a carboxylic acid or or salts thereof.

"a-hydroxyacyl-CoA:formate CoA-transferase" refers a peptide catalyzing the transfer of the coenzyme A of the mandelyl-CoA thioester or a derivative thereof to the formate, and thus forms a formyl-CoA thioester and a carboxylic acid or salts thereof.

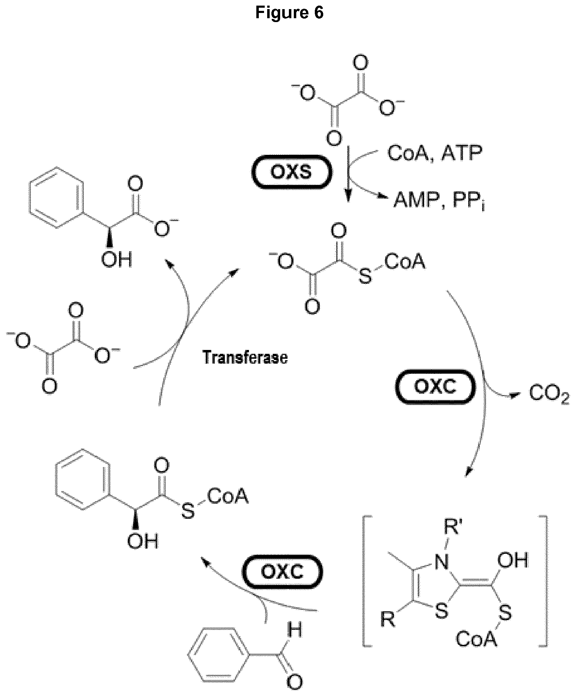

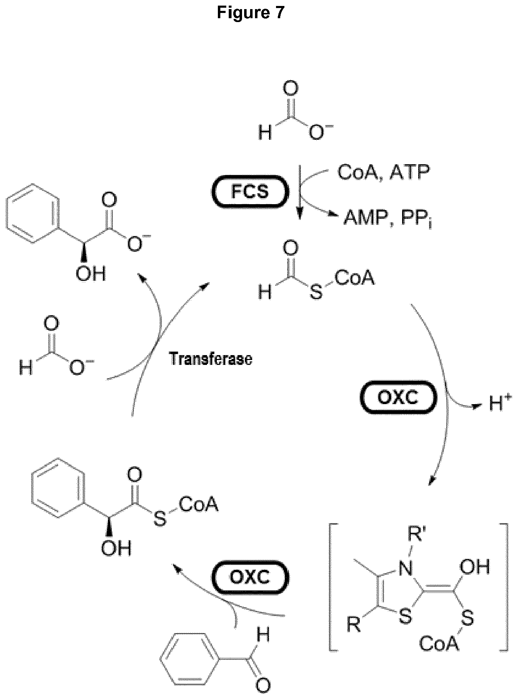

In case the method is carried out as a cascade reaction in one-pot system, the catalytic cycle using oxalate and formate is depicted in figure 6 and 7, respectively. It is apparent that stoichiometric amounts of ATP are not required.

"Mandelyl-CoA:oxalate CoA-transferase" refers a peptide catalyzing the transfer of the coenzyme A of the mandelyl-CoA thioester or a derivative thereof to the oxalate, and thus forms an oxalyl-CoA thioester and the mandelic acid or mandelic acid derivative or salts thereof.

"Mandelyl-CoA:formate CoA-transferase" refers a peptide catalyzing the transfer of the coenzyme A of the mandelyl-CoA thioester or a derivative thereof to the formate, and thus forms a formyl-CoA thioester and the mandelic acid or mandelic acid derivative or salts thereof.

The α-hydroxyacyl-CoA:oxalate CoA-transferase or α-hydroxyacyl-CoA:formate CoA-transferase can be a mutant of formyl-CoA:oxalate CoA-transferase in particular encoded by a gene selected from *Oxalobacter formigenes*, a cinnamoyl-CoA:phenyllactate transferase or a mutant thereof in particular encoded by a gene selected from *Clostridium botulinum*, isocaprenoyl-CoA:α-hydroxyisocaproate CoA-transferase or a mutant thereof in particular encoded by a gene selected from *Clostridium difficile.*

The mandelyl-CoA:oxalate transferase or mandelyl-CoA:formate transferase can be a mutant of formyl-CoA:oxalate CoA-transferase in particular encoded by a gene selected from *Oxalobacter formigenes*, a cinnamoyl-CoA:phenyllactate transferase or a mutant thereof in particular encoded by a gene selected from *Clostridium botulinum*, isocaprenoyl-CoA:α-hydroxyisocaproate CoA-transferase or a mutant thereof in particular encoded by a gene selected from *Clostridium difficile.*

A preferred embodiment of the present invention is thus directed to method for the preparation of an α-hydroxyacyl compound of the formula (**I'**) 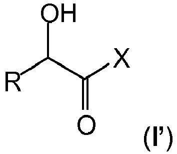 comprising:
**A**) providing a compound R-CHO (**1**) and an acyl-CoA (**2**) 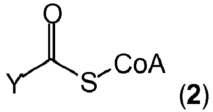
   wherein Y represents -H, or -COO⁻;
   in a buffer solution with a pH-value in the range of 5.0 to 7.5 optionally together with at least one co-solvent;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the α-hydroxyacyl-CoA thioester (**3'**) 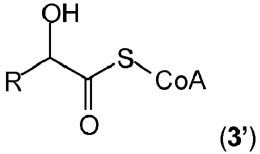 wherein the oxalyl-CoA decarboxylase (**3a**) is a peptide encoded by a gene selected from *Methylobacterium extorquens* or a mutant thereof; and optionally comprising the additional step C)
**C**) hydrolysing said α-hydroxyacyl-CoA thioester (**3'**) by a hydrolase to obtain the compound of the formula (**II'**) or a salt thereof 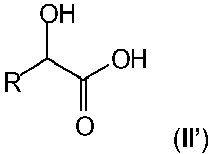 wherein the hydrolase is acyl-CoA thioester hydrolase YciA (**4**) encoded by a gene selected from *E. coli.*

Another preferred embodiment of the present invention is related to to a method for the preparation of α-hydroxyacyl compound of the formula (**I**) 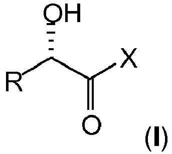
**A)** providing a compound R-CHO **(1)** and an acyl-CoA (**2**) 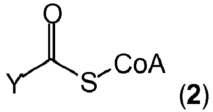
   wherein Y represents -H, or -COO⁻,
   in a buffer solution with a pH-value in the range of 5.0 to 7.5 optionally together with at least one co-solvent;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the (S)-α-hydroxyacyl-CoA thioester (**3**) 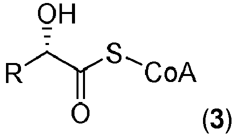 wherein the oxalyl-CoA decarboxylase (**3a**) is a peptide encoded by a gene selected from *Methylobacterium extorquens* or a mutant thereof; or additionally comprising the step **C**)
**C**)hydrolysing said *(S)*-α-hydroxyacyl-CoA thioester (**3**) by a hydrolase to obtain the compound of the formula (**II**) or a salt thereof 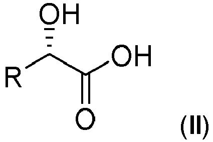 wherein the hydrolase is acyl-CoA thioester hydrolase YciA (**4**) encoded by a gene selected from *E. coli.*

A further preferred embodiment of the present invention is directed to a method for preparation of a compound of the formula (**I'**) 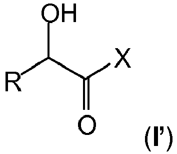 comprising:
**A**) providing a compound R-CHO (**1**) and an oxalyl-CoA (**2'**) 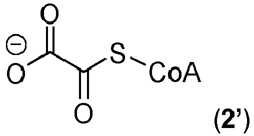 in a buffer solution with a pH-value in the range of 5.0 to 7.5, optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the α-hydroxyacyl-CoA thioester (**3'**) 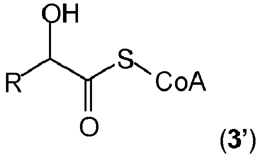 wherein the oxalyl-CoA decarboxylase (**3a**) is a peptide encoded by a gene selected from *Methylobacterium extorquens* or a mutant thereof; or additionally comprising the step **C**)
**C**) hydrolysing said α-hydroxyacyl-CoA thioester (**3'**) by a hydrolase to obtain the compound of the formula (**II'**) or a salt thereof 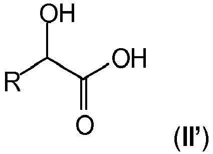 wherein the hydrolase is acyl-CoA thioester hydrolase YciA (**4**) encoded by a gene selected from *E. coli.*

Further, a preferred embodiment of the invention is directed to a method for preparation of a compound of the formula (I) 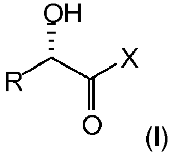 comprising:
**A**) providing a compound R-CHO (**1**) and an oxalyl-CoA (**2'**) 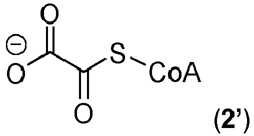 in a buffer solution with a pH-value in the range of 5.0 to 7.5, optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 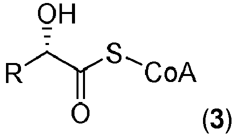 wherein the oxalyl-CoA decarboxylase (**3a**) is a peptide encoded by a gene selected from *Methylobacterium extorquens* or a mutant thereof; or additionally comprising the step **C**)
**C**)hydrolysing said *(S)*-α-hydroxyacyl-CoA thioester (**3**) by a hydrolase to obtain the compound of the formula (**II**) or a salt thereof 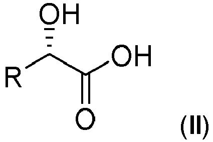 wherein the hydrolase is acyl-CoA thioester hydrolase YciA (**4**) encoded by a gene selected from *E. coli.*

Another preferred embodiment of the present invention is related to a method for preparation of a compound of the formula (II') 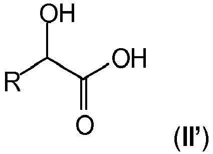 comprising:
**A**) providing a compound R-CHO (**1**) and an oxalyl-CoA (**2'**) 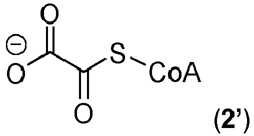 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the α-hydroxyacyl-CoA thioester (**3'**) 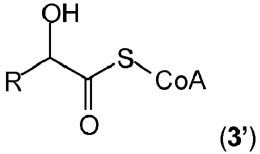 wherein the oxalyl-CoA decarboxylase (**3a**) is a peptide encoded by a gene selected from *Methylobacterium extorquens* or a mutant thereof; and
**C**) hydrolysing said α-hydroxyacyl-CoA thioester (**3'**) by a hydrolase to obtain the compound of the formula (**II'**) or a salt thereof 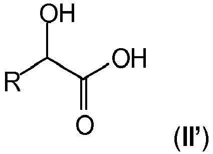 wherein the hydrolase is acyl-CoA thioester hydrolase YciA (**4**) encoded by a gene selected from *E. coli.*

In step **C**) of a method according to the invention as described herein, a concentration of the acyl-CoA thioester hydrolase YciA encoded by a gene selected from *E. coli* (**4**) can be in the range of 0.25 to 5 µM, preferably 0.5 to 3, and more preferably 1 to 2.5.

An embodiment of the invention is related to a method for the preparation of α-hydroxyacyl compound of the formula (I) 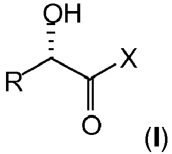 comprising:
**A**) providing a compound R-CHO (**1**) and an acyl-CoA (**2**) 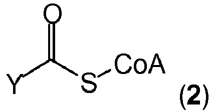
   wherein Y represents -H, or -COO⁻,
   in a buffer solution with a pH-value in the range of 5.0 to 7.5 optionally together with at least one co-solvent;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the (S)-α-hydroxyacyl-CoA thioester (**3**) 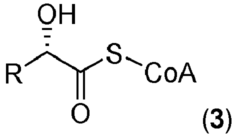 wherein the oxalyl-CoA decarboxylase (**3a**) is a peptide encoded by a gene selected from *Methylobacterium extorquens* or a mutant thereof; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 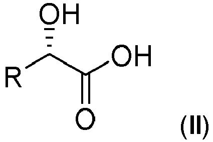 wherein the hydrolase is acyl-CoA thioester hydrolase YciA (**4**) encoded by a gene selected from *E. coli,* and wherein a concentration of the acyl-CoA thioester hydrolase YciA (**4**) is in the range of 0.25 to 5 µM.

An embodiment of the invention is directed to a method for preparation of a compound of the formula (**I**) 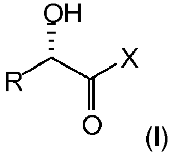 comprising:
**A**) providing a compound R-CHO (**1**) and an oxalyl-CoA (**2'**) 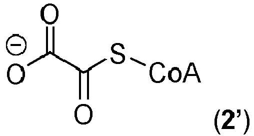 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 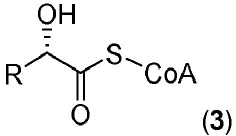 wherein the oxalyl-CoA decarboxylase (**3a**) is a peptide encoded by a gene selected from *Methylobacterium extorquens* or a mutant thereof; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 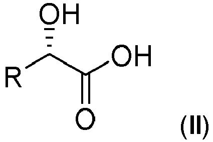 wherein the hydrolase is acyl-CoA thioester hydrolase YciA (**4**) encoded by a gene selected from *E. coli,* and wherein a concentration of the acyl-CoA thioester hydrolase YciA (**4**) is in the range of 0.25 to 5 µM (2 µM).

Another embodiment of the present invention is related to a method for preparation of a compound of the formula (**II**) 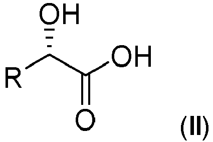 comprising:
**A**) providing a compound R-CHO (**1**) and an oxalyl-CoA (**2'**) 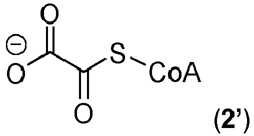 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 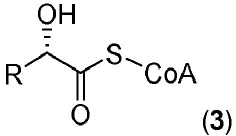 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 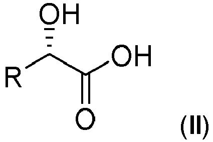 wherein the hydrolase is acyl-CoA thioester hydrolase YciA (**4**) encoded by a gene selected from *E. coli,* and wherein a concentration of the acyl-CoA thioester hydrolase YciA (**4**) is in the range of 0.25 to 5 µM.

A preferred embodiment of the invention is related to a method for preparation of a compound of the formula (**II**) 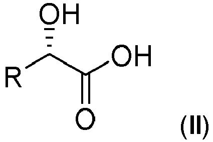 comprising:
**A**) providing a compound R-CHO (**1**) and an oxalyl-CoA (**2'**) 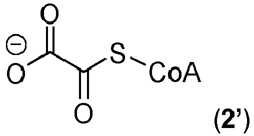 in a buffer solution with a pH-value in the range of 6.5 to 7.5 optionally together with at least one co-solvent, wherein a concentration of R-CHO (**1**) is in the range of 5 mM to 50 mM, wherein the buffer solution comprises 2-[[1,3-dihydroxy-2-(hydroxymethyl)propan-2-yl]amino]ethanesulfonic acid (TES) in the range of 0.25 wt% to 2.5 wt%, and wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**);
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 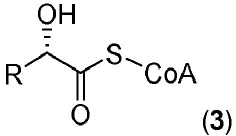 wherein the oxalyl-CoA decarboxylase (**3a**) is a peptide encoded by a gene selected from *Methylobacterium extorquens* or a mutant thereof, wherein a molar ratio of the thiamine diphosphate (**3b**) compared to R-CHO (**1**) is 0.001 to 0.01 with a concentration in the range of 0.05 mM to 0.5 mM, wherein a concentration of the oxalyl-CoA decarboxylase is in the range of 1 to 25 µM and/or a concentration of the magnesium (II) cation is in the range of 2.5 mM to 20 mM, and wherein the mutant preferably is selected from the group comprises or consists of OXC_{Me-Y497A} having the sequence SEQ ID NO. 2, OXC_{Me-S568A} having the sequence SEQ ID NO. 3 or OXC_{Me-Y497A-S568A} having the sequence SEQ ID NO. 4; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (II) or a salt thereof 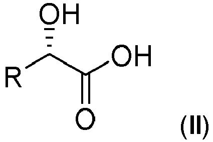 wherein the hydrolase is acyl-CoA thioester hydrolase YciA (**4**) encoded by a gene selected from *E. coli,* and wherein a concentration of the acyl-CoA thioester hydrolase YciA (**4**) is in the range of 0.25 to 5 µM*;* and
   wherein steps **A'**), **A**), **B**), and **C**) are carried out as cascade reaction in one-pot system, and wherein a ratio of R-CHO (**1**) and the free coenzyme A (**2b**) is 10 to 1000.

In the methods according to the invention, in step **A'**) a concentration of the carboxylate (**2a**) can be in the range of 1 to 100 mM, preferably 5 mM to 50 mM, more preferably 10 mM to 25 mM, and/or a concentration of acyl-CoA synthetase (**2c**) can be in the range of 1 to 25 µM, preferably 2 to 15, more preferably 5 to 10, and/or ATP can be in the range of 1 mM to 50 mM preferably 5 to 25, more preferably 10 to 20.

An embodiment of the present invention is directed to a method for preparation of a compound of the formula (I) 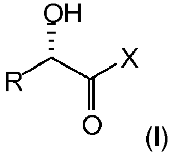 comprising:
**A**) providing a compound R-CHO (**1**) and an acyl-CoA (**2**) 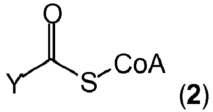
   wherein Y represents -H, or -COO⁻,
   in a buffer solution with a pH-value in the range of 5.0 to 7.5, optionally together with at least one co-solvent, and wherein acyl-CoA (**2**) can be prepared by the following step A'):
**A'**) reacting an carboxylate (**2a**) with a free coenzyme A (**2b**) by acyl-CoA synthetase (**2c**) in the presence of ATP (**2e**) to produce an acyl-CoA (**2**) wherein a concentration of the carboxylate (**2a**) is in the range of 1 to 100 mM and/or a concentration of acyl-CoA synthetase (**2c**) is in the range of 1 to 25 µM (5 µM) and/or ATP is in the range of 1 mM to 50 mM;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the (S)-α-hydroxyacyl-CoA thioester (**3**) 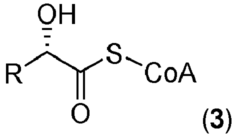 wherein the oxalyl-CoA decarboxylase (**3a**) is a peptide encoded by a gene selected from *Methylobacterium extorquens* or a mutant thereof; or additionally comprising the step **C**)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 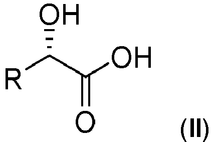 wherein the hydrolase is acyl-CoA thioester hydrolase YciA (**4**) encoded by a gene selected from *E. coli*; and
   wherein the steps **A'**), **A**), and **B**), and **C**) are carried out as cascade reaction in one-pot system, and wherein a ratio of the carboxylate (**2a**) and the free coenzyme A (**2b**) is preferably 10 to 10,000

In the methods according to the invention, in step **A'**) a concentration of the oxalate can be in the range of 1 to 100 mM and/or a concentration of oxalyl-CoA synthetase (**2c'**) can be in the range of 1 to 25 µM (5 µM) and/or ATP can in the range of 1 mM to 50 mM.

An embodiment of the invention is directed to a method for preparation of a compound of the formula (**I**) 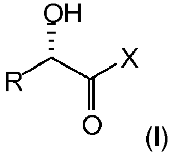 comprising:
**A**) providing a compound R-CHO (**1**) and an oxalyl-CoA (**2'**) 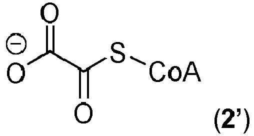 in a buffer solution with a pH-value in the range of 5.0 to 7.5 optionally together with at least one co-solvent, wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**), wherein a concentration of the oxalate is in the range of 1 to 100 mM and/or a concentration of oxalyl-CoA synthetase (**2c'**) is in the range of 1 to 25 µM and/or ATP is in the range of 1 mM to 50 mM;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 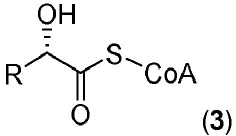 wherein the oxalyl-CoA decarboxylase (**3a**) is a peptide encoded by a gene selected from *Methylobacterium extorquens* or a mutant thereof; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 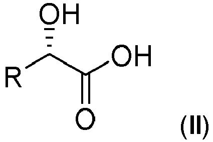 wherein the hydrolase is acyl-CoA thioester hydrolase YciA (**4**) encoded by a gene selected from *E. coli.*

Another embodiment of the present invention is related to a method for preparation of a compound of the formula (**II**) 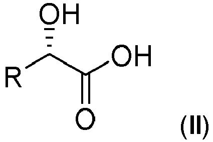 comprising:
**A**) providing a compound R-CHO (**1**) and an oxalyl-CoA (**2'**) 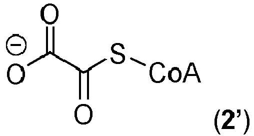 in a buffer solution with a pH-value in the range of 5.0 to 7.5 optionally together with at least one co-solvent, wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**), wherein a concentration of the oxalate is in the range of 1 to 100 mM and/or a concentration of oxalyl-CoA synthetase (**2c'**) is in the range of 1 to 25 µM (5 µM) and/or ATP is in the range of 1 mM to 50 mM;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 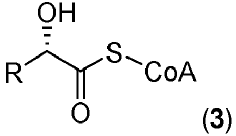 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 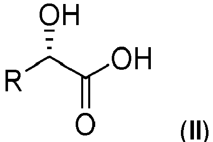 wherein the hydrolase is acyl-CoA thioester hydrolase YciA (**4**) encoded by a gene selected from *E. coli.*

A preferred embodiment of the invention is related to a method for preparation of a compound of the formula (**II**) 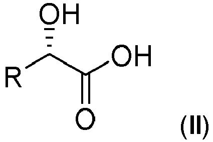 comprising:
**A**) providing a compound R-CHO (**1**) and an oxalyl-CoA (**2'**) 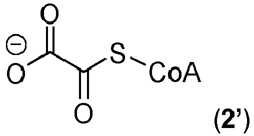 in a buffer solution with a pH-value in the range of 6.5 to 7.5 optionally together with at least one co-solvent, wherein a concentration of R-CHO (**1**) is in the range of 5 mM to 50 mM, wherein the buffer solution comprises 2-[[1,3-dihydroxy-2-(hydroxymethyl)propan-2-yl]amino]ethanesulfonic acid (TES) in the range of 0.25 wt% to 2.5 wt%, and wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**), wherein a concentration of the oxalate is in the range of 1 to 100 mM and/or a concentration of oxalyl-CoA synthetase (**2c'**) is in the range of 1 to 25 µM (5 µM) and/or ATP is in the range of 1 mM to 50 mM;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 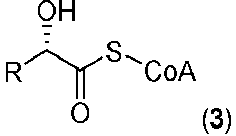 wherein the oxalyl-CoA decarboxylase (**3a**) is a peptide encoded by a gene selected from *Methylobacterium extorquens* or a mutant thereof, wherein a molar ratio of the thiamine diphosphate (**3b**) compared to R-CHO (**1**) is 0.001 to 0.01 with a concentration in the range of 0.05 mM to 0.5 mM, wherein a concentration of the oxalyl-CoA decarboxylase is in the range of 1 to 25 µM and/or a concentration of the magnesium (II) cation is in the range of 2.5 mM to 20 mM, and wherein the mutant preferably is selected from the group comprises or consists of OXC_{Me-Y497A} having the sequence SEQ ID NO. 2, OXC_{Me-S568A} having the sequence SEQ ID NO. 3 or OXC_{Me-Y497A-S568A} having the sequence SEQ ID NO. 4; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 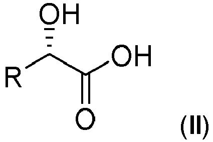 wherein the hydrolase is acyl-CoA thioester hydrolase YciA (**4**) encoded by a gene selected from *E. coli*, and wherein a concentration of the acyl-CoA thioester hydrolase YciA (**4**) is in the range of 0.25 to 5 µM*;* and
   wherein steps **A'**), **A**), **B**), and **C**) are carried out as cascade reaction in one-pot system, and wherein a ratio of R-CHO (**1**) and the free coenzyme A (**2b**) is 10 to 1000.

In step **A'**) of the method according to the invention, the carboxylate (**2a**) have a cationic counterion selected from the group comprising or consisting of NH₄⁺, Li⁺, Na⁺, K⁺, Rb⁺, Cs⁺, Mg²⁺, Ca²⁺, Zn²⁺, Fe²⁺, Mn²⁺, Ni²⁺, Cu²⁺, and Co²⁺. Preferably NH₄⁺, Na⁺, K⁺, Mg²⁺, and Ca²⁺.

An embodiment of the present invention is directed to a method for preparation of a compound of the formula (**I**) 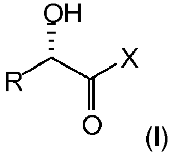 comprising:
**A**) providing a compound R-CHO (**1**) and an acyl-CoA (**2**) 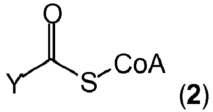
   wherein Y represents -H, or -COO⁻,
   in a buffer solution with a pH-value in the range of 5.0 to 7.5, optionally together with at least one co-solvent, and wherein acyl-CoA (**2**) can be prepared by the following step A'):
      **A'**) reacting an carboxylate (**2a**) with a free coenzyme A (**2b**) by acyl-CoA synthetase (**2c**) in the presence of ATP (**2e**) to produce an acyl-CoA (**2**), and wherein the carboxylate (**2a**) have a cationic counterion selected from the group consisting of NH₄⁺, Li⁺, Na⁺, K⁺, Rb⁺, Cs⁺, Mg²⁺, Ca²⁺, Zn²⁺, Fe²⁺, Mn²⁺, Ni²⁺, Co²⁺ and Cu²⁺;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the (S)-α-hydroxyacyl-CoA thioester (**3**) 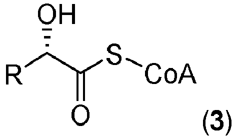 wherein the oxalyl-CoA decarboxylase (**3a**) is a peptide encoded by a gene selected from *Methylobacterium extorquens* or a mutant thereof; or additionally comprising the step **C**)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 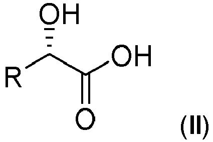 wherein the hydrolase is acyl-CoA thioester hydrolase YciA (**4**) encoded by a gene selected from *E. coli.*

In step **A'**) of the method according to the invention, the oxalate (**2a'**) can be selected from the group comprising or consisting of (NH₄)₂C₂O₄, Li₂C₂O₄, Na₂C₂O₄, K₂C₂O₄, Rb₂C₂O₄, Cs₂C₂O₄, MgC₂O₄, CaC₂O₄, ZnC₂O₄, FeC₂O₄, MnC₂O₄, NiC₂O₄, CuC₂O₄, and CoC₂O₄.

An embodiment of the invention is directed to a method for preparation of a compound of the formula (**I**) 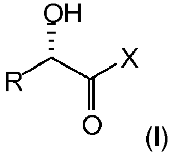 comprising:
**A**) providing a compound R-CHO (**1**) and an oxalyl-CoA (**2'**) 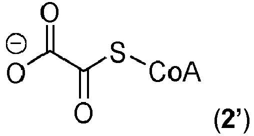 in a buffer solution with a pH-value in the range of 5.0 to 7.5 optionally together with at least one co-solvent, wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**), wherein the oxalate is selected from the group comprising or consisting of (NH₄)₂C₂O₄, Li₂C₂O₄, Na₂C₂O₄, K₂C₂O₄, Rb₂C₂O₄, Cs₂C₂O₄, MgC₂O₄, CaC₂O₄, ZnC₂O₄, FeC₂O₄, MnC₂O₄, NiC₂O₄, CuC₂O₄; and CoC₂O₄
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 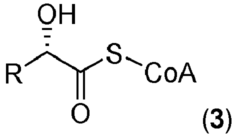 wherein the oxalyl-CoA decarboxylase (3a) is a peptide encoded by a gene selected from *Methylobacterium extorquens* or a mutant thereof; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 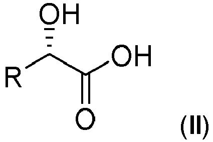 wherein the hydrolase is acyl-CoA thioester hydrolase YciA (**4**) encoded by a gene selected from *E. coli.*

Another embodiment of the present invention is related to a method for preparation of a compound of the formula (**II**) 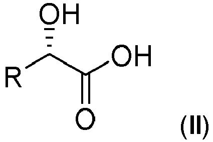 comprising:
**A**) providing a compound R-CHO (**1**) and an oxalyl-CoA (**2'**) 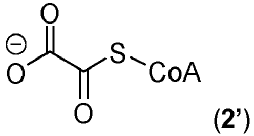 in a buffer solution with a pH-value in the range of 5.0 to 7.5 optionally together with at least one co-solvent, wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**), wherein the oxalate is selected from the group comprising or consisting of (NH₄)₂C₂O₄, Li₂C₂O₄, Na₂C₂O₄, K₂C₂O₄, MgC₂O₄, CaC₂O₄, ZnC₂O₄, FeC₂O₄, MnC₂O₄, NiC₂O₄, CuC₂O₄, Rb₂C₂O₄, Cs₂C₂O₄, and CoC₂O₄
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 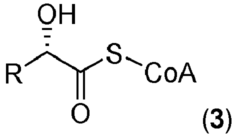 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 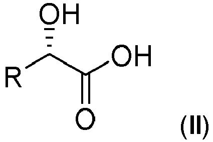 wherein the hydrolase is acyl-CoA thioester hydrolase YciA (**4**) encoded by a gene selected from *E. coli.*

A preferred embodiment of the invention is related to a method for preparation of a compound of the formula (**II**) 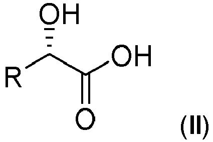 comprising:
**A**) providing a compound R-CHO (**1**) and an oxalyl-CoA (**2'**) 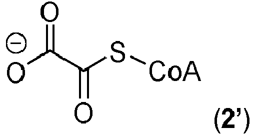 in a buffer solution with a pH-value in the range of 6.5 to 7.5 optionally together with at least one co-solvent, wherein a concentration of R-CHO (**1**) is in the range of 5 mM to 50 mM, wherein the buffer solution comprises 2-[[1,3-dihydroxy-2-(hydroxymethyl)propan-2-yl]amino]ethanesulfonic acid (TES) in the range of 0.25 wt% to 2.5 wt%, and wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**) wherein a concentration of the oxalate is in the range of 1 to 100 mM and/or a concentration of oxalyl-CoA synthetase (**2c'**) is in the range of 1 to 25 µM and/or ATP is in the range of 1 mM to 50 mM, and wherein the oxalate is selected from the group comprising or consisting of (NH₄)₂C₂O₄, Li₂C₂O₄, Na₂C₂O₄, K₂C₂O₄, MgC₂O₄, CaC₂O₄, ZnC₂O₄, FeC₂O₄, MnC₂O₄, NiC₂O₄, CuC₂O₄, Rb₂C₂O₄, Cs₂C₂O₄, and CoC₂O₄, preferably (NH₄)₂C₂O₄, Li₂C₂O₄, Na₂C₂O₄, K₂C₂O₄, more preferably Na₂C₂O₄;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 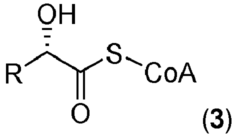 wherein the oxalyl-CoA decarboxylase (**3a**) is a peptide encoded by a gene selected from *Methylobacterium extorquens* or a mutant thereof, wherein a molar ratio of the thiamine diphosphate (**3b**) compared to R-CHO (**1**) is 0.001 to 0.01 with a concentration in the range of 0.05 mM to 0.5 mM, wherein a concentration of the oxalyl-CoA decarboxylase is in the range of 1 to 25 µM and/or a concentration of the magnesium (II) cation is in the range of 2.5 mM to 20 mM, and wherein the mutant preferably is selected from the group comprises or consists of OXC_{Me-Y497A} having the sequence SEQ ID NO. 2, OXC_{Me-S568A} having the sequence SEQ ID NO. 3 or OXC_{Me-Y497A-S568A} having the sequence SEQ ID NO. 4; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 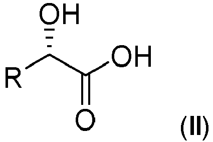 wherein the hydrolase is acyl-CoA thioester hydrolase YciA (**4**) encoded by a gene selected from *E. coli,* and wherein a concentration of the acyl-CoA thioester hydrolase YciA (**4**) is in the range of 0.25 to 5 µM*;* and
   wherein steps **A'**), **A**), **B**), and **C**) are carried out as cascade reaction in one-pot system, and wherein a ratio of R-CHO (**1**) and the free coenzyme A (**2b**) is 10 to 1000.

In principal each co-solvent can be used when it does not denaturize the enzyme under the used conditions. A skilled person in the art knows how to choose a suitable co-solvent. A co-solvent can be selected from the group comprising or consisting of dimethyl sulfoxide, acetone, acetonitrile (ACN), 2-butanol, tert-butyl alcohol, 1,2-dimethoxy-ethane (DME), dimethyl-formamide (DMF), 1,4-dioxane, ethanol, methanol, 1-propanol, 2-propanol, tetrahydrofuran (THF), and ethylene glycol (EG). Preferably, one of the at least one co-solvent is dimethyl sulfoxide. Preferably, the at least one co-solvent is dimethyl sulfoxide.

Preferably, **R** represents 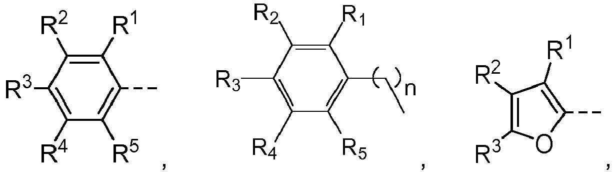 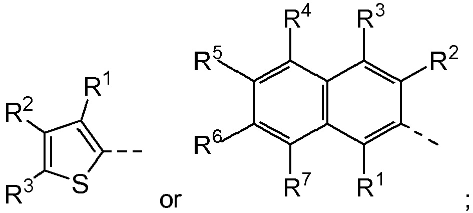 and
**R¹**, **R²**, **R³**, **R⁴**, **R⁵**, **R⁶** and **R⁷** represents independently of each other -H, -Cl, -OH, -OCH₃, -NO₂, -N(CH₃)₂, -C≡CH, -CHO, -COCH₃ or -COOH, and n represents an integer 1.

An embodiment of the invention is related to a method for the preparation of α-hydroxyacyl compound of the formula (I) 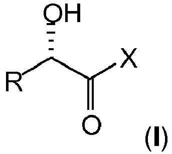 wherein R represents 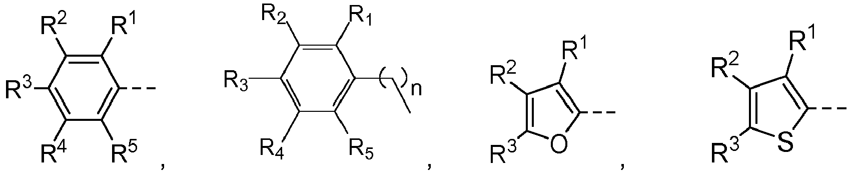 or 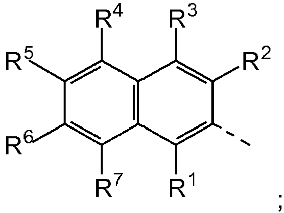 ; and
**R¹**, **R²**, **R³**, **R⁴**, **R⁵**, **R⁶** and **R⁷** represents independently of each other -H, -Cl, -OH, -OCH₃, -NO₂, -N(CH₃)₂, -C≡CH, -CHO, -COCH₃ or -COOH, and represents an integer 1; or a salt thereof.
comprising:
**A**) providing a compound R-CHO (**1**) and an acyl-CoA (**2**) 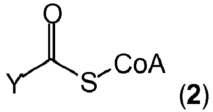
   wherein Y represents -H, or -COO⁻,
   in a buffer solution with a pH-value in the range of 5.0 to 7.5 optionally together with at least one co-solvent;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the (S)-α-hydroxyacyl-CoA thioester (**3**) 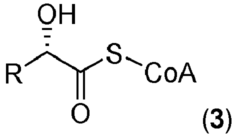 wherein the oxalyl-CoA decarboxylase (**3a**) is a peptide encoded by a gene selected from *Methylobacterium extorquens* or a mutant thereof; and optionally comprising the additional step C)
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 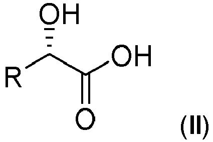 wherein the hydrolase is acyl-CoA thioester hydrolase YciA (**4**) encoded by a gene selected from *E. coli.*

A preferred embodiment of the invention is related to a method for the preparation of α-hydroxyacyl compound of the formula (**I**) 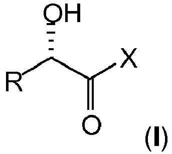 wherein R represents 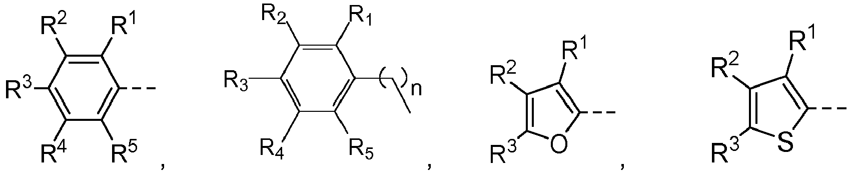 or 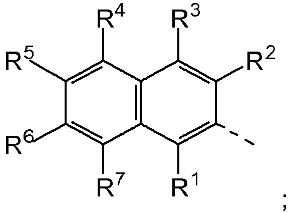 and
**R¹**, **R²**, **R³**, **R⁴**, **R⁵**, **R⁶** and **R⁷** represents independently of each other -H, -Cl, -OH, -OCH₃, -NO₂, -N(CH₃)₂, -C≡CH, -CHO, -COCH₃ or -COOH, and represents an integer 1; or a salt thereof.
comprising:
**A**) providing a compound R-CHO (**1**) and an acyl-CoA (**2**) 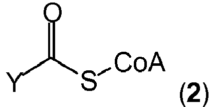
   wherein Y represents -H, or -COO⁻,
   in a buffer solution with a pH-value in the range of 5.0 to 7.5 optionally together with at least one co-solvent;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the (S)-α-hydroxyacyl-CoA thioester (**3**) 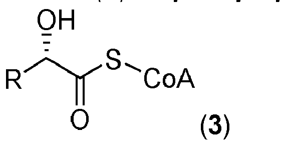 wherein the oxalyl-CoA decarboxylase (**3a**) is a peptide encoded by a gene selected from *Methylobacterium extorquens* or a mutant thereof; and optionally comprising the additional step C)
**C**) hydrolysing said *(S)*-α-hydroxyacyl-CoA thioester (**3**) by a hydrolase to obtain the compound of the formula (II) or a salt thereof, 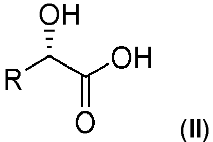 wherein the hydrolase is acyl-CoA thioester hydrolase YciA (**4**) encoded by a gene selected from *E. coli.*

An embodiment of the invention is directed to a method for preparation of a compound of the formula (**I**) 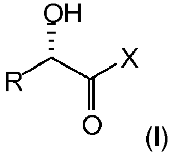 wherein **R** represents 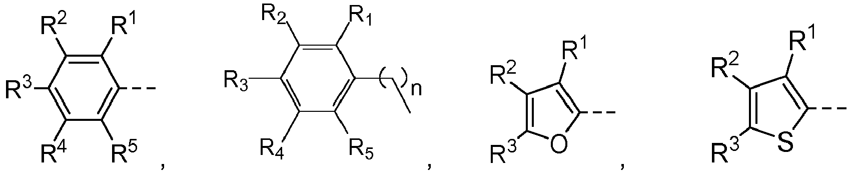 or 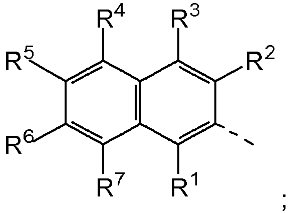 and
**R¹**, **R²**, **R³**, **R⁴**, **R⁵**, **R⁶** and **R⁷** represents independently of each other -H, -Cl, -OH, -OCH₃, -NO₂, -N(CH₃)₂, -C≡CH, -CHO, -COCH₃ or -COOH, and represents an integer 1; or a salt thereof.
comprising:
**A**) providing a compound R-CHO (**1**) and an oxalyl-CoA (**2'**) 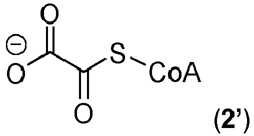 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 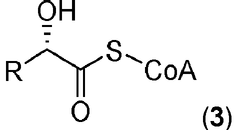 wherein the oxalyl-CoA decarboxylase (**3a**) is a peptide encoded by a gene selected from *Methylobacterium extorquens* or a mutant thereof; and optionally comprising the additional step C)
**C**) cleaving the coenzyme A moiety of said *(S)*-α-hydroxyacyl-CoA thioester (**3**) by a hydrolase , α-hydroxyacyl-CoA:oxalate CoA-transferase and/or α-hydroxyacyl-CoA:formate CoA-transferase to obtain the compound of the formula (**II**) or a salt thereof 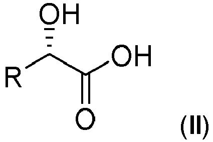 wherein the hydrolase is acyl-CoA thioester hydrolase YciA (**4**) encoded by a gene selected from *E. coli.*

Another embodiment of the present invention is related to a method for preparation of a compound of the formula (**II**) 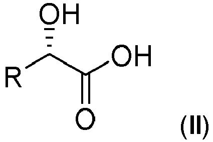 wherein **R** represents 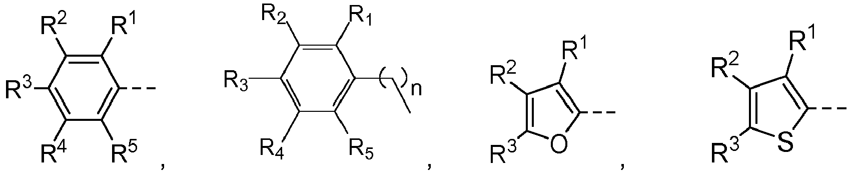 or 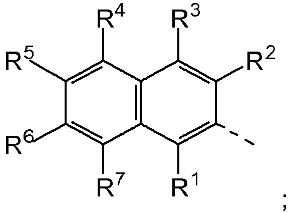 and
**R¹**, **R²**, **R³**, **R⁴**, **R⁵**, **R⁶** and **R⁷** represents independently of each other -H, -Cl, -OH, -OCH₃, -NO₂, -N(CH₃)₂, -C≡CH, -CHO, -COCH₃ or -COOH, and represents an integer 1; or a salt thereof.
comprising:
**A**) providing a compound R-CHO (**1**) and an oxalyl-CoA (**2'**) 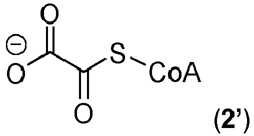 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 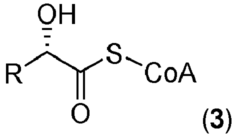 wherein the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 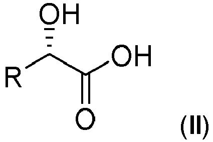 wherein the hydrolase is acyl-CoA thioester hydrolase YciA (**4**) encoded by a gene selected from *E. coli.*

A preferred embodiment of the present invention is related to a method for preparation of a compound of the formula (**II**) 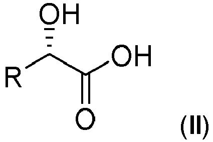 wherein **R** represents 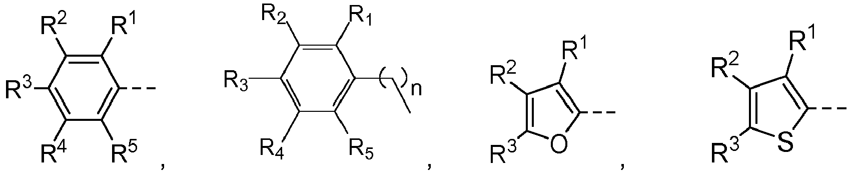 or 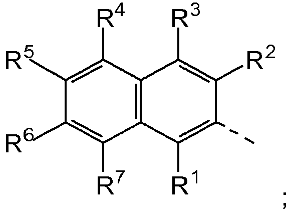 and
**R¹**, **R²**, **R³**, **R⁴**, **R⁵**, **R⁶** and **R⁷** represents independently of each other -H, -Cl, -OH, -OCH₃, -NO₂, -N(CH₃)₂, -C≡CH, -CHO, -COCH₃ or -COOH, and represents an integer 1; or a salt thereof.
comprising:
**A**) providing a compound R-CHO (**1**) and an oxalyl-CoA (**2'**) 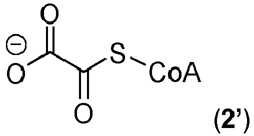 in a buffer solution with a pH-value in the range of 5.0 to 7.5 optionally together with at least one co-solvent;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 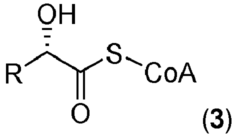 wherein the oxalyl-CoA decarboxylase (**3a**) is a peptide encoded by a gene selected from *Methylobacterium extorquens* or a mutant thereof; and
**C**) hydrolyzing said *(S)*-α-hydroxyacyl-CoA thioester (**3**) by a hydrolase to obtain the compound of the formula (**II**) or a salt thereof 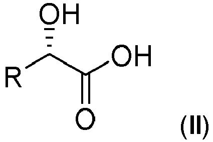 wherein the hydrolase is acyl-CoA thioester hydrolase YciA (**4**) encoded by a gene selected from *E. coli.*

A preferred embodiment of the invention is related to a method for preparation of a compound of the formula (**II**) 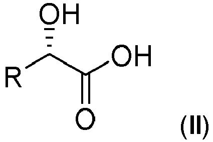 wherein **R** represents 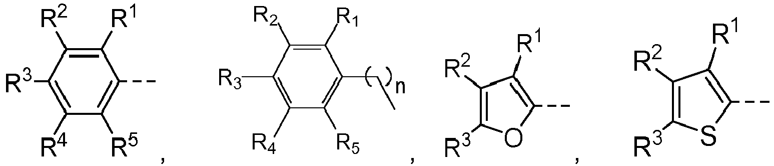 or 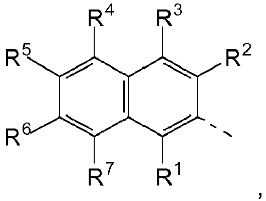 and
wherein **R¹**, **R²**, **R³**, **R⁴**, **R⁵**, **R⁶** and **R⁷** represent independently -H, -Cl, -OH, -OCH₃, -NO₂, -N(CH₃)₂, -C≡CH, -CHO, -COCH₃ or -COOH, wherein n represents an integer 1; or a salt thereof.
comprising:
**A**) providing a compound R-CHO (**1**) and an oxalyl-CoA (**2'**) 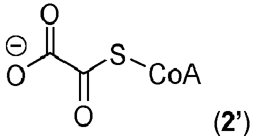 in a buffer solution with a pH-value in the range of 6.5 to 7.5 optionally together with at least one co-solvent, wherein a concentration of R-CHO (**1**) is in the range of 5 mM to 50 mM , wherein the buffer solution comprises 2-[[1,3-dihydroxy-2-(hydroxymethyl)propan-2-yl]amino]ethanesulfonic acid (TES) in the range of 0.25 wt% to 2.5 wt%, and wherein the oxalyl-CoA (2') is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**) wherein a concentration of the oxalate is in the range of 1 to 100 mM (Fig1.C) and/or a concentration of oxalyl-CoA synthetase (**2c'**) is in the range of 1 to 25 µM and/or ATP is in the range of 1 mM to 50 mM, and wherein the oxalate is selected from the group comprising or consisting of (NH₄)₂C₂O₄, Li₂C₂O₄, Na₂C₂O₄, K₂C₂O₄, Rb₂C₂O₄, Cs₂C₂O₄ MgC₂O₄, CaC₂O₄, ZnC₂O₄, FeC₂O₄, MnC₂O₄, NiC₂O₄, CuC₂O₄, and CoC₂O₄, preferably (NH₄)₂C₂O₄, Li₂C₂O₄, Na₂C₂O₄, K₂C₂O₄, more preferably Na₂C₂O₄;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 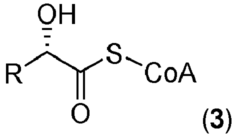 wherein the oxalyl-CoA decarboxylase (**3a**) is a peptide encoded by a gene selected from *Methylobacterium extorquens* or a mutant thereof, wherein a molar ratio of the thiamine diphosphate (**3b**) compared to R-CHO (**1**) is 0.001 to 0.01 with a concentration in the range of 0.05 mM to 0.5 mM, wherein a concentration of the oxalyl-CoA decarboxylase is in the range of 1 to 25 µM and/or a concentration of the magnesium (II) cation is in the range of 2.5 mM to 20 mM, and wherein the mutant preferably is selected from the group comprises or consists of OXC_{Me-Y497A} having the sequence SEQ ID NO. 2, OXC_{Me-S568A} having the sequence SEQ ID NO. 3 or OXC_{Me-Y497A}-_{S568A} having the sequence SEQ ID NO. 4; and
**C**) cleaving the coenzyme A moiety of said *(S)*-α-hydroxyacyl-CoA thioester (**3**) by a hydrolase, α-hydroxyacyl-CoA:oxalate CoA-transferase and/or α-hydroxyacyl-CoA:formate CoA-transferase to obtain the compound of the formula (**II**) or a salt thereof 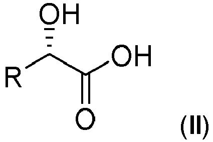 wherein the hydrolase is acyl-CoA thioester hydrolase YciA (**4**) encoded by a gene selected from *E. coli,* and wherein a concentration of the acyl-CoA thioester hydrolase YciA (**4**) is in the range of 0.25 to 5 µM (2 µM); and
   wherein steps **A'**), **A**), **B**), and **C**) are carried out as cascade reaction in one-pot system, and wherein a ratio of R-CHO (**1**) and the free coenzyme A (**2b**) is 10 to 1000.

A more preferred embodiment of the invention is related to a method for preparation of a compound of the formula (**II**) 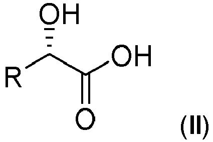 wherein **R** represents 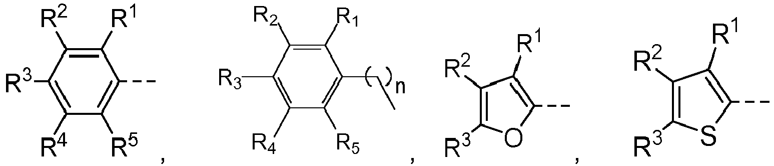 or 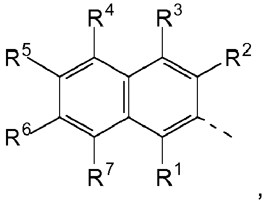 and wherein **R¹**, **R²**, **R³**, **R⁴**, **R⁵**, **R⁶** and **R⁷** represent independently -H, -Cl, -OH, -OCH₃, -NO₂, -N(CH₃)₂, -C≡CH, -CHO, -COCH₃ or -COOH, wherein n represents an integer 1; or a salt thereof.
comprising:
**A**) providing a compound R-CHO (**1**) and an oxalyl-CoA (**2'**) 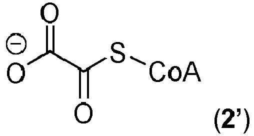 in a buffer solution with a pH-value in the range of 6.5 to 7.5 optionally together with at least one co-solvent, wherein a concentration of R-CHO (**1**) is in the range of 5 mM to 50 mM , wherein the buffer solution comprises 2-[[1,3-dihydroxy-2-(hydroxymethyl)propan-2-yl]amino]ethanesulfonic acid (TES) in the range of 0.25 wt% to 2.5 wt%, and wherein the oxalyl-CoA (**2'**) is prepared by the following step **A'**)
   **A'**) reacting an oxalate (C₂O₄²⁻) (**2a'**) with a free coenzyme A (**2b**) by oxalyl-CoA synthetase (**2c'**) in the presence of ATP (**2e**) to produce an oxalyl-CoA (**2'**) wherein a concentration of the oxalate is in the range of 1 to 100 mM and/or a concentration of oxalyl-CoA synthetase (**2c'**) is in the range of 1 to 25 µM and/or ATP is in the range of 1 mM to 50 mM, and wherein the oxalate is selected from the group comprising or consisting of (NH₄)₂C₂O₄, Li₂C₂O₄, Na₂C₂O₄, K₂C₂O₄, Rb₂C₂O₄, Cs₂C₂O₄, MgC₂O₄, CaC₂O₄, ZnC₂O₄, FeC₂O₄, MnC₂O₄, NiC₂O₄, CuC₂O₄, and CoC₂O₄, preferably (NH₄)₂C₂O₄, Li₂C₂O₄, Na₂C₂O₄, K₂C₂O₄, more preferably Na₂C₂O₄;
**B**) performing a coupling reaction of R-CHO (**1**) and oxalyl-CoA (**2'**) by an oxalyl-CoA decarboxylase (**3a**) in the presence of thiamine diphosphate (**3b**) and a magnesium (II) cation to obtain the (S)-α-hydroxyacyl-CoA thioester (**3**) 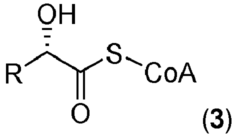 wherein the oxalyl-CoA decarboxylase (**3a**) is a peptide encoded by a gene selected from *Methylobacterium extorquens* or a mutant thereof, wherein a molar ratio of the thiamine diphosphate (**3b**) compared to R-CHO (**1**) is 0.001 to 0.01 with a concentration in the range of 0.05 mM to 0.5 mM, wherein a concentration of the oxalyl-CoA decarboxylase is in the range of 1 to 25 µM and/or a concentration of the magnesium (II) cation is in the range of 2.5 mM to 20 mM, and wherein the mutant preferably is selected from the group comprises or consists of OXC_{Me-Y497A} having the sequence SEQ ID NO. 2, OXC_{Me-S568A} having the sequence SEQ ID NO. 3 or OXC_{Me-Y497A-S568A} having the sequence SEQ ID NO. 4; and
**C**) hydrolysing said *(S)*-α-hydroxyacyl-CoA thioester (**3**) by a hydrolase to obtain the compound of the formula (**II**) or a salt thereof 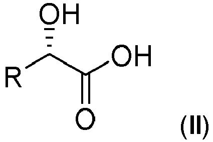 wherein the hydrolase is acyl-CoA thioester hydrolase YciA (**4**) encoded by a gene selected from *E. coli*, and wherein a concentration of the acyl-CoA thioester hydrolase YciA (**4**) is in the range of 0.25 to 5 µM (2 µM); and
   wherein steps **A'**), **A**), **B**), and **C**) are carried out as cascade reaction in one-pot system, and wherein a ratio of R-CHO (**1**) and the free coenzyme A (**2b**) is 10 to 1000.

### Description of the figures:

- Figure 1: shows the reaction of an aldehyde R-CHO with formyl-CoA or oxalyl-CoA to an α-hydroxyacyl-CoA thioester, and a possible subsequent formation of an α-hydroxy carboxylic acid or an α-hydroxy carboxylate.
- Figure 2: shows the hydrolysis of an α-hydroxyacyl-CoA thioester to the α-hydroxy carboxylate (first reaction), and the transfer of the coenzyme A moiety from the α-hydroxyacyl-CoA thioester to a formate (second reaction) resulting in the formation of an α-hydroxy carboxylate and a formyl-CoA thioester as well as the transfer of the coenzyme A moiety from α-hydroxyacyl-CoA thioester to an oxalate resulting in the formation of an α-hydroxy carboxylate and an oxalyl-CoA thioester.
- Figure 3: shows the catalytic cycle of a one-pot system performed as a cascade reaction using a thioesterase YciA in order to hydrolyse an α-hydroxyacyl-CoA thioester to an α-hydroxy carboxylate.
- Figure 4: shows the generation of oxalyl-CoA thioester by a transfer of the coenzyme A moiety from formyl-CoA thioester by means of a formyl-CoA:oxalate CoA transferase (FRC).
- Figure 5: shows the catalytic cycle of a one-pot system performed as a cascade reaction using a thioesterase YciA in order to hydrolyse an α-hydroxyacyl-CoA thioester to an α-hydroxy carboxylate, wherein a formyl-CoA thioester is transferred to a oxalyl-CoA thioester by means of a formyl-CoA:oxalate CoA-transferase (FRC).
- Figure 6: shows the catalytic cycle of a one-pot system performed as a cascade reaction using a transferase in order to transfer the coenzyme A moiety from an α-hydroxyacyl-CoA thioester to an oxalate resulting in the formation of an α-hydroxy carboxylate and oxalyl-CoA thioester.
- Figure 7: shows the catalytic cycle of a one-pot system performed as a cascade reaction using a transferase in order to transfer the coenzyme A moiety from an α-hydroxyacyl-CoA thioester to a formate resulting in the formation of an α-hydroxy carboxylate and formyl-CoA thioester.
- Figure 8: shows the peptide sequence SEQ ID No. 1 of oxalyl-CoA decarboxylase encoded by a gene selected from *Methylobacterium extorquens* (OXC_{Me}).
- Figure 9: shows the peptide sequence SEQ ID No. 2 (OXC_{Me-Y497A}) of a mutation of oxalyl-CoA decarboxylase encoded by a gene selected from *Methylobacterium extorquens.*
- Figure 10: shows the peptide sequence SEQ ID No. 3 (OXC_{Me-S568A}) of a mutation of oxalyl-CoA decarboxylase encoded by a gene selected from *Methylobacterium extorquens.*
- Figure 11: shows the peptide sequence SEQ ID No. 4 (OXC_{Me-Y497A-S568A}) of a mutation of oxalyl-CoA decarboxylase encoded by a gene selected from *Methylobacterium extorquens.*

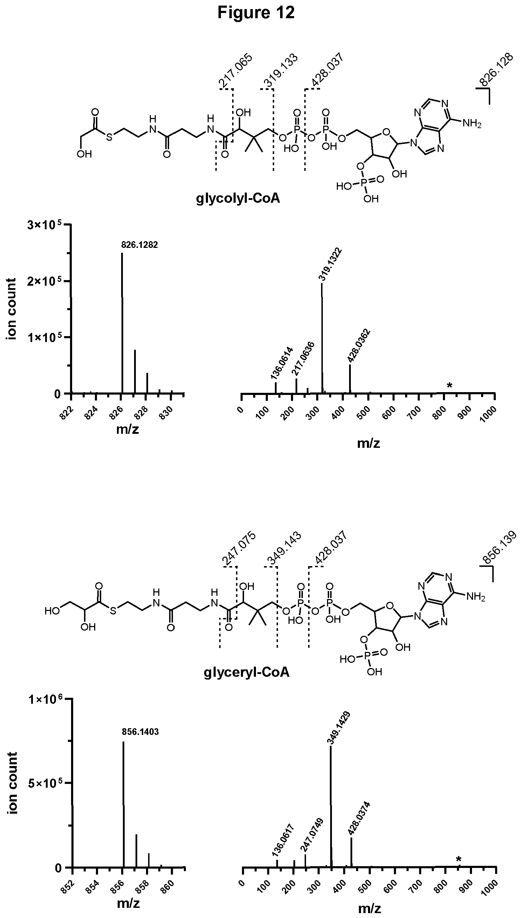

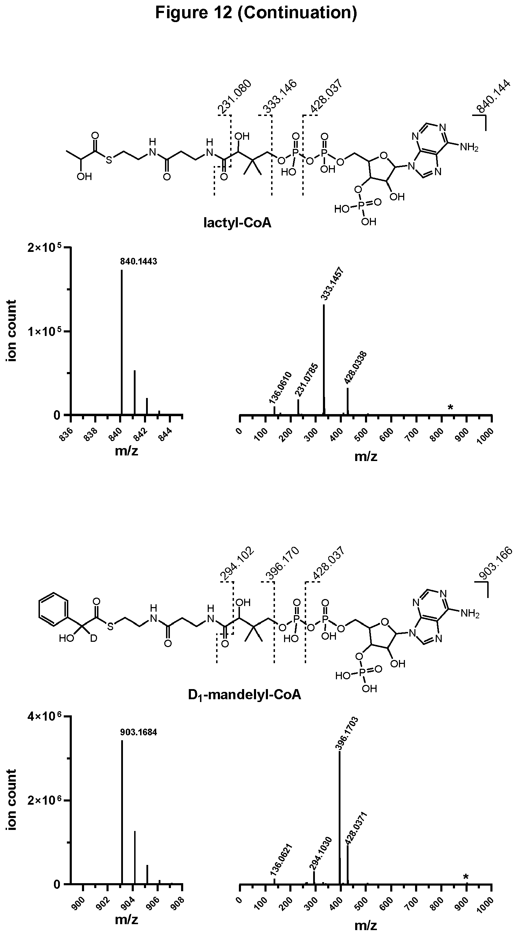

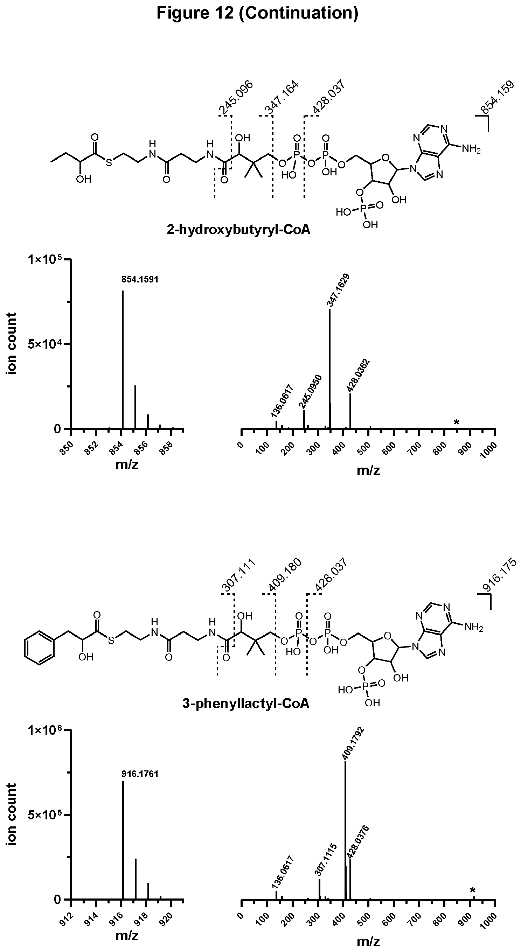

- Figure 12: shows the MS/MS spectra of the α-hydroxyacyl-CoA thioester products. Calculated *m*/*z* values of the fragmentation products are indicated on top. Spectra on the left show the parent ion. Spectra on the right show the fragmentation products of the parent ion (asterisk). The numbers in the spectrum indicate measured *m*/*z* values. Mandelyl-CoA was produced from deuterated benzaldehyde; the deuterium is retained on the α-carbon in the product.
- Figure 13: shows Michaelis-Menten graphs of OXC_{Me} and mutants thereof according to example 6. Error bars show standard deviation of three replicates.
- Figure 14: shows competing reaction pathways of OXC. Assays were carried out at 30 °C in reaction buffer consisting of 50 mM TES-KOH pH 6.8, 10 mM MgCl₂. 0.5 mM ADP, 0.15 mM ThDP and 25 mM benzaldehyde, 1 µM OXC_{Me} or 1 µM OXC_{Me-Y497A}. The reaction was started by adding either 1 mM formyl-CoA (**A**) or 1 mM oxalyl-CoA (**B**). Samples were taken after 0, 2, 5, 10 and 20 minutes and analyzed with the LC-MS detection of CoA esters. CoA esters were quantified by comparison to synthetic standards of formyl-CoA, oxalyl-CoA and mandelyl-CoA, respectively. Concentration of CoA-esters was determined by comparison to a standard curve obtained from chemically synthesized formyl-CoA, oxalyl-CoA and mandelyl-CoA, respectively. Error bars show standard deviation of three replicates. **C**, apparent turnover numbers were determined by linear regression of the mandelyl-CoA formation rate over 20 min. wt = wild type = OXC_{Me}; Y497A = OXC_{Me-Y497A}.
- Figure 15: shows the screen for mandelyl-CoA Thioesterase activity. CoA formation was detected with the Ellman's reagent (5,5'-dithiobis-(2-nitrobenzoic acid)), which reacts with free thiols under release of 2-nitro-5-thiobenzoate (ε₄₁₂ₙₘ = 14,150 M⁻¹ cm⁻¹). Assays were carried out at 30 °C in 50 mM MES-KOH pH 6.8 and contained 1 mM Ellman's reagent and 0.5 µM YciA or 0.5 µM TesB or 0.5 µM Paal. The reaction was started by adding 0.1 mM mandelyl-CoA (time point indicated with the arrow).

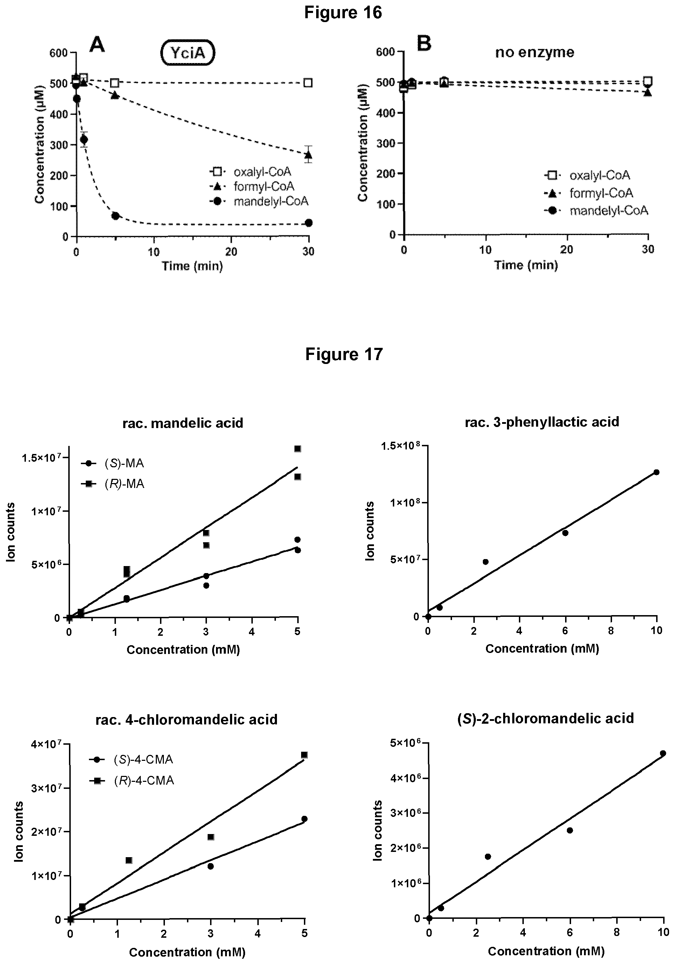

- Figure 16: shows the thioesterase activity of YciA towards oxalyl-CoA, formyl-CoA and racemic mandelyl-CoA. Assays were carried out at 30 °C in reaction buffer consisting of 50 mM TES-KOH pH 6.8, 10 mM MgCl₂. 0.5 mM ADP, 0.15 mM ThDP and 25 mM benzaldehyde, 2 µM YciA (A) or no YciA (B). The reaction was started by adding either 0.5 mM formyl-CoA or 0.5 mM oxalyl-CoA or 0.5 mM mandelyl-CoA. Samples were taken after 0, 1, 5 and 30 minutes and analyzed with the LC-MS detection of CoA esters. CoA esters were quantified by comparison to synthetic standards of formyl-CoA, oxalyl-CoA and mandelyl-CoA, respectively. Error bars show standard deviation of two.
- Figure 17: shows calibration curves of commercially obtained rac. mandelic acid, rac. 3-phenyllactic acid, rac. 4-chloromandelic acid and (S)-2-chloromandelic acid. The lines show linear fit to the data.
- Figure 18: shows Michaelis-Menten graph of OXS with oxalate as substrate according to example 5. Oxalyl-CoA production was followed by coupling OXS to purified PanE2, an NADPH-dependent oxalyl-CoA reductase from *M. extorquens.* An assay containing 50 mM potassium phosphate pH 6.5, 0.3 mM NADPH, 10 mM MgCl₂, 1 mM ATP, 2.5 mM CoA, 1.2 µM PanE2, 176 nM OXS was preincubated for 2 min and the reaction started by adding disodium oxalate to a final concentration of 5, 10, 25, 100, 250, 1,000 µM, respectively. Reaction procedure was monitored by following the oxidation of NADPH at 340 nm. Error bars show standard deviation of three replicates.
- Figure 19: shows mandelic acid formation of the cascade over time. *ee* of (*S*)-mandelic acid is indicated for the last time point (22 h) according to example 13. Mandelic acid formation of the OXS-OXC-YciA cascade over time. Enantiomeric excess of (S)-mandelic acid is indicated for the last time point (22 h). Assays were carried out at 30 °C in reaction buffer consisting of 50 mM TES-KOH pH 6.8, 10 mM MgCl₂. 0.5 mM ADP, 0.15 mM ThDP, 0.5 mM CoA, 10 mM ATP, 25 mM benzaldehyde, 5 µM OXS, 5 µM OXC and 2 µM YciA. The reaction was started by adding 10 mM oxalate. As negative controls, YciA, OXS or OXC were omitted in separate reactions. Samples were taken after 0, 0.05, 0.25, 0.66, 1, 2, 3, 22 hours and analyzed with the LC-MS detection of mandelic acid derivatives. Mandelic acid was quantified by comparison to commercially obtained standards of mandelic acid.

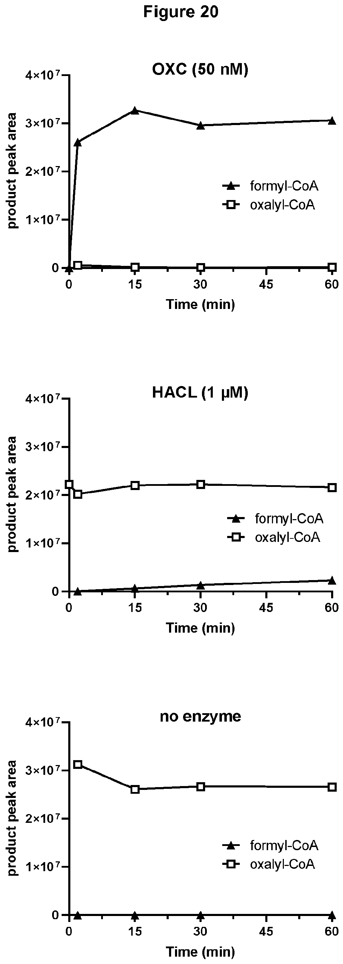

- Figure 20: shows the oxalyl-CoA decarboxylation activity of OXC_{Me} and HACL_{Hs}. Assays were carried out at 30 °C in 100 mM MES-KOH pH 6.8 and contained 10 mM MgCl₂. 0.5 mM ADP, 0.15 mM ThDP, 0.05 µM OXC_{Me} or 1 µM HACL_{Hs} or no enzyme. The reaction was started by adding 0.5 mM oxalyl-CoA. Samples were taken after 1, 15, 30, 60 min and analyzed with the LC-MS detection of CoA esters.
- Figure 21: shows the peptide sequence of oxalyl-CoA synthetase OXS encoded by a gene selected by *Methylobacterium extorquens* (SEQ ID NO. 5).
- Figure 22: shows the peptide sequence of YciA by a gene selected from *E. coli* (SEQ ID NO.6).
- Figure 23: shows the amino acid sequence SEQ ID NO. 18 of acyl-CoA synthetase (eryACS-1) from *Erythrobacter* sp. NAP1 used as a formyl-CoA synthetase.
- Figure 24: shows the amino acid sequence SEQ ID NO. 19 of a mutant of acyl-CoA synthetase used as a formyl-CoA synthetase.
- Figure 25: shows the nucleotide sequence of eryACS-1 (ENA|EAQ27819|EAQ27819.1acetyl-CoA synthetase from *Erythrobacter* sp. NAP1 a; herein SEQ ID NO. 20).

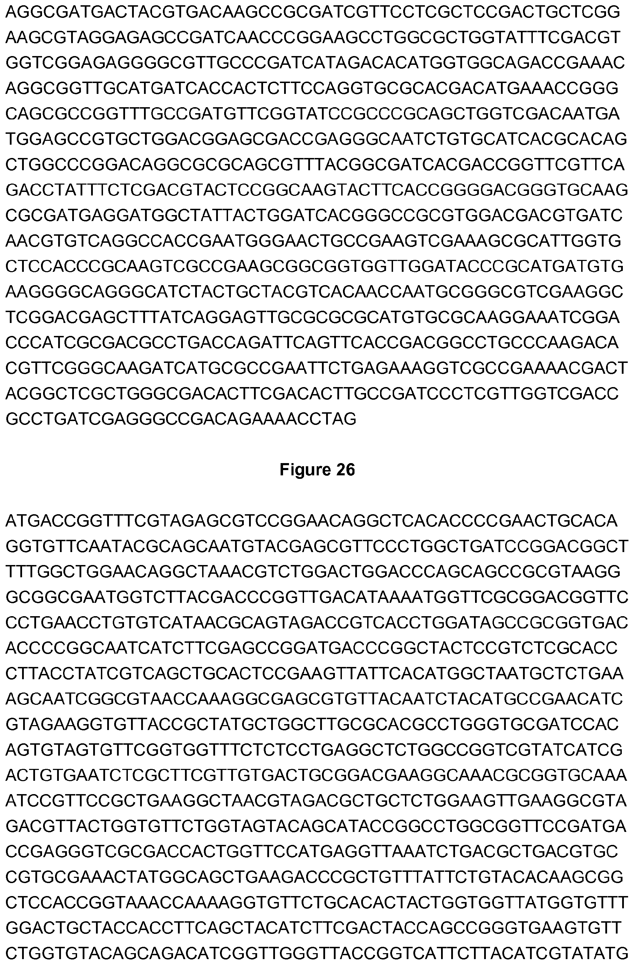

- Figure 26: shows the nucleotide sequence of eryACS-1 (ENA|EAQ27819|EAQ27819.1 acetyl-CoA synthetase from *Erythrobacter* sp. NAP1; herein SEQ ID NO. 21) being codon-optimized for *E. coli.*

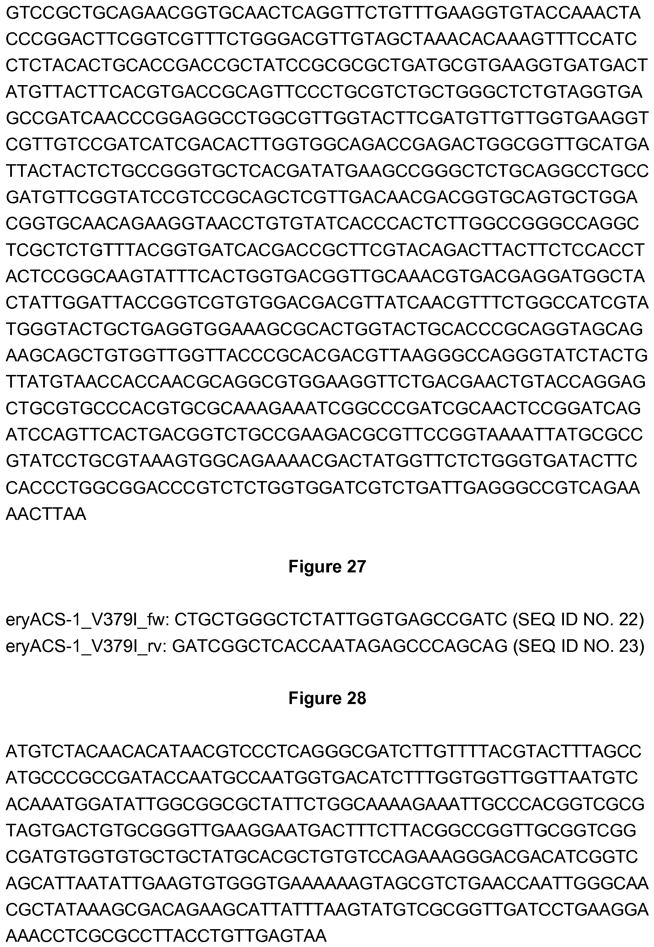

- Figure 27: shows the primers used for site directed mutagenesis of eryACS-1 (from *Erythrobacter* sp. NAP1)
- Figure 28: shows the DNA sequence SEQ ID NO. 25 of the thioesterase YciA encoded by a gene selected from *E. coli.*

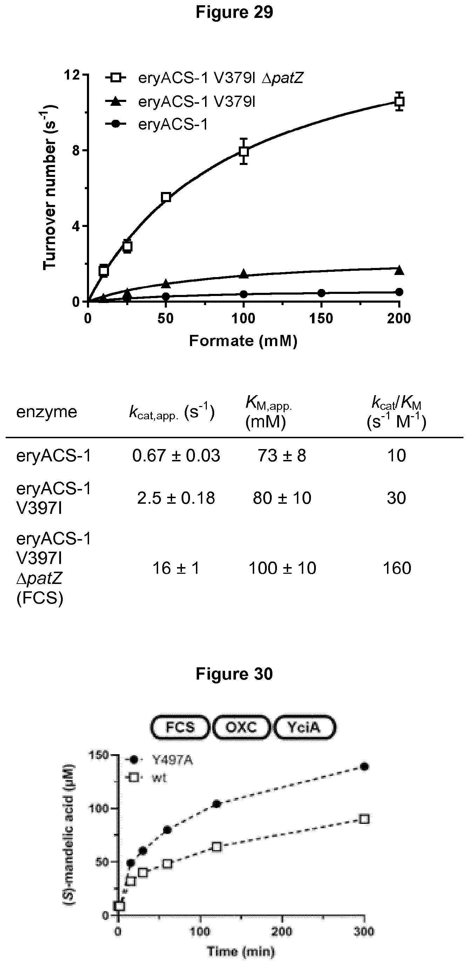

- Figure 29: shows Michaelis-Menten graph of eryACS-1 and variants thereof with formate as substrate according to example 6. AMP production was followed at 30 °C by coupling eryACS-1 to purified adenylate kinase (ADK), pyruvate kinase (PK) and lactate dehydrogenase (LDH). An assay containing 200 mM MOPS-KOH pH 7.8, 0.3 mM NADH, 10 mM MgCl₂, 2 mM ATP, 0.5 mM CoA, 2.5 mM PEP, 0.2 mg/mL ADK, 15 U/mL PK from rabbit muscle (Sigma), 23 U/mL LDH from rabbit muscle (Sigma), and an appropriate concentration of eryACS-1 was preincubated for 2 min and the reaction started by adding sodium formate to a final concentration of 10, 25, 50, 100, 150, 200 mM, respectively. Reaction procedure was monitored by following the oxidation of NADH at 340 nm. Error bars show standard deviation of three replicates. eryACS-1 V379I = eryACS-1, wherein at position 379 valine is replaced by isoleucine. eryACS-1 V379I Δpatz = eryACS-1, wherein at position 379 valine is replaced by isoleucine, and the enzyme were expressed by an *E. coli* strain lacking lysine acetyltransferase *patZ.*
- Figure 30: shows (S)-mandelic acid formation of the cascade over time. (*S*)-Mandelic acid formation of the FCS-OXC-YciA cascade over time. Assays were carried out at 30 °C in reaction buffer consisting of 50 mM TES-KOH pH 6.8, 10 mM MgCl₂, 0.5 mM ADP, 0.15 mM ThDP, 0.5 mM CoA, 5 mM ATP, 25 mM benzaldehyde, 1 µM FCS, 2 µM OXC and 2 µM YciA. The reaction was started by adding 50 mM sodium formate. Samples were taken after 0, 2, 15, 30, 60, 120, 300 min and analyzed with the LC-MS detection of mandelic acid derivatives. Mandelic acid was quantified by comparison to commercially obtained standards of mandelic acid.

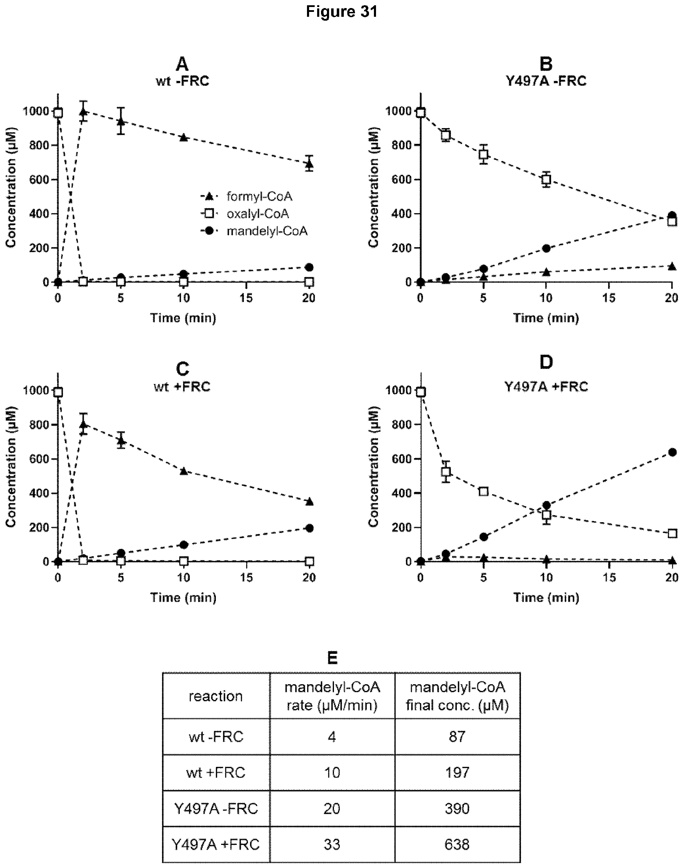

- Figure 31: shows formyl-CoA 'proofreading' by FRC. FRC converts formyl-CoA and oxalate into formate and oxalyl-CoA. Because mandelyl-CoA formation rate proceeds faster with oxalyl-CoA than with formyl-CoA as C₁ donor, addition of FRC results in increased mandelyl-CoA formation rate. Assays were carried out at 30 °C in reaction buffer consisting of 50 mM TES-KOH pH 6.8, 10 mM MgCl₂, 0.5 mM ADP, 0.15 mM ThDP and 25 mM benzaldehyde, 1 µM OXC_{Me} (**A,C**) or 1 µM OXC_{Me-Y497A} (**B,D**). In **B** and **D** the reaction additionally contained 20 µM FRC. The reaction was started by adding 1 mM oxalyl-CoA. Samples were taken after 0, 2, 5, 10 and 20 minutes and analyzed with the LC-MS detection of CoA esters. CoA esters were quantified by comparison to synthetic standards of formyl-CoA, oxalyl-CoA and mandelyl-CoA, respectively. Error bars show standard deviation of three replicates. **E**, mandelyl-CoA formation rates were determined by linear regression (slope of mandelyl-CoA in the graphs). Final concentration refers to the last time point (20 minutes). wt = wild type = OXC_{Me}; Y497A = OXC_{Me-Y497A}.

### EXAMPLES

### Abbreviations

- LC-MS: liquid chromatography mass spectrometry
- rcf: relative centrifugal force
- HPLC: high performance liquid chromatography
- UPLC: ultra performance liquid chromatography
- TOF: time of flight
- ESI: electron spray ionization
- HEPES: 2-[4-(2-hydroxyethyl)piperazin-1-yl]ethanesulfonic acid
- MES: 2-(N-morpholino)ethanesulfonic acid
- MOPS: 3-Morpholinopropane-1-sulfonic acid
- TES: 2-[[1,3-dihydroxy-2-(hydroxymethyl)propan-2-yl]amino]ethanesulfonic acid
- ThDP: thiamine diphosphate
- UV: ultraviolet
- CoA: coenzyme A
- ATP: adenosine triphosphate
- dNTP: deoxy nucleoside triphosphate
- NADH: nicotinamide adenine dinucleotide
- NADPH: nicotinamide adenine dinucleotide phosphate
- CDI: carbonyldiimidazole
- THF: tetrahydrofuran
- ACN: acetonitrile
- DMF: dimethylformamide
- EG: ethylene glycol
- DMSO: dimethyl sulfoxide
- FCS: formyl-CoA synthetase
- OXS: oxalyl-CoA synthetase
- OXC: oxalyl-CoA decarboxylase
- OXC_{Me}: oxalyl-CoA decarboxylase from *Methylobacterium extorquens*
- HACL: *hydroxyacyl-CoA lyases*
- HACL_{Hs}: human *hydroxyacyl-CoA lyases*
- ADK: adenylate kinase
- LDH: lactate dehydrogenase
- PK: pyruvate kinase
- NdeI: endonuclease isolated from *Neisseria denitrificans*
- BamHI: endonuclease isolated from *Bacillus amyloliquefaciens*
- DpnI: endonuclease isolated from *Diplococcus pneumoniae*
- PCR: polymerase chain reaction
- LB: lysogeny broth
- TB: terrific broth
- IPTG: Isopropyl β-D-1-thiogalactopyranoside
- OD₆₀₀: optical density at 600 nm
- FPLC: fast protein liquid chromatography
- SDS-PAGE: sodium dodecyl sulfate-polyacrylamide gel electrophoresis

### Material and methods

### Chemicals

Unless stated otherwise, standard laboratory reagents were obtained from Sigma-Aldrich® (Steinheim, Germany) or Carl Roth GmbH & Co. KG (Karlsruhe, Germany) with the highest purity available. Propionaldehyde, Vanillin, (*R*)-mandelic acid were *(S)*-mandelic acid were obtained from Tokyo Chemical Industry (Zwijndrecht, Belgium). 4-chloromandelic acid was obtained from Alfa Aesar (ThermoFisher (Kandel) GmbH; Kandel, Germany).

### Method for detection of α-hydroxyacl-CoA thioester (that is the method described in LC-MS detection of CoA esters) or α-hydroxy carboxylic acids (that is the method described in LC-MS detection of mandelic acid derivatives).

LC-MS analyses was used to investigate the formation of the product and side products.

### LC-MS analyses

Samples were prepared for LC-MS analysis by quenching an aliquot of a reaction with formic acid (final concentration 4%) and centrifuging for 10 min at 17,000 rcf, to remove precipitated proteins. LC-MS data were analyzed and quantified using MassHunter Qualitative Navigator and Quantitative Analysis software (Agilent, Waldbronn, Germany).

### LC-MS detection of CoA esters

Samples were diluted 1:10 in H₂O. UPLC-high resolution MS of CoA esters was performed as described previously.^{[5]} CoA esters were analyzed using an Agilent 6550 iFunnel Q-TOF LC-MS system equipped with an electrospray ionization source set to positive ionization mode. Compounds were separated on a RP-18 column (50 mm x 2.1 mm, particle size 1.7 µm, Kinetex EVO C18, Phenomenex) using a mobile phase system comprised of 50 mM ammonium formate pH 8.1 (A) and methanol (B). Chromatographic separation was carried out using the following gradient condition at a flow rate of 250 µl/min: 0 min 2.5% B; 2.5 min 2.5% B; 8 min 23% B; 10 min 80 %B; 11 min 80%; 12 min 2.5% B; 12.5 min 0% B. The column oven was set to 40 °C and autosampler was maintained at 10 °C. Standard injection volume was 1 µl. Capillary voltage was set at 3.5 kV and nitrogen gas was used as nebulizing (20 psig), drying (13 l/min, 225 °C) and sheath gas (12 l/min, 400°C). The time of flight (kindly confirm) (TOF) was calibrated using an ESI-L Low Concentration Tuning Mix (Agilent) before measurement (residuals less than 2 ppm for five reference ions) and was recalibrated during a run using 922.0908 *m*/*z* as reference mass . The scan range for MS and MS/MS data is 100-1000 *m*/*z* and 50-1000 *m*/*z* respectively. Collision energy used for MS/MS fragmentation was 35 eV.

The MS/MS spectra are depicted in figure 12.

### LC-MS detection of mandelic acid derivatives

Samples were diluted 1:10 in H₂O. UPLC-high resolution MS analyses were performed on an Agilent 6550 iFunnel QTOF LC/MS system equipped with an electrospray ionization source to negative ionization mode. The analytes were isocratically chromatographed on a chiral column (100 mm x 2.1 mm, particle size 2.7 µm, Poroshell 120 Chiral-T, Agilent) kept at ambient tempature using a mobile phase system comprised of 30:70 20 mM ammonium formate pH 4 / methanol at a flow rate of 250 µl/min for 10 min. Samples were held at 10°C and injection volume was 1 µl. Capillary voltage was set at 3.5 kV and nitrogen gas was used as nebulizing (20 psig), drying (13 l/min, 225 °C) and sheath gas (12 l/min, 40°C). MS data were acquired with a scan range of 100-1100 *m*/*z.* For quantification the calculated *m*/*z* value of [M-H]⁻ was used to obtain the extracted ion count from the total ion count.

### Enzyme assays with LC-MS detection

Unless noted otherwise, all LC-MS assays were carried out at 30 °C in reaction buffer consisting of 50 mM TES-KOH pH 6.8, 10 mM MgCl₂. 0.5 mM ADP, 0.15 mM ThDP.

### Kinetic investigations

### Spectrophotometric enzyme assays

Assays were performed on a Cary-60 UV/Vis spectrophotometer (Agilent) at 30°C using quartz cuvettes (10 mm path length; Hellma, Müllheim, Germany). For the determination of steady-state kinetic parameters, each concentration was measured in triplicates and the obtained curves were fit using GraphPad Prism 7. Hyperbolic curves were fit to the Michaelis-Menten equation to obtain apparent *k*_{cat} and *K*_{M} values.

### Synthesis of the starting materials and reference samples

### Example 1 - Formyl-CoA synthesis

Formyl-CoA was synthesized as described previously (W. S. Sly, E. R. Stadtman, J. Biol. Chem. 1963, 238, 2632-2638; b) S. Jonsson, S. Ricagno, Y. Lindqvist, N. G. Richards, J. Biol. Chem. 2004, 279, 36003-36012.). After Extraction with diethylether formyl-CoA was purified by preparative HPLC-MS with an acetonitrile gradient in 25 mM ammonium formate pH 4.2. The fractions containing the product were lyophilized and stored at -20 °C. Formyl-CoA was dissolved in aq. HCl (pH 4). The concentration was determined by enzymatic depletion with PduP (J. Zarzycki, M. Sutter, N. S. Cortina, T. J. Erb, C. A. Kerfeld, Sci. Rep. 2017, 7, 42757.), following NADH consumption at 340 nm.

### Example 2 - Oxalyl-CoA synthesis

Oxalyl-CoA was synthesized enzymatically with OXS. A 5 mL reaction containing 50 mg mM CoA (0.064 mmol, 1 eq.), 52 mg ATP (0.086 mmol, 1.3 eq.), 10 mg disodium oxalate (0.075 mmol, 1.2 eq.) in buffer (100 mM MES-KOH pH 6.8, 15 mM MgCl₂) was started by adding OXS to a final concentration of 0.4 mg/mL and incubated at 30 °C for 1 hour. The reaction was quenched with 250 µL formic acid and the enzyme removed by centrifugation (4,000 × g, 4 °C, 10 min). Oxalyl-CoA was purified by preparative HPLC-MS with an acetonitrile gradient in 25 mM ammonium formate pH 4.2. The fractions containing the product were lyophilized and stored at -20 °C. Oxalyl-CoA was dissolved in 10 mM acetate buffer pH 4.5. The concentration was determined spectrophotometrically, using the extinction coefficient for saturated CoA esters (ε₂₆₀ₙₘ = 16.4 cm⁻¹ mM⁻¹).

### Example 3 - Mandelyl-CoA synthesis (Reference sample)

Mandelyl-CoA and *(S)*-mandelyl-CoA were synthesized chemically with the carbonyldiimidazole (CDI) CoA-acylation method described previously (D. M. Peter, B. Vögeli, N. S. Cortina, T. J. Erb, Molecules 2016, 21, 517.). 21 mg CDI (0.127 mmol, 4 eq.) was dissolved in 1 mL tetrahydrofuran, mandelic acid or (*S*)-mandelic acid was added (0.127 mmol, 4 eq.) and the mixture stirred at 22 °C for 15 min. 25 mg CoA (0.032 mmol, 1 eq.) was dissolved in 1 mL 0.5 M NaHCO₃ and added to the reaction mixture, followed by stirring at 22 °C for 30 min. THF was removed by applying vacuum (100 mbar) for 5 min. The mixture was then purified by preparative HPLC with a methanol gradient in 25 mM ammonium formate pH 8.0. The fractions containing the product were lyophilized and stored at -20 °C. Mandelyl-CoA was dissolved in 10 mM acetate buffer pH 4.5. The concentration was determined spectrophotometrically, using the extinction coefficient for saturated CoA esters (ε₂₆₀ₙₘ = 16.4 cm⁻¹ mM⁻¹).

### Example 4 - Synthesis of the peptides

### Example 4a - Cloning and Mutagenesis

Oligonucleotides were obtained from Eurofins Genomics (Ebersbach, Germany). oxc (MexAM1_META1 p0990), oxs (MexAM1_META1p2130), and *panE2* (MexAM1_META1p3141) were PCR-amplified from *M. extorquens* chromosomal DNA using the corresponding primers (Table 1). The purified PCR products were digested with Ndel and BamHI and ligated into pET-16b. Correct cloning was confirmed by sequencing (Eurofins Genomics).

The gene encoding eryACS-1 (GenBank: EAQ27819.1) was obtained by gene synthesis performed by the Joint Genome Institute (Walnut Creek, CA, USA). The gene was codon optimized for *E. coli and* sub-cloned into pSEVA141.

Human *Hacl1* (GenBank: CAB60200.1) was obtained by gene synthesis (Table 2), performed by BaseClear (Leiden, The Netherlands). The gene was codon optimized for *E. coli and* sub-cloned into pET-16b.

*yciA* and *adk* were obtained from the ASKA collection ([M. Kitagawa, T. Ara, M. Arifuzzaman, T. loka-Nakamichi, E. Inamoto, H. Toyonaga, H. Mori, DNA Res. 2005, 12, 291-299).

Point mutations were introduced into *oxc* by PCR using mismatch primers (Table 1). A 50 µL reaction contained 60 ng of pET-16b_OXC_{Me}, 0.25 µM forward and reverse primer, 200 µM dNTP, 5 µL 10x reaction buffer, 1 µL Phusion polymerase (2 U/µL). Template plasmid was removed by DpnI digest (10 U) at 37 °C immediately after PCR amplification. Mutations were confirmed by sequencing.

**Table 1: Primers used for cloning of OXC and OXS and site-directed mutagenesis of OXC.**

| **Primer name** | **Nucleotide sequence (5' to 3') restriction sites or mismatches are underlined)** |
|---|---|
| oxc_fw_NdeI | GTTCACATATGACCGTCCAGGCCCAG (SEQ ID NO. 7) |
| oxc_rv_BamHI | CGCTGGATCCTCACTTCTTCTTCAAGGTGCTC (SEQ ID NO. 8) |
| oxs_fw_NdeI | GTTCACATATGACGATGCTTCTGCC (SEQ ID NO. 9) |
| oxs_rv_BamHI | CAATGGATCCTCAGACCAGCCCGAG (SEQ ID NO. 10) |
| panE2_fw_Ndel | GCGCACATATGAGCATCGCGATCGTCG (SEQ ID NO. 11) |
| panE2_rv_BamHI | CAGAGGATCCTCATGCTCCCTGGATCGC (SEQ ID NO. 12) |
| oxc_Y497A_fw | CAACAACGGCATCGCTCGCGGCACCGAC (SEQ ID NO. 13) |
| oxc_Y497A_rv | GTCGGTGCCGCGAGCGATGCCGTTGTTG (SEQ ID NO. 14) |
| oxc_S568A_fw | CCGGCAGCGAGGCCGGCAATATCGG (SEQ ID NO. 15) |
| oxc_S568A_rv | CCGATATTGCCGGCCTCGCTGCCGG (SEQ ID NO. 16) |

**Table 2: Synthetic gene sequences**

| Gene name | DNA sequence |
|---|---|
| *hacl1* (GenBank: CAB60200) (SEQ ID NO. 24) | ATGCCGGACAGTAACTTCGCAGAGCGCAGCGAGGAGCAGG TGTCTGGTGCTAAAGTCATCGCTCAGGCCCTGAAAACGCAA GATGTGGAGTACATATTTGGCATCGTAGGCATCCCAGTGAC CGAAATCGCCATTGCTGCCCAGCAGCTAGGCATCAAGTACA TCGGGATGAGGAATGAGCAAGCGGCTTGTTATGCTGCCTCC GCGATTGGATATCTGACAAGCAGGCCAGGAGTCTGCCTTGT TGTTTCTGGCCCAGGTCTCATCCATGCCTTGGGCGGTATGG CAAATGCAAACATGAACTGCTGGCCCTTGCTTGTGATTGGTG GTTCCTCTGAAAGAAACCAAGAAACAATGGGAGCTTTCCAGG AGTTTCCTCAGGTTGAAGCTTGTAGATTATATACCAAGTTCTC TGCCCGCYCAAGCAGCATAGAAGCTATTCCTTTTGTTATTGA AAAGGCAGTGAGAAGCAGTATCTATGGTCGTCCAGGTGCTT GCTATGTTGACATACCAGCAGATTTTGTGAACCTTCAGGTGA ATGTGAATTCTATAAAGTACATGGAACGCTGCATGTCACCTC CTATTAGCATGGCAGAAACCTCTGCTGTGTGCACGGCGGCT TCTGTTATTAGGAATGCCAAACAACCCCTTCTTATCATCGGG AAAGGTGCTGCTTACGCTCATGCAGAAGAGAGTATCAAGAAA TTGGTGGAGCAATATAAACTGCCATTTTTGCCCACCCCTATG GGAAAGGGTGTTGTCCCTGACAACCATCCATACTGTGTAGG TGCAGCCAGATCCAGGGCTTTGCAATTTGCTGATGTAATTGT GTTATTTGGTGCCAGACTAAATTGGATTTTACATTTTGGACTG CCTCCAAGATATCAGCCAGATGTGAAGTTTATCCAGGTTGAT |
| | ATCTGTGCAGAAGAATTGGGGAATAATGTAAAGCCCGCTGTT ACTTTGCTAGGAAACATACATGCTGTCACTAAGCAGCTTTTA GAGGAACTTGATAAAACACCATGGCAGTATCCTCCAGAGAG CAAGTGGTGGAAAACTCTGAGAGAAAAAATGAAGAGCAATG AAGCTGCATCCAAGGAACTAGCTTCTAAAAATCCCTGCCTA TGAATTATTACACAGTATTCTACCATGTTCAAGAACAACTACC TAGAGACTGTTTCGTGGTAAGTGAAGGAGCAAATACTATGGA CATTGGACGGACTGTGCTTCAGAACTACCTTCCTCGTCACAG GCTTGATGCTGGTACTTTCGGAACAATGGGAGTTGGTTTGG GATTTGCTATTGCAGCTGCCGTGGTGGCTAAAGATAGAAGC CCTGGGCATTGGATCATCTGTGTGGAAGGAGACAGTGCATT TGGGTTTTCTGGCATGGAGGTAGAAACCATCTGCAGGTACA ACTTGCCAATCATACTGTTGGTAGTGAATAACAATGGAATTTA CCAAGGTTTTGATACAGATACTTGGAAAGAAATGTTAAAATTT CAAGATGCTACTGCAGTGGTCCCTCCAATGTGTTTGCTGCCA AATTCACATTATGAGCAAGTCATGACTGCATTTGGAGGCAAA GGGTATTTTGTACAAACACCAGAAGAACTCCAAAAATCCCTG GAGCAGAGCCTAGCAGACACAACTAAACCTTCTCTTATCAAC ATCATGATTGAGCCACAAGCCACACGGAAGGCCCAGGATTT TCATTGGCTGACCCGCTCTAATATGTAA |
| *hacl1* codon optimized for *E. coli* (SEQ ID NO. 17) | ATGCCGGATTCAAACTTTGCTGAACGTTCAGAAGAACAGGTT TCTGGCGCAAAAGTGATTGCGCAAGCGCTAAAAACCCAGGA TGTCGAATATATCTTCGGTATAGTGGGTATTCCGGTGACGGA AATCGCCATCGCGGCTCAGCAACTCGGTATTAAATACATCGG TATGCGTAATGAGCAGGCGGCTTGTTACGCAGCAAGCGCGA TTGGCTATCTGACCTCAAGACCGGGGGTATGCCTTGTTGTCA GCGGCCCGGGCCTGATTCATGCCCTGGGTGGTATGGCGAA TGCTAACATGAACTGCTGGCCGCTGTTAGTTATAGGCGGCA GCAGTGAACGCAATCAGGAGACCATGGGCGCATTCCAAGAG TTTCCTCAGGTGGAAGCCTGCCGCCTGTATACCAAATTTTCT GCTAGACCTTCGTCAATAGAAGCCATCCCCTTCGTGATTGAA AAAGCGGTCCGTAGTTCTATCTATGGGCGCCCTGGGGCTTG CTATGTTGACATTCCCGCCGACTTCGTAAACCTCCAAGTGAA TGTCAATTCCATTAAATACATGGAACGCTGTATGTCGCCGCC GATCAGTATGGCCGAGACCAGCGCCGTGTGCACAGCGGCG AGTGTTATCCGCAACGCCAAACAGCCATTACTAATAATTGGG AAAGGAGCCGCCTATGCTCACGCTGAAGAGTCCATCAAAAA ACTGGTGGAACAATACAAGTTGCCCTTTTTACCTACTCCCAT |
| | GGGTAAAGGTGTTGTACCTGATAATCATCCATACTGCGTCGG TGCAGCCCGTTCCCGCACTACAGTTCGCTGACGTGATTG TGCTGTTTGGTGCGCGTTTAAACTGGATACTCCACTTCGGTT TACCGCCACGCTACCAGCCGGATGTGAAATTTATACAGGTTG ACATCTGTGCCGAAGAACTGGGCAATAATGTGAAACCAGCA GTCACTTTGCTGGGGAACATCCATGCAGTCACCAAACAACTG CTTGAAGAGCTGGACAAGACGCCGTGGCAATATCCGCCAGA GTCAAAATGGTGGAAGACGCTACGTGAGAAAATGAAGAGCA ACGAAGCGGCTTCGAAAGAGTTGGCCTCCAAAAAGTCATTG CCTATGAATTACTATACCGTCTTTTATCATGTCCAAGAGCAGC TGCCGCGGGATTGCTTTGTCGTGTCGGAGGGCGCCAACACC ATGGATATAGGTCGCACTGTCCTTCAGAACTATCTGCCCCGG CATCGCCTGGACGCTGGTACCTTCGGAACTATGGGCGTAGG GCTGGGCTTTGCTATTGCGGCGGCAGTGGTAGCAAAGGATC GCAGCCCGGGGCAGTGGATTATTTGTGTAGAAGGGGACTCC GCATTTGGATTCTCTGGTATGGAGGTAGAGACAATATGCCGT TATAATTTGCCAATTATCCTTCTAGTGGTGAATAACAATGGTA TTTATCAGGGCTTTGATACAGATACGTGGAAGGAAATGCTGA AATTTCAAGATGCCACAGCCGTGGTACCCCCCATGTGTTTGC TGCCGAACTCTCATTACGAGCAAGTGATGACAGCATTCGGC GGCAAGGGTTACTTTGTGCAGACACCGGAAGAACTTCAAAA GAGCCTTCGCCAGAGCTTGGCGGACACCACGAAACCGAGTC TGATTAATATAATGATTGAACCACAGGCCACAAGGAAGGCAC AAGACTTTCACTGGCTCACGCGTTCAAATATGTAA |

### Example 4b - Protein Production and Purification

All proteins except HACL_{Hs} and FCS were heterologously produced in *E. coli* BL21 (DE3). 500 mL TB containing 100 µg/mL ampicillin (in the case of OXC_{Me}, OXS, PduP, PanE2, eryACS-1) or 34 µg/mL chloramphenicol (in the case of YciA and ADK) was inoculated with freshly-transformed cells and incubated at 37 °C. After reaching an OD₆₀₀ of 0.8, expression was induced by adding IPTG to a final concentration of 0.25 mM and the incubation temperature was lowered to 25 °C. FCS was produced in *E. coli* BL21-Al (ThermoFisher Scientific) in which the peptidyl-lysine N-acetyltransferase gene *patZ* had been replaced by a kanamycin resistance cassette *kanR.* 1L TB containing 100 µg/mL ampicillin and 50 µg/mL kanamycin was inoculated with freshly-transformed cells and incubated at 37 °C. After reaching an OD₆₀₀ of 0.6, L-arabinose was added to a final concentration of 0.2% (w/v) and the incubation temperature was lowered to 25 °C. After 30 minutes, expression was induced by IPTG to a final concentration of 0.5 mM. HACL_{Hs} was produced in *E. coli* ArcticExpress (DE3). 1L LB containing 100 µg/mL ampicillin and 15 µg/mL gentamycin was inoculated with freshly-transformed cells and incubated at 37 °C. After reaching an OD₆₀₀ of 0.8 the culture was cooled on ice for 15 min. Then expression was induced by adding IPTG to a final concentration of 0.1 mM and the incubation temperature was lowered to 15 °C. Cells were harvested after 16 h (24 h for HACL_{Hs}) by centrifugation (4500× g, 10 min) and resuspended in buffer A (500 mM KCI, 50 mM HEPES-KOH pH 7.6). If not used immediately, cell pellets were flash-frozen in liquid nitrogen and stored at -20 °C. The cell lysate obtained by sonication was clarified by centrifugation 75,000× g at 4 °C for 45 min. The supernatant was filtered through a 0.4 µm syringe tip filter (Sarstedt, Nümbrecht, Germany). Ni-affinity purification was performed with an Äkta FPLC system from GE Healthcare (GE Healthcare, Freiburg, Germany). The filtered soluble lysate was loaded onto a 1 mL Ni-Sepharose Fast Flow column (HisTrap FF, GE Healthcare, Little Chalfont, UK) that had been equilibrated with 10 mL buffer A. After washing with 20 mL 85% buffer A, 15% buffer B (500 mM KCI, 50 mM HEPES-KOH pH 7.6, 500 mM imidazole), the protein was eluted with 100% buffer B. Fractions containing purified protein were pooled and the buffer was exchanged to storage buffer (150 mM KCI, 50 mM HEPES-KOH pH 7.6) with a desalting column (HiTrap, GE Healthcare). Proteins were concentrated by ultrafiltration (Amicon Ultra). Concentration was determined on a NanoDrop 2000 Spectrophotometer (Thermo Scientific, Waltham, MA, USA) using the extinction coefficient at 280 nm, as calculated by protparam (https://web.expasy.org/protparam/). Enzyme purity was confirmed by SDS-PAGE. The purified proteins were stored in 50 vol% glycerol at -20 °C. OXC_{Me} wild-type and mutants were flash-frozen in liquid nitrogen and stored at -80 °C.

### Kinetic investigations

### Example 5 - Michaelis-Menten kinetics of OXS

Oxalyl-CoA production was followed by coupling OXS to purified PanE2, an NADPH-dependent oxalyl-CoA reductase from *M. extorquens.* An assay containing 50 mM potassium phosphate pH 6.5, 0.3 mM NADPH, 10 mM MgCl₂, 1 mM ATP, 2.5 mM CoA, 1.2 µM PanE2, 176 nM OXS was preincubated for 2 min and the reaction started by adding disodium oxalate to a final concentration of 5, 10, 25, 100, 250, 1'000 µM, respectively. Reaction procedure was monitored by following the oxidation of NADPH at 340 nm.

Result: The steady-state kinetic parameters are shown in figure 18 (*k*_{cat} = 1.30 ± 0.02 s⁻¹; *K*_{M}(oxalate) = 9 ± 1 µM)

### Example 6 - Michaelis-Menten kinetics OXC_{Me} - Decarboxylation

Formyl-CoA production was followed by coupling OXC to purified PduP, a promiscuous CoA-dependent aldehyde dehydrogenase that reduces formyl-CoA to formaldehyde under NADH consumption. An assay containing 50 mM MES-KOH pH 6.5, 0.3 mM NADH, 10 mM MgCl₂, 0.5 mM ADP, 0.15 mM ThDP, 5 µM PduP, and OXC_{Me} or mutant thereof like OXC_{Me-Y497A}, OXC_{Me-S568A} or OXC_{Me-Y497A-S568A} (concentration depending on the mutant) was preincubated for 2 min and the reaction started by adding oxalyl-CoA (concentrations depending on the mutant). Reaction procedure was monitored by following the oxidation of NADH at 340

The formyl-CoA formed after the decarboxylation of the oxalyl-CoA by ThDP and OXC_{Me} was removed from the reaction mixture in order to investigate the decarboxylation without the influence of the equilibrium.

Results: The results are depicted in the following table 3 and in curve of the kinetic investigations in Figure 13.

**Table 3: Steady-state kinetic parameters of oxalyl-CoA decarboxylation catalyzed by OXCMe.^{[a]}**

| Mutation | *k*_{cat} (s⁻¹) | *K*_{M} (µM) | *k*_{cat}*lK*_{M} (s⁻¹ M⁻¹) |
|---|---|---|---|
| wild-type | 98 ± 3 | 105 ± 11 | 9.3 × 105 |
| Y497A (OXC_{Me-Y497A}) | 1.32 ± 0.04 | 180 ± 17 | 7.3 × 103 |
| S568A (OXC_{Me-S568A}) | 5.53 ± 0.11 | 23 ± 2 | 1.1 × 105 |
| Y497A S568A (OXC_{Me-Y497A-S568A}) | 0.32 ± 0.01 | 103 ± 15 | 3.1 × 103 |

| | | | |
|---|---|---|---|
| ^{[a]}Michaelis-Menten graphs are shown in Figure 13. | | | |

In OXC_{Me} variants OXC_{Me-Y497A} having the sequence SEQ ID NO. 2, and OXC_{Me-}S568A having the sequence SEQ ID NO. 3, formyl-CoA formation was decreased 20 to 50-fold, while the *K*_{M} of both variants was largely unaffected (Table 3 and Figure 13). When starting with oxalyl-CoA and benzaldehyde OXC_{Me-Y497A} showed 5-fold increased mandelyl-CoA production rate (20 min⁻¹; figure 14C), suggesting that activity of the enzyme was successfully redirected towards carboligation by suppressing protonation of thiamine-α-hydroxyl-CoA-adduct (see figure 3).

### Example 7 - OXC_{Me} aldehyde and ketone screen

In reaction buffer, 1 mM formyl-CoA and 10 mM aldehyde (formaldehyde, acetaldehyde, glycolaldehyde, propionaldehyde, glyceraldehyde, glyoxylate, succinic semialdehyde, benzaldehyde, phenylacetaldehyde, and acetone) was mixed with 5 µM OXC_{Me} or HACL_{Hs}. The reaction was stopped after 1 h and products analyzed with the CoA ester method described above.

Result: Glyceraldehyde, glyoxylate and acetone were not accepted by OXC_{Me}, i.e. a carbon-carbon-bond was not formed. The results are listed in table 4:

**Table 4: Comparison of the aldehyde substrate scope of OXC_{Me}**

| | | | | |
|---|---|---|---|---|
| 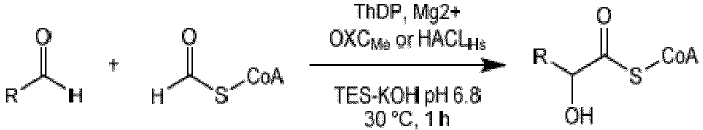 | | | | |

| **Entry** | **R** | **Product name** | **OXCMe^{[c]}** | HACL_{Hs}^{[c]} |
|---|---|---|---|---|
| 1 | H | glycolyl-CoA | 100 | 11 |
| 2 | CH₃ | lactyl-CoA | 74 | 100 |
| 3 | CH₂CH₃ | 2-hydroxybutyryl-CoA | 5 | 100 |
| 4 | CH₂OH | glyceryl-CoA | 1 | 100 |
| 5 | CHOHCH₂OH | erythronyl-CoA | n.d.^{[b]} | n.d.^{[b]} |
| 6 | COOH | tartronyl-CoA | n.d. | n.d.^{[b]} |
| 7 | (CH2)2COOH | 2-hydroxyglutaryl-CoA | 1 | 100 |
| 8 | Ph | mandelyl-CoA | 100 | 3 |
| 9 | CH2Ph | phenyllactyl-CoA | 22 | 100 |

| | | | | |
|---|---|---|---|---|
| [a]The reaction contained 2a-2i (10 mM), formyl-CoA (1 mM), OXC_{Me}. Products were analyzed by LC-MS after 1 h reaction time. [b] Product not detected. [c] Relative activity in %. Relative activity refers to the comparison of OXC and HACL. | | | | |

### Example 8 - Comparison of the Decarboxylation activity of OXC_{Me} and HACL_{Hs}

Oxalyl-CoA decarboxylation activity of OXC_{Me} and HACL_{Hs}. Assays were carried out at 30 °C in 100 mM MES-KOH pH 6.8 and contained 10 mM MgCl₂. 0.5 mM ADP, 0.15 mM ThDP, 0.05 µM OXC_{Me} or 1 µM HACL_{Hs} or no enzyme. The reaction was started by adding 0.5 mM oxalyl-CoA. Samples were taken after 1, 15, 30, 60 min and analyzed with the LC-MS detection of CoA esters.

Results: The results are depicted in figure 20. In the control experiment without an enzyme the concentration of the oxalyl-CoA does not change, and thus formyl-CoA is not formed.

### Example 9 - Comparison OXC wild type and OXC_{Me-Y497A} starting form formyl-CoA or oxalyl-CoA

Assays were carried out at 30 °C in reaction buffer consisting of 50 mM TES-KOH pH 6.8, 10 mM MgCl₂. 0.5 mM ADP, 0.15 mM ThDP and 25 mM benzaldehyde, 1 µM OXC_{Me} or 1 µM OXC_{Me-Y497A}. The reaction was started by adding either 1 mM formyl-CoA or 1 mM oxalyl-CoA. Samples were taken after 0, 2, 5, 10 and 20 minutes and analyzed with the LC-MS detection of CoA esters. CoA esters were quantified by comparison to synthetic standards of formyl-CoA, oxalyl-CoA and mandelyl-CoA, respectively. Concentration of CoA-esters was determined by comparison to a standard curve obtained from chemically synthesized formyl-CoA, oxalyl-CoA and mandelyl-CoA, respectively.

Results: The results are depicted in figure 14A and 14B. Starting from the formyl-CoA the formation is faster by means of OXC_{Me-Y497A} than OXC_{Me} (wild type). When oxalyl-CoA is used as a C₁-source, the mandelyl-CoA is formed much faster in case of the OXC_{Me-Y497A}.

### Example 10 - YciA substrate screen (Selectivity of YciA towards formyl-CoA, oxalyl-CoA and mandelyl-CoA)

Thioesterase activity of YciA towards oxalyl-CoA, formyl-CoA and racemic mandelyl-CoA. Assays were carried out at 30 °C in reaction buffer consisting of 50 mM TES-KOH pH 6.8, 10 mM MgCl₂. 0.5 mM ADP, 0.15 mM ThDP and 25 mM benzaldehyde, 2 µM YciA (**A**) or no YciA (**B**). The reaction was started by adding either 0.5 mM formyl-CoA or 0.5 mM oxalyl-CoA or 0.5 mM mandelyl-CoA. Samples were taken after 0, 1, 5 and 30 minutes and analyzed with the LC-MS detection of CoA esters. CoA esters were quantified by comparison to synthetic standards of formyl-CoA, oxalyl-CoA and mandelyl-CoA, respectively.

Results: The results are depicted in figure 16.

YciA from *E. coli* has shown a relatively high α-hydroxyacyl-CoA in particular mandelyl-CoA thioesterase activity, low activity with formyl-CoA and no activity with oxalyl-CoA.

### Example 11 - Comparison of the activity of the thioesterase activity of YciA, TesB and Paal

Screen for mandelyl-CoA Thioesterase activity. CoA formation was detected with the Ellman's reagent (5,5'-dithiobis-(2-nitrobenzoic acid)), which reacts with free thiols under release of 2-nitro-5-thiobenzoate (ε₄₁₂ₙₘ = 14,150 M⁻¹ cm⁻¹). Assays were carried out at 30 °C in 50 mM MES-KOH pH 6.8 and contained 1 mM Ellman's reagent and 0.5 µM YciA or 0.5 µM TesB or 0.5 µM Paal. The reaction was started by adding 0.1 mM mandelyl-CoA.

Results: The result is depicted in Figure 15. YciA exhibits a higher thioesterase activity than TesB and Paal.

### Example 12 - OXS- OXC_{Me}-YciA cascade prototyping

In a 1.5 mL microfuge tube, reaction buffer containing 50 mM TES-KOH pH 6.8, 10 mM MgCl₂. 0.5 mM ADP, 0.15 mM ThDP, 0.5 mM CoA, 10 mM ATP, 25 mM benzaldehyde, 2 µM YciA, 5 µM OXC_{Me} and 5 µM OXS were mixed and the reaction was initiated by the addition of 10 mM disodium oxalate. For the negative controls each enzyme was omitted in a separate reaction. Samples were taken after 0, 3, 15, 40 min, 1, 2, 3 and 22 h and analyzed with the mandelic acid derivatives method described above. For quantification commercial, racemic mandelic acid was diluted in reaction buffer to appropriate concentrations to obtain a calibration curve

### Example 13 - Comparison of OXC_{Me} with its mutant

Assays were carried out at 30 °C in reaction buffer consisting of 50 mM TES-KOH pH 6.8, 10 mM MgCl₂. 0.5 mM ADP, 0.15 mM ThDP, 0.5 mM CoA, 10 mM ATP, 25 mM benzaldehyde, 5 µM OXS, 5 µM OXC and 2 µM YciA. The reaction was started by adding 10 mM oxalate. As negative controls, YciA, OXS or OXC were omitted in separate reactions. Samples were taken after 0, 0.05, 0.25, 0.66, 1, 2, 3, 22 hours and analyzed with the LC-MS detection of mandelic acid derivatives. Mandelic acid was quantified by comparison to commercially obtained standards of mandelic acid.

Results: The results are depicted in figure 19.

When OXC_{Me} is replaced by the OXC_{Me-Y497A} variant having the sequence SEQ ID NO. 2, mandelic acid production rate increased 5-fold and yield increased 4-fold.

### Example 14 - OXS- OXC_{Me}-YciA cascade aldehyde scope

The aldehyde substrate screen of the OXS-OXC_{Me-Y497A}-YciA cascade was carried out as described above (example 13), except that the aromatic aldehydes were prepared as 33 mM stocks in 20 vol% DMSO and diluted to final concentration of 25 mM into the assay. Samples were analyzed with the mandelic acid derivatives method described above. Mandelic acid (**3a**), 4-chloromandelic acid (**3c**), 2-chloromandelic acid (**3d**) and 3-phenyllactic acid (**3b**) were quantified by comparison to commercial standards.

The results are depicted in the following table 5.

**Table 5: Scope of the OXS-OXC_{Me-Y497A}-YciA cascade for the synthesis of chiral α-hydroxy acids.^{[a]}**

| | | | | |
|---|---|---|---|---|
| 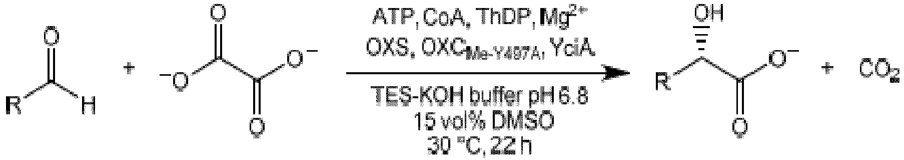 | | | | |

| **Entry** | **R** | **Product name** | **Conversion [%]^{[b]}** | **ee [%]^{[b]}** |
|---|---|---|---|---|
| 1 | Ph | Mandelic acid | 75 | 99 |
| 2 | CH₂Ph | 3-phenyllactic acid | 58 | n.d.^{[c]} |
| 3 | 4-OH-Ph | 4-hydroxymandelic acid | n.d. | 95 |
| 4 | 4-NO₂-Ph | 4-nitromandelic acid | n.d. | 80 |
| 5 | 4-Cl-Ph | 4-chloromandelic acid | 57 | 95 |
| 6 | 3-Cl-Ph | 3-chloromandelic acid | n.d. | 98 |
| 7 | 2-Cl-Ph | 2-chloromandelic acid | 93 | 44 |
| 8 | 4-CHO-Ph | 4-aldehydemandelic acid | n.d. | 46 |
| 9 | 4-COOH-Ph | 4-formylmandelic acid | n.d. | n.d. |
| 10 | 4-COCH₃Ph | 4-acetylmandelic acid | n.d. | 51 |
| 11 | 4-N(CH₃)₂Ph | 4-(dimethylamino)mandelic acid | n.d. | n.d. |
| 12 | 4-C≡CH-Ph | 4-ethnylnylmandelic acid | n.d. | 75 |
| 13 | 3-OCH₃-4-OH-Ph | 4-hydroxy-3-methoxymandelic acid | n.d., product detected | n.d. |
| 14 | 2-furyl | Hydroxy(2-furyl)acetic acid | n.d. | 75 |
| 15 | 2-thiopheneyl | Hydroxy(2-thiopheneyl)acetic acid | n.d. | 65 |
| 16 | 3-pyridinyl | Hydroxy(3-pyridinyl)acetic acid | n.d., product detected | n.d. |
| 17 | 2-naphthyl | Hydroxy(2-naphthyl)acetic acid | n.d. | 80 |

| | | | | |
|---|---|---|---|---|
| [a] The reaction contained aldehyde (25 mM), disodium oxalate (10 mM), ATP (10 mM), OXS(5 µM), OXC_{Me-Y497A} (5 µM), YciA (2 µM). [b] Determined by chiral HPLC (see Figure S5-7 for detailed quantification). [c] Not determined. | | | | |

### Synthesis of α-hydroxyacyl-CoA thioester and α-hydroxy carboxylic acids Example 15 - Preparation of α-hydroxyacyl-CoA thioester in a one step procedure according to the invention - starting from formyl-CoA and oxalyl-CoA (one pot)

Assays were carried out at 30 °C in a 1.5 mL microfuge tube. Reactions contained 50 mM TES-KOH pH 6.8, 10 mM MgCl₂, 0.5 mM ADP, 0.15 mM ThDP, 10 mM aldehyde (formaldehyde, acetaldehyde, glycolaldehyde, propionaldehyde, glyceraldehyde, glyoxylate, succinic semialdehyde, benzaldehyde, and phenylacetaldehyde) or 10 mM acetone and 5 µM OXC_{Me} or HACL_{Hs}, respectively. The reaction was started by adding 1 mM formyl-CoA or oxalyl-CoA. The reaction was stopped after 1 h by quenching with 4% formic acid and products analyzed with the CoA ester method described above.

### Example 16 - Preparation of α-hydroxy carboxylic acid starting from oxalyl-CoA by enzymatic catalysis in a two step procedure according to the invention (one pot)

Assays were carried out at 30 °C in a 1.5 mL microfuge tube. Reactions contained 50 mM TES-KOH pH 6.8, 10 mM MgCl₂, 0.5 mM ADP, 0.15 mM ThDP, 10 mM aldehyde, 5 µM OXC_{Me} or OXC_{Me-Y497A}, 2 µM YciA. The reaction was started by adding 1 mM formyl-CoA or oxalyl-CoA. The reaction was stopped after 1 h by quenching with 4% formic acid and products analyzed with the mandelic acid derivatives method described above.

### Example 17 - Preparation of α-hydroxy carboxylic acid starting from oxalate by enzymatic catalysis in a three step according to the invention (one pot)

Assays were carried out at 30 °C in a 1.5 mL microfuge tube. Reactions contained 15vol% DMSO, 50 mM TES-KOH pH 6.8, 10 mM MgCl₂ 0.5 mM ADP, 0.15 mM ThDP, 0.5 mM CoA, 10 mM ATP, 25 mM aldehyde, 5 µM OXS, 5 µM OXC_{Me} or OXC_{Me-Y497A} and 2 µM YciA. The reaction was started by adding 10 mM oxalate. Samples were taken after 0, 0.05, 0.25, 0.66, 1, 2, 3, 22 hours, quenched with 4% formic acid and analyzed with the LC-MS detection of mandelic acid derivatives.

### Example 18 - Michaelis-Menten kinetics of the formation of formyl-CoA catalyzed by formyl-CoA synthetase (acetyl-CoA synthetase)

The Michaelis-Menten kinetic of eryACS-1 (an acyl-CoA synthetase from *Erythrobacter* sp.) and variants thereof as formyl-CoA synthetase (FCS) were investigated with formate as substrate according to example 6.

AMP production was followed at 30 °C by coupling eryACS-1 to purified adenylate kinase (ADK), pyruvate kinase (PK) and lactate dehydrogenase (LDH). An assay containing 200 mM MOPS-KOH pH 7.8, 0.3 mM NADH, 10 mM MgCl₂, 2 mM ATP, 0.5 mM CoA, 2.5 mM PEP, 0.2 mg/mL ADK, 15 U/mL PK from rabbit muscle (Sigma), 23 U/mL LDH from rabbit muscle (Sigma), and an appropriate concentration of eryACS-1 was preincubated for 2 min and the reaction started by adding sodium formate to a final concentration of 10, 25, 50, 100, 150, 200 mM, respectively. Reaction procedure was monitored by following the oxidation of NADH at 340 nm.

Results: The results are depicted in figure 29.

Acyl-CoA synthetase from *Erythrobacter* sp. NAP1 (eryACS-1) exhibits a favorable activity towards formate (*k*_{cat} = 0.67 ± 0.03 s⁻¹; *K*_{M}(formate) = 73 ± 8 mM). The enzyme was engineered by replacing valine 379 by isoleucine, which increased catalytic efficiency, mainly through an improved turnover number. Activity of the enzyme was further increased by expressing the gene in an *E. coli* strain lacking lysine acetyltransferase *patZ*, which inhibits acetyl-CoA synthetase, to finally obtain a highly active variant (*k*_{cat} = 16 ± 1 s⁻¹; *K*_{M}(formate) = 100 ± 10 mM).

### Example 19 - FCS- OXC_{Me}-YciA cascade prototyping

The investigation of (*S*)-mandelic acid formation of the cascade FCS- OXC_{Me}-YciA using formate as starting material were performed according to example 12.

Assays were carried out at 30 °C in reaction buffer consisting of 50 mM TES-KOH pH 6.8, 10 mM MgCl₂, 0.5 mM ADP, 0.15 mM ThDP, 0.5 mM CoA, 5 mM ATP, 25 mM benzaldehyde, 1 µM FCS, 2 µM OXC and 2 µM YciA. The reaction was started by adding 50 mM sodium formate. Samples were taken after 0, 2, 15, 30, 60, 120, 300 min and analyzed with the LC-MS detection of mandelic acid derivatives. Mandelic acid was quantified by comparison to commercially obtained standards of mandelic acid.

Results: The results are depicted in figure 30.

The formyl-CoA synthetase can be used in a cascade reaction in a one-pot system along with a thioesterase YciA and oxalyl-CoA synthetase for obtaining mandelic acid.

### Example 20 - FRC formyl-CoA 'proofreading'

Formyl-CoA:oxalate CoA-transferase (FRC) from *Oxalobacter formigenes* catalyzes the reaction formyl-CoA + oxalate = oxalyl-CoA + formate. This reaction is fully reversible. Converting formyl-CoA into oxalyl-CoA in the context of the cascade should have 2 beneficial effects: First, YciA has (low) thioesterase activity towards formyl-CoA, but none towards oxalyl-CoA (Fig. 16). This means that no (or less) ATP equivalents are lost to formyl-CoA hydrolysis, resulting in higher yield. Secondly, OXC_{Me-Y497A} is faster in the carboligation with oxalyl-CoA than with formyl-CoA (Fig. 14). Therefore, turning formyl-CoA into oxalyl-CoA should increase the rate of OXC, and thus the rate of the whole cascade. In order to test these hypothesis FRC was added to a reaction containing OXC, benzaldehyde, oxalate and oxalyl-CoA.

Results: The results are depicted in figure 31. OXC_{Me-Y497A} with FRC gave mandelyl-CoA formation rate of 33 µM/min, a 1.65-fold improvement over no FRC. Also note how the formyl-CoA is not accumulating in the reaction with OXC_{Me-Y497A}, whereas it is still formed rapidly in the case of OXC_{Me}. This can be explained by the *k*_{cat} for decarboxylation (OXC_{Me}, 98 s⁻¹ vs. OXC_{Me-Y497A}, 1.3 s⁻¹).

## Claims

1. A method for preparation of a compound of the formula (**I'**) 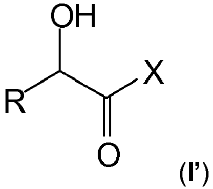 wherein **R** represents 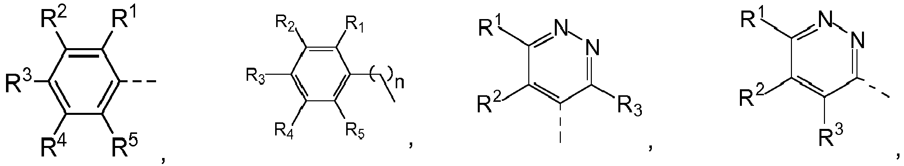 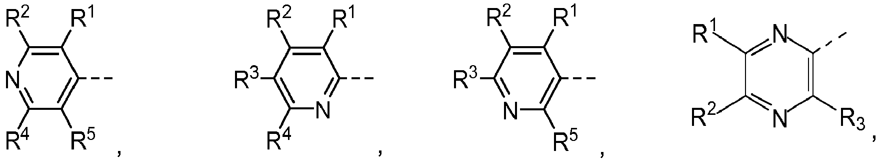 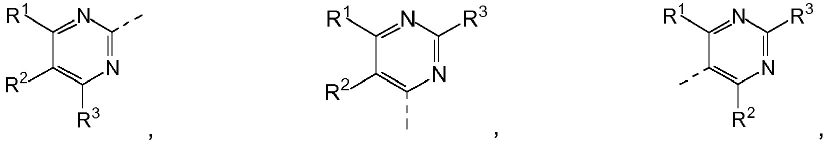 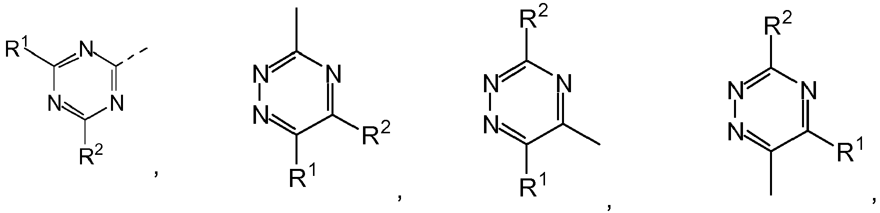 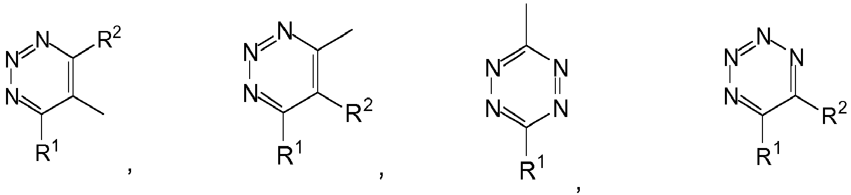 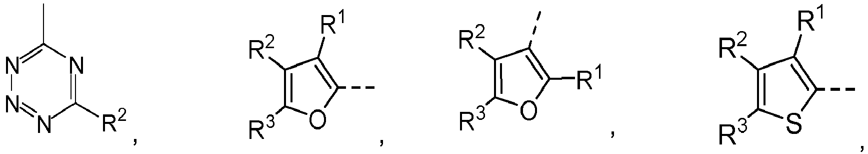 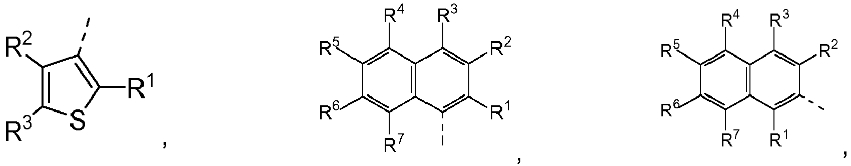 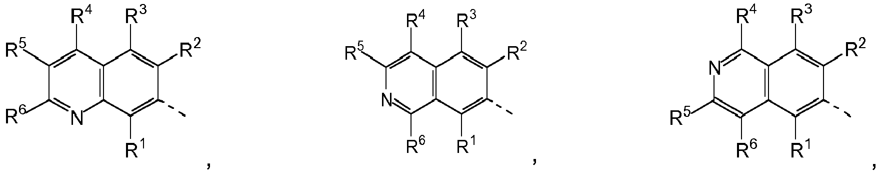 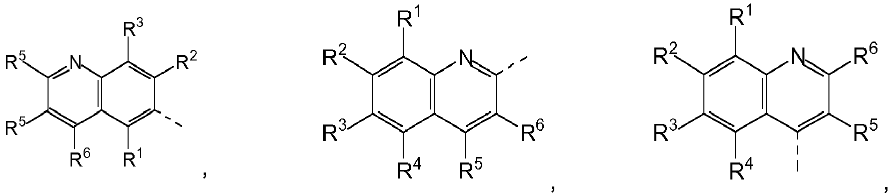 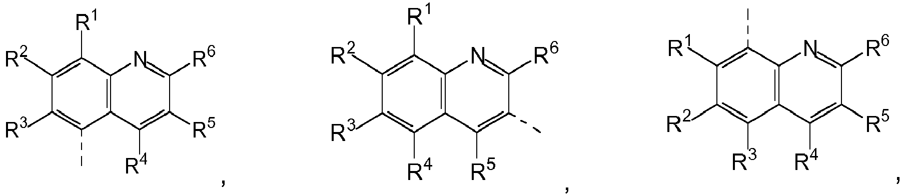 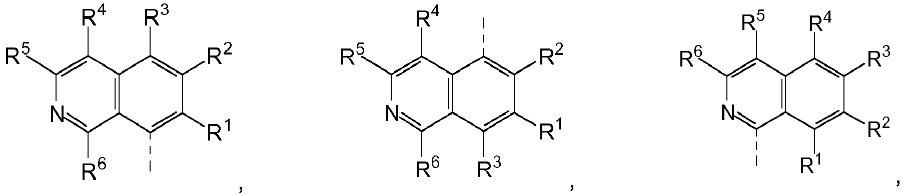 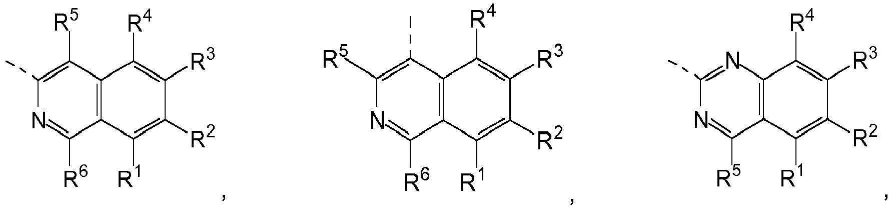 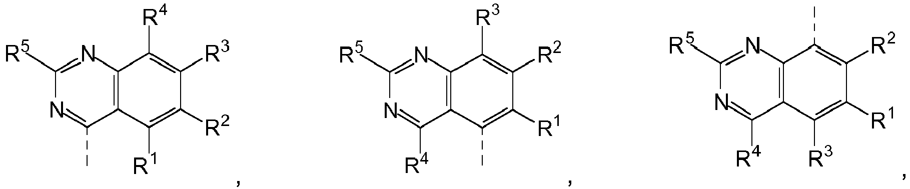 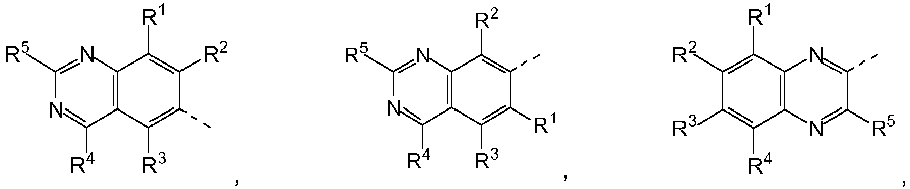 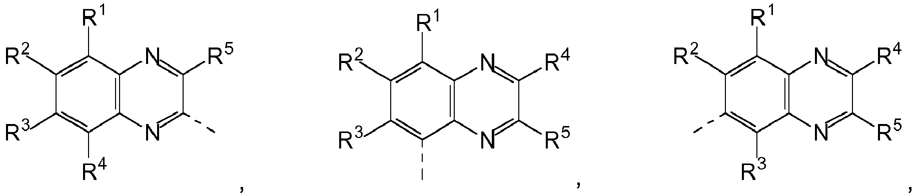 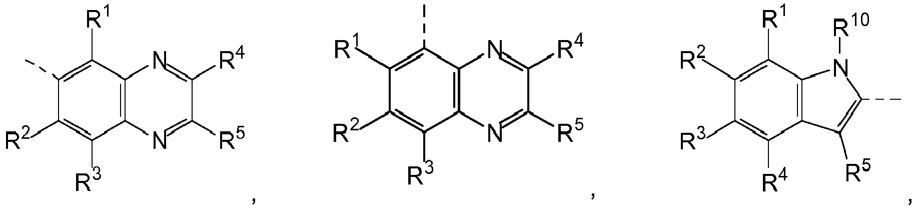 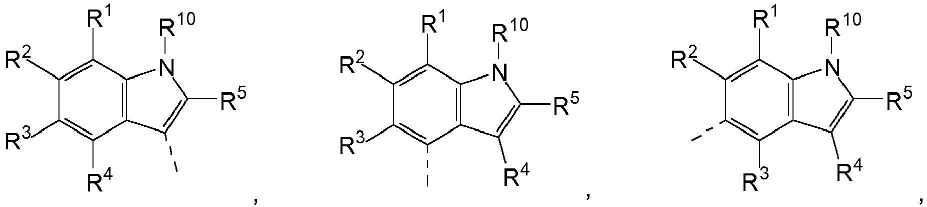 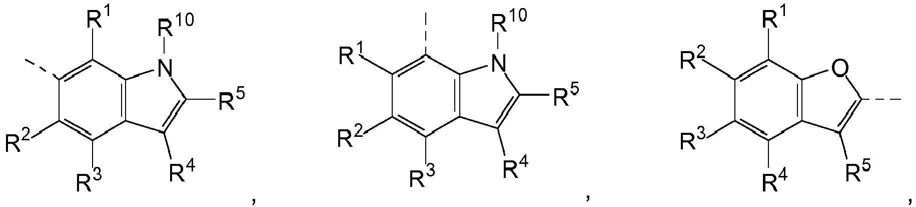 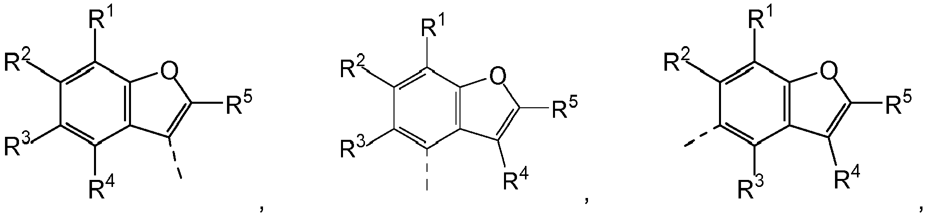 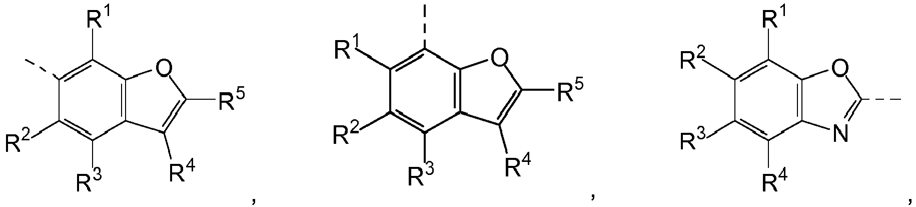 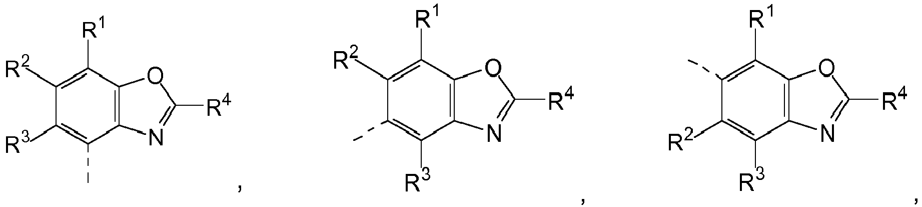 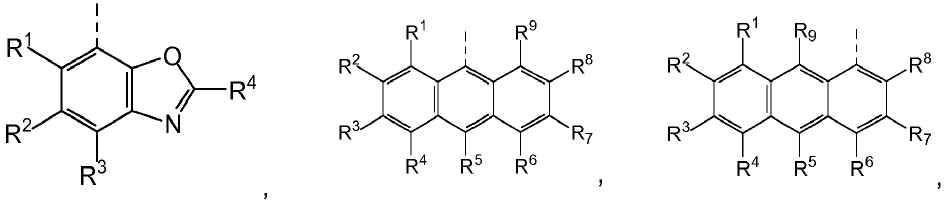 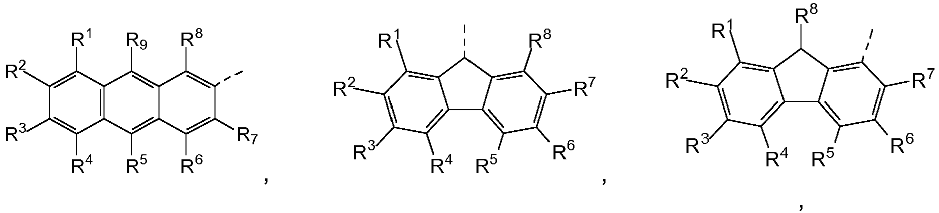 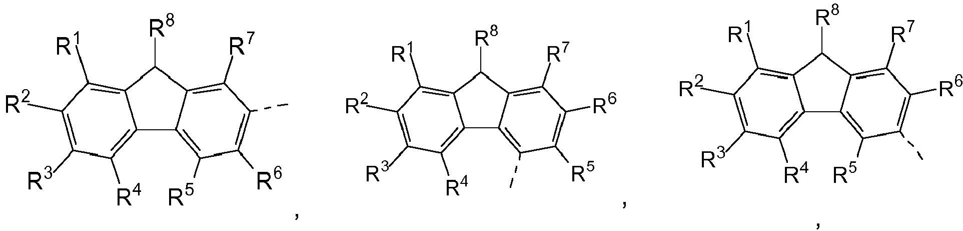 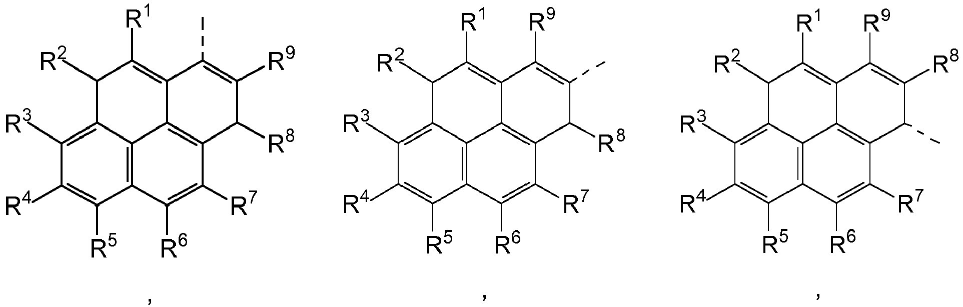 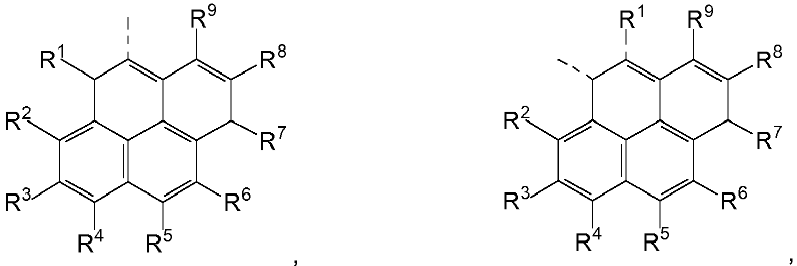 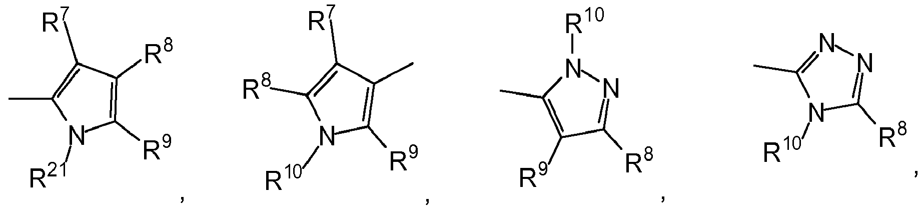 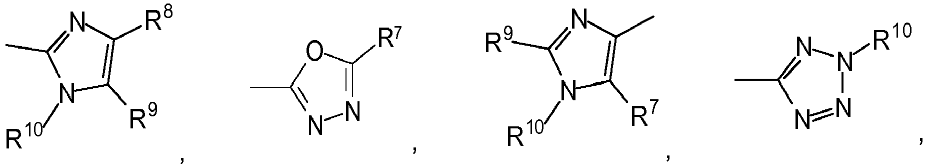 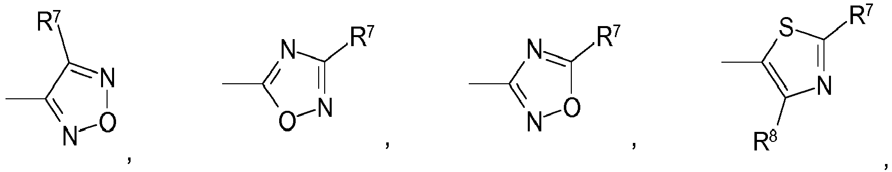 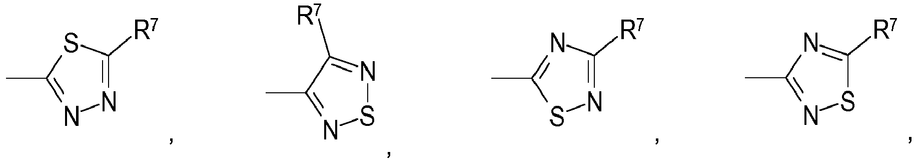 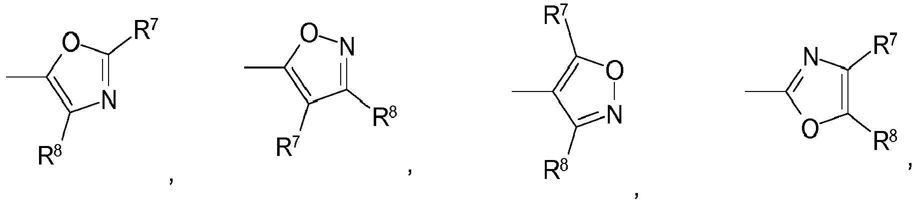 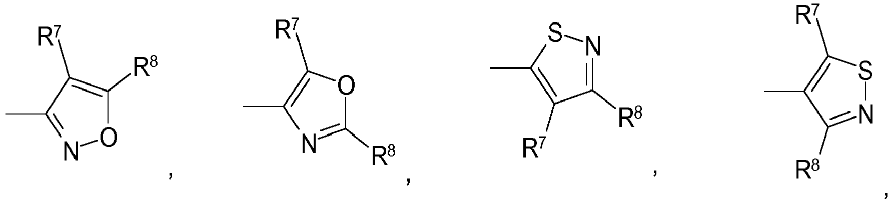 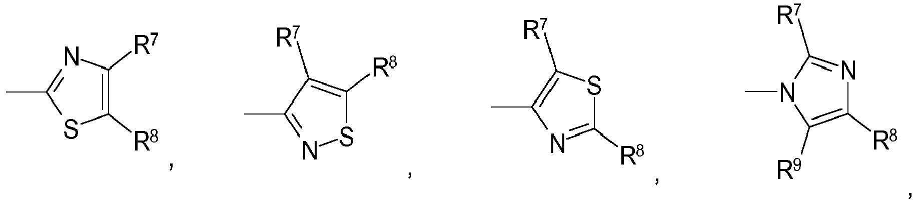 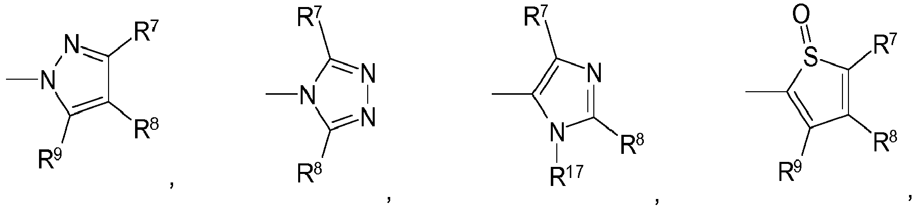 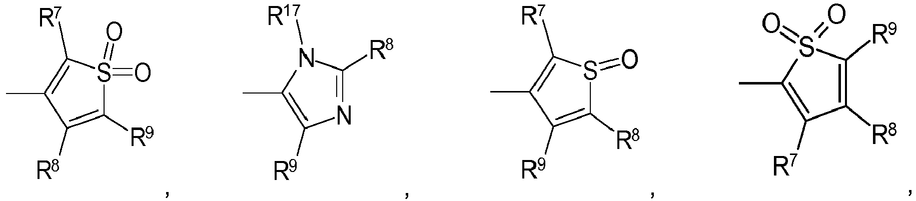 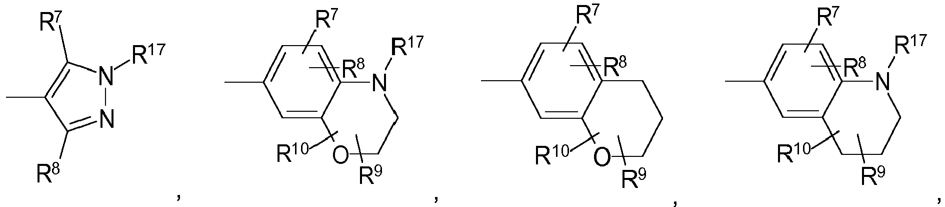 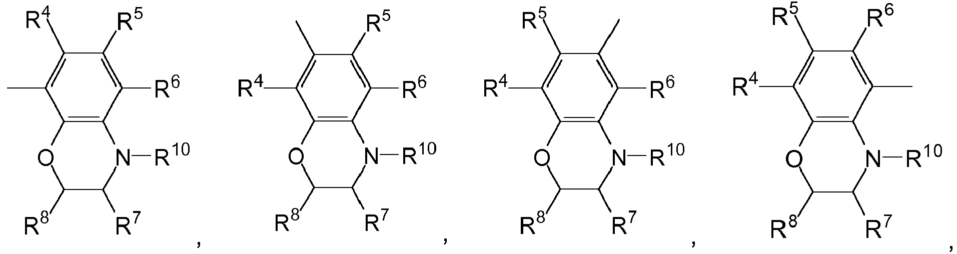 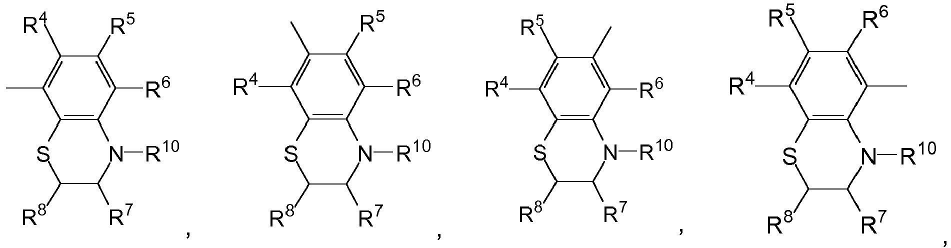 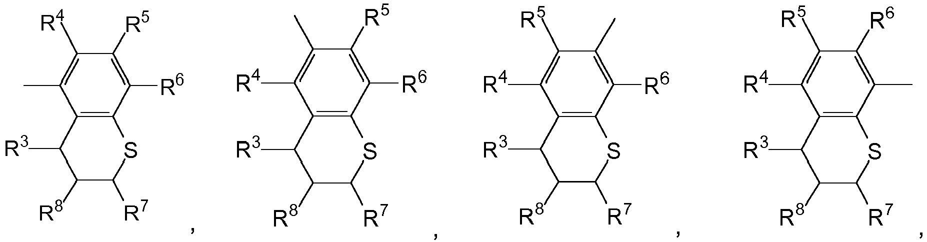 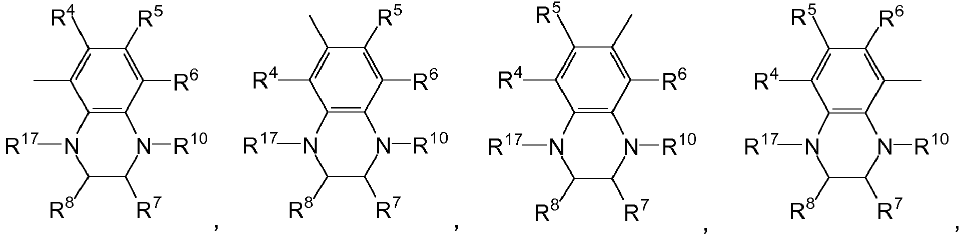 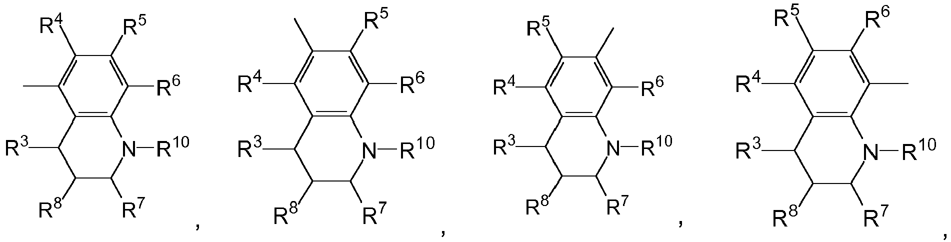 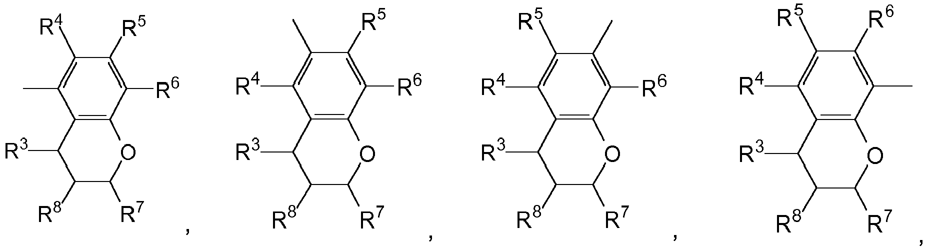 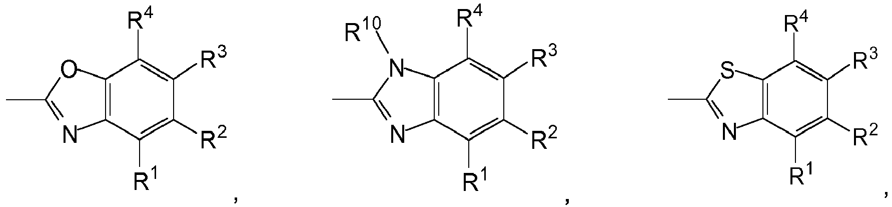 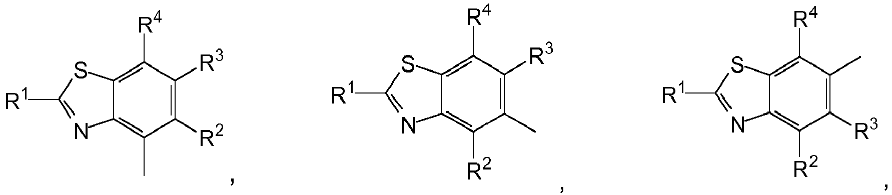 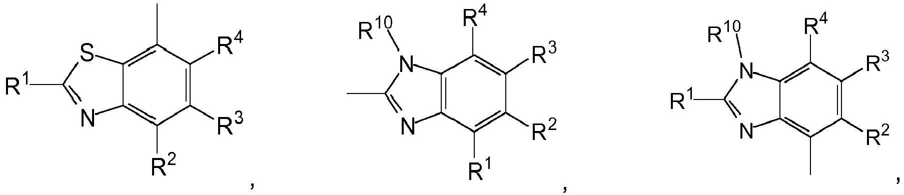 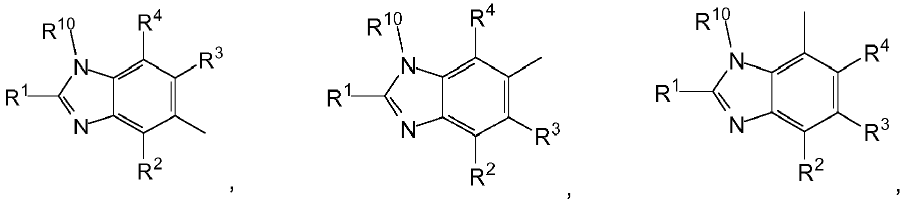 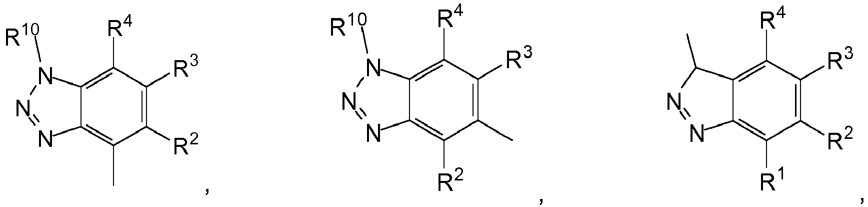 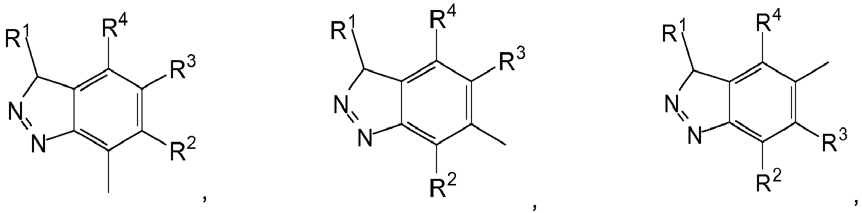 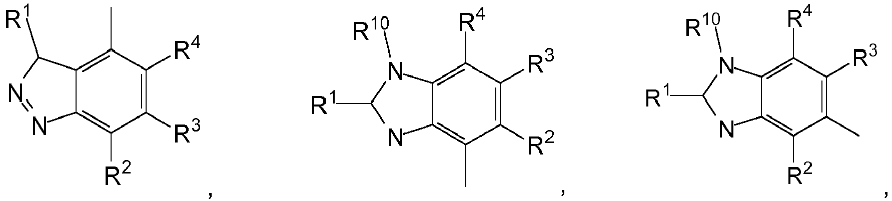 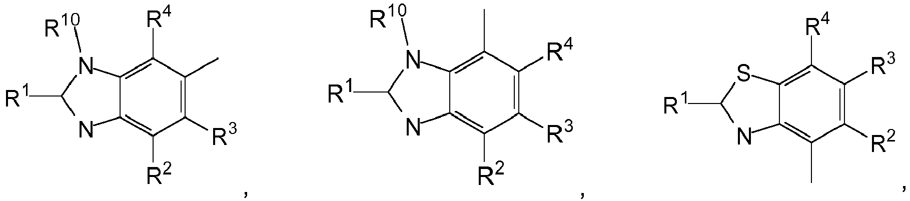 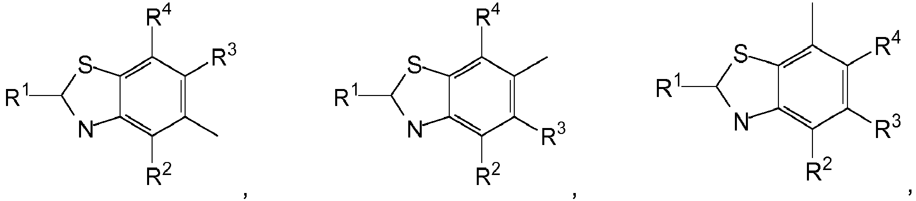 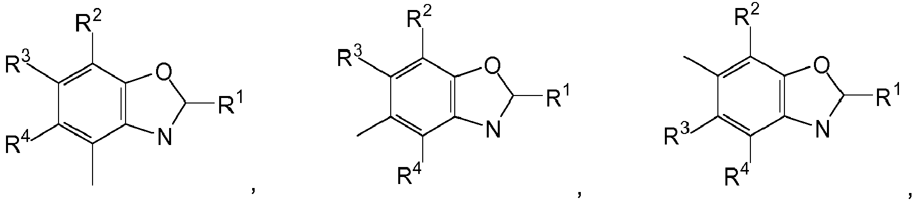 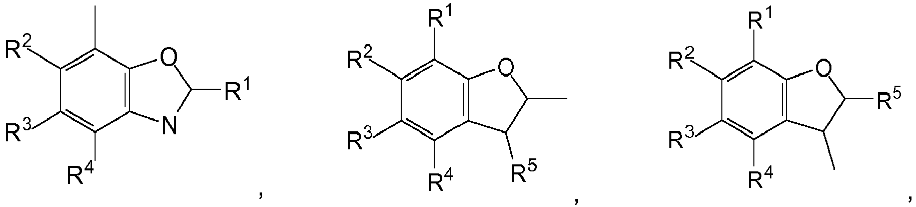 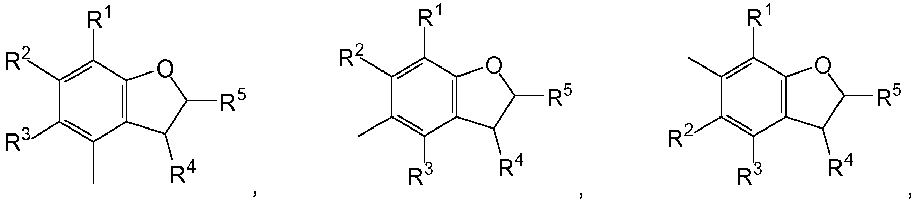 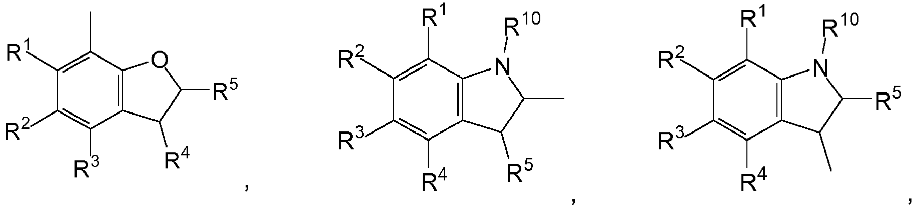 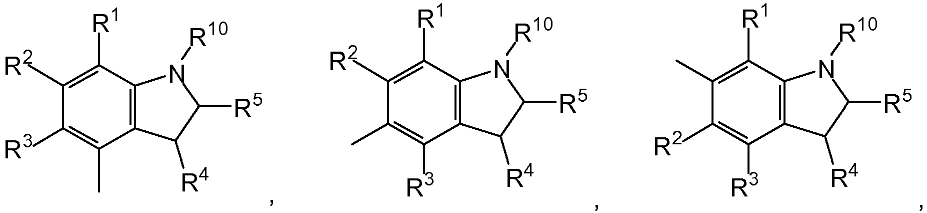 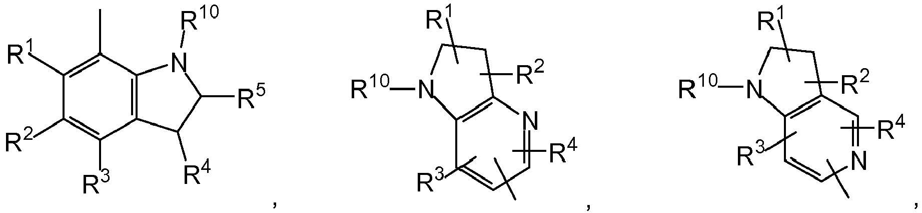 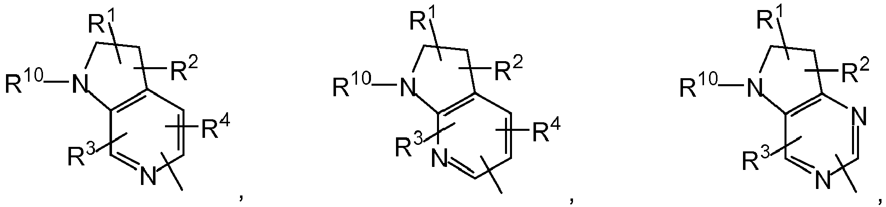 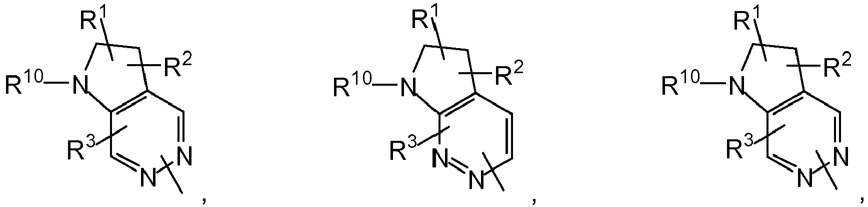 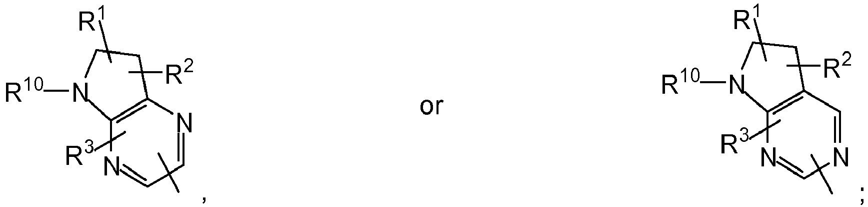 wherein **R¹**, **R²**, **R³**, **R⁴**, **R⁵**, **R⁶**, **R⁷**, **R⁸**, **and R⁹** represent independently of each other -H, -F, -CI, -Br, -I, -Ph, -OH, -OCH₃, -OC₂H₅, -OC₃H₇, -OCH(CH₃)₂, -OC₄H₉, -OCH(CH₃)CH₂CH₃, -OCH₂CH(CH₃)₂, -OC(CH₃)₃, -O-cyclo-C₃H₅, -O-cyclo-C₄H₇, -O-cyclo-C₅H₉, -O-cyclo-C₆H₁₁, -OPh, -OCH₂-Ph, -OCH₂CH₂-Ph -OCH=CH₂, -OCH₂-CH=CH₂, -OCH₂CH₂-CH=CH₂, -OCF₃, -OC₂F₅, -SCH₃, -SC₂H₅, -SC₃H₇, -SCH(CH₃)₂, -SC₄H₉, -SCH(CH₃)CH₂CH₃, -SCH₂CH(CH₃)₂, -SC(CH₃)₃, -NO₂, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇₎₂, -N(C₄H₉)₂, -N(cyclo-C₃H₅)₂, -N[CH(CH₃)₂]₂, -N[C(CH₃)₃]₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH₂-CH₂-CH(CH₃)₂, -CH(CH₃)-CH(CH₃)-CH₃, -C(CH₃)₂-C₂H₅, -C₆H₁₃, -CH(CH₃)-C₄H₉, -CH₂-CH(CH₃)-C₃H₇, -CH₂-CH₂-CH₂-CH(CH₃)₂, -CH(CH₃)-CH(CH₃)-C₂H₅, -C(CH₃)₂-C₃H₇, -C₇H₁₅, -CH(CH₃)-C₅H₁₁, -CH₂-CH(CH₃)-C₄H₉, -CH₂-CH₂-CH₂-CH₂-CH(CH₃)₂, -C₈H₁₇, -C₉H₁₉, -C₁₀H₂₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -CH₂F, -CF₂I, -CHF₂, -CF₃, -CH₂I, -CH₂Br, -CH₂I, -CH₂-CH₂F, -CH₂-CHF₂, -CH₂-CF₃, -CH₂-CH₂I, -CH₂-CH₂Br, -CH₂-CH₂I, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CHO, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COO-cyclo-C₄H₇, -COO-Cyclo-C₅H₉ -COO-cyclo-C₆H₁₁, -COOCH(CH₃)₂, -COOC(CH₃)₃, -O-COOCH₃, -O-COOC₂H₅, -O-COOC₃H₇, -O-COO-cyclo-C₃H₅, -O-COOCH(CH₃)₂, -O-COOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -NHCO-OCH₃, -NHCO-OC₂H₅, -NHCO-OC₃H₇, -NHCO-O-cyclo-C₃H₅ -NHCO-OCH(CH₃)₂, -NHCO-OC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, 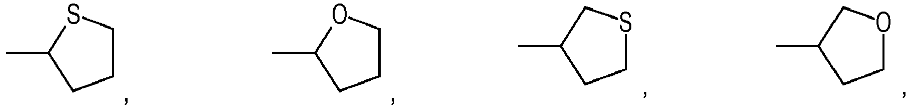 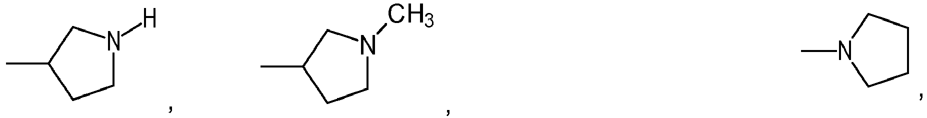 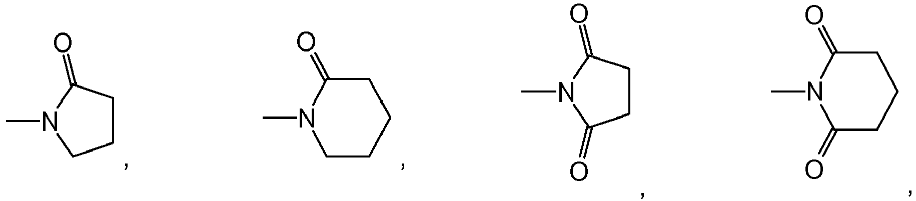 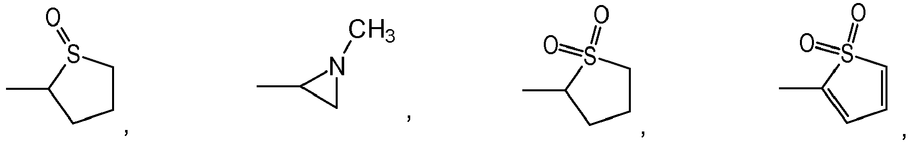 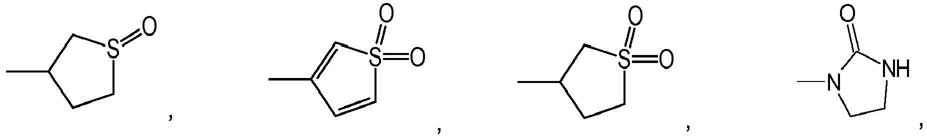 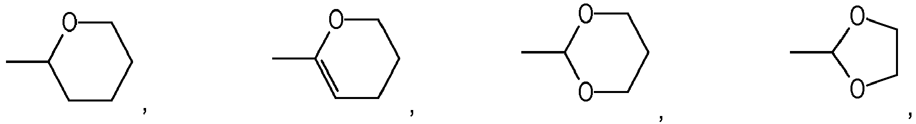 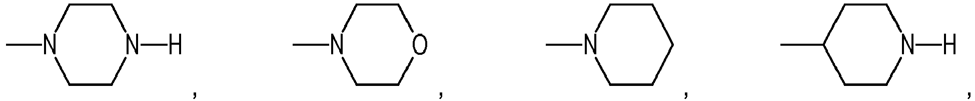 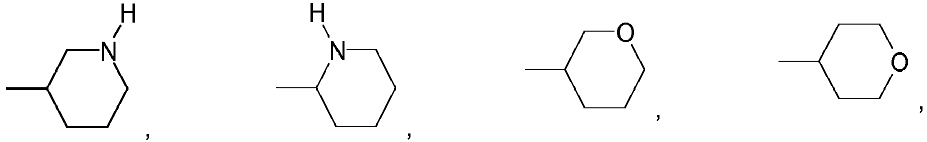 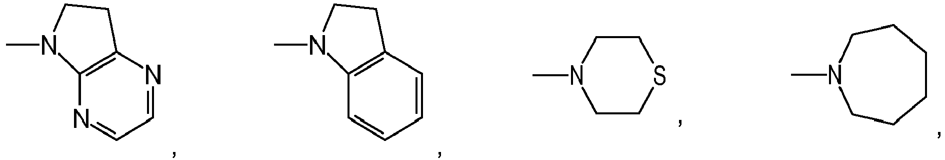 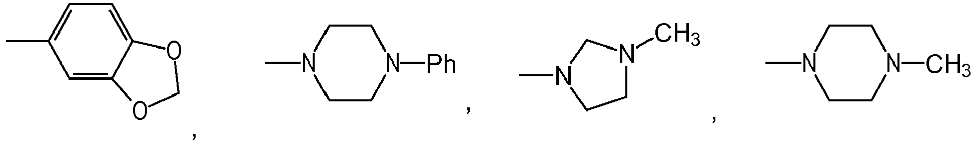 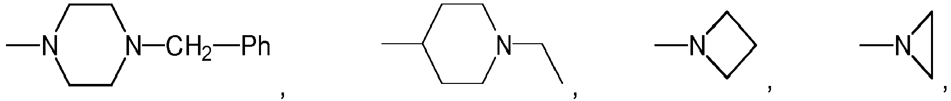 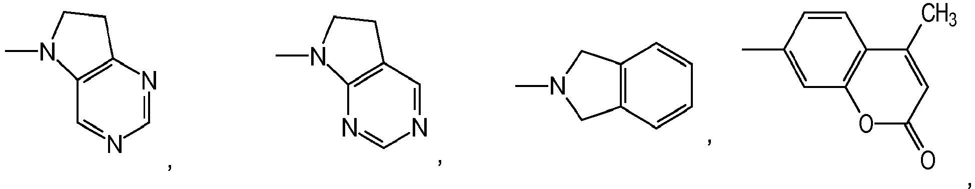 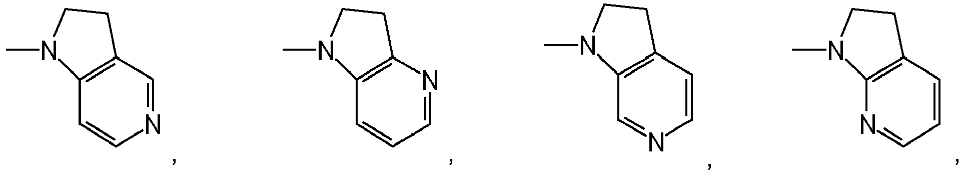 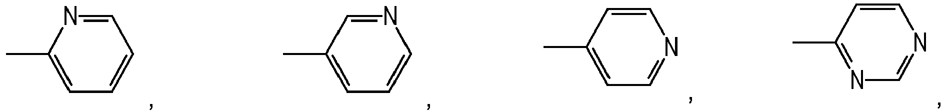 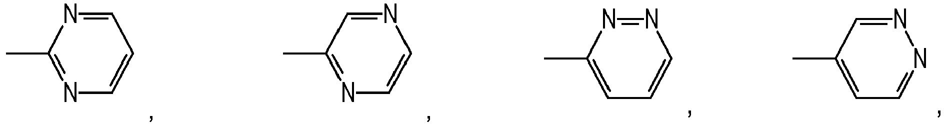 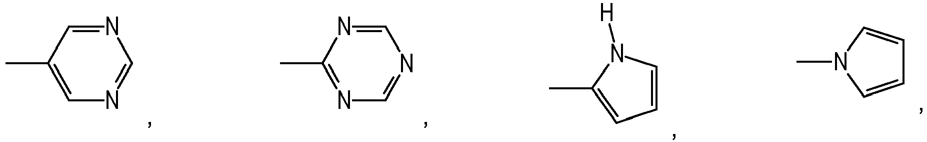 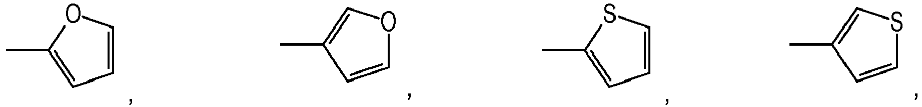 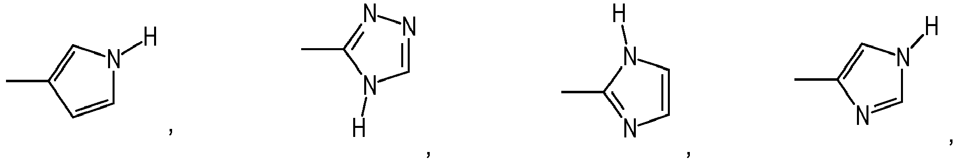 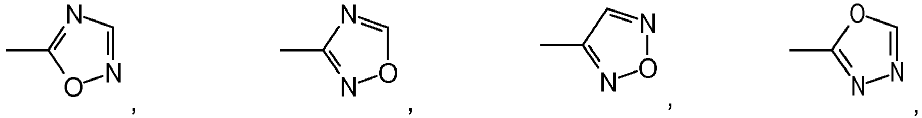 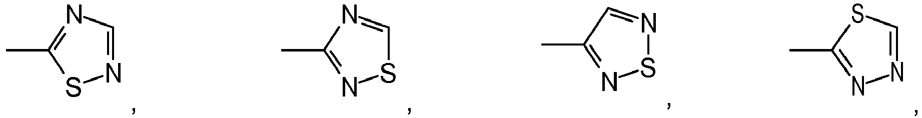 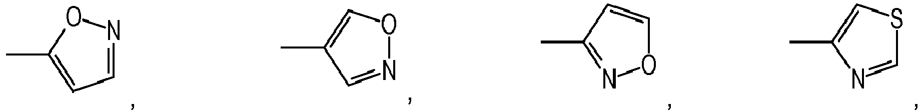 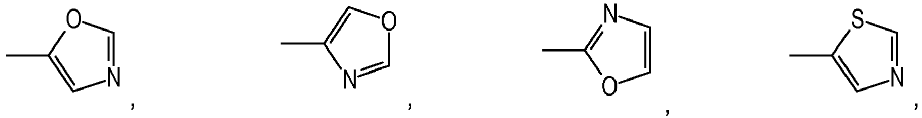 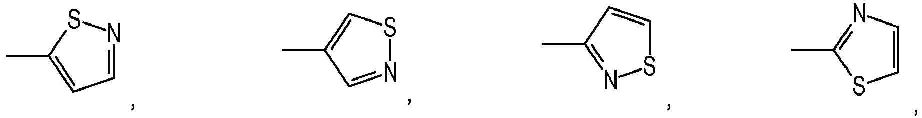 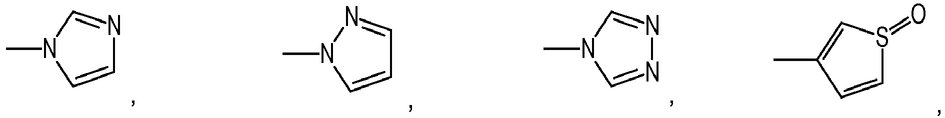 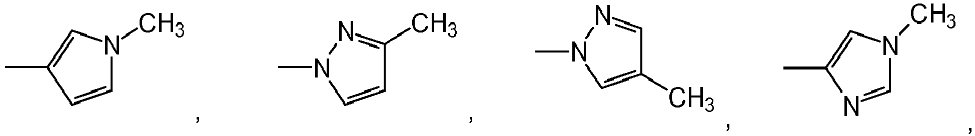 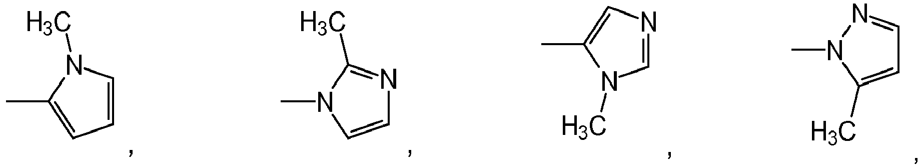 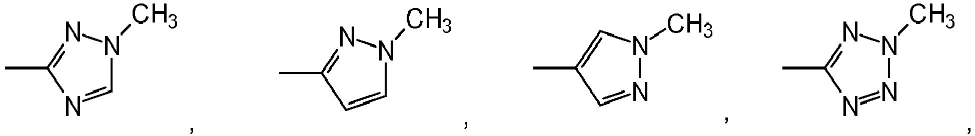 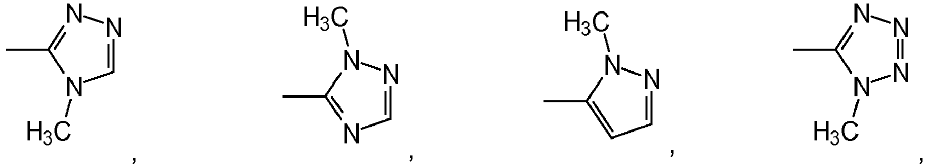 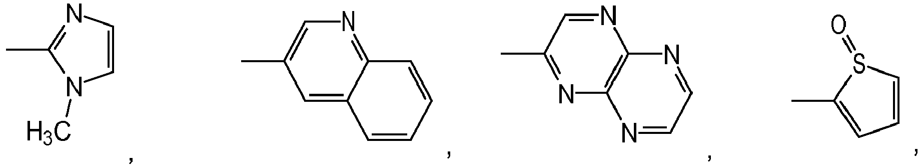 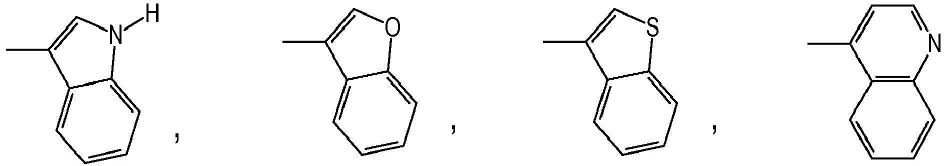 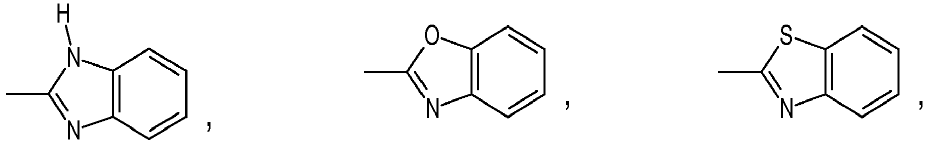 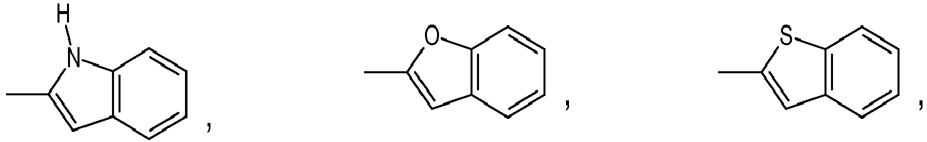 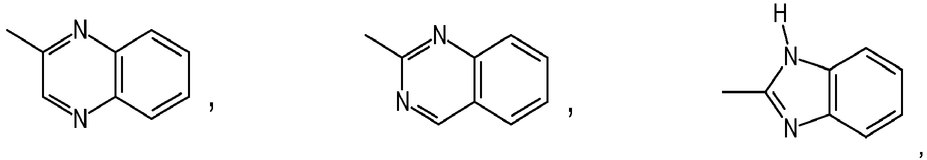 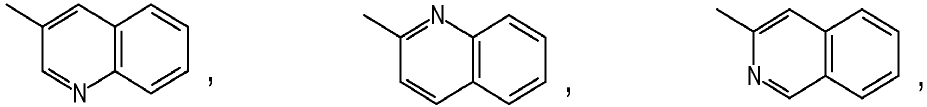 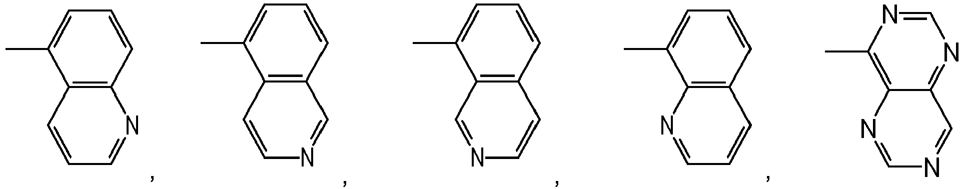 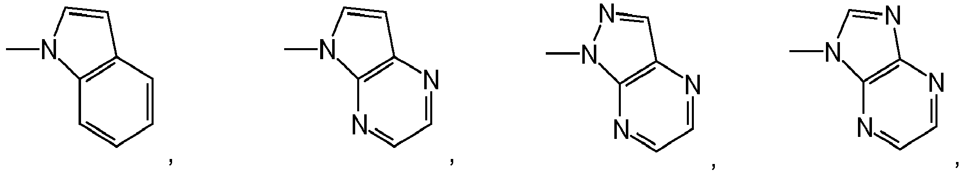 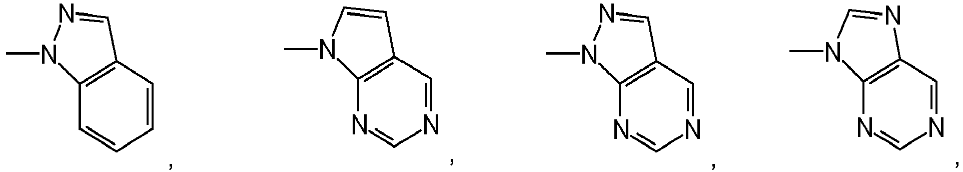 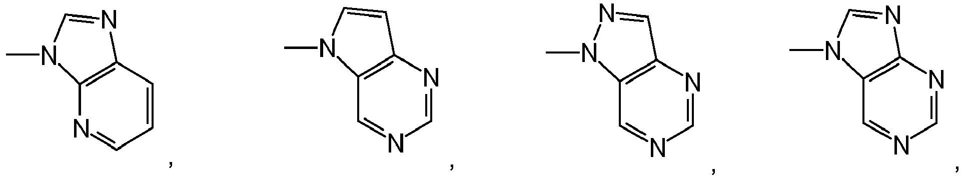 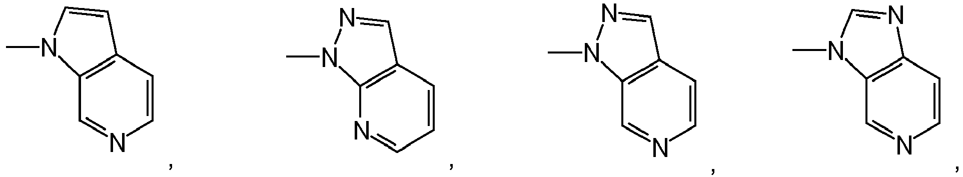 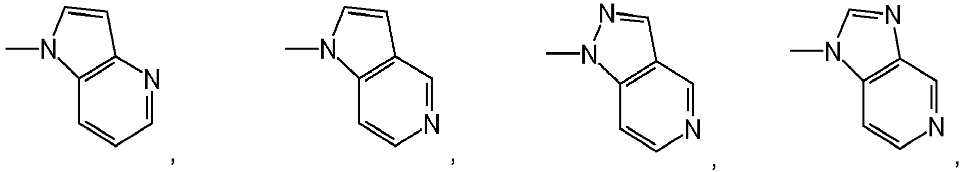 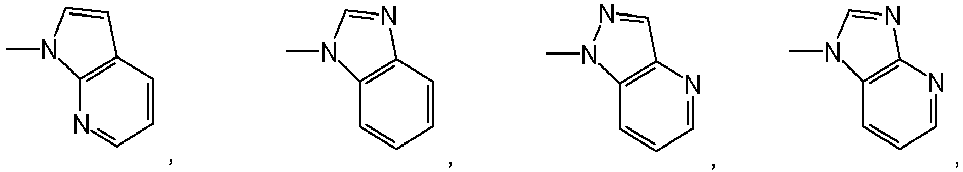 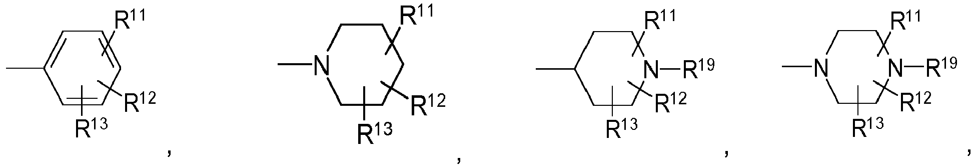 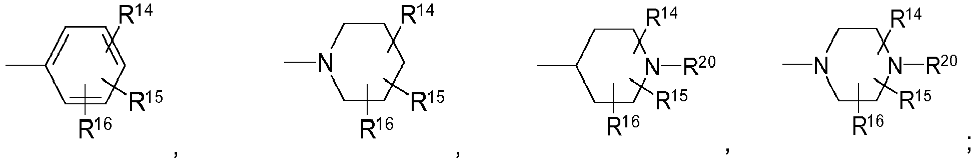 **R¹¹** to **R¹⁶** represents independently of each other:
-H, -NH₂, -OH, -OCH₃, -OC₂H₅, -OC₃H₇, -OCF₃, -CF₃, -F, -Cl, -Br, -I, -CH₃, -C₂H₅, -C₃H₇, -CH(CH₃)₂, -Ph, or -CN;
**R¹⁰**, **R¹⁷** , **R¹⁸**, **R¹⁹**, **R²⁰** , **and R²¹** represents independently of each other -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, CH₂-CH₂F, -CH₂-CHF₂, -CH₂-CF₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CHO, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, preferably R²¹ cannot be -H;
**X** represents -OH, -O⁻ or -SCoA,
wherein n is an integer 1, 2, or 3; or a salt thereof,
comprising:
**A**) providing a compound R-CHO (1) and an acyl-CoA (**2**) 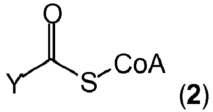
wherein Y represents -H or -COO⁻,
in a buffer solution with a pH value in the range of 5.0 to 7.5 optionally together with at least one co-solvent;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the α-hydroxyacyl-CoA thioester (I') 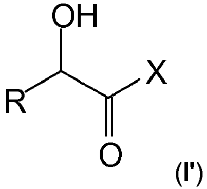
wherein X represents -S-CoA,
and the oxalyl-CoA decarboxylase (**3a**) is a peptide encoded by a gene selected from *Methylobacterium extorquens* or a mutant thereof; and optionally comprising the additional step **C**)
**C**) cleaving the coenzyme A moiety of said α-hydroxyacyl-CoA thioester (**I'**) by a hydrolase, α-hydroxyacyl-CoA:oxalate CoA-transferase and/or α-hydroxyacyl-CoA:formate CoA-transferase to obtain the compound of the formula (I') or a salt thereof 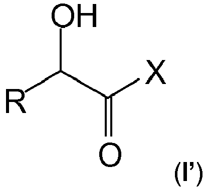
wherein X represents -OH or O⁻,
and the hydrolase is acyl-CoA thioester hydrolase YciA (**4**) encoded by a gene selected from *E. coli.*

2. The method for the preparation of the compounds according to claim 1 represented by formula (I) 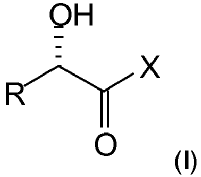 comprising:
**A**) providing a compound R-CHO (**1**) and an acyl-CoA (**2**) 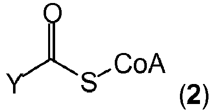
wherein Y represents -H, or -COO⁻,
in a buffer solution with a pH-value in the range of 5.0 to 7.5, optionally together with at least one co-solvent;
**B**) performing a coupling reaction of R-CHO (**1**) and acyl-CoA (**2**) by an oxalyl-CoA decarboxylase (**3a**) in the presence of thiamine diphosphate (**3b**) and a magnesium (II) cation to obtain the *(S)*-α-hydroxyacyl-CoA thioester (**I**) 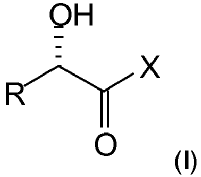
wherein X represents -S-CoA,
and the oxalyl-CoA decarboxylase (**3a**) is a peptide encoded by a gene selected from *Methylobacterium extorquens* or a mutant thereof; and optionally comprising the additional step **C**)
**C**) cleaving the coenzyme A moiety of said *(S)*-α-hydroxyacyl-CoA thioester (**I**) by a hydrolase, α-hydroxyacyl-CoA:oxalate CoA-transferase and/or α-hydroxyacyl-CoA:formate CoA-transferase to obtain the compound of the formula (**I**) or a salt thereof 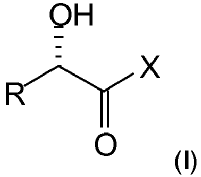
wherein X represents -OH or O⁻,
and the hydrolase is acyl-CoA thioester hydrolase YciA (**4**) encoded by a gene selected from *E. coli.*

3. The method for preparation of the compounds according to claim 2 represented by formula (**I**) 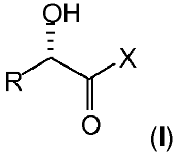 comprising:
**A)** providing a compound R-CHO **(1)** and an oxalyl-CoA (2) 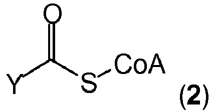
wherein Y represents -COO⁻,
in a buffer solution with a pH-value in the range of 5.0 to 7.5 optionally together with at least one co-solvent;
**B**) performing a coupling reaction of R-CHO (1) and oxalyl-CoA (**2**) by an oxalyl-CoA decarboxylase (**3a**) in the presence of thiamine diphosphate (**3b**) and a magnesium (II) cation to obtain the *(S)*-α-hydroxyacyl-CoA thioester (I) 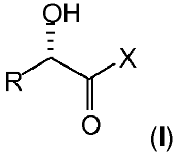
wherein X represents -S-CoA,
and the oxalyl-CoA decarboxylase (**3a**) is a peptide encoded by a gene selected from *Methylobacterium extorquens* or a mutant thereof; and optionally comprising the additional step **C**)
**C**) cleaving the coenzyme A moiety of said *(S)*-α-hydroxyacyl-CoA thioester (**I**) by a hydrolase, α-hydroxyacyl-CoA:oxalate CoA-transferase and/or α-hydroxyacyl-CoA:formate CoA-transferase to obtain the compound of the formula (**I**) or a salt thereof 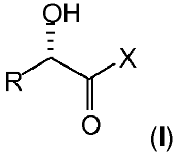
wherein X represents -OH or O⁻,
and the hydrolase is acyl-CoA thioester hydrolase YciA (4) encoded by a gene selected from *E. coli.*

4. The method for the preparation of the compounds according to claim 2 or 3 represented by formula (**II**) 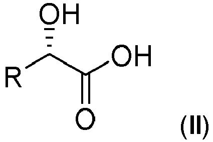 comprising:
**A)** providing a compound R-CHO **(1)** and an oxalyl-CoA (**2**) 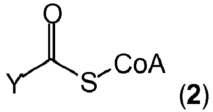
wherein Y represents -COO⁻,
in a buffer solution with a pH-value in the range of 5.0 to 7.5 optionally together with at least one co-solvent;
**B**) performing a coupling reaction of R-CHO (**1**) and oxalyl-CoA (**2**) by an oxalyl-CoA decarboxylase (**3a**) in the presence of thiamine diphosphate (**3b**) and a magnesium (II) cation to obtain the (*S*)-α-hydroxyacyl-CoA thioester (**I**) 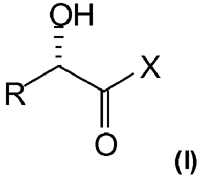
wherein X represents -S-CoA,
and the oxalyl-CoA decarboxylase (**3a**) is a peptide encoded by a gene selected from *Methylobacterium extorquens* or a mutant thereof; and
**C**) cleaving the coenzyme A moiety of said *(S)*-α-hydroxyacyl-CoA thioester (**I**) by a hydrolase, α-hydroxyacyl-CoA:oxalate CoA-transferase and/or α-hydroxyacyl-CoA:formate CoA-transferase to obtain the compound of the formula (**I**) or a salt thereof 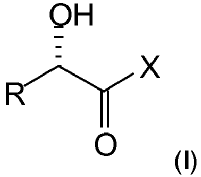
wherein X represents -OH or O⁻,
and the hydrolase is acyl-CoA thioester hydrolase YciA (**4**) encoded by a gene selected from *E. coli.*

5. The method according to claim 4, wherein steps A), B) and C) are carried out as cascade reaction in one-pot system.

6. The method according to claim 3 or 5, wherein the oxalyl-CoA (**2**') is prepared by the following step **A**') **A**'
) reacting an oxalate (C₂O₄²⁻) **(2a')** with a free coenzyme A (**2b**) by oxalyl-CoA synthetase (**2c**') in the presence of ATP (**2e**) to produce an oxalyl-CoA (**2**').

7. The method according to claim 6, wherein steps **A**'), **A**), **B**), and **C**) are carried out as cascade reaction in one-pot system.

8. The method according to claim 7, wherein in step A') a ratio of the oxalate (**2a**') and the free coenzyme A (**2b**) is 10 to 10,000.

9. The method according to any one of the claims 1 to 8, wherein in step **B**), a concentration of the oxalyl-CoA decarboxylase is in the range of 1 to 25 µM and/or a concentration of the magnesium (II) cation is in the range of 2.5 mM to 20 mM.

10. The method according to any one of the claims 1 to 9, wherein in step **B**), the mutant comprises OXC_{Me-Y497A}, OXC_{Me-S568A}, or OXC_{Me-Y497A-S568A}.

11. The method according to any one of the claims 5 to 12, wherein in step **A**') the oxalate is selected from the group consisting of (NH₄⁺)₂C₂CO₄, Li₂C₂O₄, Na₂C₂O₄, K₂C₂O₄, Rb₂C₂O₄, Cs₂C₂O₄, MgC₂O₄, CaC₂O₄, ZnC₂O₄, FeC₂O₄, MnC₂O₄, NiC₂O₄, CuC₂O₄, and CoC₂O₄.

12. The method according to any one of the claims 1 to 13, wherein R represents 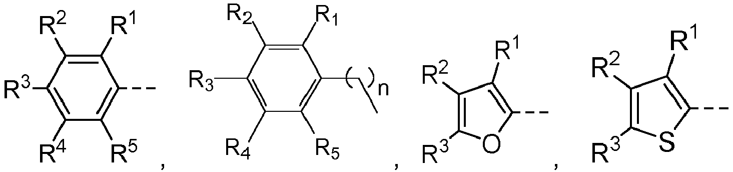 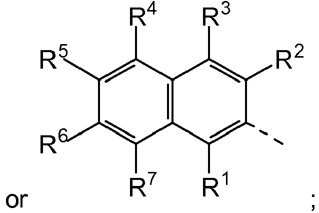 and
**R¹, R², R³, R⁴, R⁵, R⁶** and **R⁷** represents independently of each other -H, -CI, -OH, -OCH₃, -NO₂, -N(CH₃)₂, -C=CH, -CHO, -COCH₃ or -COOH, wherein n represents an integer 1.

13. A peptide OXC_{Me-Y497A} having the sequence SEQ ID NO. 2, OXC_{Me-S568A} having the sequence SEQ ID NO. 3 or OXC_{Me-Y497A-S568A} having the sequence SEQ ID NO. 4.

14. Composition comprising the peptide OXC_{Me} having the sequence SEQ ID NO. 1, OXC_{Me-Y497A} having the sequence SEQ ID NO. 2, OXC_{Me-S568A} having the sequence SEQ ID NO. 3 or OXC_{Me-Y497A-S568A} having the sequence SEQ ID NO. 4 and a thioesterase YciA encoded by a gene selected from *E. coli.*

15. Composition comprising the peptide OXC_{Me} having the sequence SEQ ID NO. 1, OXC_{Me-Y497A} having the sequence SEQ ID NO. 2, OXC_{Me-S568A} having the sequence SEQ ID NO. 3 or OXC_{Me-Y497A-S568A} having the sequence SEQ ID NO. 4, a thioesterase YciA encoded by a gene selected from *E*. *coli* and oxalyl-CoA synthetase.


SEQUENCE LISTING

<110>  Max-Planck-Gesellschaft zur Foerderung der Wissenschaften

<120>  Peptides and methods for the carbon-carbon bond formation

<130>  GAR-P04190EP

<160>  25

<170>  PatentIn version 3.5

<210>  1
<211>  583
<212>  PRT
<213>  Methylobacterium extorquens

<400>  1

Met Thr Val Gln Ala Gln Asn Ile Asp Ala Ile Thr Ala Gly Ala Met
1               5                   10                  15

Pro His Glu Glu Pro Glu Leu Thr Asp Gly Phe His Leu Val Ile Asp
            20                  25                  30

Ala Leu Lys Leu Asn Gly Ile Glu Thr Ile Tyr Asn Val Pro Gly Ile
        35                  40                  45

Pro Ile Thr Asp Leu Gly Arg Leu Ala Gln Ala Glu Gly Leu Arg Val
    50                  55                  60

Ile Ser Phe Arg His Glu Gln Asn Ala Gly Asn Ala Ala Ala Ile Ala
65                  70                  75                  80

Gly Phe Leu Thr Lys Lys Pro Gly Ile Cys Leu Thr Val Ser Ala Pro
                85                  90                  95

Gly Phe Leu Asn Gly Leu Thr Ala Leu Ala Asn Ala Thr Thr Asn Cys
            100                 105                 110

Phe Pro Met Ile Leu Ile Ser Gly Ser Ser Glu Arg Glu Ile Val Asp
        115                 120                 125

Leu Gln Gln Gly Asp Tyr Glu Glu Met Asp Gln Leu Ala Ile Ala Lys
    130                 135                 140

Pro Leu Cys Lys Ala Ala Phe Arg Val Leu His Ala Ala Asp Ile Gly
145                 150                 155                 160

Ile Gly Val Ala Arg Ala Ile Arg Ala Ala Val Ser Gly Arg Pro Gly
                165                 170                 175

Gly Val Tyr Leu Asp Leu Pro Ala Lys Leu Phe Ser Gln Val Ile Asp
        180                 185                 190

Ala Asp Leu Gly Ala Arg Ser Leu Val Lys Val Ile Asp Ala Ala Pro
    195                 200                 205

Ala Gln Leu Pro Ala Pro Ala Ala Ile Ala Arg Ala Leu Asp Val Leu
    210                 215                 220

Lys Ser Ala Glu Arg Pro Leu Ile Ile Leu Gly Lys Gly Ala Ala Tyr
225                 230                 235                 240

Ala Gln Ala Asp Glu Ala Val Arg Ala Leu Val Glu Glu Ser Gly Ile
                245                 250                 255

Pro Tyr Val Pro Met Ser Met Ala Lys Gly Leu Leu Pro Asp Thr His
            260                 265                 270

Pro Leu Ser Ala Gly Ala Ala Arg Ser Thr Ala Leu Lys Asp Ser Asp
        275                 280                 285

Val Val Leu Leu Val Gly Ala Arg Leu Asn Trp Leu Leu Ser His Gly
    290                 295                 300

Lys Gly Lys Thr Trp Gly Glu Pro Gly Ser Lys Arg Phe Ile Gln Ile
305                 310                 315                 320

Asp Ile Glu Pro Arg Glu Met Asp Ser Asn Val Glu Ile Val Ala Pro
                325                 330                 335

Val Val Gly Asp Ile Gly Ser Cys Val Glu Ala Leu Leu Asp Gly Ile
            340                 345                 350

Arg Lys Asp Trp Lys Gly Ala Pro Ser Asn Trp Leu Glu Thr Leu Arg
        355                 360                 365

Gly Lys Arg Glu Ala Asn Ile Ala Lys Met Ala Pro Lys Leu Met Lys
    370                 375                 380

Asn Ser Ser Pro Met Cys Phe His Ser Ala Leu Gly Ala Leu Arg Thr
385                 390                 395                 400

Val Ile Lys Glu Arg Pro Asp Ala Ile Leu Val Asn Glu Gly Ala Asn
                405                 410                 415

Thr Leu Asp Leu Ala Arg Gly Ile Ile Asp Met Tyr Gln Pro Arg Lys
            420                 425                 430

Arg Leu Asp Val Gly Thr Trp Gly Val Met Gly Ile Gly Met Gly Phe
        435                 440                 445

Ala Val Ala Ala Ala Val Glu Thr Gly Lys Pro Val Leu Ala Val Glu
    450                 455                 460

Gly Asp Ser Ala Phe Gly Phe Ser Gly Met Glu Val Glu Thr Ile Cys
465                 470                 475                 480

Arg Tyr Glu Leu Pro Val Cys Ile Val Ile Phe Asn Asn Asn Gly Ile
                485                 490                 495

Tyr Arg Gly Thr Asp Thr Asp Pro Thr Gly Arg Asp Pro Gly Thr Thr
            500                 505                 510

Val Phe Val Lys Asn Ser Arg Tyr Asp Lys Met Met Glu Ala Phe Gly
        515                 520                 525

Gly Val Gly Val Asn Val Thr Thr Pro Asp Glu Leu Lys Arg Ala Val
    530                 535                 540

Asp Glu Ala Met Asn Ser Gly Lys Pro Thr Leu Ile Asn Ala Glu Ile
545                 550                 555                 560

Asp Pro Ala Ala Gly Ser Glu Ser Gly Asn Ile Gly Ser Leu Asn Pro
                565                 570                 575

Gln Ser Thr Leu Lys Lys Lys
            580

<210>  2
<211>  583
<212>  PRT
<213>  Artificial

<220>
<223>  Mutant of oxalyl-CoA decarboxylase from Methylobacterium
       extorquens

<400>  2

Met Thr Val Gln Ala Gln Asn Ile Asp Ala Ile Thr Ala Gly Ala Met
1               5                   10                  15

Pro His Glu Glu Pro Glu Leu Thr Asp Gly Phe His Leu Val Ile Asp
            20                  25                  30

Ala Leu Lys Leu Asn Gly Ile Glu Thr Ile Tyr Asn Val Pro Gly Ile
        35                  40                  45

Pro Ile Thr Asp Leu Gly Arg Leu Ala Gln Ala Glu Gly Leu Arg Val
    50                  55                  60

Ile Ser Phe Arg His Glu Gln Asn Ala Gly Asn Ala Ala Ala Ile Ala
65                  70                  75                  80

Gly Phe Leu Thr Lys Lys Pro Gly Ile Cys Leu Thr Val Ser Ala Pro
                85                  90                  95

Gly Phe Leu Asn Gly Leu Thr Ala Leu Ala Asn Ala Thr Thr Asn Cys
            100                 105                 110

Phe Pro Met Ile Leu Ile Ser Gly Ser Ser Glu Arg Glu Ile Val Asp
        115                 120                 125

Leu Gln Gln Gly Asp Tyr Glu Glu Met Asp Gln Leu Ala Ile Ala Lys
    130                 135                 140

Pro Leu Cys Lys Ala Ala Phe Arg Val Leu His Ala Ala Asp Ile Gly
145                 150                 155                 160

Ile Gly Val Ala Arg Ala Ile Arg Ala Ala Val Ser Gly Arg Pro Gly
                165                 170                 175

Gly Val Tyr Leu Asp Leu Pro Ala Lys Leu Phe Ser Gln Val Ile Asp
            180                 185                 190

Ala Asp Leu Gly Ala Arg Ser Leu Val Lys Val Ile Asp Ala Ala Pro
        195                 200                 205

Ala Gln Leu Pro Ala Pro Ala Ala Ile Ala Arg Ala Leu Asp Val Leu
    210                 215                 220

Lys Ser Ala Glu Arg Pro Leu Ile Ile Leu Gly Lys Gly Ala Ala Tyr
225                 230                 235                 240

Ala Gln Ala Asp Glu Ala Val Arg Ala Leu Val Glu Glu Ser Gly Ile
                245                 250                 255

Pro Tyr Val Pro Met Ser Met Ala Lys Gly Leu Leu Pro Asp Thr His
            260                 265                 270

Pro Leu Ser Ala Gly Ala Ala Arg Ser Thr Ala Leu Lys Asp Ser Asp
        275                 280                 285

Val Val Leu Leu Val Gly Ala Arg Leu Asn Trp Leu Leu Ser His Gly
    290                 295                 300

Lys Gly Lys Thr Trp Gly Glu Pro Gly Ser Lys Arg Phe Ile Gln Ile
305                 310                 315                 320

Asp Ile Glu Pro Arg Glu Met Asp Ser Asn Val Glu Ile Val Ala Pro
                325                 330                 335

Val Val Gly Asp Ile Gly Ser Cys Val Glu Ala Leu Leu Asp Gly Ile
            340                 345                 350

Arg Lys Asp Trp Lys Gly Ala Pro Ser Asn Trp Leu Glu Thr Leu Arg
        355                 360                 365

Gly Lys Arg Glu Ala Asn Ile Ala Lys Met Ala Pro Lys Leu Met Lys
    370                 375                 380

Asn Ser Ser Pro Met Cys Phe His Ser Ala Leu Gly Ala Leu Arg Thr
385                 390                 395                 400

Val Ile Lys Glu Arg Pro Asp Ala Ile Leu Val Asn Glu Gly Ala Asn
                405                 410                 415

Thr Leu Asp Leu Ala Arg Gly Ile Ile Asp Met Tyr Gln Pro Arg Lys
            420                 425                 430

Arg Leu Asp Val Gly Thr Trp Gly Val Met Gly Ile Gly Met Gly Phe
        435                 440                 445

Ala Val Ala Ala Ala Val Glu Thr Gly Lys Pro Val Leu Ala Val Glu
    450                 455                 460

Gly Asp Ser Ala Phe Gly Phe Ser Gly Met Glu Val Glu Thr Ile Cys
465                 470                 475                 480

Arg Tyr Glu Leu Pro Val Cys Ile Val Ile Phe Asn Asn Asn Gly Ile
                485                 490                 495

Ala Arg Gly Thr Asp Thr Asp Pro Thr Gly Arg Asp Pro Gly Thr Thr
            500                 505                 510

Val Phe Val Lys Asn Ser Arg Tyr Asp Lys Met Met Glu Ala Phe Gly
        515                 520                 525

Gly Val Gly Val Asn Val Thr Thr Pro Asp Glu Leu Lys Arg Ala Val
    530                 535                 540

Asp Glu Ala Met Asn Ser Gly Lys Pro Thr Leu Ile Asn Ala Glu Ile

545                 550                 555                 560

Asp Pro Ala Ala Gly Ser Glu Ser Gly Asn Ile Gly Ser Leu Asn Pro
                565                 570                 575

Gln Ser Thr Leu Lys Lys Lys
            580

<210>  3
<211>  583
<212>  PRT
<213>  Artificial

<220>
<223>  Mutant of oxalyl-CoA decarboxylase from Methylobacterium
       extorquens

<400>  3

Met Thr Val Gln Ala Gln Asn Ile Asp Ala Ile Thr Ala Gly Ala Met
1               5                   10                  15

Pro His Glu Glu Pro Glu Leu Thr Asp Gly Phe His Leu Val Ile Asp
            20                  25                  30

Ala Leu Lys Leu Asn Gly Ile Glu Thr Ile Tyr Asn Val Pro Gly Ile
        35                  40                  45

Pro Ile Thr Asp Leu Gly Arg Leu Ala Gln Ala Glu Gly Leu Arg Val
    50                  55                  60

Ile Ser Phe Arg His Glu Gln Asn Ala Gly Asn Ala Ala Ala Ile Ala
65                  70                  75                  80

Gly Phe Leu Thr Lys Lys Pro Gly Ile Cys Leu Thr Val Ser Ala Pro
                85                  90                  95

Gly Phe Leu Asn Gly Leu Thr Ala Leu Ala Asn Ala Thr Thr Asn Cys
            100                 105                 110

Phe Pro Met Ile Leu Ile Ser Gly Ser Ser Glu Arg Glu Ile Val Asp
        115                 120                 125

Leu Gln Gln Gly Asp Tyr Glu Glu Met Asp Gln Leu Ala Ile Ala Lys
    130                 135                 140

Pro Leu Cys Lys Ala Ala Phe Arg Val Leu His Ala Ala Asp Ile Gly
145                 150                 155                 160

Ile Gly Val Ala Arg Ala Ile Arg Ala Ala Val Ser Gly Arg Pro Gly

                165                 170                 175

Gly Val Tyr Leu Asp Leu Pro Ala Lys Leu Phe Ser Gln Val Ile Asp
            180                 185                 190

Ala Asp Leu Gly Ala Arg Ser Leu Val Lys Val Ile Asp Ala Ala Pro
        195                 200                 205

Ala Gln Leu Pro Ala Pro Ala Ala Ile Ala Arg Ala Leu Asp Val Leu
    210                 215                 220

Lys Ser Ala Glu Arg Pro Leu Ile Ile Leu Gly Lys Gly Ala Ala Tyr
225                 230                 235                 240

Ala Gln Ala Asp Glu Ala Val Arg Ala Leu Val Glu Glu Ser Gly Ile
                245                 250                 255

Pro Tyr Val Pro Met Ser Met Ala Lys Gly Leu Leu Pro Asp Thr His
            260                 265                 270

Pro Leu Ser Ala Gly Ala Ala Arg Ser Thr Ala Leu Lys Asp Ser Asp
        275                 280                 285

Val Val Leu Leu Val Gly Ala Arg Leu Asn Trp Leu Leu Ser His Gly
    290                 295                 300

Lys Gly Lys Thr Trp Gly Glu Pro Gly Ser Lys Arg Phe Ile Gln Ile
305                 310                 315                 320

Asp Ile Glu Pro Arg Glu Met Asp Ser Asn Val Glu Ile Val Ala Pro
                325                 330                 335

Val Val Gly Asp Ile Gly Ser Cys Val Glu Ala Leu Leu Asp Gly Ile
            340                 345                 350

Arg Lys Asp Trp Lys Gly Ala Pro Ser Asn Trp Leu Glu Thr Leu Arg
        355                 360                 365

Gly Lys Arg Glu Ala Asn Ile Ala Lys Met Ala Pro Lys Leu Met Lys
    370                 375                 380

Asn Ser Ser Pro Met Cys Phe His Ser Ala Leu Gly Ala Leu Arg Thr
385                 390                 395                 400

Val Ile Lys Glu Arg Pro Asp Ala Ile Leu Val Asn Glu Gly Ala Asn
                405                 410                 415

Thr Leu Asp Leu Ala Arg Gly Ile Ile Asp Met Tyr Gln Pro Arg Lys
        420                 425                 430

Arg Leu Asp Val Gly Thr Trp Gly Val Met Gly Ile Gly Met Gly Phe
    435                 440                 445

Ala Val Ala Ala Ala Val Glu Thr Gly Lys Pro Val Leu Ala Val Glu
    450                 455                 460

Gly Asp Ser Ala Phe Gly Phe Ser Gly Met Glu Val Glu Thr Ile Cys
465                 470                 475                 480

Arg Tyr Glu Leu Pro Val Cys Ile Val Ile Phe Asn Asn Asn Gly Ile
                485                 490                 495

Tyr Arg Gly Thr Asp Thr Asp Pro Thr Gly Arg Asp Pro Gly Thr Thr
            500                 505                 510

Val Phe Val Lys Asn Ser Arg Tyr Asp Lys Met Met Glu Ala Phe Gly
        515                 520                 525

Gly Val Gly Val Asn Val Thr Thr Pro Asp Glu Leu Lys Arg Ala Val
    530                 535                 540

Asp Glu Ala Met Asn Ser Gly Lys Pro Thr Leu Ile Asn Ala Glu Ile
545                 550                 555                 560

Asp Pro Ala Ala Gly Ser Glu Ala Gly Asn Ile Gly Ser Leu Asn Pro
                565                 570                 575

Gln Ser Thr Leu Lys Lys Lys
            580

<210>  4
<211>  583
<212>  PRT
<213>  Artificial

<220>
<223>  Mutant of oxalyl-CoA decarboxylase from Methylobacterium
       extorquens

<400>  4

Met Thr Val Gln Ala Gln Asn Ile Asp Ala Ile Thr Ala Gly Ala Met
1               5                   10                  15

Pro His Glu Glu Pro Glu Leu Thr Asp Gly Phe His Leu Val Ile Asp
            20                  25                  30

Ala Leu Lys Leu Asn Gly Ile Glu Thr Ile Tyr Asn Val Pro Gly Ile
        35                  40                  45

Pro Ile Thr Asp Leu Gly Arg Leu Ala Gln Ala Glu Gly Leu Arg Val
    50                  55                  60

Ile Ser Phe Arg His Glu Gln Asn Ala Gly Asn Ala Ala Ala Ile Ala
65                  70                  75                  80

Gly Phe Leu Thr Lys Lys Pro Gly Ile Cys Leu Thr Val Ser Ala Pro
                85                  90                  95

Gly Phe Leu Asn Gly Leu Thr Ala Leu Ala Asn Ala Thr Thr Asn Cys
            100                 105                 110

Phe Pro Met Ile Leu Ile Ser Gly Ser Ser Glu Arg Glu Ile Val Asp
        115                 120                 125

Leu Gln Gln Gly Asp Tyr Glu Glu Met Asp Gln Leu Ala Ile Ala Lys
    130                 135                 140

Pro Leu Cys Lys Ala Ala Phe Arg Val Leu His Ala Ala Asp Ile Gly
145                 150                 155                 160

Ile Gly Val Ala Arg Ala Ile Arg Ala Ala Val Ser Gly Arg Pro Gly
                165                 170                 175

Gly Val Tyr Leu Asp Leu Pro Ala Lys Leu Phe Ser Gln Val Ile Asp
            180                 185                 190

Ala Asp Leu Gly Ala Arg Ser Leu Val Lys Val Ile Asp Ala Ala Pro
        195                 200                 205

Ala Gln Leu Pro Ala Pro Ala Ala Ile Ala Arg Ala Leu Asp Val Leu
    210                 215                 220

Lys Ser Ala Glu Arg Pro Leu Ile Ile Leu Gly Lys Gly Ala Ala Tyr
225                 230                 235                 240

Ala Gln Ala Asp Glu Ala Val Arg Ala Leu Val Glu Glu Ser Gly Ile
                245                 250                 255

Pro Tyr Val Pro Met Ser Met Ala Lys Gly Leu Leu Pro Asp Thr His
            260                 265                 270

Pro Leu Ser Ala Gly Ala Ala Arg Ser Thr Ala Leu Lys Asp Ser Asp
        275                 280                 285

Val Val Leu Leu Val Gly Ala Arg Leu Asn Trp Leu Leu Ser His Gly
    290                 295                 300

Lys Gly Lys Thr Trp Gly Glu Pro Gly Ser Lys Arg Phe Ile Gln Ile
305                 310                 315                 320

Asp Ile Glu Pro Arg Glu Met Asp Ser Asn Val Glu Ile Val Ala Pro
                325                 330                 335

Val Val Gly Asp Ile Gly Ser Cys Val Glu Ala Leu Leu Asp Gly Ile
            340                 345                 350

Arg Lys Asp Trp Lys Gly Ala Pro Ser Asn Trp Leu Glu Thr Leu Arg
        355                 360                 365

Gly Lys Arg Glu Ala Asn Ile Ala Lys Met Ala Pro Lys Leu Met Lys
    370                 375                 380

Asn Ser Ser Pro Met Cys Phe His Ser Ala Leu Gly Ala Leu Arg Thr
385                 390                 395                 400

Val Ile Lys Glu Arg Pro Asp Ala Ile Leu Val Asn Glu Gly Ala Asn
                405                 410                 415

Thr Leu Asp Leu Ala Arg Gly Ile Ile Asp Met Tyr Gln Pro Arg Lys
            420                 425                 430

Arg Leu Asp Val Gly Thr Trp Gly Val Met Gly Ile Gly Met Gly Phe
        435                 440                 445

Ala Val Ala Ala Ala Val Glu Thr Gly Lys Pro Val Leu Ala Val Glu
    450                 455                 460

Gly Asp Ser Ala Phe Gly Phe Ser Gly Met Glu Val Glu Thr Ile Cys
465                 470                 475                 480

Arg Tyr Glu Leu Pro Val Cys Ile Val Ile Phe Asn Asn Asn Gly Ile
                485                 490                 495

Ala Arg Gly Thr Asp Thr Asp Pro Thr Gly Arg Asp Pro Gly Thr Thr
            500                 505                 510

Val Phe Val Lys Asn Ser Arg Tyr Asp Lys Met Met Glu Ala Phe Gly
        515                 520                 525

Gly Val Gly Val Asn Val Thr Thr Pro Asp Glu Leu Lys Arg Ala Val
    530                 535                 540

Asp Glu Ala Met Asn Ser Gly Lys Pro Thr Leu Ile Asn Ala Glu Ile
545                 550                 555                 560

Asp Pro Ala Ala Gly Ser Glu Ala Gly Asn Ile Gly Ser Leu Asn Pro
                565                 570                 575

Gln Ser Thr Leu Lys Lys Lys
            580

<210>  5
<211>  528
<212>  PRT
<213>  Methylobacterium extorquens

<400>  5

Met Thr Met Leu Leu Pro Glu Pro Ser Ala Leu Leu Pro Thr Asp Ser
1               5                   10                  15

Lys Glu Ala Ser Val Pro Ala Thr Thr Leu His Glu Leu Ile Gln Ala
            20                  25                  30

Gly Ala Asp Ala Ala Pro Ala Leu Ser Ser Pro Gly Gly Val Pro Leu
        35                  40                  45

Thr Phe Gln Ala Leu Arg Ala Leu Thr Glu Arg Thr Val Ala Asp Leu
    50                  55                  60

Asn Ala Arg Gly Ile Gly Arg Gly Asp Arg Val Ala Ile Val Leu Pro
65                  70                  75                  80

Asn Gly Pro Glu Met Ala Ala Ala Phe Ile Ala Val Ala Ala Gly Thr
                85                  90                  95

Thr Ser Ala Pro Leu Asn Pro Ser Tyr Lys Ala Asp Glu Phe Glu Phe
            100                 105                 110

Tyr Met Ser Asp Leu Gly Ala Lys Leu Leu Leu Val Ala Glu Gly Ser
        115                 120                 125

Glu Thr Pro Ala Val Ala Val Ala Glu Lys Leu Gly Val Ser Val Ala
    130                 135                 140

Arg Leu Arg Pro Thr Pro Asp Glu Gly Ala Gly Ser Phe Thr Leu His
145                 150                 155                 160

Phe Ala Ser Glu Ser Thr Gly Pro Thr Glu Lys Ser Gly Pro Ala Gly
                165                 170                 175

Ser Asp Asp Ile Ala Leu Val Leu His Thr Ser Gly Thr Thr Ser Arg
        180                 185                 190

Pro Lys Ile Val Pro Leu Thr Gln Ala Asn Val Cys Ala Ser Ala Arg
    195                 200                 205

Asn Ile Arg Thr Ala Leu Ala Phe Gly Pro Glu Asp Arg Gly Leu Asn
    210                 215                 220

Ile Met Pro Leu Phe His Ile His Gly Leu Ile Ala Gly Ile Leu Ala
225                 230                 235                 240

Pro Leu Ser Val Gly Gly Gln Val Ser Cys Thr Pro Gly Phe Asn Ala
                245                 250                 255

Leu Lys Phe Phe Gly Trp Met Asp Glu Val Asn Pro Thr Trp Tyr Thr
        260                 265                 270

Gly Val Pro Thr Met His Gln Ala Ile Leu Gly Arg Ala Ala Arg Asn
    275                 280                 285

Lys Glu Ile Ile Ala Arg Asn Pro Leu Arg Phe Ile Arg Ser Ser Ser
    290                 295                 300

Ser Ser Leu Pro Pro Gln Val Met Lys Glu Leu Glu Glu Thr Phe Gly
305                 310                 315                 320

Ala Pro Val Ile Glu Ala Tyr Gly Met Thr Glu Ala Ala His Gln Met
                325                 330                 335

Ala Ser Asn Pro Leu Pro Pro Lys Pro His Tyr Ala Gly Ser Val Gly
        340                 345                 350

Leu Ala Ala Gly Pro Glu Ile Ala Val Val Asp Leu Asp Gly Glu Pro
    355                 360                 365

Leu Pro Ala Gly Glu Thr Gly Glu Ile Val Ile Arg Gly Asp Asn Val
    370                 375                 380

Met Lys Gly Tyr Glu Asn Asn Glu Lys Ala Asn Ala Glu Ala Phe Thr
385                 390                 395                 400

Lys Gln Gly Trp Phe Arg Thr Gly Asp Gln Gly Val Leu Ser Pro Glu
                405                 410                 415

Gly Tyr Leu Ser Ile Thr Gly Arg Leu Lys Glu Ile Ile Asn Arg Gly

420                 425                 430

Gly Glu Lys Ile Ser Pro Arg Glu Val Asp Glu Ile Leu Met Asp His
        435                 440                 445

Pro Ala Val Ser Gln Cys Val Thr Phe Ala Val Pro His Asp Lys Leu
    450                 455                 460

Gly Glu Asp Val Ala Ala Ala Ile Val Leu Arg Glu Gly Val Glu Ala
465                 470                 475                 480

Val Glu Lys Asp Ile Arg Ser Phe Ala Ser Glu Arg Leu Ala Ala Phe
                485                 490                 495

Lys Val Pro Ala Lys Ile Leu Ile Leu Asp Glu Ile Pro Lys Gly Ala
            500                 505                 510

Thr Gly Lys Leu Gln Arg Ile Gly Leu Ala Gln Lys Leu Gly Leu Val
        515                 520                 525

<210>  6
<211>  132
<212>  PRT
<213>  E. coli

<400>  6

Met Ser Thr Thr His Asn Val Pro Gln Gly Asp Leu Val Leu Arg Thr
1               5                   10                  15

Leu Ala Met Pro Ala Asp Thr Asn Ala Asn Gly Asp Ile Phe Gly Gly
            20                  25                  30

Trp Leu Met Ser Gln Met Asp Ile Gly Gly Ala Ile Leu Ala Lys Glu
        35                  40                  45

Ile Ala His Gly Arg Val Val Thr Val Arg Val Glu Gly Met Thr Phe
    50                  55                  60

Leu Arg Pro Val Ala Val Gly Asp Val Val Cys Cys Tyr Ala Arg Cys
65                  70                  75                  80

Val Gln Lys Gly Thr Thr Ser Val Ser Ile Asn Ile Glu Val Trp Val
                85                  90                  95

Lys Lys Val Ala Ser Glu Pro Ile Gly Gln Arg Tyr Lys Ala Thr Glu
            100                 105                 110

Ala Leu Phe Lys Tyr Val Ala Val Asp Pro Glu Gly Lys Pro Arg Ala

115                 120                 125

Leu Pro Val Glu
    130

<210>  7
<211>  26
<212>  DNA
<213>  Artificial

<220>
<223>  Primer for oxc (DNA of the oxalyl-CoA decarboxylase; oxc_fw_NDEI)

<400>  7
gttcacatat gaccgtccag gcccag                                          26

<210>  8
<211>  32
<212>  DNA
<213>  Artificial

<220>
<223>  Primer for oxc (DNA of the oxalyl-CoA decarboxylase;
       oxc_rv_BamHI)

<400>  8
cgctggatcc tcacttcttc ttcaaggtgc tc                                   32

<210>  9
<211>  25
<212>  DNA
<213>  Artificial

<220>
<223>  Primer for oxs (DNA of the acetyl-CoA synthetase; oxs_fw_NdeI)

<400>  9
gttcacatat gacgatgctt ctgcc                                           25

<210>  10
<211>  25
<212>  DNA
<213>  Artificial

<220>
<223>  Primer for oxs (DNA of the acetyl-CoA synthetase; oxs_rv_BamHI)

<400>  10
caatggatcc tcagaccagc ccgag                                           25

<210>  11
<211>  27
<212>  DNA
<213>  Artificial

<220>
<223>  Primer for panE2 (DNA of the oxalyl-CoA reductase; panE2_fw_NdeI)

<400>  11
gcgcacatat gagcatcgcg atcgtcg                                          27


<210>  12
<211>  28
<212>  DNA
<213>  Artificial

<220>
<223>  Primer for panE2 (DNA of the oxalyl-CoA reductase;
       panE2_rv_BamHI)

<400>  12
cagaggatcc tcatgctccc tggatcgc                                         28


<210>  13
<211>  28
<212>  DNA
<213>  Artificial

<220>
<223>  Primer for oxcY497A (DNA of a mutant of the oxalyl-CoA
       decarboxylase; oxc_Y497A_fw)

<400>  13
caacaacggc atcgctcgcg gcaccgac                                         28


<210>  14
<211>  28
<212>  DNA
<213>  Artificial

<220>
<223>  Primer for oxcY497A (DNA of a mutant of the oxalyl-CoA
       decarboxylase; oxc_Y497A_rv)

<400>  14
gtcggtgccg cgagcgatgc cgttgttg                                         28


<210>  15
<211>  25
<212>  DNA
<213>  Artificial

<220>
<223>  Primer for oxc S568A (DNA of a mutant of the oxalyl-CoA
       decarboxylase; oxc_S568A_fw)

<400>  15
ccggcagcga ggccggcaat atcgg                                            25


<210>  16
<211>  25
<212>  DNA
<213>  Artificial

<220>

<223>  Primer for oxc S568A (DNA of a mutant of the oxalyl-CoA
       decarboxylase; oxc_S568A_rv)

<400>  16
ccgatattgc cggcctcgct gccgg                                            25


<210>  17
<211>  1737
<212>  DNA
<213>  Artificial

<220>
<223>  Hacl1 codon optimized DNA for E. coli

<400>  17
atgccggatt caaactttgc tgaacgttca gaagaacagg tttctggcgc aaaagtgatt      60

gcgcaagcgc taaaaaccca ggatgtcgaa tatatcttcg gtatagtggg tattccggtg     120

acggaaatcg ccatcgcggc tcagcaactc ggtattaaat acatcggtat gcgtaatgag     180

caggcggctt gttacgcagc aagcgcgatt ggctatctga cctcaagacc gggggtatgc     240

cttgttgtca gcggcccggg cctgattcat gccctgggtg gtatggcgaa tgctaacatg     300

aactgctggc cgctgttagt tataggcggc agcagtgaac gcaatcagga gaccatgggc     360

gcattccaag agtttcctca ggtggaagcc tgccgcctgt ataccaaatt ttctgctaga     420

ccttcgtcaa tagaagccat ccccttcgtg attgaaaaag cggtccgtag ttctatctat     480

gggcgccctg gggcttgcta tgttgacatt cccgccgact tcgtaaacct ccaagtgaat     540

gtcaattcca ttaaatacat ggaacgctgt atgtcgccgc cgatcagtat ggccgagacc     600

agcgccgtgt gcacagcggc gagtgttatc cgcaacgcca aacagccatt actaataatt     660

gggaaaggag ccgcctatgc tcacgctgaa gagtccatca aaaaactggt ggaacaatac     720

aagttgccct ttttacctac tcccatgggt aaaggtgttg tacctgataa tcatccatac     780

tgcgtcggtg cagcccgttc ccgcgcacta cagttcgctg acgtgattgt gctgtttggt     840

gcgcgtttaa actggatact ccacttcggt ttaccgccac gctaccagcc ggatgtgaaa     900

tttatacagg ttgacatctg tgccgaagaa ctgggcaata atgtgaaacc agcagtcact     960

ttgctgggga acatccatgc agtcaccaaa caactgcttg aagagctgga caagacgccg    1020

tggcaatatc cgccagagtc aaaatggtgg aagacgctac gtgagaaaat gaagagcaac    1080

gaagcggctt cgaaagagtt ggcctccaaa aagtcattgc ctatgaatta ctataccgtc    1140

ttttatcatg tccaagagca gctgccgcgg gattgctttg tcgtgtcgga gggcgccaac    1200

accatggata taggtcgcac tgtccttcag aactatctgc cccggcatcg cctggacgct    1260

ggtaccttcg gaactatggg cgtagggctg ggctttgcta ttgcggcggc agtggtagca    1320

aaggatcgca gcccggggca gtggattatt tgtgtagaag gggactccgc atttggattc    1380

tctggtatgg aggtagagac aatatgccgt tataatttgc caattatcct tctagtggtg    1440

aataacaatg gtatttatca gggctttgat acagatacgt ggaaggaaat gctgaaattt 1500

caagatgcca cagccgtggt accccccatg tgtttgctgc cgaactctca ttacgagcaa 1560

gtgatgacag cattcggcgg caagggttac tttgtgcaga caccggaaga acttcaaaag 1620

agccttcgcc agagcttggc ggacaccacg aaaccgagtc tgattaatat aatgattgaa 1680

ccacaggcca caaggaaggc acaagacttt cactggctca cgcgttcaaa tatgtaa 1737

<210> 18
<211> 641
<212> PRT
<213> Erythrobacter sp. NAP1

<400> 18

Met Thr Gly Phe Val Glu Arg Pro Glu Gln Ala His Thr Pro Asn Cys
1               5                   10                  15

Thr Gly Val Gln Tyr Ala Ala Met Tyr Glu Arg Ser Leu Ala Asp Pro
            20                  25                  30

Asp Gly Phe Trp Leu Glu Gln Ala Lys Arg Leu Asp Trp Thr Gln Gln
        35                  40                  45

Pro Arg Lys Gly Gly Glu Trp Ser Tyr Asp Pro Val Asp Ile Lys Trp
    50                  55                  60

Phe Ala Asp Gly Ser Leu Asn Leu Cys His Asn Ala Val Asp Arg His
65                  70                  75                  80

Leu Asp Ser Arg Gly Asp Thr Pro Ala Ile Ile Phe Glu Pro Asp Asp
                85                  90                  95

Pro Ala Thr Pro Ser Arg Thr Leu Thr Tyr Arg Gln Leu His Ser Glu
            100                 105                 110

Val Ile His Met Ala Asn Ala Leu Lys Ala Ile Gly Val Thr Lys Gly
        115                 120                 125

Glu Arg Val Thr Ile Tyr Met Pro Asn Ile Val Glu Gly Val Thr Ala
    130                 135                 140

Met Leu Ala Cys Ala Arg Leu Gly Ala Ile His Ser Val Val Phe Gly
145                 150                 155                 160

Gly Phe Ser Pro Glu Ala Leu Ala Gly Arg Ile Ile Asp Cys Glu Ser
                165                 170                 175

Arg Phe Val Val Thr Ala Asp Glu Gly Lys Arg Gly Ala Lys Ser Val
            180                 185                 190

Pro Leu Lys Ala Asn Val Asp Ala Ala Leu Glu Val Glu Gly Val Asp
        195                 200                 205

Val Thr Gly Val Leu Val Val Gln His Thr Gly Leu Ala Val Pro Met
    210                 215                 220

Thr Glu Gly Arg Asp His Trp Phe His Glu Val Lys Ser Asp Ala Asp
225                 230                 235                 240

Val Pro Cys Glu Thr Met Ala Ala Glu Asp Pro Leu Phe Ile Leu Tyr
                245                 250                 255

Thr Ser Gly Ser Thr Gly Lys Pro Lys Gly Val Leu His Thr Thr Gly
            260                 265                 270

Gly Tyr Gly Val Trp Thr Ala Thr Thr Phe Ser Tyr Ile Phe Asp Tyr
        275                 280                 285

Gln Pro Gly Glu Val Phe Trp Cys Thr Ala Asp Ile Gly Trp Val Thr
    290                 295                 300

Gly His Ser Tyr Ile Val Tyr Gly Pro Leu Gln Asn Gly Ala Thr Gln
305                 310                 315                 320

Val Leu Phe Glu Gly Val Pro Asn Tyr Pro Asp Phe Gly Arg Phe Trp
                325                 330                 335

Asp Val Val Ala Lys His Lys Val Ser Ile Leu Tyr Thr Ala Pro Thr
            340                 345                 350

Ala Ile Arg Ala Leu Met Arg Glu Gly Asp Asp Tyr Val Thr Ser Arg
        355                 360                 365

Asp Arg Ser Ser Leu Arg Leu Leu Gly Ser Val Gly Glu Pro Ile Asn
    370                 375                 380

Pro Glu Ala Trp Arg Trp Tyr Phe Asp Val Val Gly Glu Gly Arg Cys
385                 390                 395                 400

Pro Ile Ile Asp Thr Trp Trp Gln Thr Glu Thr Gly Gly Cys Met Ile
                405                 410                 415

Thr Thr Leu Pro Gly Ala His Asp Met Lys Pro Gly Ser Ala Gly Leu
            420                 425                 430

Pro Met Phe Gly Ile Arg Pro Gln Leu Val Asp Asn Asp Gly Ala Val
        435                 440                 445

Leu Asp Gly Ala Thr Glu Gly Asn Leu Cys Ile Thr His Ser Trp Pro
    450                 455                 460

Gly Gln Ala Arg Ser Val Tyr Gly Asp His Asp Arg Phe Val Gln Thr
465                 470                 475                 480

Tyr Phe Ser Thr Tyr Ser Gly Lys Tyr Phe Thr Gly Asp Gly Cys Lys
                485                 490                 495

Arg Asp Glu Asp Gly Tyr Tyr Trp Ile Thr Gly Arg Val Asp Asp Val
            500                 505                 510

Ile Asn Val Ser Gly His Arg Met Gly Thr Ala Glu Val Glu Ser Ala
        515                 520                 525

Leu Val Leu His Pro Gln Val Ala Glu Ala Ala Val Val Gly Tyr Pro
    530                 535                 540

His Asp Val Lys Gly Gln Gly Ile Tyr Cys Tyr Val Thr Thr Asn Ala
545                 550                 555                 560

Gly Val Glu Gly Ser Asp Glu Leu Tyr Gln Glu Leu Arg Ala His Val
                565                 570                 575

Arg Lys Glu Ile Gly Pro Ile Ala Thr Pro Asp Gln Ile Gln Phe Thr
            580                 585                 590

Asp Gly Leu Pro Lys Thr Arg Ser Gly Lys Ile Met Arg Arg Ile Leu
        595                 600                 605

Arg Lys Val Ala Glu Asn Asp Tyr Gly Ser Leu Gly Asp Thr Ser Thr
    610                 615                 620

Leu Ala Asp Pro Ser Leu Val Asp Arg Leu Ile Glu Gly Arg Gln Lys
625                 630                 635                 640

Thr

<210>  19
<211>  641
<212>  PRT
<213>  Artificial

<220>

<223>  Mutant of acyl-CoA synthetase from Erythrobacter sp. NAP1

<400>  19

Met Thr Gly Phe Val Glu Arg Pro Glu Gln Ala His Thr Pro Asn Cys
1               5                   10                  15

Thr Gly Val Gln Tyr Ala Ala Met Tyr Glu Arg Ser Leu Ala Asp Pro
            20                  25                  30

Asp Gly Phe Trp Leu Glu Gln Ala Lys Arg Leu Asp Trp Thr Gln Gln
        35                  40                  45

Pro Arg Lys Gly Gly Glu Trp Ser Tyr Asp Pro Val Asp Ile Lys Trp
    50                  55                  60

Phe Ala Asp Gly Ser Leu Asn Leu Cys His Asn Ala Val Asp Arg His
65                  70                  75                  80

Leu Asp Ser Arg Gly Asp Thr Pro Ala Ile Ile Phe Glu Pro Asp Asp
                85                  90                  95

Pro Ala Thr Pro Ser Arg Thr Leu Thr Tyr Arg Gln Leu His Ser Glu
            100                 105                 110

Val Ile His Met Ala Asn Ala Leu Lys Ala Ile Gly Val Thr Lys Gly
        115                 120                 125

Glu Arg Val Thr Ile Tyr Met Pro Asn Ile Val Glu Gly Val Thr Ala
    130                 135                 140

Met Leu Ala Cys Ala Arg Leu Gly Ala Ile His Ser Val Val Phe Gly
145                 150                 155                 160

Gly Phe Ser Pro Glu Ala Leu Ala Gly Arg Ile Ile Asp Cys Glu Ser
                165                 170                 175

Arg Phe Val Val Thr Ala Asp Glu Gly Lys Arg Gly Ala Lys Ser Val
            180                 185                 190

Pro Leu Lys Ala Asn Val Asp Ala Ala Leu Glu Val Glu Gly Val Asp
        195                 200                 205

Val Thr Gly Val Leu Val Val Gln His Thr Gly Leu Ala Val Pro Met
    210                 215                 220

Thr Glu Gly Arg Asp His Trp Phe His Glu Val Lys Ser Asp Ala Asp
225                 230                 235                 240

Val Pro Cys Glu Thr Met Ala Ala Glu Asp Pro Leu Phe Ile Leu Tyr
                245                 250                 255

Thr Ser Gly Ser Thr Gly Lys Pro Lys Gly Val Leu His Thr Thr Gly
            260                 265                 270

Gly Tyr Gly Val Trp Thr Ala Thr Thr Phe Ser Tyr Ile Phe Asp Tyr
        275                 280                 285

Gln Pro Gly Glu Val Phe Trp Cys Thr Ala Asp Ile Gly Trp Val Thr
    290                 295                 300

Gly His Ser Tyr Ile Val Tyr Gly Pro Leu Gln Asn Gly Ala Thr Gln
305                 310                 315                 320

Val Leu Phe Glu Gly Val Pro Asn Tyr Pro Asp Phe Gly Arg Phe Trp
                325                 330                 335

Asp Val Val Ala Lys His Lys Val Ser Ile Leu Tyr Thr Ala Pro Thr
            340                 345                 350

Ala Ile Arg Ala Leu Met Arg Glu Gly Asp Asp Tyr Val Thr Ser Arg
        355                 360                 365

Asp Arg Ser Ser Leu Arg Leu Leu Gly Ser Ile Gly Glu Pro Ile Asn
    370                 375                 380

Pro Glu Ala Trp Arg Trp Tyr Phe Asp Val Val Gly Glu Gly Arg Cys
385                 390                 395                 400

Pro Ile Ile Asp Thr Trp Trp Gln Thr Glu Thr Gly Gly Cys Met Ile
                405                 410                 415

Thr Thr Leu Pro Gly Ala His Asp Met Lys Pro Gly Ser Ala Gly Leu
            420                 425                 430

Pro Met Phe Gly Ile Arg Pro Gln Leu Val Asp Asn Asp Gly Ala Val
        435                 440                 445

Leu Asp Gly Ala Thr Glu Gly Asn Leu Cys Ile Thr His Ser Trp Pro
    450                 455                 460

Gly Gln Ala Arg Ser Val Tyr Gly Asp His Asp Arg Phe Val Gln Thr
465                 470                 475                 480

Tyr Phe Ser Thr Tyr Ser Gly Lys Tyr Phe Thr Gly Asp Gly Cys Lys
                485                 490                 495

Arg Asp Glu Asp Gly Tyr Tyr Trp Ile Thr Gly Arg Val Asp Asp Val
            500                 505                 510

Ile Asn Val Ser Gly His Arg Met Gly Thr Ala Glu Val Glu Ser Ala
        515                 520                 525

Leu Val Leu His Pro Gln Val Ala Glu Ala Ala Val Val Gly Tyr Pro
    530                 535                 540

His Asp Val Lys Gly Gln Gly Ile Tyr Cys Tyr Val Thr Thr Asn Ala
545                 550                 555                 560

Gly Val Glu Gly Ser Asp Glu Leu Tyr Gln Glu Leu Arg Ala His Val
                565                 570                 575

Arg Lys Glu Ile Gly Pro Ile Ala Thr Pro Asp Gln Ile Gln Phe Thr
            580                 585                 590

Asp Gly Leu Pro Lys Thr Arg Ser Gly Lys Ile Met Arg Arg Ile Leu
        595                 600                 605

Arg Lys Val Ala Glu Asn Asp Tyr Gly Ser Leu Gly Asp Thr Ser Thr
    610                 615                 620

Leu Ala Asp Pro Ser Leu Val Asp Arg Leu Ile Glu Gly Arg Gln Lys
625                 630                 635                 640

Thr

<210>  20
<211>  1926
<212>  DNA
<213>  Erythrobacter sp. NAP1

<400>  20
atgactggat ttgtcgagcg gcccgagcag gcgcacactc ccaattgcac cggtgttcaa      60

tacgctgcga tgtatgagcg ttcgctggct gatcctgatg gcttctggct tgaacaggcc     120

aagcgcctcg attggaccca gcagccaagg aaaggtggcg agtggagcta cgacccggtc     180

gatatcaagt ggtttgccga cggttcgctg aacctttgcc acaacgcggt cgatcggcat     240

ctcgacagcc gcggtgacac acccgctatt atctttgaac ccgacgaccc agcaacgcct     300

tcgcgcacgc tcacctatcg ccagcttcac tccgaagtca tccacatggc caacgccctc     360

aaggccatcg gggtgaccaa gggcgagcgc gtgacgatct acatgccgaa catagtcgag     420

ggcgtgacgg ctatgctcgc ctgcgcgcgg ctgggcgcga tccactccgt tgtctttggc     480

ggcttctcgc ctgaggcgct tgctggtcgg atcattgatt gtgaaagccg cttcgtggtc 540

accgcggacg aaggcaagcg cggggcaaag agcgttcctt tgaaagccaa tgtggacgcc 600

gccttggaag tcgaaggggt cgatgtaact ggagtgctgg tcgtccagca caccggatta 660

gccgtgccga tgaccgaagg gcgagaccac tggttccatg aggtcaaatc ggacgcagat 720

gtgccctgcg agaccatggc agccgaagac ccgctcttca ttctttacac atcgggctca 780

accggaaagc ccaagggtgt gctccacaca accggcggat acggcgtgtg gaccgcgacg 840

acgttcagtt acattttcga ctatcagccc ggtgaggtgt tctggtgcac ggccgatatc 900

ggctgggtca ctggccacag ctacatcgtc tatggcccac tacagaacgg cgcgacgcag 960

gtgctgttcg agggtgttcc caactacccc gattttggtc ggttctggga tgttgtcgca 1020

aaacacaagg tcagcatcct ctacaccgcg ccaaccgcga tccgggccct gatgcgtgaa 1080

ggcgatgact acgtgacaag ccgcgatcgt tcctcgctcc gactgctcgg aagcgtagga 1140

gagccgatca acccggaagc ctggcgctgg tatttcgacg tggtcggaga ggggcgttgc 1200

ccgatcatag acacatggtg gcagaccgaa acaggcggtt gcatgatcac cactcttcca 1260

ggtgcgcacg acatgaaacc gggcagcgcc ggtttgccga tgttcggtat ccgcccgcag 1320

ctggtcgaca atgatggagc cgtgctggac ggagcgaccg agggcaatct gtgcatcacg 1380

cacagctggc ccggacaggc gcgcagcgtt tacggcgatc acgaccggtt cgttcagacc 1440

tatttctcga cgtactccgg caagtacttc accggggacg ggtgcaagcg cgatgaggat 1500

ggctattact ggatcacggg ccgcgtggac gacgtgatca acgtgtcagg ccaccgaatg 1560

ggaactgccg aagtcgaaag cgcattggtg ctccacccgc aagtcgccga agcggcggtg 1620

gttggatacc cgcatgatgt gaaggggcag ggcatctact gctacgtcac aaccaatgcg 1680

ggcgtcgaag gctcggacga gctttatcag gagttgcgcg cgcatgtgcg caaggaaatc 1740

ggacccatcg cgacgcctga ccagattcag ttcaccgacg gcctgcccaa gacacgttcg 1800

ggcaagatca tgcgccgaat tctgagaaag gtcgccgaaa acgactacgg ctcgctgggc 1860

gacacttcga cacttgccga tccctcgttg gtcgaccgcc tgatcgaggg ccgacagaaa 1920

acctag 1926

<210> 21
<211> 1926
<212> DNA
<213> Artificial

<220>
<223> Codon-optimized nucleotide sequence encoding acyl-CoA synthetase
 from Erythrobacter sp. NAP1 for E. coli

<400> 21
atgaccggtt tcgtagagcg tccggaacag gctcacaccc cgaactgcac aggtgttcaa 60

tacgcagcaa tgtacgagcg ttccctggct gatccggacg gcttttggct ggaacaggct 120

aaacgtctgg actggaccca gcagccgcgt aagggcggcg aatggtctta cgacccggtt 180

gacataaaat ggttcgcgga cggttccctg aacctgtgtc ataacgcagt agaccgtcac 240

ctggatagcc gcggtgacac cccggcaatc atcttcgagc cggatgaccc ggctactccg 300

tctcgcaccc ttacctatcg tcagctgcac tccgaagtta ttcacatggc taatgctctg 360

aaagcaatcg gcgtaaccaa aggcgagcgt gttacaatct acatgccgaa catcgtagaa 420

ggtgttaccg ctatgctggc ttgcgcacgc ctgggtgcga tccacagtgt agtgttcggt 480

ggtttctctc ctgaggctct ggccggtcgt atcatcgact gtgaatctcg cttcgttgtg 540

actgcggacg aaggcaaacg cggtgcaaaa tccgttccgc tgaaggctaa cgtagacgct 600

gctctggaag ttgaaggcgt agacgttact ggtgttctgg tagtacagca taccggcctg 660

gcggttccga tgaccgaggg tcgcgaccac tggttccatg aggttaaatc tgacgctgac 720

gtgccgtgcg aaactatggc agctgaagac ccgctgttta ttctgtacac aagcggctcc 780

accggtaaac caaaaggtgt tctgcacact actggtggtt atggtgtttg gactgctacc 840

accttcagct acatcttcga ctaccagccg ggtgaagtgt tctggtgtac agcagacatc 900

ggttgggtta ccggtcattc ttacatcgta tatggtccgc tgcagaacgg tgcaactcag 960

gttctgtttg aaggtgtacc aaactacccg gacttcggtc gtttctggga cgttgtagct 1020

aaacacaaag tttccatcct ctacactgca ccgaccgcta tccgcgcgct gatgcgtgaa 1080

ggtgatgact atgttacttc acgtgaccgc agttccctgc gtctgctggg ctctgtaggt 1140

gagccgatca acccggaggc ctggcgttgg tacttcgatg ttgttggtga aggtcgttgt 1200

ccgatcatcg acacttggtg gcagaccgag actggcggtt gcatgattac tactctgccg 1260

ggtgctcacg atatgaagcc gggctctgca ggcctgccga tgttcggtat ccgtccgcag 1320

ctcgttgaca acgacggtgc agtgctggac ggtgcaacag aaggtaacct gtgtatcacc 1380

cactcttggc cgggccaggc tcgctctgtt tacggtgatc acgaccgctt cgtacagact 1440

tacttctcca cctactccgg caagtatttc actggtgacg gttgcaaacg tgacgaggat 1500

ggctactatt ggattaccgg tcgtgtggac gacgttatca acgtttctgg ccatcgtatg 1560

ggtactgctg aggtggaaag cgcactggta ctgcacccgc aggtagcaga agcagctgtg 1620

gttggttacc cgcacgacgt taagggccag ggtatctact gttatgtaac caccaacgca 1680

ggcgtggaag gttctgacga actgtaccag gagctgcgtg cccacgtgcg caaagaaatc 1740

ggcccgatcg caactccgga tcagatccag ttcactgacg gtctgccgaa gacgcgttcc 1800

ggtaaaatta tgcgccgtat cctgcgtaaa gtggcagaaa acgactatgg ttctctgggt 1860

gatacttcca ccctggcgga cccgtctctg gtggatcgtc tgattgaggg ccgtcagaaa 1920

acttaa                                                                1926

<210>  22
<211>  27
<212>  DNA
<213>  Artificial

<220>
<223>  Primer for the preparation of a mutant of the gene from
       Erythrobacter sp. NAP1 encoding acyl-CoA synthetase
       (GATCGGCTCACCAATAGAGCCCAGCAG)

<400>  22
ctgctgggct ctattggtga gccgatc                                          27

<210>  23
<211>  27
<212>  DNA
<213>  Artificial

<220>
<223>   Primer for the preparation of a mutant of the gene from
       Erythrobacter sp. NAP1 encoding acyl-CoA synthetase
       (eryACS-1_V379I_rv)

<400>  23
gatcggctca ccaatagagc ccagcag                                          27

<210>  24
<211>  1737
<212>  DNA
<213>  Humane

<400>  24
atgccggaca gtaacttcgc agagcgcagc gaggagcagg tgtctggtgc taaagtcatc      60

gctcaggccc tgaaaacgca agatgtggag tacatatttg gcatcgtagg catcccagtg     120

accgaaatcg ccattgctgc ccagcagcta ggcatcaagt acatcgggat gaggaatgag     180

caagcggctt gttatgctgc ctccgcgatt ggatatctga caagcaggcc aggagtctgc     240

cttgttgttt ctggcccagg tctcatccat gccttgggcg gtatggcaaa tgcaaacatg     300

aactgctggc ccttgcttgt gattggtggt tcctctgaaa gaaaccaaga aacaatggga     360

gctttccagg agtttcctca ggttgaagct tgtagattat ataccaagtt ctctgcccgc     420

ycaagcagca tagaagctat tccttttgtt attgaaaagg cagtgagaag cagtatctat     480

ggtcgtccag gtgcttgcta tgttgacata ccagcagatt ttgtgaacct tcaggtgaat     540

gtgaattcta taaagtacat ggaacgctgc atgtcacctc ctattagcat ggcagaaacc     600

tctgctgtgt gcacggcggc ttctgttatt aggaatgcca aacaacccct tcttatcatc     660

gggaaaggtg ctgcttacgc tcatgcagaa gagagtatca agaaattggt ggagcaatat     720

aaactgccat ttttgcccac ccctatggga aagggtgttg tccctgacaa ccatccatac     780

tgtgtaggtg cagccagatc cagggctttg caatttgctg atgtaattgt gttatttggt 840

gccagactaa attggatttt acattttgga ctgcctccaa gatatcagcc agatgtgaag 900

tttatccagg ttgatatctg tgcagaagaa ttggggaata atgtaaagcc cgctgttact 960

ttgctaggaa acatacatgc tgtcactaag cagcttttag aggaacttga taaaacacca 1020

tggcagtatc ctccagagag caagtggtgg aaaactctga gagaaaaaat gaagagcaat 1080

gaagctgcat ccaaggaact agcttctaaa aaatccctgc ctatgaatta ttacacagta 1140

ttctaccatg ttcaagaaca actacctaga gactgtttcg tggtaagtga aggagcaaat 1200

actatggaca ttggacggac tgtgcttcag aactaccttc ctcgtcacag gcttgatgct 1260

ggtactttcg gaacaatggg agttggtttg ggatttgcta ttgcagctgc cgtggtggct 1320

aaagatagaa gccctgggca ttggatcatc tgtgtggaag gagacagtgc atttgggttt 1380

tctggcatgg aggtagaaac catctgcagg tacaacttgc caatcatact gttggtagtg 1440

aataacaatg gaatttacca aggttttgat acagatactt ggaaagaaat gttaaaattt 1500

caagatgcta ctgcagtggt ccctccaatg tgtttgctgc caaattcaca ttatgagcaa 1560

gtcatgactg catttggagg caaagggtat tttgtacaaa caccagaaga actccaaaaa 1620

tccctggagc agagcctagc agacacaact aaaccttctc ttatcaacat catgattgag 1680

ccacaagcca cacggaaggc ccaggatttt cattggctga cccgctctaa tatgtaa 1737

<210> 25
<211> 399
<212> DNA
<213> E. coli

<400> 25
atgtctacaa cacataacgt ccctcagggc gatcttgttt tacgtacttt agccatgccc 60

gccgatacca atgccaatgg tgacatcttt ggtggttggt taatgtcaca aatggatatt 120

ggcggcgcta ttctggcaaa agaaattgcc cacggtcgcg tagtgactgt gcgggttgaa 180

ggaatgactt tcttacggcc ggttgcggtc ggcgatgtgg tgtgctgcta tgcacgctgt 240

gtccagaaag ggacgacatc ggtcagcatt aatattgaag tgtgggtgaa aaaagtagcg 300

tctgaaccaa ttgggcaacg ctataaagcg acagaagcat tatttaagta tgtcgcggtt 360

gatcctgaag gaaaacctcg cgccttacct gttgagtaa 399